# EUROPEAN PATENT SPECIFICATION

(11) **EP 4 031 542 B1**
(45) Date of publication and mention of the grant of the patent: **15.10.2025**
(21) Application number: 20865605.8
(22) Date of filing: 18.09.2020
(51) Int. Cl.: C07D 471/04, A61K 31/4375, A61P 35/00

(54) **SMALL MOLECULE INHIBITORS OF KRAS G12C MUTANT**
KLEINMOLEKÜLIGE INHIBITOREN DER KRAS-G12C-MUTANTE
PETITES MOLÉCULES INHIBITRICES DE MUTANT DE KRAS G12C

(30) Priority: 18.09.2019 US 201962902022 P
(43) Date of publication of application: 27.07.2022
(73) Proprietor: Merck Sharp & Dohme LLC, Rahway, New Jersey 07065 (US)
(72) Inventor: HUSSAIN, Zahid, Kenilworth, NJ 07033-1310 (US); HAN, Yongxin, Boston, MA 02115-5727 (US); MANDAL, Mihir, Kenilworth, NJ 07033-1310 (US); GRAHAM, Thomas, H., Boston, MA 02115-5727 (US); HOOVER, Andrew, J., Boston, MA 02115-5727 (US); MA, Xiaoshen, Boston, MA 02115-5727 (US); GATHIAKA, Symon, Boston, MA 02115-5727 (US); SLOMAN, David, L., Boston, MA 02115-5727 (US)
(74) Representative: Bradley, Josephine Mary
(86) International application number: PCT/US2020/051464
(87) International publication number: WO 2021/055728

(56) References cited:
- WO-A1-2017/172979
- WO-A1-2017/201161
- WO-A1-2019/099524
- US-A- 4 034 075
- US-A1- 2006 135 532
- US-A1- 2015 239 900
- DATABASE PubChem COMPOUND 25 October 2006 (2006-10-25), "4-[[4-[4-(Carboxymethyl)piperazin-1-yl]quinoline-2-carbonyl]amino]-5-(4-ethoxycarbonylpiperazin-1-yl)-5-oxopentanoic acid", XP055807399, retrieved from ncbi Database accession no. CID10121096

## Description

### CROSS REFERENCE TO RELATED APPLICATIONS

This application claims the benefit of priority of provisional Appl. No. 62/902,022, filed September 18, 2019.

### FIELD OF THE INVENTION

The present disclosure relates to certain heteroaryl compounds and pharmaceutically acceptable salts thereof that inhibit the G12C mutant of Kirsten rat sarcoma (KRAS) protein and are expected to have utility as therapeutic agents, for example, for treatment of cancer. The present application also relates to pharmaceutical compounds containing such compounds as well as the compounds for use in methods for treating cancer.

### BACKGROUND OF THE INVENTION

RAS proteins are membrane-associated guanine nucleotide-binding proteins which function as molecular switches. RAS proteins function as components of signalling pathways transmitting signals from cell-surface receptors to regulate cellular proliferation, survival and differentiation. RAS proteins cycle between an inactive GDP-bound state and an active GTP-bound state.

The most notable members of the RAS subfamily are HRAS, KRAS and NRAS, mainly for being implicated in many types of cancer. However, there are many other members including DIRAS1; DIRAS2; DIRAS3; ERAS; GEM; MRAS; KIRAS1; KIRAS2; NRAS; RALA; RALB; RAPIA; RAPID; RAP2A; RAP2B; RAP2C; RASD1; RASD2; RASL10A; RASL10B; RASL11A; RASL11B; RASL12; REM1; REM2; RERG; RERGL; RRAD; RRAS and RRAS2.

Mutations in any one of the three main isoforms of RAS (HRAS, NRAS, or KRAS) genes are among the most common events in human tumorigenesis. KRAS mutations are detected in 25-30% of tumors. By comparison, the rates of oncogenic mutation occurring in the NRAS and HRAS family members are much lower (8% and 3% respectively).

Exchange of a glycine for a cysteine at residue 12 of RAS (the G12C mutation) results from a mutation commonly found in RAS genes. Large-scale cancer sequencing studies indicate that the G12C mutation appeared most frequently in lung, colorectal and pancreatic cancers. Histological analysis of seven cancer types indicated non-small cell lung cancer contributed the most, 70-75%, to cancer cases having the KRAS G12C mutation. *See* Lindsay, C.R., et al., Br J Cancer 121, 197-198 (2019).

Accordingly, while progress has been made in this field, there remains a need in the art for improved compounds and methods for treatment of cancer, for example, by inhibition of a mutant KRAS, HRAS or NRAS protein (*e.g*., KRAS G12C). Embodiments of the present disclosure fulfill this need and provide further related advantages.
WO2019099524A1 relates to compounds that inhibit KRas G12C. In particular, WO2019099524A1 relates to compounds that irreversibly inhibit the activity of KRas G12C, pharmaceutical compositions comprising the compounds and methods of use therefor.

### SUMMARY OF THE INVENTION

The present disclosure provides compounds which modulate mutant KRAS, HRAS, and/or NRAS proteins and may be valuable pharmaceutically active compounds for the treatment of cancer. In some embodiments the disclosed compounds selectively inhibit the KRAS (G12C) protein. The compounds of Formula (I) 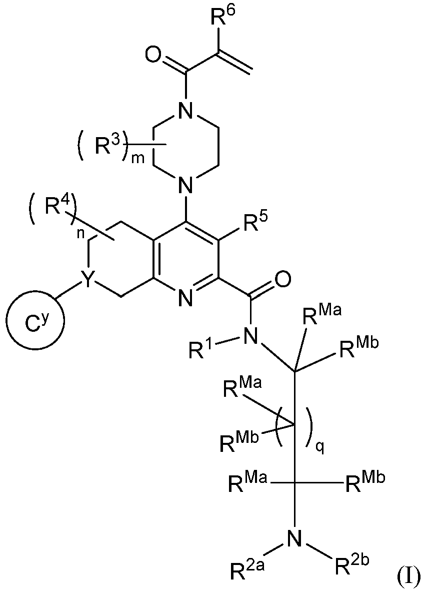 and their pharmaceutically acceptable salts, can modulate the activity of KRAS, HRAS and/or NRAS activity and thereby affect the signaling pathway which regulates cell growth, differentiation, and proliferation associated with oncological disorders. In certain embodiments, the compounds of Formula (I) can inhibit the KRAS (G12C) protein. The disclosure furthermore provides processes for preparing compounds of Formula (I), to such compounds for use in the treatment of oncological disorders and for preparing compounds for this purpose, and to pharmaceutical compositions which comprise compounds of Formula (I).

### DETAILED DESCRIPTION OF THE INVENTION

### Compounds of the Disclosure

In embodiment no. 1, the present disclosure provides a compound having structural Formula (I) as shown above:
wherein:
Y is N;
R¹ is:
   (a) H,
   (b) C₁-C₄ alkyl, wherein said C₁-C₄ alkyl is unsubstituted or substituted by 1 to 2 hydroxy, cyano, carboxy, amino, or C₁-C₄ alkoxy, or
   (c) C₁-C₄ fluoroalkyl, wherein said C₁-C₄ fluoroalkyl is unsubstituted or substituted by 1 to 2 hydroxy, cyano, carboxy, amino, or C₁-C₄ alkoxy;
each occurrence of R^{Ma} and R^{Mb} is independently:
   (a) H,
   (b) C₁-C₄ alkyl, wherein said C₁-C₄ alkyl is unsubstituted or substituted by 1 to 2 fluoro, hydroxy, cyano, carboxy, amino, or C₁-C₄ alkoxy;
   (c) C₁-C₄ fluoroalkyl, wherein said C₁-C₄ fluoroalkyl is unsubstituted or substituted by 1 to 2 hydroxy, cyano, carboxy, amino, or C₁-C₄ alkoxy;
   (d) alternatively, R^{Ma} and R^{Mb} , when geminally substituted on a common carbon atom, together with the atom to which they are attached, form a ring M^{c}, wherein ring M^{c} is a 3- to 8-membered saturated ring optionally containing one heteroatom selected from N and O,
      wherein ring M^{c} is unsubstituted or substituted by 1 to 6 fluoro, hydroxy, cyano, carboxy, amino, oxo, C₁-C₃ alkoxy, C₁-C₃ alkyl, or C₁-C₃ fluoroalkyl;
      wherein said C₁-C₃ alkyl or C₁-C₃ fluoroalkyl peripheral substituent of ring M^{c} is unsubstituted or substituted by 1 to 2 hydroxy, cyano, carboxy, amino, oxo, or C₁-C₃ alkoxy;
   (e) alternatively, R^{Ma} and R^{Mb}, when substituted on vicinal or hominal carbon atoms, together with the atoms to which they are attached, form a ring M^{d}, wherein ring M^{d} is phenyl, a 5- to 6-membered heteroaryl containing one to three heteroatoms selected from N, O, and S, or a 3- to 6-membered saturated ring optionally containing one heteroatom selected from N and O,
      wherein ring M^{d} is unsubstituted or substituted by 1 to 6 fluoro, hydroxy, cyano, carboxy, amino, oxo, C₁-C₃ alkoxy, C₁-C₃ alkyl, or C₁-C₃ fluoroalkyl;
      wherein said C₁-C₃ alkyl or C₁-C₃ fluoroalkyl peripheral substituent of ring M^{d} is unsubstituted or substituted by 1 to 2 hydroxy, cyano, carboxy, amino, oxo, or C₁-C₃ alkoxy;
(f) alternatively, any R^{Ma} and R¹, together with the atoms to which they are attached, form ring M^{e}, wherein ring M^{e} is a 3- to 6-membered heterocycloalkyl ring optionally containing one additional heteroatom selected from N and O,
   wherein ring M^{e} is unsubstituted or substituted by 1 to 6 fluoro, hydroxy, cyano, carboxy, amino, oxo, C₁-C₃ alkoxy, C₁-C₃ alkyl, or C₁-C₃ fluoroalkyl;
   wherein said C₁-C₃ alkyl or C₁-C₃ fluoroalkyl peripheral substituent of ring M^{e} is unsubstituted or substituted by 1 to 2 hydroxy, cyano, carboxy, amino, oxo, or C₁-C₃ alkoxy; or
(g) alternatively, any R^{Ma} and R^{2b}, together with the atoms to which they are attached, form ring M^{f}, wherein ring M^{f} is a 4- to 7-membered heterocycloalkyl ring optionally containing one additional heteroatom selected from N and O,
   wherein ring M^{f} is unsubstituted or substituted by 1 to 6 fluoro, hydroxy, cyano, carboxy, amino, oxo, C₁-C₃ alkoxy, C₁-C₃ alkyl, or C₁-C₃ fluoroalkyl;
   wherein said C₁-C₃ alkyl or C₁-C₃ fluoroalkyl peripheral substituent of ring M^{f} is unsubstituted or substituted by 1 to 2 hydroxy, cyano, carboxy, amino, oxo, or C₁-C₃ alkoxy;
R^{2a} is:
   (a) H,
   (b) C₁-C₄ alkyl, wherein said C₁-C₄ alkyl is unsubstituted or substituted by 1 to 2 hydroxy, cyano, carboxy, amino, or C₁-C₄ alkoxy, or
   (c) C₁-C₄ fluoroalkyl, wherein said C₁-C₄ fluoroalkyl is unsubstituted or substituted by 1 to 2 hydroxy, cyano, carboxy, amino, or C₁-C₄ alkoxy;
R2b is:
   (a) H,
   (b) C₁-C₄ alkyl, wherein said C₁-C₄ alkyl is unsubstituted or substituted by 1 to 2 hydroxy, cyano, carboxy, amino, or C₁-C₄ alkoxy,
   (c) C₁-C₄ fluoroalkyl, wherein said C₁-C₄ fluoroalkyl is unsubstituted or substituted by 1 to 2 hydroxy, cyano, carboxy, amino, or C₁-C₄ alkoxy; or
   (d) R^{2b} and R^{Ma}, together with the atoms to which they are attached, form ring M^{f} as described above,
   or alternatively R^{2a} and R^{2b}, together with the nitrogen atom to which they are attached, form ring C^{Z}, wherein ring C^{Z} is a 3- to 7-membered heterocycloalkyl optionally containing one additional heteroatom selected from N and O,
   wherein ring C^{Z} is unsubstituted or substituted by 1 to 6 fluoro, hydroxy, cyano, carboxy, amino, oxo, C₁-C₃ alkoxy, C₁-C₃ alkyl, or C₁-C₃ fluoroalkyl;
   wherein said C₁-C₃ alkyl or C₁-C₃ fluoroalkyl peripheral substituent of ring C^{Z} is unsubstituted or substituted by 1 to 2 hydroxy, cyano, carboxy, amino, oxo, or C₁-C₃ alkoxy;
ring Cy is:
   (a) C₆-C₁₀ aryl, or
   (b) a five- to ten-membered heteroaryl containing one to four heteroatoms selected from N, O, and S;
   wherein ring C^{y} is unsubstituted or substituted by one to four halo, hydroxy, C₁-C₃ alkyl, C₁-C₃ fluoroalkyl, C₁-C₃ alkoxy, cyano, or C₁-C₄ hydroxyalkyl;
R³ is C₁-C₄ alkyl, C₁-C₄ cyanoalkyl, C₁-C₄ fluoroalkyl, C₁-C₄ hydroxyalkyl, oxo, carboxy, or alternatively, two R³ substituents, together with the carbon atoms to which they are attached, can form a bicyclo- or spirocyclic ring system with the illustrated piperazine ring;
R⁴ is C₁-C₃ alkyl, C₁-C₃ cyanoalkyl, C₁-C₃ fluoroalkyl, hydroxy, oxo, or fluoro;
R⁵ is H, halo, cyano, C₁-C₃ alkyl, C₁-C₃ fluoroalkyl or C₁-C₃ alkoxy;
R⁶ is H or halo;
the subscript m is 0, 1, 2, or 3;
the subscript n is 0, 1 or 2; and
the subscript q is 0 or 1;
or a pharmaceutically acceptable salt thereof.

In embodiment no. 2, the present disclosure provides a compound of Formula (I), wherein each occurrence of R^{Ma} and R^{Mb} is independently:
(a) H,
(b) C₁-C₄ alkyl, wherein said C₁-C₄ alkyl is unsubstituted or substituted by 1 to 2 fluoro, hydroxy, cyano, carboxy, amino, or C₁-C₄ alkoxy;
(c) C₁-C₄ fluoroalkyl, wherein said C₁-C₄ fluoroalkyl is unsubstituted or substituted by 1 to 2 hydroxy, cyano, carboxy, amino, or C₁-C₄ alkoxy;
(d) alternatively, R^{Ma} and R^{Mb} , when geminally substituted on a common carbon atom, together with the atom to which they are attached, form a ring M^{c}, wherein ring M^{c} is a 3- to 6-membered saturated ring optionally containing one heteroatom selected from N and O,
   wherein ring M^{c} is unsubstituted or substituted by 1 to 6 fluoro, hydroxy, cyano, carboxy, amino, oxo, C₁-C₃ alkoxy, C₁-C₃ alkyl, or C₁-C₃ fluoroalkyl;
   wherein said C₁-C₃ alkyl or C₁-C₃ fluoroalkyl peripheral substituent of ring M^{c} is unsubstituted or substituted by 1 to 2 hydroxy, cyano, carboxy, amino, oxo, or C₁-C₃ alkoxy;
(e) alternatively, R^{Ma} and R^{Mb}, when substituted on vicinal or hominal carbon atoms, together with the atoms to which they are attached, form a ring M^{d}, wherein ring M^{d} is phenyl, a 5- to 6-membered heteroaryl containing one to three heteroatoms selected from N, O, and S, or a 3- to 6-membered saturated ring optionally containing one heteroatom selected from N and O,
   wherein ring M^{d} is unsubstituted or substituted by 1 to 6 fluoro, hydroxy, cyano, carboxy, amino, oxo, C₁-C₃ alkoxy, C₁-C₃ alkyl, or C₁-C₃ fluoroalkyl;
   wherein said C₁-C₃ alkyl or C₁-C₃ fluoroalkyl peripheral substituent of ring M^{d} is unsubstituted or substituted by 1 to 2 hydroxy, cyano, carboxy, amino, oxo, or C₁-C₃ alkoxy;
(f) alternatively, any R^{Ma} and R¹, together with the atoms to which they are attached, form ring M^{e}, wherein ring M^{e} is a 3- to 6-membered heterocycloalkyl ring optionally containing one additional heteroatom selected from N and O,
   wherein ring M^{e} is unsubstituted or substituted by 1 to 6 fluoro, hydroxy, cyano, carboxy, amino, oxo, C₁-C₃ alkoxy, C₁-C₃ alkyl, or C₁-C₃ fluoroalkyl;
   wherein said C₁-C₃ alkyl or C₁-C₃ fluoroalkyl peripheral substituent of ring M^{e} is unsubstituted or substituted by 1 to 2 hydroxy, cyano, carboxy, amino, oxo, or C₁-C₃ alkoxy; or
(g) alternatively, any R^{Ma} and R^{2b}, together with the atoms to which they are attached, form ring M^{f}, wherein ring M^{f} is a 4- to 7-membered heterocycloalkyl ring optionally containing one additional heteroatom selected from N and O,
   wherein ring M^{f} is unsubstituted or substituted by 1 to 6 fluoro, hydroxy, cyano, carboxy, amino, oxo, C₁-C₃ alkoxy, C₁-C₃ alkyl, or C₁-C₃ fluoroalkyl;
   wherein said C₁-C₃ alkyl or C₁-C₃ fluoroalkyl peripheral substituent of ring M^{f} is unsubstituted or substituted by 1 to 2 hydroxy, cyano, carboxy, amino, oxo, or C₁-C₃ alkoxy;

R⁶ is H; and
the remaining variables are as set forth in embodiment no. 1.

In embodiment no. 3, the present disclosure provides a compound of Formula (I), wherein the subscript q is 0, and the remaining variables are as set forth in any one of embodiment nos. 1-2.

In embodiment no. 4 the present disclosure provides a compound of Formula (I), wherein R¹ is H, and the remaining variables are as set forth in any one of embodiment nos. 1

In embodiment no. 5 the present disclosure provides a compound of Formula (I),
wherein Y is N, the subscript q is 0, R¹ is H, and the remaining variables are as set forth in embodiment no. 1.

In embodiment no. 6,
the present disclosure provides a compound of Formula (I) as set forth in embodiment no. 5, wherein each R^{Ma} and R^{Mb} are independently:
(a) H,
(b) C₁-C₄ alkyl, wherein said C₁-C₄ alkyl is unsubstituted or substituted by 1 to 2 fluoro, hydroxy, cyano, carboxy, amino, or C₁-C₄ alkoxy; or
(c) C₁-C₄ fluoroalkyl, wherein said C₁-C₄ fluoroalkyl is unsubstituted or substituted by 1 to 2 hydroxy, cyano, carboxy, amino, or C₁-C₄ alkoxy.

In embodiment no. 7 the present disclosure provides a compound of Formula (I) as set forth in embodiment no. 5,
wherein R^{Ma} and R^{Mb} are each independently H or C₁-C₄ alkyl.

In embodiment no. 8 the present disclosure provides a compound of Formula (I) as set forth in embodiment no. 5, wherein R2a and R^{2b} are each independently:
(a) H,
(b) C₁-C₄ alkyl, wherein said C₁-C₄ alkyl is unsubstituted or substituted by 1 to 2 hydroxy, cyano, carboxy, amino, or C₁-C₄ alkoxy, or
(c) C₁-C₄ fluoroalkyl, wherein said C₁-C₄ fluoroalkyl is unsubstituted or substituted by 1 to 2 hydroxy, cyano, carboxy, amino, or C₁-C₄ alkoxy.

In embodiment no. 9, the present disclosure provides a compound of Formula (I) as set forth in embodiment no. 5,
wherein R^{2a} and R^{2b} are each independently H or C₁-C₄ alkyl.

In embodiment no. 10, the present disclosure provides a compound of Formula (I) as set forth in any one of embodiment nos. 1-5, wherein the group

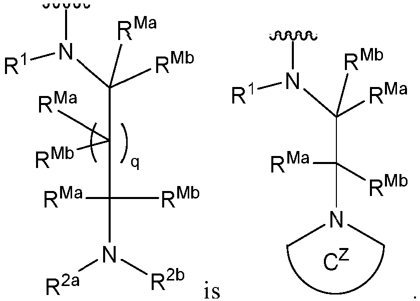

In embodiment no. 11, the present disclosure provides a compound of Formula (I) as set forth in embodiment no. 10, wherein ring C^{Z} is azetidine, pyrrolidine, piperidine, piperazine or morpholine.

In embodiment no. 12, the present disclosure provides a compound of Formula (I) as set forth in any one of embodiment nos. 1-5, wherein the group

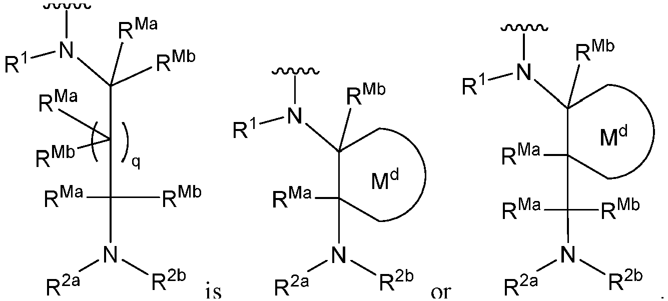

In embodiment no. 13, the present disclosure provides a compound of Formula (I) as set forth in embodiment no. 12, wherein the group

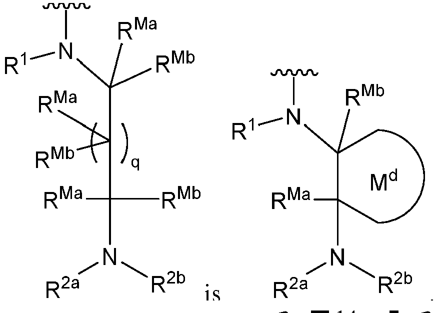

In embodiment no. 14, the present disclosure provides a compound of Formula (I) as set forth in embodiment no. 12, wherein ring M^{d} is a 3- to 6-membered cycloalkyl ring or a 3- to 6-membered saturated heterocycloalkyl ring containing one heteroatom selected from N and O.

In embodiment no. 15, ring M^{d} is a 3- to 6-membered cycloalkyl ring.

In embodiment no. 16, ring M^{d} is phenyl.

In embodiment no. 17, the present disclosure provides a compound of Formula (I) as set forth in any one of embodiment nos. 1-5, wherein the group

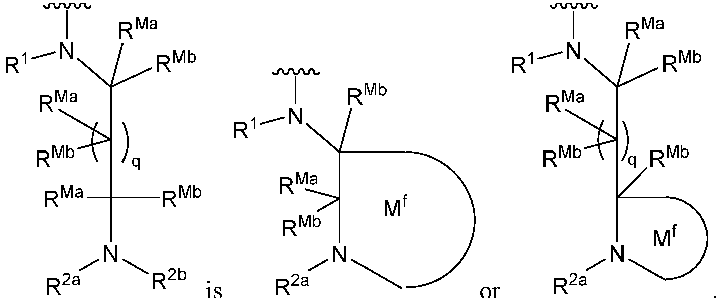

In embodiment no. 18, the present disclosure provides a compound of Formula (I) as set forth in any one of embodiment nos. 1-5, wherein the group

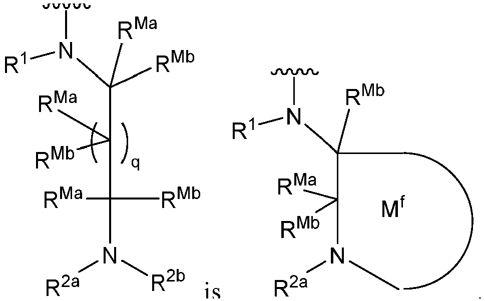

In embodiment no. 19, the present disclosure provides a compound of Formula (I) as set forth in embodiment no. 18, wherein ring M^{f} is an azetidine, pyrrolidine, piperidine, or azepine ring.

In embodiment no. 20, ring M^{f} is a pyrrolidine or piperidine ring.

In embodiment no. 21, the present disclosure provides a compound of Formula (I) as set forth in any one of embodiment nos. 18-20, wherein R2a is methyl.

In embodiment no. 22, the present disclosure provides a compound of Formula (I) as set forth in any one of embodiment nos. 1-2, wherein the group

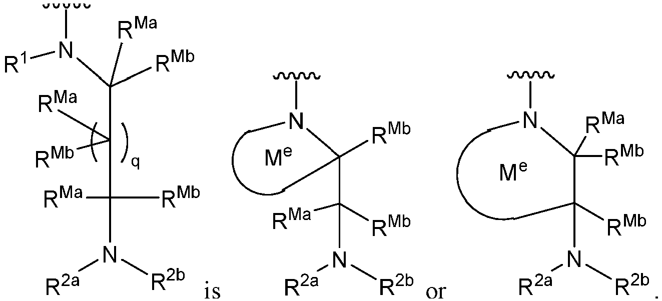

In embodiment no. 23, the present disclosure provides a compound of Formula (I) as set forth in embodiment no. 22, wherein ring M^{e} is a pyrrolidine ring.

In embodiment no. 24, the present disclosure provides a compound of Formula (I) as set forth in any one of embodiment nos. 1-5, wherein the group

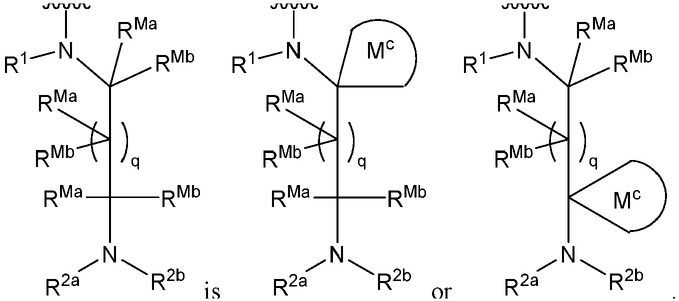

In embodiment no. 25, the present disclosure provides a compound of Formula (I) as set forth in any one of embodiment nos. 1-2, wherein the group

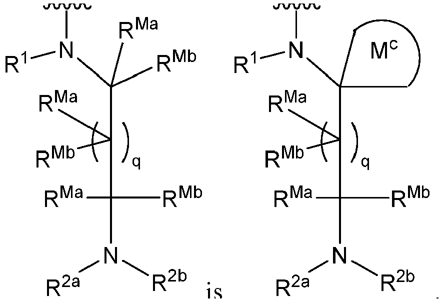

In embodiment no. 26, the present disclosure provides a compound as set forth in embodiment no. 25, wherein ring M^{c} is cyclopropane, q = 0, and R¹ = H.

In embodiment no. 27, the present disclosure provides a compound of Formula (I) as set forth in any one of embodiment nos. 1-26, wherein the subscript m is 0 or 1.

In embodiment no. 28, the present disclosure provides a compound of Formula (I) as set forth in any one of embodiment nos. 27, wherein the subscript n is 0.

In embodiment no. 29, the present disclosure provides a compound of Formula (I) as set forth in any one of embodiment nos. 1-28, wherein R⁵ is H.

In embodiment no. 30, the present disclosure provides a compound as described in any one of Examples 1-107 as set forth below, or a pharmaceutically acceptable salt thereof.

The present disclosure includes the pharmaceutically acceptable salts of the compounds defined herein, including the pharmaceutically acceptable salts of all structural formulas, embodiments and classes defined herein. Reference to the compounds of structural Formula (I) includes the compounds of other generic structural Formulas and embodiments that fall within the scope of Formula (I).

### Definitions

Unless defined otherwise, all technical and scientific terms used herein have the same meaning as is commonly understood by one of skill in the art to which this disclosure belongs.

As used throughout this disclosure, "a compound of Formula (I)" is to be understood to include "a compound of Formula (I) or a pharmaceutically acceptable salt thereof".

"Alkyl", as well as other groups having the prefix "alk", such as alkoxy, and the like, means carbon chains which may be linear or branched, or combinations thereof, containing the indicated number of carbon atoms. If no number is specified, 1-6 carbon atoms are intended for linear and 3-7 carbon atoms for branched alkyl groups. Examples of alkyl groups include methyl, ethyl, propyl, isopropyl, butyl, *sec-* and *tert*-butyl, pentyl, hexyl, heptyl, octyl, nonyl and the like.

"Alkoxy" and "alkyl-O-" are used interchangeably and refer to an alkyl group linked to oxygen.

"Aryl" means phenyl or naphthyl.

"Bicyclo- ring system" refers to two joined rings. The rings may be fused, i.e., share two adjacent atoms, or "spirocyclic", i.e., share only a single atom.

"Cyanoalkyl" refers to an alkyl group substituted with a cyano group.

"Fluoroalkyl" includes mono-substituted as well as multiple fluoro-substituted alkyl groups, up to perfluoro substituted alkyl. For example, fluoromethyl, 1,1-difluoroethyl, trifluoromethyl or 1,1,1,2,2-pentafluorobutyl are included.

"Cycloalkyl" means a saturated cyclic hydrocarbon radical having the number of carbon atoms designated if no number of atoms is specified, 3-12 carbon atoms are intended, forming 1-3 carbocyclic rings that are fused. Examples of cycloalkyl include cyclopropyl, cyclobutyl, cyclopentyl, cyclohexyl, cycloheptyl, adamantyl, and the like.

"Diagnostic signals", as used herein in reference to describing ¹H NMR spectra, refer to signals that are characteristic for a given compound, where such signals are a subset of all signals in an NMR spectrum.

"Geminally substituted" refers to substitutions (e.g., functional groups) that are attached to the same atom in a molecule. For instance, 1,1-dichloroethane is an ethane molecule which is geminally substituted with two chlorine atoms.

"Heterocycloalkyl" refers to nonaromatic monocyclic and bicyclic ring structures in which one or more atoms in the ring, the heteroatom(s), is an element other than carbon. Such nonaromatic cyclic ring structures can be saturated or unsaturated. Heteroatoms are typically O, S or N atoms. Examples of heterocycloalkyl groups include piperidine, piperazinyl, morpholinyl, pyrrolidinyl, tetrahydrofuranyl, azetidinyl, oxiranyl, or aziridinyl, and the like.

"Heteroaryl" refers to aromatic monocyclic and bicyclic ring structures in which one or more atoms in the ring, the heteroatom(s), is an element other than carbon. Heteroatoms are typically O, S, or N atoms. Examples of heteroaromatic groups include pyridinyl, pyrimidinyl, pyrrolyl, pyridazinyl, isoxazolyl, thiazolyl, oxazolyl, indolyl, benzoxazolyl, benzothiazolyl, or imidazolyl.

"Halogen" (or "halo") unless otherwise indicated, includes fluorine (fluoro), chlorine (chloro), bromine (bromo) and iodine (iodo). In one embodiment, halo is fluoro (-F) or chloro (-Cl).

"Hominally substituted" refers to substitutions or groups that are attached on a molecule in a 1,3-relationship. For instance, 1,3-dichloropropane ClCH₂CH₂CH₂Cl is a propane molecule which is hominally substituted with two chlorine atoms.

"Hydroxyalkyl" includes mono-substituted as well as multiple hydroxy-substituted alkyl groups.

When any variable (*e.g*., R³, R⁴, etc.) occurs more than one time in any constituent or in Formula (I) or other generic formulas herein, its definition on each occurrence is independent of its definition at every other occurrence. Combinations of substituents and/or variables are permissible only if such combinations result in stable compounds. In choosing compounds of the present disclosure, one of ordinary skill in the art will recognize that the various substituents, *i.e.,* R³, R⁴, etc., are to be chosen in conformity with well-known principles of chemical structure connectivity and stability. Unless expressly stated to the contrary, substitution by a named substituent is permitted on any atom in a ring (*e.g.,* aryl, a heteroaryl ring, or a saturated heterocycloalkyl ring) provided such ring substitution is chemically allowed and results in a stable compound. A "stable" compound is a compound which can be prepared and isolated and whose structure and properties remain or can be caused to remain essentially unchanged for a period of time sufficient to allow use of the compound for the purposes described herein (*e.g.,* therapeutic or prophylactic administration to a subject).

The term "substituted" shall be deemed to include multiple degrees of substitution by a named substituent. Where multiple substituent moieties are disclosed or claimed, the substituted compound can be independently substituted by one or more of the disclosed or claimed substituent moieties, singly or plurally. By independently substituted, it is meant that the (two or more) substituents can be the same or different.

"Vicinally substituted" refers to substitutions or groups that are attached to the adjacent atoms in a molecule. For instance, 1,2-dichloroethane is an ethane molecule which is vicinally substituted with two chlorine atoms.

Unless expressly depicted or described otherwise, variables depicted in a structural formula with a "floating" bond, such as R³ or R⁴ in Formula (I), are permitted on any available carbon atom in the ring to which the variable is attached. When a moiety is noted as being "optionally substituted" in Formula (I) or any embodiment thereof, it means that Formula (I) or the embodiment thereof encompasses compounds that contain the noted substituent (or substituents) on the moiety and also compounds that do not contain the noted substituent (or substituents) on the moiety.

The wavy line 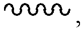 as used herein, indicates a point of attachment to the rest of the compound.

Compounds of Formula (I) may contain one or more asymmetric centers and can thus occur as racemates and racemic mixtures, single enantiomers, diastereoisomeric mixtures and individual diastereoisomers. Centers of asymmetry that are present in the compounds of Formula (I) can all independently of one another have S configuration or R configuration. The compounds of this disclosure include all possible enantiomers and diastereomers and mixtures of two or more stereoisomers, for example, mixtures of enantiomers and/or diastereomers, in all ratios. Thus, enantiomers are a subject of the disclosure in enantiomerically pure form, both as levorotatory and as dextrorotatory antipodes, in the form of racemates and in the form of mixtures of the two enantiomers in all ratios. In the case of a cis/trans isomerism, the disclosure includes both the cis form and the trans form as well as mixtures of these forms in all ratios. The present disclosure is meant to comprehend all such stereoisomeric forms of the compounds of Formula (I). Where a structural formula or chemical name specifies a particular configuration at a stereocenter, the enantiomer or stereoisomer of the compound resulting from that specified stereocenter is intended. Where a structural formula of the compounds of Formula (I) indicates a straight line at a chiral center, the structural formula includes both the S and R stereoisomers associated with the chiral center and mixtures thereof.

Compounds of Formula (I) may be separated into their individual diastereoisomers by, for example, fractional crystallization from a suitable solvent, for example, methanol or ethyl acetate or a mixture thereof, or via chiral chromatography using an optically active stationary phase. Absolute stereochemistry may be determined by X-ray crystallography of crystalline products or crystalline intermediates which are derivatized, if necessary, with a reagent containing an asymmetric center of known absolute configuration. Vibrational circular dichroism (VCD) may also be used to determine the absolute stereochemistry. Alternatively, any stereoisomer or isomers of a compound of Formula (I) may be obtained by stereospecific synthesis using optically pure starting materials or reagents of known absolute configuration.

If desired, racemic mixtures of the compounds may be separated so that the individual enantiomers are isolated. The separation can be carried out by methods well known in the art, such as the coupling of a racemic mixture of compounds to an enantiomerically pure compound to form a diastereoisomeric mixture, followed by separation of the individual diastereoisomers by standard methods, such as fractional crystallization or chromatography. The coupling reaction is often the formation of salts using an enantiomerically pure acid or base. The diastereomeric derivatives may then be converted to the pure enantiomers by cleavage of the added chiral residue. The racemic mixture of the compounds can also be separated directly by chromatographic methods utilizing chiral stationary phases, which methods are well known in the art.

For compounds of Formula (I) described herein which contain olefinic double bonds, unless specified otherwise, they are meant to include both E and Z geometric isomers.

Some of the compounds described herein may exist as tautomers which have different points of attachment of hydrogen accompanied by one or more double bond shifts. For example, a ketone and its enol form are keto-enol tautomers. The individual tautomers as well as mixtures thereof are encompassed with compounds of Formula (I) of the present disclosure.

In the compounds of Formula (I), the atoms may exhibit their natural isotopic abundances, or one or more of the atoms may be artificially enriched in a particular isotope having the same atomic number, but an atomic mass or mass number different from the atomic mass or mass number predominantly found in nature. The present disclosure as described and claimed herein is meant to include all suitable isotopic variations of the compounds of Formula (I) and embodiments thereof. For example, different isotopic forms of hydrogen (H) include protium (¹H) and deuterium (²H, also denoted herein as D). Protium is the predominant hydrogen isotope found in nature. Enriching for deuterium may afford certain therapeutic advantages, such as increasing *in vivo* half-life or reducing dosage requirements, or may provide a compound useful as a standard for characterization of biological samples. Isotopically-enriched compounds of Formula (I) can be prepared without undue experimentation by conventional techniques well known to those skilled in the art or by processes analogous to those described in the Schemes and Examples herein using appropriate isotopically-enriched reagents and/or intermediates.

The term "pharmaceutically acceptable salts" refers to salts prepared from pharmaceutically acceptable non-toxic bases or acids. When the compound of the present disclosure is acidic, its corresponding salt can be conveniently prepared from pharmaceutically acceptable non-toxic bases, including inorganic bases and organic bases. Salts derived from such inorganic bases include aluminum, ammonium, calcium, copper (ic and ous), ferric, ferrous, lithium, magnesium, manganese (ic and ous), potassium, sodium, zinc and the like salts. Preferred are the ammonium, calcium, magnesium, potassium and sodium salts. Salts prepared from pharmaceutically acceptable organic non-toxic bases include salts of primary, secondary, and tertiary amines derived from both naturally occurring and synthetic sources. Pharmaceutically acceptable organic non-toxic bases from which salts can be formed include, for example, arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, dicyclohexylamine, lysine, methylglucamine, morpholine, piperazine, piperidine, polyamine resins, procaine, purines, theobromine, triethylamine, trimethylamine, tripropylamine, tromethamine and the like.

When the compound of the present disclosure is basic, its corresponding salt can be conveniently prepared from pharmaceutically acceptable non-toxic inorganic and organic acids. Such acids include, for example, acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid and the like. Preferred are citric, hydrobromic, hydrochloric, maleic, phosphoric, sulfuric, and tartaric acids. If the compounds of Formula (I) simultaneously contain acidic and basic groups in the molecule, the disclosure also includes, in addition to the salt forms mentioned, inner salts or betaines (zwitterions). Salts can be obtained from the compounds of Formula (I) by customary methods which are known to the person skilled in the art, for example, by combination with an organic or inorganic acid or base in a solvent or dispersant, or by anion exchange or cation exchange from other salts. The present disclosure also includes all salts of the compounds of Formula (I) which, owing to low physiological compatibility, are not directly suitable for use in pharmaceuticals but which can be used, for example, as intermediates for chemical reactions or for the preparation of pharmaceutically acceptable salts.

Furthermore, compounds of the present disclosure may exist in amorphous form and/or one or more crystalline forms, and, as such, all amorphous and crystalline forms and mixtures thereof of the compounds of Formula (I), including the Examples, are intended to be included within the scope of the present disclosure. In addition, some of the compounds of the instant disclosure may form solvates with water (*i.e.,* a hydrate) or common organic solvents such as, but not limited to, ethyl acetate. Such solvates and hydrates, particularly the pharmaceutically acceptable solvates and hydrates, of the instant compounds are likewise encompassed within the scope of this disclosure, along with un-solvated and anhydrous forms.

The present disclosure also relates to processes for the preparation of the compounds of Formula (I) which are described in the following and by which the compounds of the disclosure are obtainable.

The terms "therapeutically effective (or efficacious ) amount" and similar descriptions such as "an amount efficacious for treatment" are intended to mean that amount of a pharmaceutical drug that will elicit the biological or medical response of a tissue, a system, animal or human that is being sought by a researcher, veterinarian, medical doctor or other clinician. In a preferred embodiment, the term "therapeutically effective amount" means an amount of a pharmaceutical drug that alleviates at least one clinical symptom in a human patient. The terms "prophylactically effective (or efficacious) amount" and similar descriptions such as "an amount efficacious for prevention" are intended to mean that amount of a pharmaceutical drug that will prevent or reduce the risk of occurrence of the biological or medical event that is sought to be prevented in a tissue, a system, animal or human by a researcher, veterinarian, medical doctor or other clinician.

### Dosages of the Compounds of Formula (I)

The dosage regimen utilizing a compound of the instant disclosure is selected in accordance with a variety of factors including type, species, age, weight, sex and medical condition of the patient; the severity of the condition to be treated; the potency of the compound chosen to be administered; the route of administration; and the renal and hepatic function of the patient. A consideration of these factors is well within the purview of the ordinarily skilled clinician for the purpose of determining the therapeutically effective or prophylactically effective dosage amount needed to prevent, counter, or arrest the progress of the condition. It is understood that a specific daily dosage amount can simultaneously be both a therapeutically effective amount, *e.g.,* for treatment of an oncological condition, and a prophylactically effective amount, *e.g.,* for prevention of an oncological condition.

While individual needs vary, determination of optimal ranges of effective amounts of the compound of the disclosure is within the skill of the art. For administration to a human in the curative or prophylactic treatment of the conditions and disorders identified herein, for example, typical dosages of the compounds of the present disclosure can be about 0.05 mg/kg/day to about 50 mg/kg/day, for example, at least 0.05 mg/kg, at least 0.08 mg/kg, at least 0.1 mg/kg, at least 0.2 mg/kg, at least 0.3 mg/kg, at least 0.4 mg/kg, or at least 0.5 mg/kg, and 50 mg/kg or less, 40 mg/kg or less, 30 mg/kg or less, 20 mg/kg or less, or 10 mg/kg or less, which can be about 2.5 mg/day (0.5 mg/kg x 5 kg) to about 5000 mg/day (50 mg/kg x 100 kg), for example. For example, dosages of the compounds can be about 0.1 mg/kg/day to about 50 mg/kg/day, about 0.05 mg/kg/day to about 10 mg/kg/day, about 0.05 mg/kg/day to about 5 mg/kg/day, about 0.05 mg/kg/day to about 3 mg/kg/day, about 0.07 mg/kg/day to about 3 mg/kg/day, about 0.09 mg/kg/day to about 3 mg/kg/day, about 0.05 mg/kg/day to about 0.1 mg/kg/day, about 0.1 mg/kg/day to about 1 mg/kg/day, about 1 mg/kg/day to about 10 mg/kg/day, about 1 mg/kg/day to about 5 mg/kg/day, about 1 mg/kg/day to about 3 mg/kg/day, about 3 mg/day to about 500 mg/day, about 5 mg/day to about 250 mg/day, about 10 mg/day to about 100 mg/day, about 3 mg/day to about 10 mg/day, or about 100 mg/day to about 250 mg/day. Such doses may be administered in a single dose or may be divided into multiple doses.

### Pharmaceutical Compositions

The compounds of Formula (I) and their pharmaceutically acceptable salts can be administered to animals, preferably to mammals, and in particular to humans, as pharmaceuticals by themselves, in mixtures with one another or in the form of pharmaceutical compositions. The term "subject" or "patient" includes animals, preferably mammals and especially humans, who use the instant active agents for the prevention or treatment of a medical condition. Administering of the drug to the subject includes both self-administration and administration to the patient by another person. The subject may be in need of, or desire, treatment for an existing disease or medical condition, or may be in need of or desire prophylactic treatment to prevent or reduce the risk of occurrence of said disease or medical condition. As used herein, a subject "in need" of treatment of an existing condition or of prophylactic treatment encompasses both a determination of need by a medical professional, as well as the desire of a patient for such treatment.

The present disclosure therefore also provides the compounds of Formula (I) and their pharmaceutically acceptable salts for use as pharmaceuticals, their use for modulating the activity of mutant KRAS, HRAS and/or NRAS proteins and in particular their use in the therapy and prophylaxis of the below-mentioned diseases or disorders as well as their use for preparing medicaments for these purposes. In certain embodiments, the compounds of Formula (I) and their pharmaceutically acceptable salts inhibit the KRAS G12C protein.

Furthermore, the present disclosure provides pharmaceutical compositions which comprise as active component an effective dose of at least one compound of Formula (I) and/or a pharmaceutically acceptable salt thereof and a customary pharmaceutically acceptable carrier, *i.e.,* one or more pharmaceutically acceptable carrier substances and/or additives.

Thus, the present disclosure provides, for example, said compound and its pharmaceutically acceptable salts for use as pharmaceutical compositions which comprise as active component an effective dose of the compound of Formula (I) and/or a pharmaceutically acceptable salt thereof and a customary pharmaceutically acceptable carrier, and the uses of said compound and/or a pharmaceutically acceptable salt thereof in the therapy or prophylaxis of the below-mentioned diseases or disorders, *e.g*., cancer, as well as their use for preparing medicaments for these purposes.

The pharmaceutical compositions according to the disclosure can be administered orally, for example, in the form of pills, tablets, lacquered tablets, sugar-coated tablets, granules, hard and soft gelatin capsules, aqueous, alcoholic or oily solutions, syrups, emulsions or suspensions, or rectally, for example, in the form of suppositories. Administration can also be carried out parenterally, for example, subcutaneously, intramuscularly or intravenously in the form of solutions for injection or infusion.

Other suitable administration forms are, for example, percutaneous or topical administration, for example, in the form of ointments, tinctures, sprays or transdermal therapeutic systems, or, for example, microcapsules, implants or rods. The preferred administration form depends, for example, on the disease to be treated and on its severity.

The amount of active compound of Formula (I) and/or its pharmaceutically acceptable salts in the pharmaceutical composition normally is from 0.01 to 200 mg, such as from 0.1 to 200 mg, preferably from 1 to 200 mg, per dose, but depending on the type of the pharmaceutical composition, it can also be higher. In some embodiments, the amount of active compound of Formula (I) and/or its pharmaceutically acceptable salts in the pharmaceutical composition is from 0.01 to 10 mg per dose. The pharmaceutical compositions usually comprise 0.5 to 90 percent by weight of the compound of Formula (I) and/or their pharmaceutically acceptable salts. The preparation of the pharmaceutical compositions can be carried out in a manner known *per se.* For this purpose, one or more compounds of Formula (I) and/or their pharmaceutically acceptable salts, together with one or more solid or liquid pharmaceutical carrier substances and/or additives (or auxiliary substances) and, if desired, in combination with other pharmaceutically active compounds having therapeutic or prophylactic action, are brought into a suitable administration form or dosage form which can then be used as a pharmaceutical in human or veterinary medicine.

For the production of pills, tablets, sugar-coated tablets and hard gelatin capsules, it is possible to use, for example, lactose, starch, for example, maize starch, or starch derivatives, talc, stearic acid or its salts, etc. Carriers for soft gelatin capsules and suppositories are, for example, fats, waxes, semisolid and liquid polyols, natural or hardened oils, etc. Suitable carriers for the preparation of solutions, for example, of solutions for injection, or of emulsions or syrups are, for example, water, physiologically acceptable sodium chloride solution, alcohols such as ethanol, glycerol, polyols, sucrose, invert sugar, glucose, mannitol, vegetable oils, etc. It is also possible to lyophilize the compounds of Formula (I) and their pharmaceutically acceptable salts and to use the resulting lyophilisates, for example, for preparing preparations for injection or infusion. Suitable carriers for microcapsules, implants or rods are, for example, copolymers of glycolic acid and lactic acid.

Besides the active compounds and carriers, the pharmaceutical compositions can also contain customary additives, for example, fillers, disintegrants, binders, lubricants, wetting agents, stabilizers, emulsifiers, dispersants, preservatives, sweeteners, colorants, flavorings, aromatizers, thickeners, diluents, buffer substances, solvents, solubilizers, agents for achieving a depot effect, salts for altering the osmotic pressure, coating agents or antioxidants.

The references to the methods of treatment by therapy or surgery or in vivo diagnostic methods herein are to be interpreted as references to compounds, pharmaceutical compositions, combinations and/or medicaments for use in those methods.

### Methods of Using the Compounds of Formula (I)

The present application provides a method of inhibiting RAS-mediated cell signaling comprising contacting a cell with a compound of Formula (I) or a pharmaceutically acceptable salt thereof. Inhibition of RAS-mediated signal transduction can be assessed and demonstrated by a wide variety of ways known in the art. Non-limiting examples include (a) a decrease in GTPase activity of RAS; (b) a decrease in GTP binding affinity or an increase in GDP binding affinity; (c) an increase in K_{off} of GTP or a decrease in K_{off} of GDP; (d) a decrease in the levels of signaling transduction molecules downstream in the RAS pathway, such as a decrease in pMEK, pERK, or pAKT levels; and/or (e) a decrease in binding of RAS complex to downstream signaling molecules including but not limited to Raf. Kits and commercially available assays can be utilized for determining one or more of the above.

The present application also provides methods of using the compounds of Formula (I) (or their pharmaceutically acceptable salts) or pharmaceutical compositions containing such compounds to treat disease conditions, including but not limited to, conditions implicated by mutant KRAS, HRAS and/or NRAS proteins (*e.g*., cancer), and in some embodiments the KRAS G12C mutant.

In some embodiments, a method for treatment of cancer is provided, the method comprising administering a therapeutically effective amount a compound of Formula (I) (or a pharmaceutically acceptable salt thereof) or any of the foregoing pharmaceutical compositions comprising such a compound to a subject in need of such treatment. In some embodiments, the cancer is mediated by a KRAS, HRAS or NRAS mutation, *e.g*., the KRAS G12C mutation. In various embodiments, the cancer is pancreatic cancer, colorectal cancer or lung cancer. In some embodiments, the cancer is gall bladder cancer, thyroid cancer, and bile duct cancer.

In some embodiments the present disclosure provides a method of treating a disorder in a subject in need thereof, wherein said method comprises determining if the subject has a KRAS, HRAS or NRAS mutation (*e.g*., KRAS G12C mutation) and if the subject is determined to have the KRAS, HRAS or NRAS mutation, then administering to the subject a therapeutically effective amount of a compound of Formula (I) or a pharmaceutically acceptable salt thereof.

The disclosed compounds inhibit anchorage-independent cell growth and therefore have the potential to inhibit tumor metastasis. Accordingly, another embodiment the present disclosure provides a method for inhibiting tumor metastasis, the method comprising administering an effective amount of a compound disclosed herein.

KRAS, HRAS or NRAS mutations have also been identified in hematological malignancies (*e.g*., cancers that affect blood, bone marrow and/or lymph nodes). Accordingly, certain embodiments are directed to administration of the compounds of Formula (I) (*e.g.,* in the form of a pharmaceutical composition) to a subject in need of treatment of a hematological malignancy. Such malignancies include, but are not limited to, leukemias and lymphomas. For example, the presently disclosed compounds can be used for treatment of diseases such as acute lymphoblastic leukemia (ALL), acute myelogenous leukemia (AML), chronic lymphocytic leukemia (CLL), small lymphocytic lymphoma (SLL), chronic myelogenous leukemia (CML), acute monocytic leukemia (AMoL) and/ or other leukemias. In other embodiments, the compounds are useful for treatment of lymphomas such as Hodgkins lymphoma or non-Hodgkins lymphoma. In various embodiments, the compounds are useful for treatment of plasma cell malignancies such as multiple myeloma, mantle cell lymphoma, and Waldenstrom's macroglubunemia.

Determining whether a tumor or cancer comprises a KRAS, HRAS or NRAS mutation (*e.g.,* the KRAS G12C mutation) can be undertaken by assessing the nucleotide sequence encoding the KRAS, HRAS or NRAS protein, by assessing the amino acid sequence of the KRAS, HRAS or NRAS protein, or by assessing the characteristics of a putative KRAS, HRAS or NRAS mutant protein. The sequences of wild-type human KRAS, HRAS or NRAS are known in the art.

Methods for detecting a mutation in a KRAS, HRAS or NRAS nucleotide sequence are also known by those of skill in the art. These methods include, but are not limited to, polymerase chain reaction-restriction fragment length polymorphism (PCR-RFLP) assays, polymerase chain reaction-single strand conformation polymorphism (PCR-SSCP) assays, real-time PCR assays, PCR sequencing, mutant allele-specific PCR amplification (MASA) assays, direct sequencing, primer extension reactions, electrophoresis, oligonucleotide ligation assays, hybridization assays, TaqMan assays, SNP genotyping assays, high resolution melting assays and microarray analyses. In some embodiments, samples are evaluated for KRAS, HRAS or NRAS mutations (*e.g*., the KRAS G12C mutation) by real-time PCR. In real-time PCR, fluorescent probes specific for the KRAS, HRAS or NRAS G12C mutation are used. When a mutation is present, the probe binds and fluorescence is detected. In some embodiments, the KRAS, HRAS or NRAS mutation is identified using a direct sequencing method of specific regions (*e.g*., exon 2 and/or exon 3) in the KRAS, HRAS or NRAS gene.

Methods for detecting a mutation in a KRAS, HRAS or NRAS protein (*e.g.,* the KRAS G12C mutation) are known by those of skill in the art. These methods include, but are not limited to, detection of a KRAS, HRAS or NRAS mutant using a binding agent (*e.g.,* an antibody) specific for the mutant protein, protein electrophoresis and Western blotting, and direct peptide sequencing.

A number of tissue samples can be assessed for determining whether a tumor or cancer comprises a KRAS, HRAS or NRAS mutation (*e.g*., the KRAS G12C mutation). In some embodiments, the sample is taken from a subject having a tumor or cancer. In some embodiments, the sample is a fresh tumor/cancer sample. In some embodiments, the sample is a frozen tumor/cancer sample. In some embodiments, the sample is a formalin-fixed paraffin-embedded sample. In some embodiments, the sample is a circulating tumor cell (CTC) sample. In some embodiments, the sample is processed to a cell lysate. In some embodiments, the sample is processed to DNA or RNA.

The present application also provides a method of treating a hyperproliferative disorder comprising administering a therapeutically effective amount of a compound of the disclosure, or a pharmaceutically acceptable salt thereof to a subject in need thereof. In some embodiments, said method relates to the treatment of a subject who suffers from a cancer such as acute myeloid leukemia, cancer in adolescents, adrenocortical carcinoma childhood, AIDS- related cancers (*e.g*., Lymphoma and Kaposi's Sarcoma), anal cancer, appendix cancer, astrocytomas, atypical teratoid, basal cell carcinoma, bile duct cancer, bladder cancer, bone cancer, brain stem glioma, brain tumor, breast cancer, bronchial tumors, Burkitt lymphoma, carcinoid tumor, atypical teratoid, embryonal tumors, germ cell tumor, primary lymphoma, cervical cancer, childhood cancers, chordoma, cardiac tumors, chronic lymphocytic leukemia (CLL), chronic myelogenous leukemia (CML), chronic myleoproliferative disorders, colon cancer, colorectal cancer, craniopharyngioma, cutaneous T-cell lymphoma, extrahepatic ductal carcinoma in situ (DCIS), embryonal tumors, CNS cancer, endometrial cancer, ependymoma, esophageal cancer, esthesioneuroblastoma, ewing sarcoma, extracranial germ cell tumor, extragonadal germ cell tumor, eye cancer, fibrous histiocytoma of bone, gall bladder cancer, gastric cancer, gastrointestinal carcinoid tumor, gastrointestinal stromal tumors (GIST), germ cell tumor, gestational trophoblastic tumor, hairy cell leukemia, head and neck cancer, heart cancer, liver cancer, Hodgkin lymphoma, hypopharyngeal cancer, intraocular melanoma, islet cell tumors, pancreatic neuroendocrine tumors, kidney cancer, laryngeal cancer, lip and oral cavity cancer, liver cancer, lobular carcinoma in situ (LCIS), lung cancer, lymphoma, metastatic squamous neck cancer with occult primary, midline tract carcinoma, mouth cancer; multiple endocrine neoplasia syndromes, multiple myeloma/plasma cell neoplasm, mycosis fungoides, myelodysplasia syndromes, myelodysplastic/myeloproliferative neoplasms, multiple myeloma, merkel cell carcinoma, malignant mesothelioma, malignant fibrous histiocytoma of bone and osteosarcoma, nasal cavity and paranasal sinus cancer, nasopharyngeal cancer, neuroblastoma, non-hodgkin lymphoma, non-small cell lung cancer (NSCLC), oral cancer, lip and oral cavity cancer, oropharyngeal cancer, ovarian cancer, pancreatic cancer, papillomatosis, paraganglioma, paranasal sinus and nasal cavity cancer, parathyroid cancer, penile cancer, pharyngeal cancer, pleuropulmonary blastoma, primary central nervous system (CNS) lymphoma, prostate cancer, rectal cancer, transitional cell cancer, retinoblastoma, rhabdomyosarcoma, salivary gland cancer, skin cancer, stomach (gastric) cancer, small cell lung cancer; small intestine cancer, soft tissue sarcoma, T-Cell lymphoma, testicular cancer, throat cancer, thymoma and thymic carcinoma, thyroid cancer, transitional cell cancer of the renal pelvis and ureter, trophoblastic tumor, unusual cancers of childhood, urethral cancer, uterine sarcoma, vaginal cancer, vulvar cancer, or viral-induced cancer. In some embodiments, said method relates to the treatment of a non-cancerous hyperproliferative disorder such as benign hyperplasia of the skin (*e.g*., psoriasis), restenosis, or prostate (*e.g*., benign prostatic hypertrophy (BPH)).

In some embodiments, the methods for treatment are directed to treating lung cancers, the methods comprise administering a therapeutically effective amount of the compounds of the disclosure (or pharmaceutical composition comprising such compounds) to a subject in need thereof. In certain embodiments the lung cancer is a non-small cell lung carcinoma (NSCLC), for example, adenocarcinoma, squamous-cell lung carcinoma or large-cell lung carcinoma. In some embodiments, the lung cancer is a small cell lung carcinoma. Other lung cancers which the compounds of Formula (I) may provide therapeutic benefit include, but are not limited to, glandular tumors, carcinoid tumors and undifferentiated carcinomas.

The present disclosure also provides methods of modulating a mutant KRAS, HRAS or NRAS protein activity (*e.g.,* activity resulting from the KRAS G12C mutation) by contacting the protein with an effective amount of a compound of Formula (I). Modulation can be inhibiting or activating protein activity. In some embodiments, the present disclosure provides methods of inhibiting protein activity by contacting the mutant KRAS, HRAS or NRAS protein (*e.g.,* KRAS G12C mutant) with an effective amount of a compound of Formula (I) in solution. In some embodiments, the present disclosure provides methods of inhibiting the mutant KRAS, HRAS or NRAS protein activity by contacting a cell, tissue, or organ that expresses the protein of interest. In some embodiments, the disclosure provides methods of inhibiting protein activity in subjects including, but not limited to, rodents and mammals (*e.g.,* humans) by administering into the subjects an effective amount of a compound of Formula (I).

### Combination Therapies

One or more additional pharmacologically active agents may be administered in combination with a compound of Formula (I) (or a pharmaceutically acceptable salt thereof). An additional active agent (or agents) is intended to mean a pharmaceutically active agent (or agents) that is active in the body, including pro-drugs that convert to pharmaceutically active form after administration, which are different from the compound of Formula (I). The additional active agents also include free-acid, free-base and pharmaceutically acceptable salts of said additional active agents. Generally, any suitable additional active agent or agents, including chemotherapeutic agents or therapeutic antibodies, may be used in any combination with the compound of Formula (I) in a single dosage formulation (e.g., a fixed dose drug combination), or in one or more separate dosage formulations which allows for concurrent or sequential administration of the active agents (co-administration of the separate active agents) to subjects. In addition, the compounds of Formula (I) (or pharmaceutically acceptable salts thereof) can be administered in combination with radiation therapy, hormone therapy, surgery or immunotherapy.

The present application also provides methods for combination therapies in which the additional active agent is known to modulate other pathways, or other components of the same pathway, or even overlapping sets of target enzymes which are used in combination with a compound of Formula (I), or a pharmaceutically acceptable salt thereof. In one embodiment, such therapy includes, but is not limited to, the combination of one or more compounds of Formula (I) with chemotherapeutic agents, immunotherapeutic agents, hormonal and anti-hormonal agents, targeted therapy agents, and anti-angiogenesis agents, to provide a synergistic or additive therapeutic effect. In another embodiment, such therapy includes radiation treatment to provide a synergistic or additive therapeutic effect.

Examples of additional active agents (i.e., additional anti-cancer agents) include chemotherapeutic agents (e.g., cytotoxic agents), immunotherapeutic agents, hormonal and anti-hormonal agents, targeted therapy agents, and anti-angiogenesis agents. Many anti-cancer agents can be classified within one or more of these groups. While certain anti-cancer agents have been categorized within a specific group(s) or subgroup(s) herein, many of these agents can also be listed within one or more other group(s) or subgroup(s), as would be presently understood in the art. It is to be understood that the classification herein of a particular agent into a particular group is not intended to be limiting. Many anti-cancer agents are presently known in the art and can be used in combination with the compounds of the present disclosure.

Further, an agent can be an agonist, antagonist, allosteric modulator, toxin or, more generally, may act to inhibit or stimulate its target (e.g., receptor or enzyme activation or inhibition). For example, suitable for use are one or more agents (e.g., antibodies, antigen binding regions, or soluble receptors) that specifically bind and inhibit the activity of growth factors, such as antagonists of hepatocyte growth factor (HGF, also known as Scatter Factor), and antibodies or antigen binding regions that specifically bind its receptor "c-met".

In an embodiment, the additional anti-cancer agent is a chemotherapeutic agent, an immunotherapeutic agent, a hormonal agent, an anti-hormonal agent, a targeted therapy agent, or an anti-angiogenesis agent (or angiogenesis inhibitor). In an embodiment, the additional anti-cancer agent is selected from the group consisting of a chemotherapeutic agent, a mitotic inhibitor, a plant alkaloid, an alkylating agent, an anti-metabolite, a platinum analog, an enzyme, a topoisomerase inhibitor, a retinoid, an aziridine, an antibiotic, a hormonal agent, an anti-hormonal agent, an anti-estrogen, an anti-androgen, an anti-adrenal, an androgen, a targeted therapy agent, an immunotherapeutic agent, a biological response modifier, a cytokine inhibitor, a tumor vaccine, a monoclonal antibody, an immune checkpoint inhibitor, an anti-PD-1 agent, an anti-PD-L1 agent, a colony-stimulating factor, an immunomodulator, an immunomodulatory imide (IMiD), an anti-CTLA4 agent, an anti-LAGl agent, an anti-OX40 agent, a GITR agonist, a CAR-T cell, a BiTE, a signal transduction inhibitor, a growth factor inhibitor, a tyrosine kinase inhibitor, an EGFR inhibitor, a histone deacetylase (HDAC) inhibitor, a proteasome inhibitor, a cell-cycle inhibitor, an anti-angiogenesis agent, a matrix-metalloproteinase (MMP) inhibitor, a hepatocyte growth factor inhibitor, a TOR inhibitor, a KDR inhibitor, a VEGF inhibitor, a HIF-1α inhibitor a HIF-2α inhibitor, a fibroblast growth factor (FGF) inhibitor, a RAF inhibitor, a MEK inhibitor, an ERK inhibitor, a PI3K inhibitor, an AKT inhibitor, an MCL-1 inhibitor, a BCL-2 inhibitor, an SHP2 inhibitor, a HER-2 inhibitor, a BRAF-inhibitor, a gene expression modulator, an autophagy inhibitor, an apoptosis inducer, an antiproliferative agent, and a glycolysis inhibitor.

In one embodiment, the additional anti-cancer agent(s) is a chemotherapeutic agent. Non-limiting examples of chemotherapeutic agents include mitotic inhibitors and plant alkaloids, alkylating agents, anti-metabolites, platinum analogs, enzymes, topoisomerase inhibitors, retinoids, aziridines, and antibiotics.

Non-limiting examples of mitotic inhibitors and plant alkaloids include taxanes such as cabazitaxel, docetaxel, larotaxel, ortataxel, paclitaxel, and tesetaxel; demecolcine; epothilone; eribulin; etoposide (VP- 16); etoposide phosphate; navelbine; noscapine; teniposide; thaliblastine; vinblastine; vincristine; vindesine; vinflunine; and vinorelbine.

Non-limiting examples of alkylating agents include nitrogen mustards such as chlorambucil, chlornaphazine, cholophosphamide, cytophosphane, estramustine, ifosfamide, mannomustine, mechlorethamine, mechlorethamine oxide hydrochloride, melphalan, novembichin, phenesterine, prednimustine, tris(2-chloroethyl)amine, trofosfamide, and uracil mustard; alkyl sulfonates such as busulfan, improsulfan, and piposulfan; nitrosoureas such as carmustine, chlorozotocin, fotemustine, lomustine, nimustine, ranimustine, streptozotocin, and TA-07; ethylenimines and methylamelamines such as altretamine, thiotepa, triethylenemelamine, triethylenethiophosphaoramide, trietylenephosphoramide, and trimethylolomelamine; ambamustine; bendamustine; dacarbazine; etoglucid; irofulven; mafosfamide; mitobronitol; mitolactol; pipobroman; procarbazine; temozolomide; treosulfan; and triaziquone.

Non-limiting examples of anti-metabolites include folic acid analogues such as aminopterin, denopterin, edatrexate, methotrexate, pteropterin, raltitrexed, and trimetrexate; purine analogs such as 6-mercaptopurine, 6-thioguanine, fludarabine, forodesine, thiamiprine, and thioguanine; pyrimidine analogs such as 5-fluorouracil (5-FU), 6-azauridine, ancitabine, azacytidine, capecitabine, carmofur, cytarabine, decitabine, dideoxyuridine, doxifluridine, doxifluridine, enocitabine, floxuridine, galocitabine, gemcitabine, and sapacitabine; 3-aminopyridine-2-carboxaldehyde thiosemicarbazone; broxuridine; cladribine; cyclophosphamide; cytarabine; emitefur; hydroxyurea; mercaptopurine; nelarabine; pemetrexed; pentostatin; tegafur; and troxacitabine.

Non-limiting examples of platinum analogs include carboplatin, cisplatin, dicycloplatin, heptaplatin, lobaplatin, nedaplatin, oxaliplatin, satraplatin, and triplatin tetranitrate.

Non-limiting examples of enzymes include asparaginase and pegaspargase.

Non-limiting examples of topoisomerase inhibitors include acridine carboxamide, amonafide, amsacrine, belotecan, elliptinium acetate, exatecan, indolocarbazole, irinotecan, lurtotecan, mitoxantrone, razoxane, rubitecan, SN-38, sobuzoxane, and topotecan.

Non-limiting examples of retinoids include alitretinoin, bexarotene, fenretinide, isotretinoin, liarozole, RII retinamide, and tretinoin.

Non-limiting examples of aziridines include benzodopa, carboquone, meturedopa, and uredopa.

Non-limiting examples of antibiotics include intercalating antibiotics; anthracenediones; anthracycline antibiotics such as aclarubicin, amrubicin, daunomycin, daunorubicin, doxorubicin, epirubicin, idarubicin, menogaril, nogalamycin, pirarubicin, and valrubicin; 6-diazo-5-oxo- L-norleucine; aclacinomysins; actinomycin; authramycin; azaserine; bleomycins; cactinomycin; calicheamicin; carabicin; carminomycin; carzinophilin; chromomycins; dactinomycin; detorubicin; esorubicin; esperamicins; geldanamycin; marcellomycin; mitomycins; mitomycin C; mycophenolic acid; olivomycins; novantrone; peplomycin; porfiromycin; potfiromycin; puromycin; quelamycin; rebeccamycin; rodorubicin; streptonigrin; streptozocin; *tanespimycin;* tubercidin; ubenimex; zinostatin; zinostatin stimalamer; and zorubicin.

In one embodiment, the additional anti-cancer agent(s) is a hormonal and/or anti-hormonal agent (i.e., hormone therapy). Non-limiting examples of hormonal and anti-hormonal agents include anti-androgens such as abiraterone, apalutamide, bicalutamide, darolutamide, enzalutamide, flutamide, goserelin, leuprolide, and nilutamide; anti-estrogens such as 4- hydroxy tamoxifen, aromatase inhibiting 4(5)-imidazoles, EM-800, fosfestrol, fulvestrant, keoxifene, LY 117018, onapristone, raloxifene, tamoxifen, toremifene, and trioxifene; anti-adrenals such as aminoglutethimide, dexaminoglutethimide, mitotane, and trilostane; androgens such as calusterone, dromostanolone propionate, epitiostanol, mepitiostane, and testolactone; abarelix; anastrozole; cetrorelix; deslorelin; exemestane; fadrozole; finasteride; formestane; histrelin (RL 0903); human chorionic gonadotropin; lanreotide; LDI 200 (Milkhaus); letrozole; leuprorelin; mifepristone; nafarelin; nafoxidine; osaterone; prednisone; thyrotropin alfa; and triptorelin.

In one embodiment, the additional anti-cancer agent(s) is an immunotherapeutic agent (i.e., immunotherapy). Non-limiting examples of immunotherapeutic agents include biological response modifiers, cytokine inhibitors, tumor vaccines, monoclonal antibodies, immune checkpoint inhibitors, colony-stimulating factors, and immunomodulators.

Non-limiting examples of biological response modifiers, including cytokine inhibitors (cytokines) such as interferons and interleukins, include interferon alfa/interferon alpha such as interferon alfa-2, interferon alfa-2a, interferon alfa-2b, interferon alfa-nl, interferon alfa-n3, interferon alfacon-1, peginterferon alfa-2a, peginterferon alfa-2b, and leukocyte alpha interferon; interferon beta such as interferon beta-1a, and interferon beta-1b; interferon gamma such as natural interferon gamma-1a, and interferon gamma-1b; aldesleukin; interleukin-1 beta; interleukin-2; oprelvekin; sonermin; tasonermin; and virulizin.

Non-limiting examples of tumor vaccines include APC 8015, AVICINE, bladder cancer vaccine, cancer vaccine (Biomira), gastrin 17 immunogen, Maruyama vaccine, melanoma lysate vaccine, melanoma oncolysate vaccine (New York Medical College), melanoma vaccine (New York University), melanoma vaccine (Sloan Kettering Institute), TICE^{®} BCG (Bacillus Calmette-Guerin), and viral melanoma cell lysates vaccine (Royal Newcastle Hospital). Non-limiting examples of monoclonal antibodies include abagovomab, adecatumumab, aflibercept, alemtuzumab, blinatumomab, brentuximab vedotin, CA 125 MAb (Biomira), cancer MAb (Japan Pharmaceutical Development), daclizumab, daratumumab, denosumab, edrecolomab, gemtuzumab zogamicin, HER- 2 and Fc MAb (Medarex), ibritumomab tiuxetan, idiotypic 105AD7 MAb (CRC Technology), idiotypic CEA MAb (Trilex), ipilimumab, lintuzumab, LYM-1 -iodine 131 MAb (Techni clone), mitumomab, moxetumomab, ofatumumab, polymorphic epithelial mucin-yttrium 90 MAb (Antisoma), ranibizumab, rituximab, and trastuzumab.

Non-limiting examples of immune checkpoint inhibitors include anti-PD-1 agents or antibodies such as cemiplimab, nivolumab, and pembrolizumab; anti-PD-L1 agents or antibodies such as atezolizumab, avelumab, and durvalumab; anti-CTLA-4 agents or antibodies such as ipilumumab; anti-LAG1 agents; and anti-OX40 agents.

Non-limiting examples of colony-stimulating factors include darbepoetin alfa, epoetin alfa, epoetin beta, filgrastim, granulocyte macrophage colony stimulating factor, lenograstim, leridistim, mirimostim, molgramostim, nartograstim, pegfilgrastim, and sargramostim.

Non-limiting examples of additional immunotherapeutic agents include BiTEs, CAR-T cells, GITR agonists, imiquimod, immunomodulatory imides (IMiDs), mismatched double stranded RNA (Ampligen), resiquimod, SRL 172, and thymalfasin.

In one embodiment, the additional anti-cancer agent(s) is a targeted therapy agent (i.e., targeted therapy). Targeted therapy agents include, for example, monoclonal antibodies and small molecule drugs. Non-limiting examples of targeted therapy agents include signal transduction inhibitors, growth factor inhibitors, tyrosine kinase inhibitors, EGFR inhibitors, histone deacetylase (HDAC) inhibitors, proteasome inhibitors, cell-cycle inhibitors, angiogenesis inhibitors, matrix-metalloproteinase (MMP) inhibitors, hepatocyte growth factor inhibitors, TOR inhibitors, KDR inhibitors, VEGF inhibitors, fibroblast growth factors (FGF) inhibitors, MEK inhibitors, ERK inhibitors, PI3K inhibitors, AKT inhibitors, MCL-1 inhibitors, BCL-2 inhibitors, SHP2 inhibitors, HER-2 inhibitors, BRAF-inhibitors, gene expression modulators, autophagy inhibitors, apoptosis inducers, antiproliferative agents, and glycolysis inhibitors.

Non-limiting examples of signal transduction inhibitors include tyrosine kinase inhibitors, multiple-kinase inhibitors, anlotinib, avapritinib, axitinib, dasatinib, dovitinib, imatinib, lenvatinib, lonidamine, nilotinib, nintedanib, pazopanib, pegvisomant, ponatinib, vandetanib, and EGFR inhibitory agents.

Non-limiting examples of EGFR inhibitory agents include small molecule antagonists of EGFR such as afatinib, brigatinib, erlotinib, gefitinib, lapatinib, and osimertinib; and antibody-based EGFR inhibitors, including any anti-EGFR antibody or antibody fragment that can partially or completely block EGFR activation by its natural ligand. Antibody-based EGFR inhibitory agents may include, for example, those described in Modjtahedi, H., et al., 1993, Br. J. Cancer 67:247-253; Teramoto, T., et al., 1996, Cancer 77:639-645; Goldstein et al, 1995, Clin. Cancer Res. 1 : 1311-1318; Huang, S. M., et al., 1999, Cancer Res. 15:59(8): 1935-40; and Yang, X., et al., 1999, Cancer Res. 59: 1236-1243; monoclonal antibody Mab E7.6.3 (Yang, 1999 supra); Mab C225 (ATCC Accession No. HB-8508), or an antibody or antibody fragment having the binding specificity thereof; specific antisense nucleotide or siRNA; afatinib, cetuximab; matuzumab; necitumumab; nimotuzumab; panitumumab; and zalutumumab.

Non-limiting examples of histone deacetylase (HDAC) inhibitors include belinostat, panobinostat, romidepsin, and vorinostat.

Non-limiting examples of proteasome inhibitors include bortezomib, carfilzomib, ixazomib, marizomib (salinosporamide a), and oprozomib.

Non-limiting examples of cell-cycle inhibitors, including CDK inhibitors, include abemaciclib, alvocidib, palbociclib, and ribociclib.

In one embodiment, the additional anti-cancer agent(s) is an anti-angiogenic agent (or angiogenesis inhibitor) including, but not limited to, matrix-metalloproteinase (MMP) inhibitors; VEGF inhibitors; EGFR inhibitors; TOR inhibitors such as everolimus and temsirolimus; PDGFR kinase inhibitory agents such as crenolanib; HIF-1α inhibitors such as PX 478; HIF-2α inhibitors such as belzutifan and the HIF-2α inhibitors described in WO 2015/035223; fibroblast growth factor (FGF) or FGFR inhibitory agents such as B-FGF and RG 13577; hepatocyte growth factor inhibitors; KDR inhibitors; anti-Ang1 and anti-Ang2 agents; anti-Tie2 kinase inhibitory agents; Tek antagonists (US 2003/0162712; US 6,413,932); anti-TWEAK agents (US 6,727,225); ADAM distintegrin domain to antagonize the binding of integrin to its ligands (US 2002/0042368); anti-eph receptor and/or anti-ephrin antibodies or antigen binding regions (US 5,981,245; 5,728,813; 5,969,110; 6,596,852; 6,232,447; and 6,057,124); and anti-PDGF-BB antagonists as well as antibodies or antigen binding regions specifically binding to PDGF-BB ligands.

Non-limiting examples of matrix-metalloproteinase (MMP) inhibitors include MMP-2 (matrix-metalloproteinase 2) inhibitors, MMP-9 (matrix-metalloproteinase 9) inhibitors, prinomastat, RO 32-3555, and RS 13-0830. Examples of useful matrix metalloproteinase inhibitors are described, for example, in WO 96/33172, WO 96/27583, EP 1004578 , WO 98/07697, WO 98/03516, WO 98/34918, WO 98/34915, WO 98/33768, WO 98/30566, EP 0606046, EP 0931788, WO 90/05719, WO 99/52910, WO 99/52889, WO 99/29667, WO 1999/007675 , EP 1786785, EP 1181017, US 2009/0012085 , US 5,863,949, US 5,861,510, and EP 0780386. Preferred MMP-2 and MMP-9 inhibitors are those that have little or no activity inhibiting MMP-1. More preferred, are those that selectively inhibit MMP-2 and/or MMP-9 relative to the other matrix-metalloproteinases (i.e., MAP-1, MMP-3, MMP-4, MMP-5, MMP-6, MMP- 7, MMP- 8, MMP-10, MMP-11, MMP-12, and MMP-13).

Non-limiting examples of VEGF and VEGFR inhibitory agents include bevacizumab, cediranib, CEP 7055, CP 547632, KRN 633, orantinib, pazopanib, pegaptanib, pegaptanib octasodium, semaxanib, sorafenib, sunitinib, VEGF antagonist (Borean, Denmark), and VEGF-TRAP^{™}.

The additional anti-cancer agent(s) may also be another anti-angiogenic agent including, but not limited to, 2-methoxyestradiol, AE 941, alemtuzumab, alpha-D148 Mab (Amgen, US), alphastatin, anecortave acetate, angiocidin, angiogenesis inhibitors, (SUGEN, US), angiostatin, anti-Vn Mab (Crucell, Netherlands), atiprimod, axitinib, AZD 9935, BAY RES 2690 (Bayer, Germany, BC 1 (Genoa Institute of Cancer Research, Italy), beloranib, benefin (Lane Labs, US), cabozantinib, CDP 791 (Celltech Group, UK), chondroitinase AC, cilengitide, combretastatin A4 prodrug, CP 564959 (OSI, US), CV247, CYC 381 (Harvard University, US), E 7820, EHT 0101, endostatin, enzastaurin hydrochloride, ER-68203-00 (IVAX, US), fibrinogen-E fragment, Flk-1 (ImClone Systems, US), forms of FLT 1 (VEGFR 1), FR-111142, GCS-100, GW 2286 (GlaxoSmithKline, UK), IL-8, ilomastat, IM-862, irsogladine, KM-2550 (Kyowa Hakko, Japan), lenalidomide, lenvatinib, MAb alpha5beta3 integrin, second generation (Applied Molecular Evolution, USA and Medlmmune, US), MAb VEGF (Xenova, UK), marimastat, maspin (Sosei, Japan), metastatin, motuporamine C, M-PGA, ombrabulin, OXI4503, PI 88, platelet factor 4, PPI 2458, ramucirumab, rBPI 21 and BPI-derived antiangiogenic (XOMA, US), regorafenib, SC-236, SD-7784 (Pfizer, US), SDX 103 (University of California at San Diego, US), SG 292 (Telios, US), SU-0879 (Pfizer, US), TAN-1120, TBC-1635, tesevatinib, tetrathiomolybdate, thalidomide, thrombospondin 1 inhibitor, Tie-2 ligands (Regeneron, US), tissue factor pathway inhibitors (EntreMed, US), tumor necrosis factor-alpha inhibitors, tumstatin, TZ 93, urokinase plasminogen activator inhibitors, vadimezan, vandetanib, vasostatin, vatalanib, VE-cadherin-2 antagonists, xanthorrhizol, XL 784 (Exelixis, US), ziv-aflibercept, and ZD 6126.

In embodiments, the additional anti-cancer agent(s) is an additional active agent that disrupts or inhibits RAS-RAF-ERK or PI3K-AKT-TOR signaling pathways or is a PD-1 and/or PD-L1 antagonist. In embodiments, the additional anti-cancer agent(s) is a RAF inhibitor, EGFR inhibitor, MEK inhibitor, ERK inhibitor, PI3K inhibitor, AKT inhibitor, TOR inhibitor, MCL-1 inhibitor, BCL-2 inhibitor, SHP2 inhibitor, proteasome inhibitor, or immune therapy, including monoclonal antibodies, immunomodulatory imides (IMiDs), anti-PD-1, anti-PDL-1, anti-CTLA4, anti-LAG1, and anti-OX40 agents, GITR agonists, CAR-T cells, and BiTEs. Non-limiting examples of RAF inhibitors include dabrafenib, encorafenib, regorafenib, sorafenib, and vemurafenib.

Non-limiting examples of MEK inhibitors include binimetinib, CI-1040, cobimetinib, PD318088, PD325901, PD334581, PD98059, refametinib, selumetinib, and trametinib.

Non-limiting examples of ERK inhibitors include LY3214996, LTT462, MK-8353, SCH772984, ravoxertinib, ulixertinib, and an ERKi as described in WO 2017/068412.

Non-limiting examples of PI3K inhibitors include 17-hydroxywortmannin analogs (e.g., WO 06/044453); AEZS-136; alpelisib; AS-252424; buparlisib; CAL263; copanlisib; CUDC-907; dactolisib (WO 06/122806); demethoxyviridin; duvelisib; GNE-477; GSK1059615; IC87114; idelalisib; INK1117; LY294002; Palomid 529; paxalisib; perifosine; PI-103; PI-103 hydrochloride; pictilisib (*e.g*., WO 09/036,082; WO 09/055,730); PIK 90; PWT33597; SF1126; sonolisib; TGI 00-115; TGX-221; XL147; XL-765; wortmannin; and ZSTK474.

Non-limiting examples of AKT inhibitors include Akt-1-1 (inhibits Aktl) (Barnett et al. (2005) Biochem. J., 385 (Pt. 2), 399-408); Akt-1-1,2 (Barnett et al. (2005) Biochem. J. 385 (Pt. 2), 399-408); API-59CJ-Ome (e.g., Jin et al. (2004) Br. J. Cancer 91, 1808-12); 1-H-imidazo[4,5-c]pyridinyl compounds (*e.g.,* WO05011700); indole-3-carbinol and derivatives thereof (*e.g.,* U.S. Patent No. 6,656,963; Sarkar and Li (2004) J Nutr. 134(12 Suppl), 3493S-3498S); perifosine, Dasmahapatra et al. (2004) Clin. Cancer Res. 10(15), 5242-52, 2004); phosphatidylinositol ether lipid analogues (*e.g.,* Gills and Dennis (2004) Expert. Opin. Investig. Drugs 13, 787-97); triciribine (Yang et al. (2004) Cancer Res. 64, 4394-9); imidazooxazone compounds including trans-3-amino-1-methyl-3-[4-(3-phenyl-5H-imidazo[1,2-c]pyrido[3,4-e][1,3]oxazin-2-yl)phenyl]-cyclobutanol hydrochloride (WO 2012/137870) ; afuresertib;; capivasertib; MK2206; and patasertib.

Non-limiting examples of TOR inhibitors include deforolimus; ATP-competitive TORC1/TORC2 inhibitors, including PI-103, PP242, PP30, and Torin 1; TOR inhibitors in FKBP12 enhancer, rapamycins and derivatives thereof, including temsirolimus, everolimus, WO 9409010; rapalogs, *e.g.* as disclosed in WO 98/02441 and WO 01/14387, *e.g.* AP23573, AP23464, or AP23841; 40-(2-hydroxyethyl)rapamycin, 40-[3-hydroxy(hydroxymethyl)methylpropanoate]-rapamycin ; 40-epi-(tetrazolyl)-rapamycin (also called ABT578); 32-deoxorapamycin; 16-pentynyloxy-32(S)-dihydrorapanycin, and other derivatives disclosed in WO 05/005434; derivatives disclosed in US 5,258,389, WO 94/090101, WO 92/05179, US 5,118,677, US 5,118,678, US 5,100,883, US 5,151,413, US 5,120,842, WO 93/111130, WO 94/02136, WO 94/02485, WO 95/14023, WO 94/02136, WO 95/16691, WO 96/41807, WO 96/41807 and US 5,256,790; and phosphorus-containing rapamycin derivatives (*e.g.,* WO 05/016252).

Non-limiting examples of MCL-1 inhibitors include AMG-176, MIK665, and S63845.

Non-limiting examples of SHP2 inhibitors include SHP2 inhibitors described in WO 2019/167000 and WO 2020/022323.

Additional non-limiting examples of anti-cancer agents that are suitable for use include 2-ethylhydrazide, 2,2',2"-trichlorotriethylamine, ABVD, aceglatone, acemannan, aldophosphamide glycoside, alpharadin, amifostine, aminolevulinic acid, anagrelide, ANCER, ancestim, anti-CD22 immunotoxins, antitumorigenic herbs, apaziquone, arglabin, arsenic trioxide, azathioprine, BAM 002 (Novelos), bcl-2 (Genta), bestrabucil, biricodar, bisantrene, bromocriptine, brostallicin, bryostatin, buthionine sulfoximine, calyculin, cell-cycle nonspecific antineoplastic agents, celmoleukin, clodronate, clotrimazole, cytarabine ocfosfate, DA 3030 (Dong-A), defofamine, denileukin diftitox, dexrazoxane, diaziquone, dichloroacetic acid, dilazep, discodermolide, docosanol, doxercalciferol, edelfosine, eflomithine, EL532 (Elan), elfomithine, elsamitrucin, eniluracil, etanidazole, exisulind, ferruginol, folic acid replenisher such as frolinic acid, gacytosine, gallium nitrate, gimeracil/oteracil/tegafur combination (S-1), glycopine, histamine dihydrochloride, HIT diclofenac, HLA-B7 gene therapy (Vical), human fetal alpha fetoprotein, ibandronate, ibandronic acid, ICE chemotherapy regimen, imexon, iobenguane, IT-101 (CRLX101), laniquidar, LC 9018 (Yakult), leflunomide, lentinan, levamisole + fluorouracil, lovastatin, lucanthone, masoprocol, melarsoprol, metoclopramide, miltefosine, miproxifene, mitoguazone, mitozolomide, mopidamol, motexafin gadolinium, MX6 (Galderma), naloxone + pentazocine, nitracrine, nolatrexed, NSC 631570 octreotide (Ukrain), olaparib, P-30 protein, PAC-1, palifermin, pamidronate, pamidronic acid, pentosan polysulfate sodium, phenamet, picibanil, pixantrone, platinum, podophyllinic acid, porfimer sodium, PSK (Polysaccharide-K), rabbit antithymocyte polyclonal antibody, rasburiembodiment, retinoic acid, rhenium Re 186 etidronate, romurtide, samarium (153 Sm) lexidronam, sizofiran, sodium phenylacetate, sparfosic acid, spirogermanium, strontium-89 chloride, suramin, swainsonine, talaporfin, tariquidar, tazarotene, tegafur-uracil, temoporfin, tenuazonic acid, tetrachlorodecaoxide, thrombopoietin, tin ethyl etiopurpurin, tirapazamine, TLC ELL-12, tositumomab-iodine 131, trifluridine and tipiracil combination, troponin I (Harvard University, US), urethan, valspodar, verteporfin, zoledronic acid, and zosuquidar.

The present disclosure further provides a method for using the compounds of Formula (I) or pharmaceutical compositions provided herein, in combination with radiation therapy to treat cancer. Techniques for administering radiation therapy are known in the art, and these techniques can be used in the combination therapy described herein. The administration of the compound of Formula (I) in this combination therapy can be determined as described herein.

Radiation therapy can be administered through one of several methods, or a combination of methods, including, without limitation, external-beam therapy, internal radiation therapy, implant radiation, stereotactic radiosurgery, systemic radiation therapy, radiotherapy and permanent or temporary interstitial brachy therapy. The term "brachytherapy," as used herein, refers to radiation therapy delivered by a spatially confined radioactive material inserted into the body at or near a tumor or other proliferative tissue disease site. The term is intended, without limitation, to include exposure to radioactive isotopes (*e.g.,* At-211, I-131, I -125, Y-90, Re-186, Re-188, Sm- 153, Bi-212, P-32, and radioactive isotopes of Lu). Suitable radiation sources for use as a cell conditioner of the present disclosure include both solids and liquids. By way of non-limiting example, the radiation source can be a radionuclide, such as I-125, I-131, Yb-169, Ir-192 as a solid source, I-125 as a solid source, or other radionuclides that emit photons, beta particles, gamma radiation, or other therapeutic rays. The radioactive material can also be a fluid made from any solution of radionuclide(s), e.g., a solution of I-125 or I-131, or a radioactive fluid can be produced using a slurry of a suitable fluid containing small particles of solid radionuclides, such as Au-198, Y-90. Moreover, the radionuclide(s) can be embodied in a gel or radioactive microspheres.

The present disclosure also provides methods for combination therapies in which the additional active agent is known to modulate other pathways, or other components of the same pathway, or even overlapping sets of target enzymes which are used in combination with a compound of Formula (I), or a pharmaceutically acceptable salt thereof. In one embodiment, such therapy includes, but is not limited to, the combination of one or more compounds of Formula (I) with chemotherapeutic agents, immunotherapeutic agents, hormonal therapy agents, therapeutic antibodies, targeted therapy agents, and radiation treatment, to provide a synergistic or additive therapeutic effect.

The compounds of the disclosure can be used in combination with the agents disclosed herein or other suitable agents, depending on the condition being treated. Hence, in some embodiments the one or more compounds of the disclosure will be co-administered with other agents as described above. When used in combination therapy, the compounds described herein are administered with the second agent simultaneously or separately. This administration in combination can include simultaneous administration of the two agents in the same dosage form, simultaneous administration in separate dosage forms, and separate administration. That is, a compound of Formula (I) and any of the agents described above can be formulated together in the same dosage form and administered simultaneously. Alternatively, a compound of Formula (I) and any of the agents described above can be simultaneously administered, wherein both the agents are present in separate formulations. In another alternative, a compound of Formula (I) can be administered just followed by and any of the agents described above, or vice versa. In some embodiments of the separate administration protocol, a compound of Formula (I) and any of the agents described above are administered a few minutes apart, or a few hours apart, or a few days apart.

As one aspect of the present disclosure contemplates the treatment of the disease/conditions with a combination of pharmaceutically active compounds that may be administered separately, the disclosure further relates to combining separate pharmaceutical compositions in kit form. The kit comprises two separate pharmaceutical compositions: a compound of Formula (I), and a second pharmaceutical compound. The kit comprises a container for containing the separate compositions such as a divided bottle or a divided foil packet. Additional examples of containers include syringes, boxes, and bags. In some embodiments, the kit comprises directions for the use of the separate components. The kit form is particularly advantageous when the separate components are preferably administered in different dosage forms (*e.g.,* oral and parenteral), are administered at different dosage intervals, or when titration of the individual components of the combination is desired by the prescribing health care professional.

The present disclosure also provides for the compound of Formula (I), or the pharmaceutically acceptable salt thereof, for use in therapy, or use of the compound of Formula (I), or the pharmaceutically acceptable salt thereof, in therapy. The present disclosure also provides for the compound of Formula (I), or the pharmaceutically acceptable salt thereof, for use in treating cancer, or use of a compound of Formula (I), or the pharmaceutically acceptable salt thereof, for treating cancer. The present disclosure also provides for the compound of Formula (I), or the pharmaceutically acceptable salt thereof, for the preparation of a medicament for the treatment of cancer, or use of the compound of Formula (I), or the pharmaceutically acceptable salt thereof, for the preparation of a medicament for the treatment of cancer. The present disclosure also provides for the compound of Formula (I), or the pharmaceutically acceptable salt thereof, and an additional anti-cancer agent, for use in the treatment of cancer, or use of the compound of Formula (I), or the pharmaceutically acceptable salt thereof, and the additional anti-cancer agent for treating cancer. The disclosure also provides the compound of Formula (I), or the pharmaceutically acceptable salt thereof, and an additional anti-cancer agent, for the preparation of a medicament for the treatment of cancer, or use of the compound of Formula (I), or the pharmaceutically acceptable salt thereof, and the additional anti-cancer agent, for the preparation of a medicament for the treatment of cancer. The present disclosure also provides for a pharmaceutical composition comprising the compound of Formula (I), or the pharmaceutically acceptable salt thereof, for use in the treatment of cancer, or use of the pharmaceutical composition comprising the compound of Formula (I), or the pharmaceutically acceptable salt thereof, for treating cancer. The present disclosure also provides for a pharmaceutical composition comprising the compound of Formula (I), or the pharmaceutically acceptable salt thereof, and an additional anti-cancer agent, for use in the treatment of cancer, or use of the pharmaceutical composition comprising the compound of Formula (I), or the pharmaceutically acceptable salt thereof, and the additional anti-cancer agent, for treating cancer.

### Methods of Preparing the Compounds of the Disclosure

Several methods for preparing the compounds of this disclosure are described in the following Schemes and Examples. Starting materials and intermediates are purchased, made from known procedures, or as otherwise illustrated. Some frequently applied routes to the compounds of Formula (I) are also described by the Schemes as follows. In some cases, the order of carrying out the steps of reaction schemes may be varied to facilitate the reaction or to avoid unwanted reaction products. The "R" groups and other variables in the Schemes correspond to the variables defined in Formula (I) at the same positions on the structures.

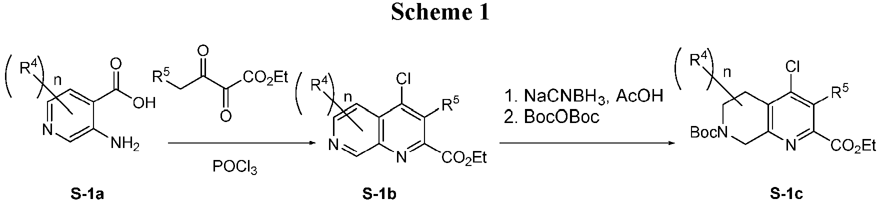

**Scheme 1** provides a general approach to assembling intermediates of type **S-1c.** Reaction of **S-1a** with an alpha-ketoester in the presence of phosphoryl chloride provides **S-1b.** Reduction of **S-1b** with subsequent Boc protection provides **S-1c.**

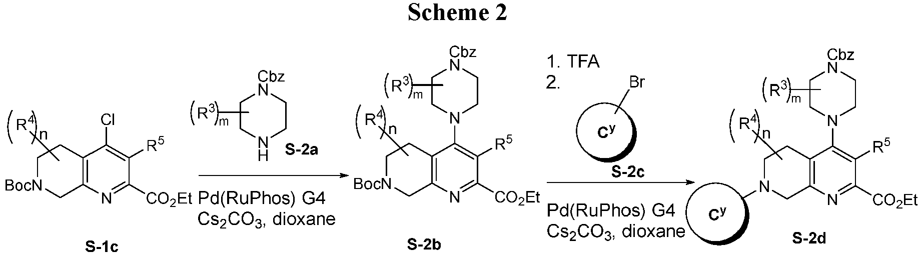

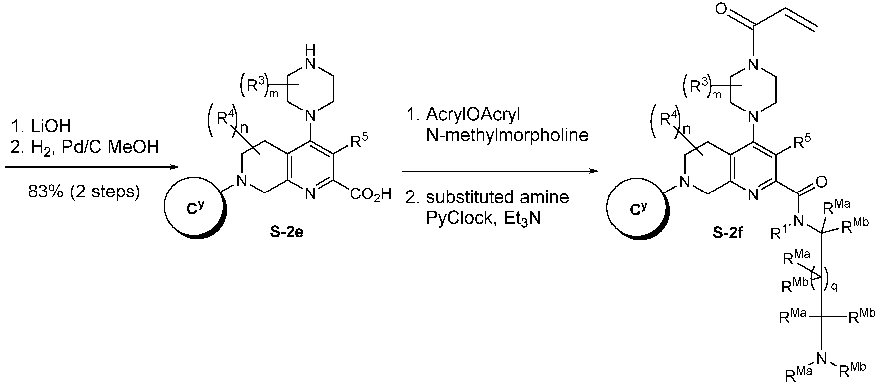

**Scheme** 2 illustrates a general approach for preparing compounds of Formula (I), wherein Y is N. Reaction of **S-1c** by C-N coupling with Cbz-protected piperazine **S-2a** affords **S-2b.** Boc-deprotection of **S-2b** with trifluoroacetic acid and subsequent C-N coupling with aryl halide **S-2c** provides **S-2d.** Ester hydrolysis of **S-2d** with subsequent Cbz-deprotection provides **S-2e.** Acrylation of **S-2e** and subsequent amide bond formation with a substituted amine provides **S-2f,** compounds of Formula (I), wherein Y is N.

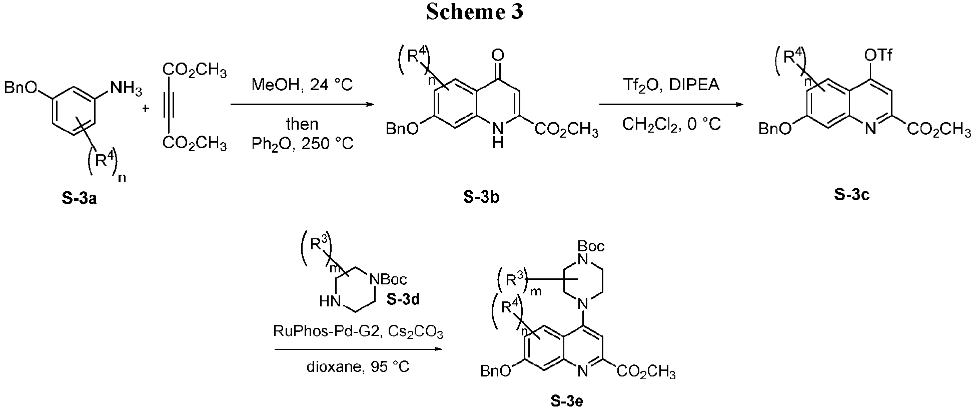

**Scheme 3** provides a general approach to assembling intermediates of type **S-3e.** Reaction of **S-3a** with a dialkyl acetylenedicarboxylate affords **S-3b.** Triflation of **S-3b** provides **S-3c.** C-N coupling of **S-3c** with a Boc-protected piperazine **S-3d** affords **S-3e.**

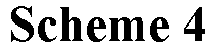

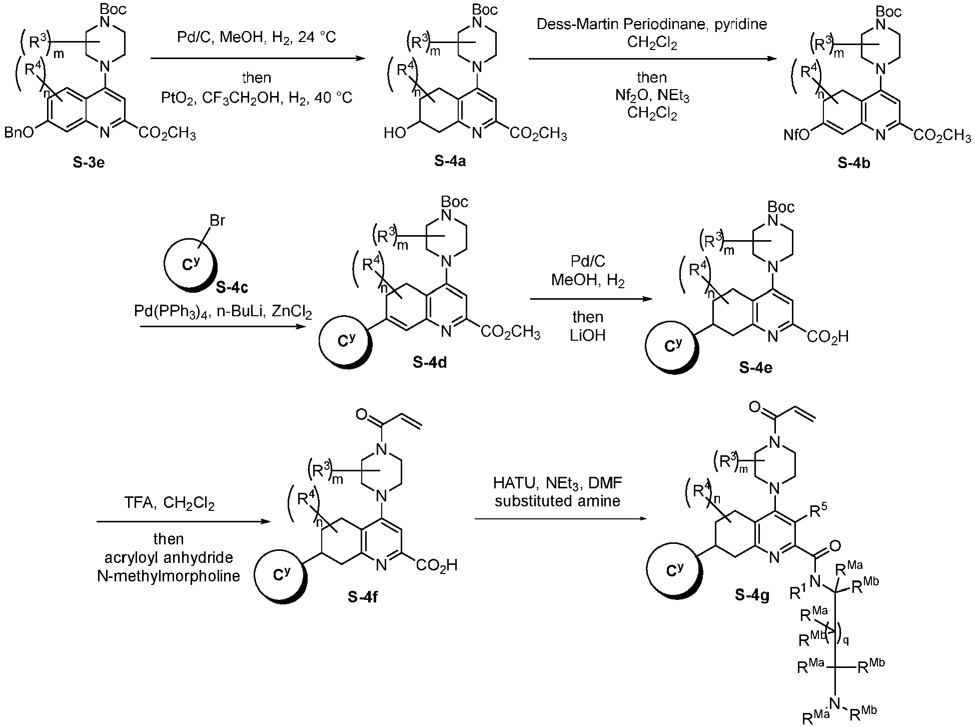

**Scheme 4** illustrates a general approach for preparing compounds of Formula (I), wherein Y is C(H). Reduction of **S-3e** provides **S-4a.** Oxidation of **S-4a** with subsequent nonaflation provides **S-4b.** Negishi coupling of nonaflate **S-4b** with arylzinc formed by lithiation and zincation of aryl bromide **S-4c** provides **S-4d.** Reduction of **S-4d** with subsequent ester hydrolysis affords **S-4e.** Boc-deprotection of **S-4e** with subsequent acrylation affords **S-4f.** Amide coupling of **S-4f** with a substituted amine affords **S-4g,** compounds of Formula (I), wherein Y is C(H).

Throughout the synthetic schemes and examples, abbreviations and acronyms may be used with the following meanings unless otherwise indicated:
Anhydr. = Anhydrous; Aq. = aqueous; atm = atmosphere; b.p. = boiling point; Bodipy-GDP = mixture of ((2R,3S,4R,5R)-5-(2-amino-6-oxo-1,6-dihydro-9H-purin-9-yl)-3-(((2-(3-(5,5-difluoro-7,9-dimethyl-5H-414,514-dipyrrolo[1,2-c:2',1'-f][1,3,2]diazaborinin-3-yl)propanamido)ethyl)carbamoyl)oxy)-4-hydroxytetrahydrofuran-2-yl)methyl hydrogen diphosphate and ((2R,3R,4R,5R)-5-(2-amino-6-oxo-1,6-dihydro-9H-purin-9-yl)-4-(((2-(3-(5,5-difluoro-7,9-dimethyl-5H-414,514-dipyrrolo[1,2-c:2',1'-f][1,3,2]diazaborinin-3-yl)propanamido)ethyl)carbamoyl)oxy)-3-hydroxytetrahydrofuran-2-yl)methyl hydrogen diphosphate (Invitrogen^{™}, catalog number G22360); br s = broad singlet; Bu = butyl; *t*-Bu = *tert*-butyl; BuLi = butyllithium; *t*-BuOH, *tert*-BuOH *= tert*-butanol; tBuOK = potassium *tert-*butoxide; Cbz = carbobenzyloxy; CDCl₃ = deuterated chloroform; CD₃OD = Tetradeuteromethanol; CELITE = diatomaceous earth; CF₃ = trifluoromethyl; cGMP = cyclic guanosine monophosphate; conc, conc. = concentrated, concentrate, concentrates; DCM = dichloromethane; 1,2-DCE, DCE = 1,2-dichloroethane; DETA-NO = Diethylenetriamine/nitric oxide adduct; DMA, DMAC = *N*,*N*-dimethylacetamide; DMF = *N*,*N-*dimethylformamide; DMSO = dimethylsulfoxide; dppf = 1,1'-bis(diphenylphosphino)ferrocene; DTT = dithiothreitol; EDTA = ethylenediaminetetraacetic acid; equiv, eq. = equivalent(s); Et = ethyl; Et₃N = triethylamine; EtOAc = ethyl acetate; EtOH = ethanol; FLORISIL = magnesium silicate; GTP = guanosine triphosphate; h, hr = hour; HATU = 1-[bis(dimethylamino)methylene]-1H-1,2,3-triazolo[4,5-b]pyridinium 3-oxide hexafluorophosphate; HEPES = 4-(2-hydroxyethyl)-1-piperazineethanesulfonic acid; HPLC = High pressure liquid chromatography; Int. = intermediate; *i*Pr = isopropyl; IPA, IP = inflection points; *i*-PrOH = Isopropanol; LCMS, LC/MS = liquid chromatography-mass spectrometry; LDA = lithium diisopropylamide; LiHMDS, LHMDS = lithium bis(trimethylsilyl)amide; min, min. = minute; M = Molar; Me = methyl; MeCN, ACN = acetonitrile; MeI = methyl iodide; MeOH = methanol; mp, m.p. = melting point; mpk = milligrams per kilogram; N = Normal; N₂ = nitrogen; NaOMe = sodium methoxide; NCS = *N*-chloro succinimide; NBS = *N*-bromo succinimide; NaHMDS = sodium bis(trimethylsilyl)amide; Nf = nonafluorobutanesulfonyl; NMR = nuclear magnetic resonance; N.D. = not determined; NIS = *N*-iodo succinimide; PDA = photodiode array; Pd₂(dba)₃ = tris(dibenzylideneacetone)dipalladium (0); Ph = phenyl; Pr = propyl; psig = pounds per square inch gauge; PTLC, prep TLC = preparative thin layer chromatography; PyClock = ((6-chloro-1H-benzo[d][1,2,3]triazol-1-yl)oxy)tri(pyrrolidin-1-yl)phosphonium hexafluorophosphate (V); rac = racemic; rt = retention time; RP-HPLC = reverse phase HPLC; RT = room temperature; sat., sat'd = saturated; SFC = supercritical fluid chromatography; SOS = Son of Sevenless; TFA = trifluoroacetic acid; TFAA = trifluoroacetic anhydride; TLC = thin layer chromatography; THF = tetrahydrofuran; TMS = trimethylsilyl; TWEEN = Polyoxyethylene (20) sorbitan monolaurate; VCD = vibrational circular dichroism; v, v/v = volume, volume to volume; w, w/w = weight, weight to weight.

The following are representative procedures for the preparation of intermediates used to prepare the final products described in the examples that follow thereafter. These examples are provided for the purpose of further illustration only and are not intended to be limitations on thedisclosure. Any intermediates described below may be referred to herein by their number preceded by "**I**-."

### EXAMPLES

### General Methods

The compounds described herein can be prepared according to the procedures of the above schemes, using appropriate materials and are further exemplified by the following specific examples. The compounds illustrated in the examples are not, however, to be construed as forming the only genus that is considered as the disclosure. The examples further illustrate details for the preparation of the compounds of the present disclosure. Those skilled in the art will readily understand that known variations of the conditions and processes of the following preparative procedures can be used to prepare these compounds.

Concentration refers to the removal of the volatile components at reduced pressure (*e.g*., by rotary evaporation) unless otherwise noted. All temperatures are in degrees Celsius unless otherwise noted. Mass spectra (MS) were measured by electrospray ion-mass spectroscopy (ESI) in positive ion detection mode and m/z refers to the [M+H]⁺ ion unless otherwise noted. ¹H NMR spectra were recorded at 400-500 MHz at ambient temperature unless otherwise noted. Diagnostic signals refer to signals that are characteristic for a given compound, where such signals are a subset of all signals in an NMR spectrum. RP-HPLC refers to reverse-phase HPLC on C18-functionalized preparative or semi-preparative columns with gradient elution using acetonitrile and water modified with trifluoroacetic acid or ammonium hydroxide as eluents and fractions were lyophilized or concentrated by rotary evaporation unless otherwise noted. Purification by column chromatography on silica gel was accomplished using a flash chromatography system (*e.g*., ISCO or Biotage) and commercial pre-packed silica gel columns with elution using the stated solvent systems. Compounds described herein were synthesized as the racemates unless otherwise noted in the experimental procedures and compound tables.

### Intermediate 1

### Preparation of ethyl 4-(4-((benzyloxy)carbonyl)piperazin-1-yl)-5,6,7,8-tetrahydro-1,7-naphthyridine-2-carboxylate (Int-1)

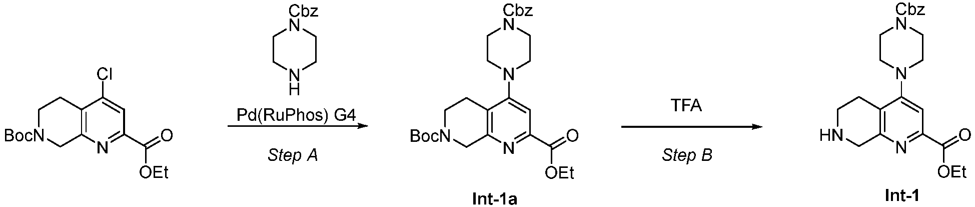

### Step A - Synthesis of 7-(tert-butyl) 2-ethyl 4-(4-((benzyloxy)carbonyl)piperazin-1-yl)-5,8-dihydro-1, 7-naphthyridine-2,7(6H)-dicarboxylate (Int-1a)

Into a 500 mL round-bottomed flask was added 7-(*tert*-butyl) 2-ethyl 4-chloro-5,8-dihydro-1,7-naphthyridine-2,7(6H)-dicarboxylate (8.86 g, 26.0 mmol), methanesulfonato(2-dicyclohexylphosphino-2',6'-di-*i*-propoxy-1,1'-biphenyl)(2'-methylamino-1,1'-biphenyl-2-yl)palladium(II) (2.21 g, 2.60 mmol), cesium carbonate (25.4 g, 78 mmol), and a teflon-coated magnetic stirbar. Under an inert atmosphere of nitrogen, the flask was charged with anhydrous 1,4-dioxane (130 mL), and benzyl piperazine-1-carboxylate (6.02 mL, 31.2 mmol). The mixture was degassed by subsurface sparging with nitrogen for 5 minutes, and the mixture was then was stirred under 1 atm of nitrogen at 100 °C for 2.5 hr, then at 90 °C for 16 hr. The mixture was then filtered through CELITE, which was rinsed with dichloromethane. The combined filtrate was concentrated to a residue, which was purified by column chromatography on silica gel (20 to 60% ethyl acetate in hexanes) to afford 7-(tert-butyl) 2-ethyl 4-(4-((benzyloxy)carbonyl)piperazin-1-yl)-5,8-dihydro-1,7-naphthyridine-2,7(6H)-dicarboxylate (**Int-1a**). ¹H NMR (500 MHz, DMSO-*d*₆) δ 7.42 (s, 1H), 7.38 (m, 4H), 7.34 (m, 1H), 5.11 (s, 2H), 4.54 (s, 2H), 4.32 (q, *J =* 7.1 Hz, 2H), 3.54 (m, 6H), 3.03 (m, 4H), 2.77 (t, *J =* 5.4 Hz, 2H), 1.45 (s, 9H), 1.31 (t, *J* = 7.1 Hz, 3H). Mass Calc'd: 524.3, found 525.3 (M+H)⁺.

### Step B - Synthesis of ethyl 4-(4-((benzyloxy)carbonyl)piperazin-1-yl)-5, 6, 7, 8-tetrahydro-1, 7-naphthyridine-2-carboxylate (Int-1)

Into a 20 mL glass vial was added 7-(tert-butyl) 2-ethyl 4-(4-((benzyloxy)carbonyl)piperazin-1-yl)-5,8-dihydro-1,7-naphthyridine-2,7(6H)-dicarboxylate (**Int-1a**) (1.098 g, 2.093 mmol), and dichloromethane (4.2 mL). After mixing, to the resulting solution was added trifluoroacetic acid (4.2 mL, 54.5 mmol). The resulting solution was allowed to stand at room temperature for 0.5 hr, and was then combined with a solution generated analogously on smaller scale from 53.1 mg of **Int-1a,** and the combined solution was then concentrated. The residue was dissolved in ethyl acetate (20 mL), and the resulting solution was washed with 2M sodium carbonate in water (20 mL), leading to a triphasic mixture. The top layer was separated from the bottom two layers, which were diluted with dichloromethane (30 mL), affording two layers which were separated. The aqueous layer was extracted with dichloromethane (10 mL), the combined dichloromethane layers were washed with brine (20 mL) and dried over anhydrous sodium sulfate and filtered. The resulting filtrate was combined with the filtrate generated by drying the top layer from the above initial triphasic mixture over anhydrous sodium sulfate and filtering. The resulting solution was concentrated to a residue, which was extracted with dichloromethane, and the extract was filtered and concentrated to a yellow foam, which was dissolved in acetonitrile and concentrated to afford ethyl 4-(4-((benzyloxy)carbonyl)piperazin-1-yl)-5,6,7,8-tetrahydro-1,7-naphthyridine-2-carboxylate (**Int-1**), which was used without further purification. Mass Calc'd: 424.2, found 425.2 (M+H)⁺.

### Intermediate 2

### Preparation of ethyl (,S)-4-(4-((benzyloxy)carbonyl)-3-(cyanomethyl)piperazin-1-yl)-5,6,7,8-tetrahydro-1,7-naphthyridine-2-carboxylate (Int-2)

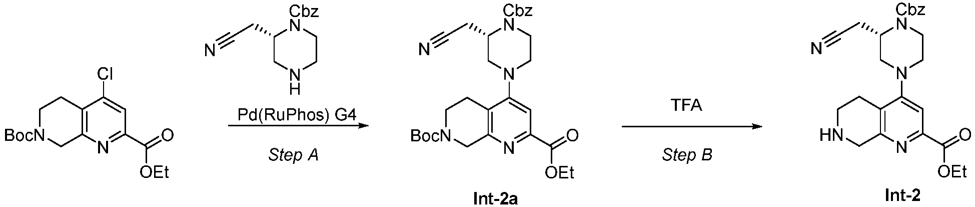

### Step A - Synthesis of 7-(tert-butyl) 2-ethyl (S)-4-(4-((benzyloxy)carbonyl)-3-(cyanomethyl)piperazin-1-yl)-5, 8-dihydro-1, 7-naphthyridine-2, 7(6H)-dicarboxylate (Int-2a)

A mixture of 7-(*tert*-butyl) 2-ethyl 4-chloro-5,8-dihydro-1,7-naphthyridine-2,7(6H)-dicarboxylate (17.6 g, 51.6 mmol), benzyl (*S*)-2-(cyanomethyl)piperazine-1-carboxylate (14.7 g, 56.8 mmol) and cesium carbonate (33.7 g, 103 mmol) in anhydrous 1,4-dioxane (50.0 mL) was degassed by subsurface sparging with nitrogen for 5 min. Then methanesulfonato(2-dicyclohexylphosphino-2',6'-di-i-propoxy-1,1'-biphenyl)(2'-methylamino-1,1'-biphenyl-2-yl)palladium(II) (4.39 g, 5.16 mmol) was added, and the mixture was stirred under nitrogen at 90 °C for 16 hr. The mixture was then filtered through a pad of FLORISIL over CELITE, rinsing with ethyl acetate. The combined filtrate was concentrated to a residue, which was purified by column chromatography on silica gel (0 to 100% ethyl acetate in hexane) to afford 7-(tert-butyl) 2-ethyl (*S*)-4-(4-((benzyloxy)carbonyl)-3-(cyanomethyl)piperazin-1-yl)-5,8-dihydro-1,7-naphthyridine-2,7(6H)-dicarboxylate (**Int-2a**). δ ¹H NMR (500 MHz, DMSO-*d*₆) δ 7.45 - 7.37 (m, 5H), 7.37 - 7.32 (m, 1H), 5.20 - 5.07 (m, 2H), 4.68 - 4.44 (m, 2H), 4.33 (q, J = 7.1 Hz, 2H), 3.70 - 3.60 (m, 1H), 3.48 - 3.34 (m, 2H), 3.32 (s, 3H), 3.32 - 3.15 (m, 2H), 3.08 - 2.93 (m, 2H), 2.93 - 2.84 (m, 1H), 2.84 - 2.66 (m, 2H), 1.46 (s, 9H), 1.32 (t, J = 7.1 Hz, 3H). Mass Calc'd: 563.3, found 564.3 (M+H)⁺.

### Step B - Synthesis of ethyl (S)-4-(4-((benzyloxy)carbonyl)-3-(cyanomethyl)piperazin-1-yl)-5,6,7,8-tetrahydro-1, 7-naphthyridine-2-carboxylate (Int-2)

To a solution of 7-(tert-butyl) 2-ethyl (S)-4-(4-((benzyloxy)carbonyl)-3-(cyanomethyl)piperazin-1-yl)-5,8-dihydro-1,7-naphthyridine-2,7(6H)-dicarboxylate (**Int-2a**) (7.65 g, 13.57 mmol) in dichloromethane (80 mL) was added trifluoroacetic acid (20 mL). The mixture was aged at room temperature for 20 h, and then was concentrated. The residue was dissolved in ethyl acetate, and washed with saturated aqueous sodium carbonate. The layers were separated, and the aqueous layer was extracted with ethyl acetate. The combined organic layers were filtered through anhydrous sodium sulfate, and the filtrate was concentrated to a residue, which was purified by column chromatography on silica gel (0 to 30% methanol in dichloromethane) to afford ethyl (*S*)-4-(4-((benzyloxy)carbonyl)-3-(cyanomethyl)piperazin-1-yl)-5,6,7,8-tetrahydro-1,7-naphthyridine-2-carboxylate (**Int-2**). ¹H NMR (500 MHz, DMSO-*d*₆) δ 7.54 - 7.26 (m, 6H), 5.76 (s, 1H), 5.25 - 4.96 (m, 2H), 4.65 (s, 1H), 4.33 (q, *J =* 7.1 Hz, 2H), 4.15 - 4.05 (m, 2H), 4.05 - 3.97 (m, 1H), 3.54 - 3.13 (m, 5H), 3.13 - 2.68 (m, 6H), 1.31 (t, *J* = 7.1 Hz, 3H). Mass Calc'd: 463.2, found 464.2 (M+H)⁺.

### Example 1

### Preparation of 4-(4-acryloylpiperazin-1-yl)-7-(naphthalen-1-yl)-N-(2-(pyrrolidin-1-yl)ethyl)-5,6,7,8-tetrahydro-1,7-naphthyridine-2-carboxamide (Example 1)

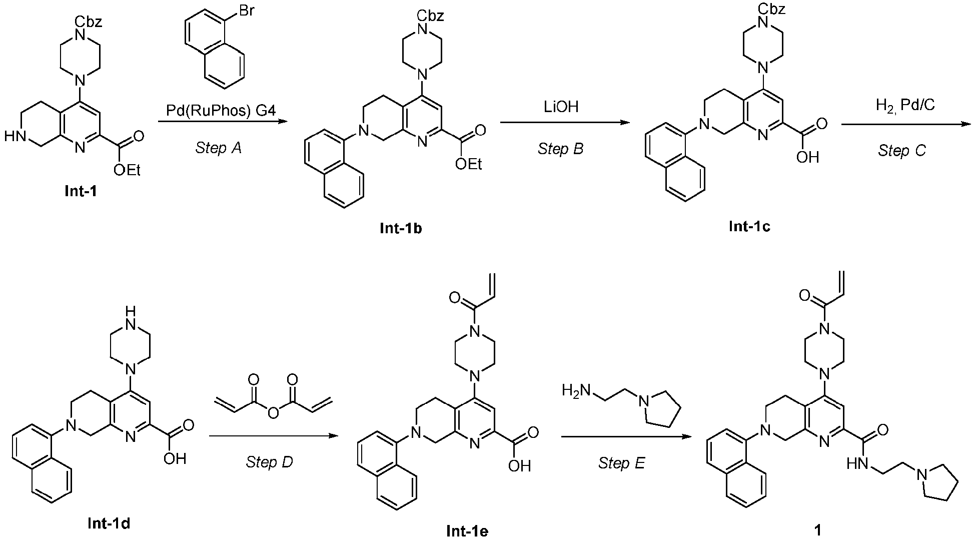

### Step A - Synthesis of ethyl 4-(4-((benzyloxy)carbonyl)piperazin-1-yl)-7-(naphthalen-1-yl)-5,6,7,8-tetrahydro-1,7-naphthyridine-2-carboxylate (Int-1b)

To a 40 mL glass vial was added ethyl 4-(4-((benzyloxy)carbonyl)piperazin-1-yl)-5,6,7,8-tetrahydro-1,7-naphthyridine-2-carboxylate (**Int-1**) (852 mg, 2.01 mmol), methanesulfonato(2-dicyclohexylphosphino-2',6'-di-i-propoxy-1,1'-biphenyl)(2'-methylamino-1,1'-biphenyl-2-yl)palladium(II) (171 mg, 0.201 mmol), cesium carbonate (2.29 g, 7.03 mmol), a teflon-coated magnetic stirbar, anhydrous 1,4-dioxane (10 mL), and 1-bromonaphthalene (1.40 mL, 10.0 mmol). The mixture was degassed by subsurface sparging with nitrogen for 5 minutes, and the vial was sealed under an atmosphere of nitrogen. The mixture was stirred at 100 °C for 1.5 h, and was then combined with a mixture generated analogously on smaller scale from 50.0 mg of**Int-1.** The combined mixture was filtered through CELITE, and the CELITE was rinsed with dichloromethane, and the combined filtrate was concentrated to a residue, which was purified by column chromatography on silica gel (10 to 30% (3:1 ethyl acetate:ethanol, v/v) in hexane) to afford ethyl 4-(4-((benzyloxy)carbonyl)piperazin-1-yl)-7-(naphthalen-1-yl)-5,6,7,8-tetrahydro-1,7-naphthyridine-2-carboxylate (**Int-1b**). Mass Calc'd: 550.3, found 551.3 (M+H)⁺.

### Step B - Synthesis of 4-(4-((benzyloxy)carbonyl)piperazin-1-yl)-7-(naphthalen-1-yl)-5, 6, 7,8-tetrahydro-1, 7-naphthyridine-2-carboxylic acid (Int-1c)

To a 20 mL glass vial was added ethyl 4-(4-((benzyloxy)carbonyl)piperazin-1-yl)-7-(naphthalen-1-yl)-5,6,7,8-tetrahydro-1,7-naphthyridine-2-carboxylate (**Int-1b**) (993 mg, 1.80 mmol), a teflon-coated magnetic stirbar, tetrahydrofuran (5.4 mL), methanol (1.8 mL), and water (1.8 mL). To the resulting stirred solution was added lithium hydroxide (216 mg, 9.01 mmol). The mixture was stirred at room temperature for 48 min, then was diluted with ethyl acetate (20 mL) and water (20 mL). To this mixture was added potassium hydrogen sulfate (1.47 g, 10.8 mmol), and the mixture was agitated and sonicated until two homogeneous layers formed. The layers were separated, the aqueous layer was extracted with ethyl acetate (10 mL). The combined organic layers were washed with brine. To the emulsified organic layer was added ethyl acetate (10 mL) and water (10 mL). To this mixture was added 6.0 mL of a solution that was prepared by dissolving lithium hydroxide (43 mg) in water (10.5 mL). The emulsified system was concentrated to a yellow solid, to which was added dichloromethane (20 mL), and water (20 mL). The mixture was sonicated, the layers were separated, the aqueous layer was extracted with dichloromethane (10 mL), the combined organic layers were washed with brine (20 mL), then dried over anhydrous sodium sulfate, filtered and concentrated. The resulting foam was sonicated under methanol (10 mL), and the mixture was concentrated. The residue was sonicated under methanol (100 mL), and the mixture was concentrated to afford 4-(4-((benzyloxy)carbonyl)piperazin-1-yl)-7-(naphthalen-1-yl)-5,6,7,8-tetrahydro-1,7-naphthyridine-2-carboxylic acid (**Int-1c**), which was used directly without further purification. Mass Calc'd: 522.2, found 523.2 (M+H)⁺.

### Step C - Synthesis of 7-(naphthalen-1-yl)-4-(piperazin-1-yl)-5,6,7,8-tetrahydro-1,7-naphthyridine-2-carboxylic acid (Int-1d)

To a stirred suspension of 4-(4-((benzyloxy)carbonyl)piperazin-1-yl)-7-(naphthalen-1-yl)-5,6,7,8-tetrahydro-1,7-naphthyridine-2-carboxylic acid (**Int-1c**) (507 mg, 0.970 mmol) in methanol (9.7 mL) was added palladium on carbon (10 wt% Pd dry basis, 50% water, 413 mg). The mixture was stirred under 1 atm of hydrogen at 50 °C for 3.5 h, then diluted with methanol (30 mL) and dichloromethane (40 mL). The mixture was sonicated and filtered. The following extraction process was performed five times: the filtered black solid was sonicated under methanol/dichloromethane (1:1 v/v, 80 mL), then filtered. Then, the combined filtrates were concentrated to afford 7-(naphthalen-1-yl)-4-(piperazin-1-yl)-5,6,7,8-tetrahydro-1,7-naphthyridine-2-carboxylic acid (**Int-1d**), which was used directly without further purification. Mass Calc'd: 388.2, found 389.2 (M+H)⁺.

### Step D - Synthesis of 4-(4-acryloylpiperazin-1-yl)-7-(naphthalen-1-yl)-5,6,7,8-tetrahydro-1,7-naphthyridine-2-carboxylic acid (Int-1e)

To a stirred suspension of 7-(naphthalen-1-yl)-4-(piperazin-1-yl)-5,6,7,8-tetrahydro-1,7-naphthyridine-2-carboxylic acid (**Int-1d**) (303 mg, 0.779 mmol) in anhydrous dichloromethane (12 mL) at 0 °C under an atmosphere of nitrogen was added a solution of acrylic anhydride (0.113 mL, 0.977 mmol) in dichloromethane (3.6 mL), followed by 4-methylmorpholine (0.26 mL, 2.4 mmol). The mixture was stirred at room temperature for 35 min, then water (15 mL) was added. Potassium bisulfate (425 mg, 3.12 mmol) was added. After thorough mixing, a biphasic system formed, and the layers were separated. The aqueous layer was extracted with dichloromethane (5 mL), the combined organic layers were washed with water (15 mL), dried over anhydrous sodium sulfate, filtered, and concentrated. The residue was sonicated under 8 mL of acetonitrile, then concentrated to afford 4-(4-acryloylpiperazin-1-yl)-7-(naphthalen-1-yl)-5,6,7,8-tetrahydro-1,7-naphthyridine-2-carboxylic acid (**Int-1e**), which was used directly without further purification. Mass Calc'd: 442.2, found 443.2 (M+H)⁺.

### Step E - Synthesis of 4-(4-acryloylpiperazin-1-yl)-7-(naphthalen-1-yl)-N-(2-(pyrrolidin-1-yl)ethyl)-5,6,7,8-tetrahydro-1,7-naphthyridine-2-carboxamide (Example 1)

A stock solution was prepared as follows, for synthesis of a library of amides including compound **1:** to a 40 mL glass vial was added 4-(4-acryloylpiperazin-1-yl)-7-(naphthalen-1-yl)-5,6,7,8-tetrahydro-1,7-naphthyridine-2-carboxylic acid (**Int-1e**) (273 mg, 0.617 mmol) and anhydrous dimethylformamide (6.2 mL). To the resulting solution was added triethylamine (0.345 mL, 2.48 mmol), then ((6-chloro-1H-benzo[d][1,2,3]triazol-1-yl)oxy)tri(pyrrolidin-1-yl)phosphonium hexafluorophosphate (V) (1.025 g, 1.85 mmol). To a 4 mL glass vial containing 2-(pyrrolidin-1-yl)ethan-1-amine (0.15 mmol) and a teflon-coated magnetic stirbar was added 0.35 mL of the above-prepared stock solution. The contents of the 4 mL vial were stirred at room temperature for 2.5 h, then diluted with dimethylsulfoxide (2.5 mL), filtered, and the filter was rinsed with dimethylsulfoxide (1.0 mL). The combined filtrate was purified directly by RP-HPLC (with ammonium hydroxide modifier) to afford 4-(4-acryloylpiperazin-1-yl)-7-(naphthalen-1-yl)-N-(2-(pyrrolidin-1-yl)ethyl)-5,6,7,8-tetrahydro-1,7-naphthyridine-2-carboxamide (Example **1**). ¹H NMR (500 MHz, DMSO-*d*₆) Diagnostic signals: δ 8.62 (s, 1H), 8.20 (m, 1H), 7.92 (m, 1H), 7.65 (d, *J* = 8.2 Hz, 1H), 7.53 (m, 2H), 7.50 - 7.44 (m, 2H), 7.25 (d, *J* = 7.3 Hz, 1H), 6.85 (dd, *J* = 16.7, 10.5 Hz, 1H), 6.16 (dd, *J =* 16.7, 2.2 Hz, 1H), 5.74 (dd, *J* = 10.5, 2.1 Hz, 1H), 4.34 (s, 2H). Mass Calc'd: 538.3, found 539.4 (M+H)⁺.

The following compounds were prepared in a similar manner to Example 1.

| **Example No.** | **Structure** | **Compound Name** | **Exact Mass** |
|---|---|---|---|
| **2** | 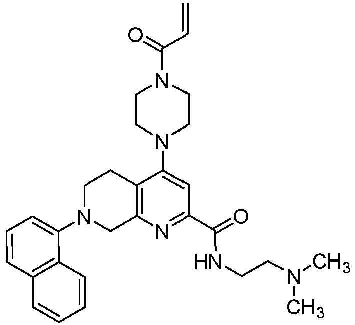 | 4-(4-acryloylpiperazin-1-yl)-N-(2-(dimethylamino)ethyl)-7-(naphthalen-1-yl)-5,6,7,8-tetrahydro-1,7-naphthyridine-2-carboxamide | Calc'd 512.3, found 513.3 (M+H)⁺ |
| **3** | 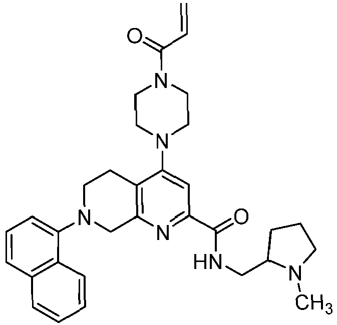 | *rac*-4-(4-acryloylpiperazin-1-yl)-N-((1-methylpyrrolidin-2-yl)methyl)-7-(naphthalen-1-yl)-5,6,7,8-tetrahydro-1,7-naphthyridine-2-carboxamide | Calc'd 538.3, found 539.5 (M+H)⁺ |
| **4** | 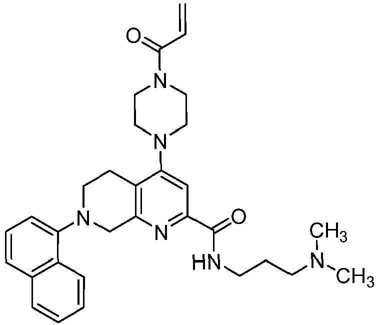 | 4-(4-acryloylpiperazin-1-yl)-N-(3-(dimethylamino)propyl )-7-(naphthalen-1-yl)-5,6,7,8-tetrahydro-1,7-naphthyridine-2-carboxamide | Calc'd 526.3, found 527.5 (M+H)⁺ |
| **5** | 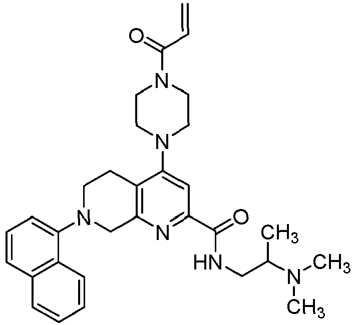 | *rac-*4-(4-acryloylpiperazin-1-yl)-N-(2-(dimethylamino)propyl )-7-(naphthalen-1-yl)-5,6,7,8-tetrahydro-1,7-naphthyridine-2-carboxamide | Calc'd 526.3, found 527.4 (M+H)⁺ |
| **6** | 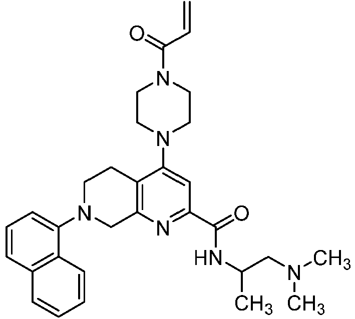 | *rac*-4-(4-acryloylpiperazin-1-yl)-N-(1-(dimethylamino)propan -2-yl)-7-(naphthalen-1-yl)-5,6,7,8-tetrahydro-1,7-naphthyridine-2-carboxamide | Calc'd 526.3, found 527.4 (M+H)⁺ |
| **7** | 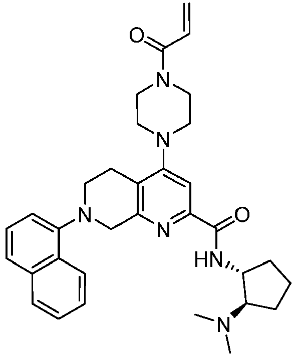 | *rac*-4-(4-acryloylpiperazin-1-*yl)-N-(trans-2-*(dimethylamino)cyclop entyl)-7-(naphthalen-1-yl)-5,6,7,8-tetrahydro-1,7-naphthyridine-2-carboxamide | Calc'd 552.3, found 553.4 (M+H)⁺ |
| **8** | 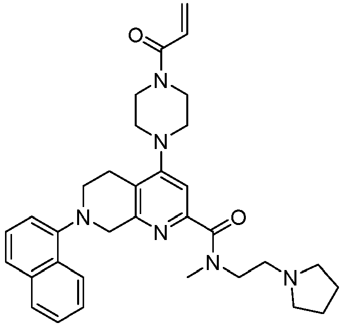 | 4-(4-acryloylpiperazin-1-yl)-N-methyl-7-(naphthalen-1-yl)-N-(2-(pyrrolidin-1-yl)ethyl)-5,6,7,8-tetrahydro-1,7-naphthyridine-2-carboxamide | Calc'd 552.3, found 553.4 (M+H)⁺ |
| **9** | 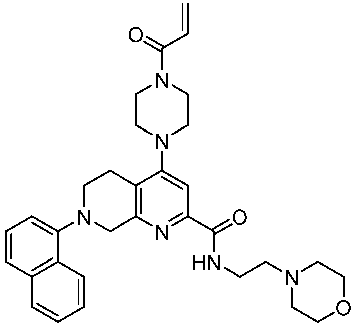 | 4-(4-acryloylpiperazin-1-yl)-N-(2-morpholinoethyl)-7-(naphthalen-1-yl)-5,6,7,8-tetrahydro-1,7-naphthyridine-2-carboxamide | Calc'd 554.3, found 555.4 (M+H)⁺ |
| **10** | 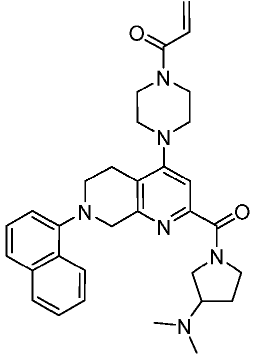 | *rac*-1-(4-(2-(3-(dimethylamino)pyrroli dine-1-carbonyl)-7-(naphthalen-1-yl)-5,6,7,8-tetrahydro-1,7-naphthyridin-4-yl)piperazin-1-yl)prop-2-en-1-one | Calc'd 538.3, found 539.4 (M+H)⁺ |
| **11** | 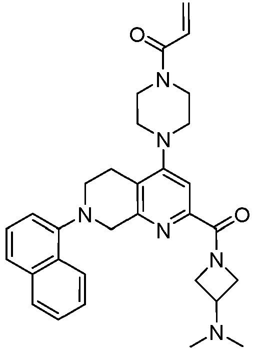 | 1-(4-(2-(3-(dimethylamino)azetidi ne-1-carbonyl)-7-(naphthalen-1-yl)-5,6,7,8-tetrahydro-1,7-naphthyridin-4-yl)piperazin-1-yl)prop-2-en-1-one | Calc'd 524.3, found 525.4 (M+H)⁺ |

### Example 12

### Preparation of (R)-4-(4-acryloylpiperazin-1-yl)-N-(1-(dimethylamino)propan-2-yl)-7-(8-methylnaphthalen-1-yl)-5,6,7,8-tetrahydro-1,7-naphthyridine-2-carboxamide (Example 12)

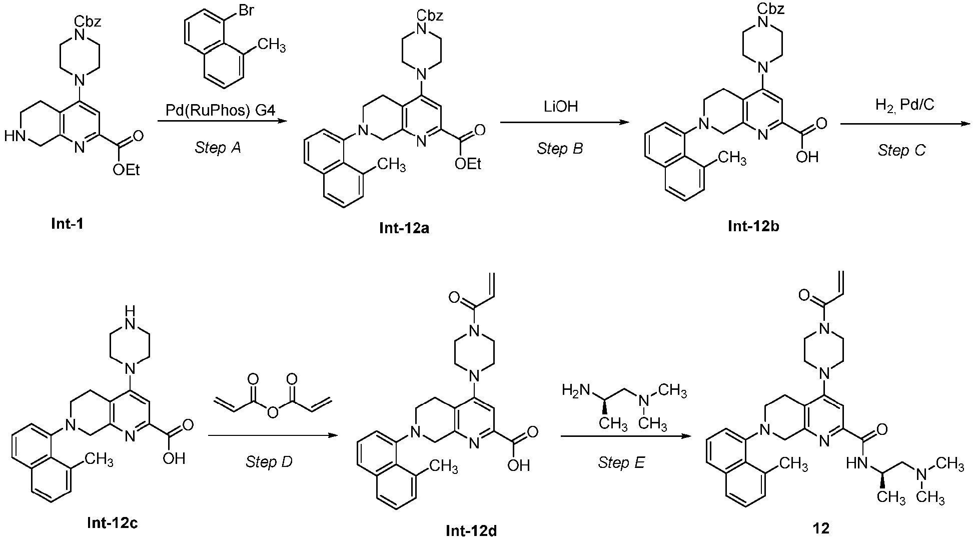

### Step A - Synthesis of ethyl 4-(4-((benzyloxy)carbonyl)piperazin-1-yl)-7-(8-methylnaphthalen-1-yl)-5,6,7,8-tetrahydro-1,7-naphthyridine-2-carboxylate (Int-12a)

To a 40 mL glass vial was added ethyl 4-(4-((benzyloxy)carbonyl)piperazin-1-yl)-5,6,7,8-tetrahydro-1,7-naphthyridine-2-carboxylate **(Int-1)** (1.00 g, 2.36 mmol), 1-bromo-8-methylnaphthalene (0.781 g, 3.53 mmol), methanesulfonato(2-dicyclohexylphosphino-2',6'-di-*i-*propoxy-1,1'-biphenyl)(2'-methylamino-1,1'-biphenyl-2-yl)palladium(II) (0.200 g, 0.236 mmol), cesium carbonate (2.69 g, 8.24 mmol), and a teflon-coated magnetic stir bar. Then anhydrous 1,4-dioxane (11.8 mL) was added, and the mixture was degassed by subsurface sparging with nitrogen for 4 min, and the vial was then sealed under 1 atm of nitrogen. The mixture was stirred at 100 °C for 3.5 h, then filtered through CELITE. The CELITE was rinsed with dichloromethane, and the combined filtrate was concentrated to a residue that was purified by column chromatography on silica gel (10% to 20% (3:1 ethyl acetate:ethanol v/v) in hexane), followed by a second purification by column chromatography on silica gel (15% to 40% ethyl acetate in hexane) to afford ethyl 4-(4-((benzyloxy)carbonyl)piperazin-1-yl)-7-(8-methylnaphthalen-1-yl)-5,6,7,8-tetrahydro-1,7-naphthyridine-2-carboxylate (**Int-12a**). ¹H NMR (500 MHz, Methanol-*d*₄) Diagnostic signals: δ 7.69 (d, *J =* 8.0 Hz, 1H), 7.65 (d, *J =* 7.3 Hz, 1H), 7.62 (s, 1H), 7.44 - 7.28 (m, 8H), 7.23 (d, *J =* 7.0 Hz, 1H), 5.17 (s, 2H), 4.47 - 4.34 (m, 3H), 3.89 (d, *J =* 17.1 Hz, 1H), 3.82 - 3.62 (m, 4H), 3.62 - 3.54 (m, 1H), 3.05 - 2.94 (m, 3H), 2.89 (s, 3H), 1.39 (t, *J* = 7.1 Hz, 3H). Mass Calc'd: 564.3, found 565.3 (M+H)⁺.

### Step B - Synthesis of 4-(4-((benzyloxy)carbonyl)piperazin-1-yl)-7-(8-methylnaphthalen-1-yl)-5,6,7,8-tetrahydro-1,7-naphthyridine-2-carboxylic acid (Int-12b)

To a solution of ethyl 4-(4-((benzyloxy)carbonyl)piperazin-1-yl)-7-(8-methylnaphthalen-1-yl)-5,6,7,8-tetrahydro-1,7-naphthyridine-2-carboxylate **(Int-12a)** (454 mg, 0.804 mmol) in THF/methanol/water (2.4 mL, 0.80 mL, 0.80 mL respectively) was added lithium hydroxide (96 mg, 4.0 mmol). The mixture was stirred at room temperature for 42 min, then potassium hydrogen sulfate (657 mg, 4.82 mmol) was added. The mixture was concentrated, then water (20 mL) and dichloromethane (15 mL) were added. The mixture was sonicated, diluted with dichloromethane (5 mL), and the layers were separated. The aqueous layer was extracted with dichloromethane (10 mL), the combined organic layers were washed with brine (20 mL), then dried over anhydrous sodium sulfate, filtered, and concentrated to afford 4-(4-((benzyloxy)carbonyl)piperazin-1-yl)-7-(8-methylnaphthalen-1-yl)-5,6,7,8-tetrahydro-1,7-naphthyridine-2-carboxylic acid **(Int-12b),** which was used directly without further purification. Mass Calc'd: 536.2, found 567.3 (M+H)⁺.

### Step C - Synthesis of 7-(8-methylnaphthalen-1-yl)-4-(piperazin-1-yl)-5, 6, 7, 8-tetrahydro-1, 7-naphthyridine-2-carboxylic acid (Int-12c)

To a stirred solution of 4-(4-((benzyloxy)carbonyl)piperazin-1-yl)-7-(8-methylnaphthalen-1-yl)-5,6,7,8-tetrahydro-1,7-naphthyridine-2-carboxylic acid **(Int-12b)** (4.32 g, 7.57 mmol) in methanol (76 mL) was added palladium on carbon (10 wt% Pd dry basis, 50% water, 1.61 g). The mixture was stirred under 1 atm of hydrogen at 50 °C for 3 h and 16 min, then concentrated. The following was performed twice: the solid was sonicated under dichloromethane (20 mL), then concentrated. In this way was obtained 7-(8-methylnaphthalen-1-yl)-4-(piperazin-1-yl)-5,6,7,8-tetrahydro-1,7-naphthyridine-2-carboxylic acid (**Int-12c**), which was used directly without further purification. Mass Calc'd: 402.2, found 402.9 (M+H)⁺.

### Step D - Synthesis of 4-(4-acryloylpiperazin-1-yl)-7-(8-methylnaphthalen-1-yl)-5, 6, 7,8-tetrahydro-1, 7-naphthyridine-2-carboxylic acid (Int-12d)

To a stirred suspension of 7-(8-methylnaphthalen-1-yl)-4-(piperazin-1-yl)-5,6,7,8-tetrahydro-1,7-naphthyridine-2-carboxylic acid (**Int-12c**) (generated from 7.57 mmol of **Int-12b,** as described in Step C above) in anhydrous dichloromethane (152 mL) was added 4-methylmorpholine (2.50 mL, 22.7 mmol), then acrylic anhydride (1.10 mL, 9.51 mmol). The mixture was stirred at room temperature for 2 h, then was filtered through CELITE, and the CELITE was rinsed four times with dichloromethane (10 mL). The combined filtrate was washed with a solution of potassium bisulfate (4.12 g, 30.3 mmol) in water (150 mL). The layers were separated, the aqueous layer was extracted with dichloromethane (10 mL), the combined organic layers were washed with water (150 mL), the aqueous wash was back-extracted with dichloromethane (5 mL), the combined organic layers were dried over anhydrous sodium sulfate, filtered, and concentrated. The resulting foam was ground to a powder with a spatula under hexane (20 mL), the supernatant was removed, and the remaining slurry was concentrated to afford 4-(4-acryloylpiperazin-1-yl)-7-(8-methylnaphthalen-1-yl)-5,6,7,8-tetrahydro-1,7-naphthyridine-2-carboxylic acid **(Int-12d),** which was used directly without further purification. Mass Calc'd: 456.2, found 457.1 (M+H)⁺.

### Step E - Synthesis of (R)-4-(4-acryloylpiperazin-1-yl)-N-(1-(dimethylamino)propan-2-yl)-7-(8-methylnaphthalen-1-yl)-5,6,7,8-tetrahydro-1,7-naphthyridine-2-carboxamide (Example 12)

A stock solution was prepared as follows, for synthesis of a library of amides including Example **12:** To a solution of 4-(4-acryloylpiperazin-1-yl)-7-(8-methylnaphthalen-1-yl)-5,6,7,8-tetrahydro-1,7-naphthyridine-2-carboxylic acid **(Int-12d)** (108.4 mg, 0.237 mmol) in anhydrous dimethylformamide (0.25 mL) was added triethylamine (0.133 mL, 0.954 mmol), then ((6-chloro-1H-benzo[d][1,2,3]triazol-1-yl)oxy)tri(pyrrolidin-1-yl)phosphonium hexafluorophosphate (V) (395.2 mg, 0.712 mmol). To a 4 mL glass vial containing (*R*)-N¹,N¹-dimethylpropane-1,2-diamine (0.150 mmol) and a teflon-coated magnetic stirbar was added 0.28 mL of the above-prepared stock solution. The contents of the 4 mL vial were stirred at room temperature for 1 h and 45 min, then diluted with dimethylsulfoxide (2.5 mL), filtered, and the filter was rinsed with dimethylsulfoxide (1.0 mL). The combined filtrate was purified directly by RP-HPLC (with ammonium hydroxide modifier) to afford (*R*)-4-(4-acryloylpiperazin-1-yl)-N-(1-(dimethylamino)propan-2-yl)-7-(8-methylnaphthalen-1-yl)-5,6,7,8-tetrahydro-1,7-naphthyridine-2-carboxamide (Example **12**). ¹H NMR (600 MHz, DMSO-*d*₆) Diagnostic signals: δ 6.93 - 6.77 (m, 1H), 6.22 - 6.11 (m, 1H), 5.79 - 5.69 (m, 1H). Mass Calc'd: 540.3, found 540.8 (M+H)⁺. The following compounds were prepared in a similar manner to Example **12.**

| **Example No.** | **Structure** | **Compound Name** | **Exact Mass** |
|---|---|---|---|
| **13** | 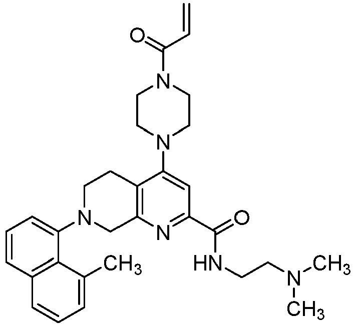 | 4-(4-acryloylpiperazin-1-yl)-N-(2-(dimethylamino)ethyl)-7-(8-methylnaphthalen-1-yl)-5,6,7,8-tetrahydro-1,7-naphthyridine-2-carboxamide | Calc'd 526.3, found 527.3 (M+H)⁺ |
| **14** | 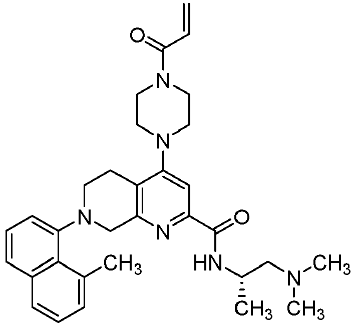 | (*S*)-4-(4-acryloylpiperazin-1-yl)-N-(1-(dimethylamino)propan-2-yl)-7-(8-methylnaphthalen-1-yl)-5,6,7,8-tetrahydro-1,7-naphthyridine-2-carboxamide | Calc'd 540.3, found 541.2 (M+H)⁺ |
| **15** | 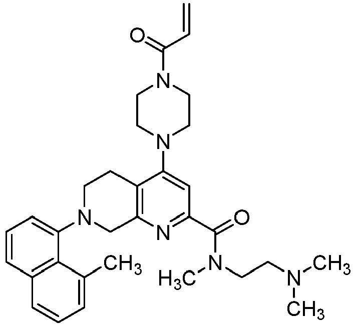 | 4-(4-acryloylpiperazin-1-yl)-N-(2-(dimethylamino)ethyl)-N-methyl-7-(8-methylnaphthalen-1-yl)-5,6,7,8-tetrahydro-1,7-naphthyridine-2-carboxamide | Calc'd 540.3, found 541.3 (M+H)⁺ |
| **16** | 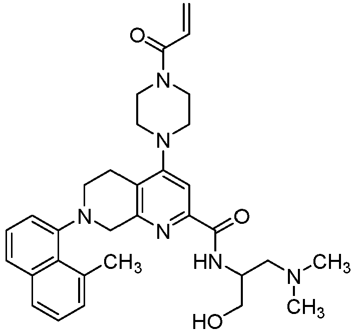 | *rac*-4-(4-acryloylpiperazin-1-yl)-N-(1-(dimethylamino)-3-hydroxypropan-2-yl)-7-(8-methylnaphthalen-1-yl)-5,6,7,8-tetrahydro-1,7-naphthyridine-2-carboxamide | Calc'd 556.3, found 556.8 (M+H)⁺ |
| **17** | 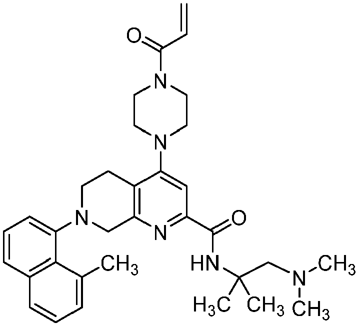 | 4-(4-acryloylpiperazin-1-yl)-N-(1-(dimethylamino)-2-methylpropan-2-yl)-7-(8-methylnaphthalen-1-yl)-5,6,7,8-tetrahydro-1,7-naphthyridine-2-carboxamide | Calc'd 554.3, found 554.8 (M+H)⁺ |
| **18** | 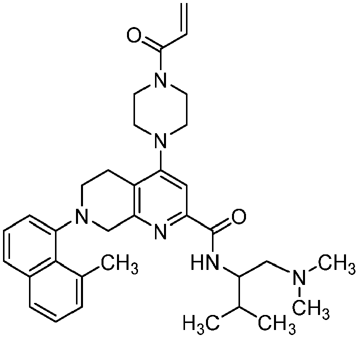 | *rac*-4-(4-acryloylpiperazin-1-yl)-N-(1-(dimethylamino)-3-methylbutan-2-yl)-7-(8-methylnaphthalen-1-yl)-5,6,7,8-tetrahydro-1,7-naphthyridine-2-carboxamide | Calc'd 568.4, found 569.2 (M+H)⁺ |
| **19** | 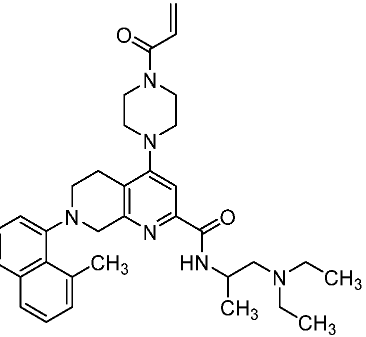 | *rac*-4-(4-acryloylpiperazin-1-yl)-N-(1-(diethylamino)propan-2-yl)-7-(8-methylnaphthalen-1-yl)-5,6,7,8-tetrahydro-1,7-naphthyridine-2-carboxamide | Calc'd 568.4, found 569.5 (M+H)⁺ |
| **20** | 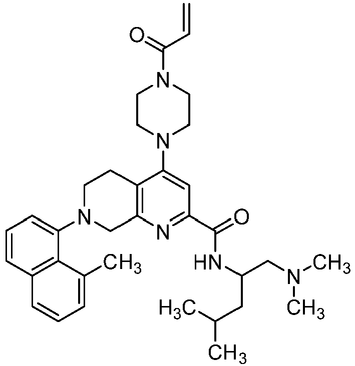 | *rac*-4-(4-acryloylpiperazin-1-yl)-N-(1-(dimethylamino)-4-methylpentan-2-yl)-7-(8-methylnaphthalen-1-yl)-5,6,7,8-tetrahydro-1,7-naphthyridine-2-carboxamide | Calc'd 582.4, found 583.5 (M+H)⁺ |
| **21** | 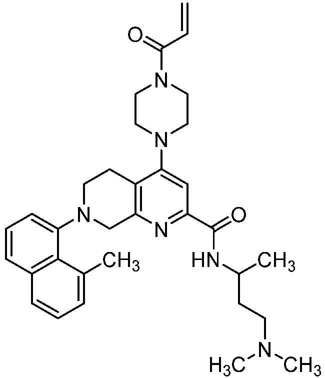 | *rac*-4-(4-acryloylpiperazin-1-yl)-N-(4-(dimethylamino)butan-methylnaphthalen-1-yl)-5,6,7,8-tetrahydro-1,7-naphthyridine-2-carboxamide | Calc'd 554.3, found 555.4 (M+H)⁺ |
| **22** | 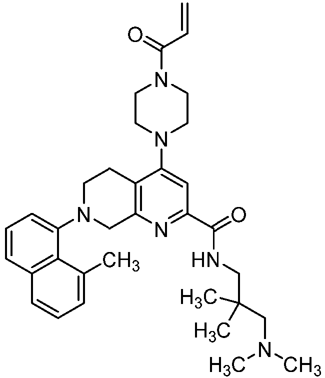 | 4-(4-acryloylpiperazin-1-yl)-N-(3-(dimethylamino)-2,2-dimethylpropyl)-7-(8-methylnaphthalen-1-yl)-5,6,7,8-tetrahydro-1,7-naphthyridine-2-carboxamide | Calc'd 568.4, found 569.5 (M+H)⁺ |
| **23** | 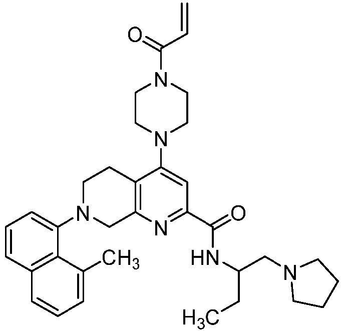 | *rac*-4-(4-acryloylpiperazin-1-yl)-7-(8-methylnaphthalen-1-yl)-N-(1-(pyrrolidin-1-yl)butan-2-yl)-5,6,7,8-tetrahydro-1,7-naphthyridine-2-carboxamide | Calc'd 580.4, found 580.9 (M+H)⁺ |
| **24** | 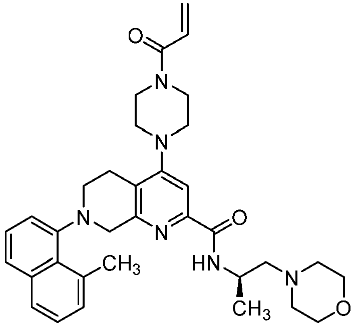 | (*R*)-4-(4-acryloylpiperazin-1-yl)-7-(8-methylnaphthalen-1-yl)-N-(1-morpholinopropan-2-yl)-5,6,7,8-tetrahydro-1,7-naphthyridine-2-carboxamide | Calc'd 582.3, found 583.4 (M+H)⁺ |
| **25** | 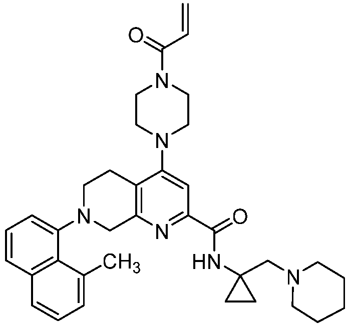 | 4-(4-acryloylpiperazin-1-yl)-7-(8-methylnaphthalen-1-yl)-N-(1-(piperidin-1-ylmethyl)cyclopropyl)-5,6,7,8-tetrahydro-1,7-naphthyridine-2-carboxamide | Calc'd 592.4, found 593.5 (M+H)⁺ |
| **26** | 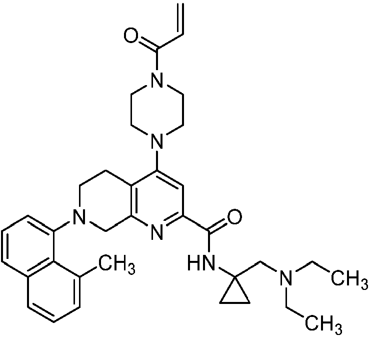 | 4-(4-acryloylpiperazin-1-yl)-N-(1-((diethylamino)methyl)cy clopropyl)-7-(8-methylnaphthalen-1-yl)-5,6,7,8-tetrahydro-1,7-naphthyridine-2-carboxamide | Calc'd 580.4, found 581.5 (M+H)⁺ |
| **27** | 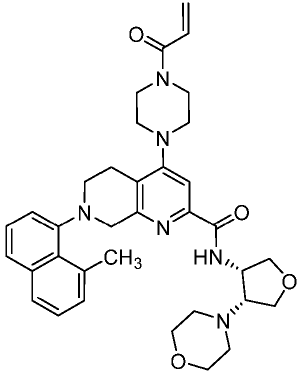 | *rac*-4-(4-acryloylpiperazin-1-yl)-7-(8-methylnaphthalen-1-yl)-N-(*cis*-4-morpholinotetrahydrofura n-3-yl)-5,6,7,8-tetrahydro-1,7-naphthyridine-2-carboxamide | Calc'd 610.3, found 611.7 (M+H)⁺ |
| **28** | 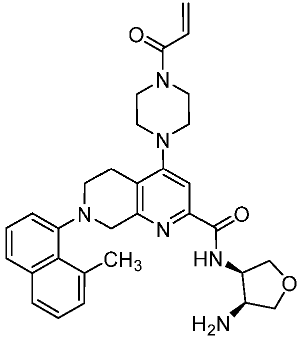 | *rac*-4-(4-acryloylpiperazin-1-yl)-N-(*cis-*4-aminotetrahydrofuran-3-yl)-7-(8-methylnaphthalen-1-yl)-5,6,7,8-tetrahydro-1,7-naphthyridine-2-carboxamide | Calc'd 540.3, found 541.5 (M+H)⁺ |
| **29** | 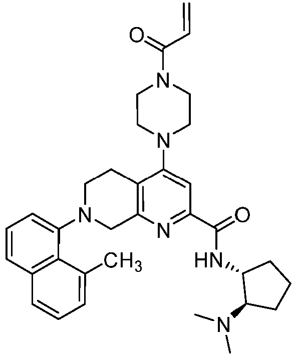 | *rac*-4-(4-acryloylpiperazin-1-yl)-N-(*trans*-2-(dimethylamino)cyclopent yl)-7-(8-methylnaphthalen-1-yl)-5,6,7,8-tetrahydro-1,7-naphthyridine-2-carboxamide | Calc'd 566.3, found 567.3 (M+H)⁺ |
| **30** | 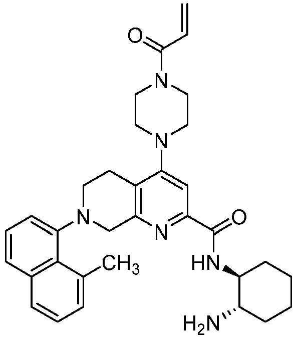 | 4-(4-acryloylpiperazin-1-yl)-N-((1*S*,2*S*)-2-aminocyclohexyl)-7-(8-methylnaphthalen-1-yl)-5,6,7,8-tetrahydro-1,7-naphthyridine-2-carboxamide | Calc'd 552.3, found 552.8 (M+H)⁺ |
| **31** | 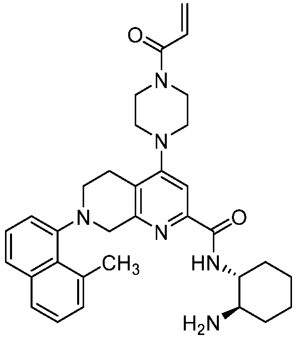 | 4-(4-acryloylpiperazin-1-yl)-N-((1*R*,2*R*)-2-aminocyclohexyl)-7-(8-methylnaphthalen-1-yl)-5,6,7,8-tetrahydro-1,7-naphthyridine-2-carboxamide | Calc'd 552.3, found 552.8 (M+H)⁺ |
| **32** | 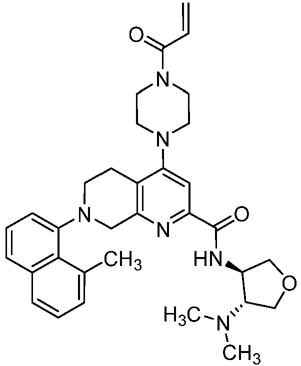 | *rac*-4-(4-acryloylpiperazin-1-yl)-N-(*trans-*4-(dimethylamino)tetrahydr ofuran-3-yl)-7-(8-methylnaphthalen-1-yl)-5,6,7,8-tetrahydro-1,7-naphthyridine-2-carboxamide | Calc'd 568.3, found 569.9 (M+H)⁺ |
| **33** | 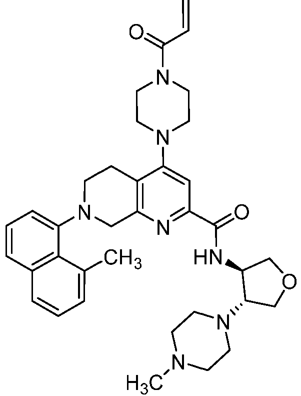 | *rac*-4-(4-acryloylpiperazin-1-yl)-7-(8-methylnaphthalen-1-yl)-*N*-(*trans*-4-(4-methylpiperazin-1-yl)tetrahydrofuran-3-yl)-5,6,7,8-tetrahydro-1,7-naphthyridine-2-carboxamide | Calc'd 623.4, found 624.5 (M+H)⁺ |
| **34** | 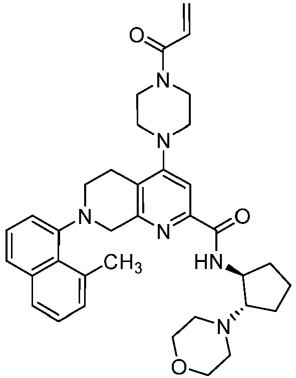 | *rac*-4-(4-acryloylpiperazin-1-yl)-7-(8-methylnaphthalen-1-yl)-N-(*trans*-2-morpholinocyclopentyl)-5,6,7,8-tetrahydro-1,7-naphthyridine-2-carboxamide | Calc'd 608.4, found 609.5 (M+H)⁺ |
| **35** | 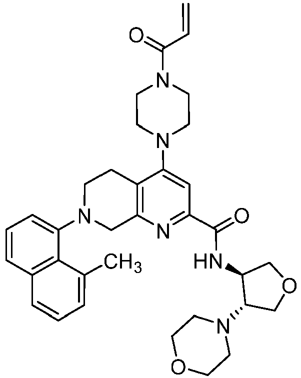 | *rac*-4-(4-acryloylpiperazin-1-yl)-7-(8-methylnaphthalen-1-yl)-N-(*trans*-4-morpholinotetrahydrofura n-3-yl)-5,6,7,8-tetrahydro-1,7-naphthyridine-2-carboxamide | Calc'd 610.3, found 611.5 (M+H)⁺ |
| **36** | 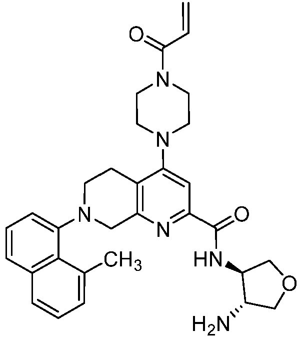 | *rac*-4-(4-acryloylpiperazin-1-yl)-N-(*trans-*4-aminotetrahydrofuran-3-yl)-7-(8-methylnaphthalen-1-yl)-5,6,7,8-tetrahydro-1,7-naphthyridine-2-carboxamide | Calc'd 540.3, found 541.4 (M+H)⁺ |
| **37** | 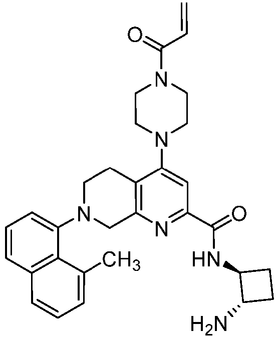 | *rac*-4-(4-acryloylpiperazin-1-yl)-N-(*trans-*2-aminocyclobutyl)-7-(8-methylnaphthalen-1-yl)-5,6,7,8-tetrahydro-1,7-naphthyridine-2-carboxamide | Calc'd 524.3, found 525.2 (M+H)⁺ |
| **38** | 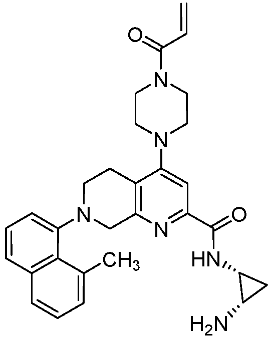 | *rac*-4-(4-acryloylpiperazin-1-yl)-N-(*cis*-2-aminocyclopropyl)-7-(8-methylnaphthalen-1-yl)-5,6,7,8-tetrahydro-1,7-naphthyridine-2-carboxamide | Calc'd 510.3, found 511.6 (M+H)⁺ |
| **39** | 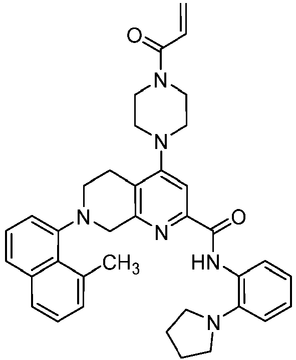 | 4-(4-acryloylpiperazin-1-yl)-7-(8-methylnaphthalen-1-yl)-N-(2-(pyrrolidin-1-yl)phenyl)-5,6,7,8-tetrahydro-1,7-naphthyridine-2-carboxamide | Calc'd 600.3, found 601.6 (M+H)⁺ |
| **40** | 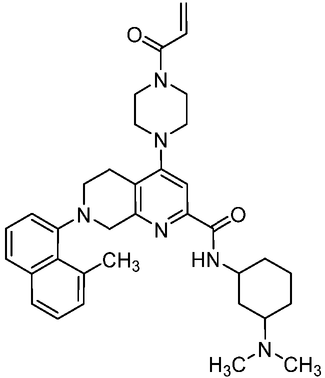 | *rac*-(*cis,trans*)-4-(4-acryloylpiperazin-1-yl)-N-(3-(dimethylamino)cyclohex yl)-7-(8-methylnaphthalen-1-yl)-5,6,7,8-tetrahydro-1,7-naphthyridine-2-carboxamide | Calc'd 580.4, found 581.2 (M+H)⁺ |
| **41** | 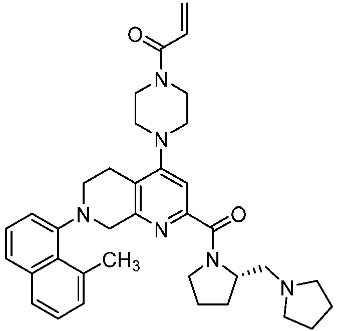 | (*S*)-1-(4-(7-(8-methylnaphthalen-1-yl)-2-(2-(pyrrolidin-1-ylmethyl)pyrrolidine-1-carbonyl)-5,6,7,8-tetrahydro-1,7-naphthyridin-4-yl)piperazin-1-yl)prop-2-en-1-one | Calc'd 592.4, found 593.7 (M+H)⁺ |
| **42** | 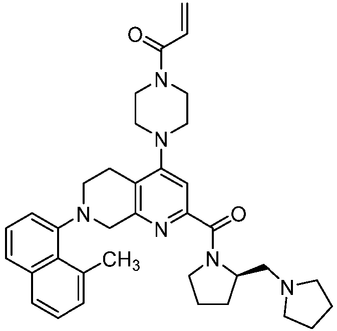 | (*R*)-1-(4-(7-(8-methylnaphthalen-1-yl)-2-(2-(pyrrolidin-1-ylmethyl)pyrrolidine-1-carbonyl)-5,6,7,8-tetrahydro-1,7-naphthyridin-4-yl)piperazin-1-yl)prop-2-en-1-one | Calc'd 592.4, found 593.6 (M+H)⁺ |
| **43** | 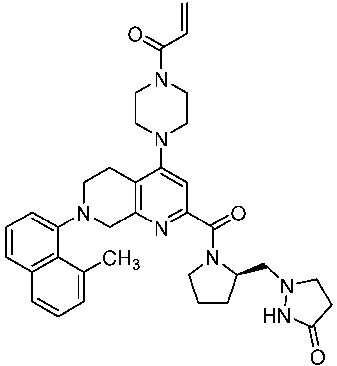 | (*R*)-1-((1-(4-(4-acryloylpiperazin-1-yl)-7-(8-methylnaphthalen-1-yl)-5,6,7,8-tetrahydro-1,7-naphthyridine-2-carbonyl)pyrrolidin-2-yl)methyl)pyrazolidin-3-one | Calc'd 607.3, found 608.5 (M+H)⁺ |
| **44** | 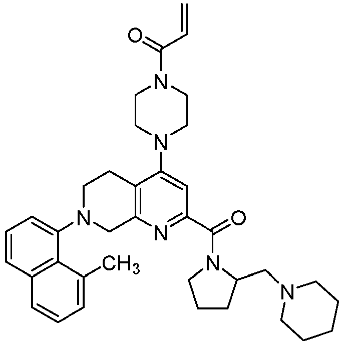 | *rac-*1-(4-(7-(8-methylnaphthalen-1-yl)-2-(2-(piperidin-1-ylmethyl)pyrrolidine-1-carbonyl)-5,6,7,8-tetrahydro-1,7-naphthyridin-4-yl)piperazin-1-yl)prop-2-en-1-one | Calc'd 606.4, found 607.5 (M+H)⁺ |
| **45** | 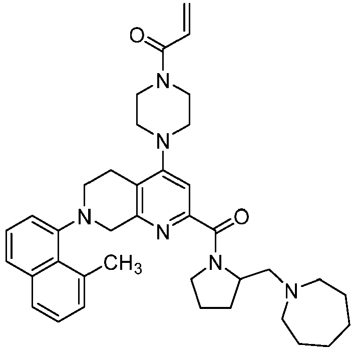 | *rac*-1-(4-(2-(2-(azepan-1-ylmethyl)pyrrolidine-1-carbonyl)-7-(8-methylnaphthalen-1-yl)-5,6,7,8-tetrahydro-1,7-naphthyridin-4-yl)piperazin-1-yl)prop-2-en-1-one | Calc'd 620.4, found 621.5 (M+H)⁺ |
| **46** | 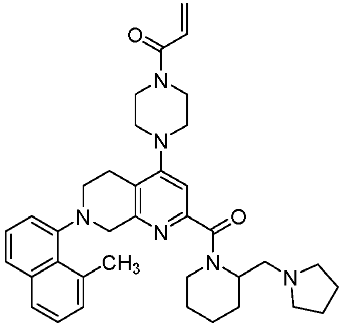 | *rac*-1-(4-(7-(8-methylnaphthalen-1-yl)-2-(2-(pyrrolidin-1-ylmethyl)piperidine-1-carbonyl)-5,6,7,8-tetrahydro-1,7-naphthyridin-4-yl)piperazin-1-yl)prop-2-en-1-one | Calc'd 606.4, found 607.5 (M+H)⁺ |
| **47** | 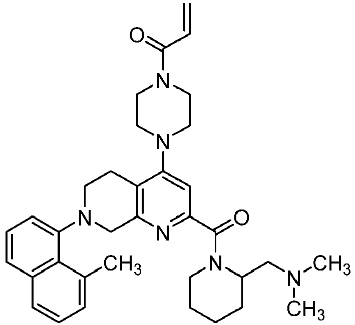 | *rac*-1-(4-(2-(2-((dimethylamino)methyl)p iperidine-1-carbonyl)-7-(8-methylnaphthalen-1-yl)-5,6,7,8-tetrahydro-1,7-naphthyridin-4-yl)piperazin-1-yl)prop-2-en-1-one | Calc'd 580.4, found 581.5 (M+H)⁺ |
| **48** | 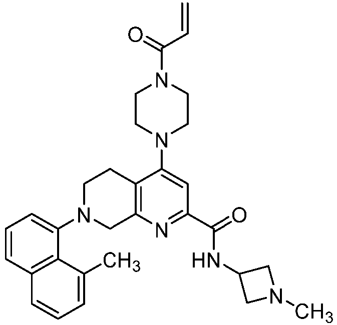 | 4-(4-acryloylpiperazin-1-yl)-N-(1-methylazetidin-3-yl)-7-(8-methylnaphthalen-1-yl)-5,6,7,8-tetrahydro-1,7-naphthyridine-2-carboxamide | Calc'd 524.3, found 525.6 (M+H)⁺ |
| **49** | 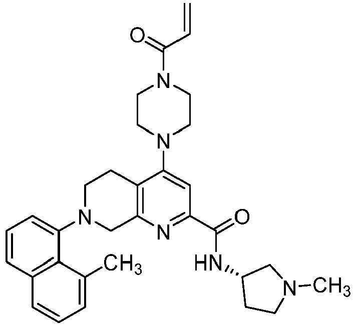 | (*S*)-4-(4-acryloylpiperazin-1-yl)-7-(8-methylnaphthalen-1-yl)-N-(1-methylpyrrolidin-3-yl)-5,6,7,8-tetrahydro-1,7-naphthyridine-2-carboxamide | Calc'd 538.3, found 539.4 (M+H)⁺ |
| **50** | 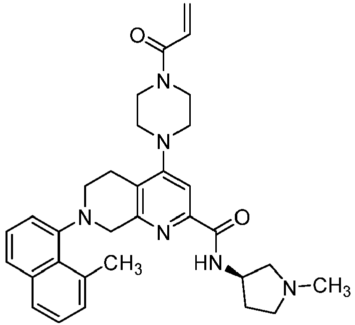 | (*R*)-4-(4-acryloylpiperazin-1-yl)-7-(8-methylnaphthalen-1-yl)-N-(1-methylpyrrolidin-3-yl)-5,6,7,8-tetrahydro-1,7-naphthyridine-2-carboxamide | Calc'd 538.3, found 539.4 (M+H)⁺ |
| **51** | 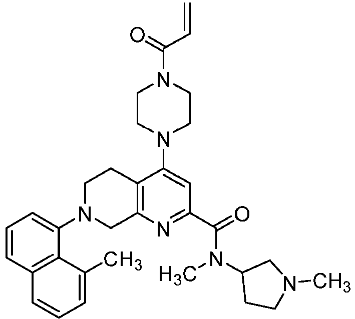 | *rac*-4-(4-acryloylpiperazin-1-yl)-N-methyl-7-(8-methylnaphthalen-1-yl)-N-(1-methylpyrrolidin-3-yl)-5,6,7,8-tetrahydro-1,7-naphthyridine-2-carboxamide | Calc'd 552.3, found 553.7 (M+H)⁺ |
| **52** | 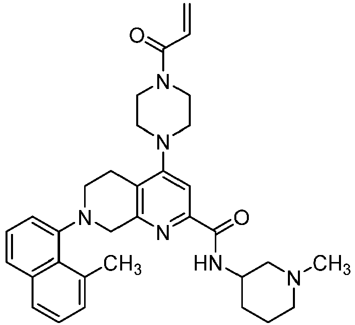 | *rac-*4-(4-acryloylpiperazin-1-yl)-7-(8-methylnaphthalen-1-yl)-N-(1-methylpiperidin-3-yl)-5,6,7,8-tetrahydro-1,7-naphthyridine-2-carboxamide | Calc'd 552.3, found 553.6 (M+H)⁺ |
| **53** | 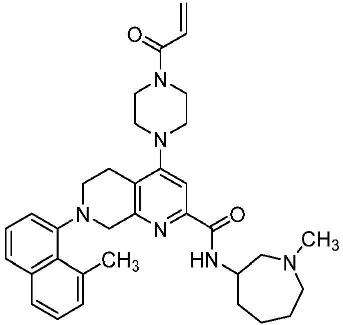 | *rac-*4-(4-acryloylpiperazin-1-yl)-N-(1-methylazepan-3-yl)-7-(8-methylnaphthalen-1-yl)-5,6,7,8-tetrahydro-1,7-naphthyridine-2-carboxamide | Calc'd 566.3, found 567.7 (M+H)⁺ |
| **54** | 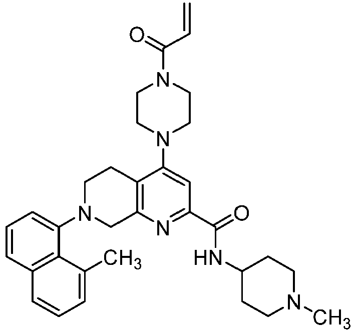 | 4-(4-acryloylpiperazin-1-yl)-7-(8-methylnaphthalen-1-yl)-N-(1-methylpiperidin-4-yl)-5,6,7,8-tetrahydro-1,7-naphthyridine-2-carboxamide | Calc'd 552.3, found 553.4 (M+H)⁺ |
| **55** | 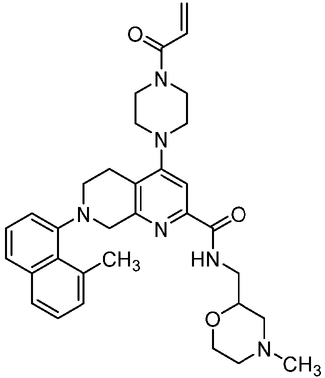 | *rac*-4-(4-acryloylpiperazin-1-yl)-N-((4-methylmorpholin-2-yl)methyl)-7-(8-methylnaphthalen-1-yl)-5,6,7,8-tetrahydro-1,7-naphthyridine-2-carboxamide | Calc'd 568.3, found 569.4 (M+H)⁺ |
| **56** | 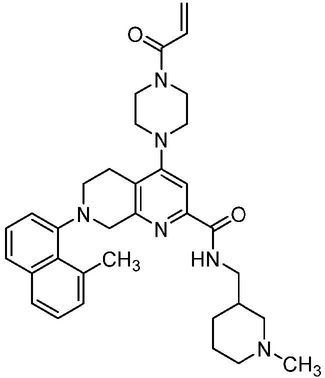 | *rac*-4-(4-acryloylpiperazin-1-yl)-7-(8-methylnaphthalen-1-yl)-N-((1-methylpiperidin-3-yl)methyl)-5,6,7,8-tetrahydro-1,7-naphthyridine-2-carboxamide | Calc'd 566.3, found 567.4 (M+H)⁺ |
| **57** | 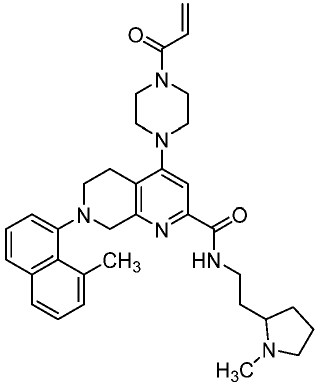 | *rac*-4-(4-acryloylpiperazin-1-yl)-7-(8-methylnaphthalen-1-yl)-N-(2-(1-methylpyrrolidin-2-yl)ethyl)-5,6,7,8-tetrahydro-1,7-naphthyridine-2-carboxamide | Calc'd 566.3, found 567.4 (M+H)⁺ |
| **58** | 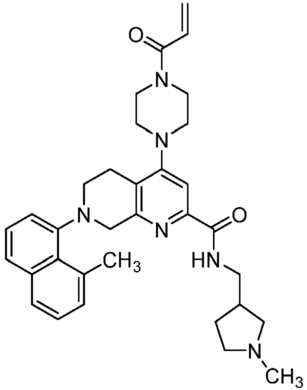 | *rac*-4-(4-acryloylpiperazin-1-yl)-7-(8-methylnaphthalen-1-yl)-N-((1-methylpyrrolidin-3-yl)methyl)-5,6,7,8-tetrahydro-1,7-naphthyridine-2-carboxamide | Calc'd 552.3, found 553.4 (M+H)⁺ |
| **59** | 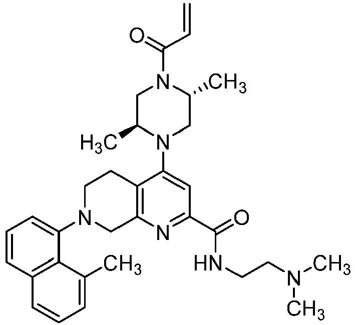 | 4-((2*S*,5*R*)-4-acryloyl-2,5-dimethylpiperazin-1-yl)-N-(2-(dimethylamino)ethyl)-7-(8-methylnaphthalen-1-yl)-5,6,7,8-tetrahydro-1,7-naphthyridine-2-carboxamide | Calc'd 554.3, found 554.8 (M+H)⁺ |

### Characterization Data for Example 59

| **Example No.** | **¹H NMR** |
|---|---|
| **59** | ¹H NMR (600 MHz, DMSO-*d*₆) δ 8.51 - 8.32 (m, 1H), 7.83 - 7.63 (m, 2H), 7.55 - 7.19 (m, 5H), 6.97 - 6.65 (m, 1H), 6.26 - 6.06 (m, 1H), 5.81 - 5.57 (m, 1H), 4.84 - 4.02 (m, 3H), 4.02 - 3.47 (m, 6H), 3.31 - 3.10 (m, 4H), 3.09 - 2.68 (m, 7H), 2.45 - 2.26 (m, 3H), 1.42 - 1.10 (m, 4H), 1.10 - 0.71 (m, 4H). |

### Example 60

### Preparation of (S)-4-(4-acryloyl-3-(cyanomethyl)piperazin-1-yl)-N-(2-(dimethylamino)ethyl)-7-(naphthalen-1-yl)-5,6,7,8-tetrahydro-1,7-naphthyridine-2-carboxamide (Example 60)

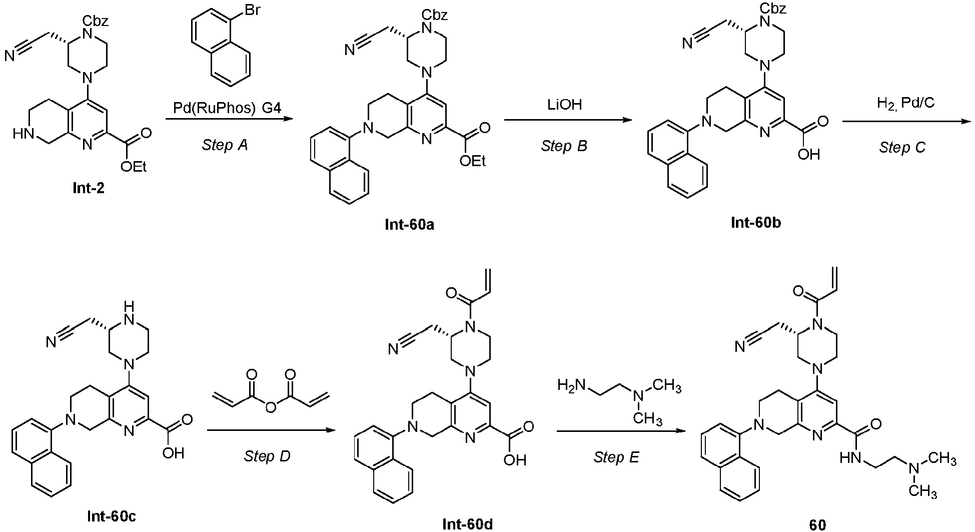

### Step A - Synthesis of ethyl (S)-4-(4-((benzyloxy)carbonyl)-3-(cyanomethyl)piperazin-1-yl)-7-(naphthalen-1-yl)-5,6,7,8-tetrahydro-1,7-naphthyridine-2-carboxylate (Int-60a)

Ethyl (*S*)-4-(4-((benzyloxy)carbonyl)-3-(cyanomethyl)piperazin-1-yl)-5,6,7,8-tetrahydro-1,7-naphthyridine-2-carboxylate **(Int-2)** (0.620 g, 1.338 mmol), 1-bromonaphthalene (0.415 g, 2.006 mmol), cesium carbonate (1.307 g, 4.01 mmol) and methanesulfonato(2-dicyclohexylphosphino-2',6'-di -i-propoxy-1,1'-biphenyl)(2'-methylamino-1,1'-biphenyl-2-yl)palladium(II) (0.114 g, 0.134 mmol) in anhydrous 1,4-dioxane (6.7 mL) subsurface sparged with nitrogen for 3 minutes. The vial was capped and placed in a preheated hotplate reaction block and stirred at 100 °C for 3 h. The mixture was cooled to room temperature and filtered through a pad of Florisil, with ethyl acetate dispacement rinse. Combined filtrates were concentrated to a residue, which was purified by column chromatography on silica gel (0 to 100% EtOAc in hexanes) to afford ethyl (S)-4-(4-((benzyloxy)carbonyl)-3-(cyanomethyl)piperazin-1-yl)-7-(naphthalen-1-yl)-5,6,7,8-tetrahydro-1,7-naphthyridine-2-carboxylate **(Int-60a).** ¹H NMR (500 MHz, Acetone-*d*₆) δ 8.34 - 8.23 (m, 1H), 8.00 - 7.88 (m, 1H), 7.66 (d, *J =* 9.1 Hz, 2H), 7.57 - 7.45 (m, 5H), 7.41 (t, *J =* 7.3 Hz, 2H), 7.36 (t, *J =* 7.2 Hz, 1H), 7.31 (d, *J =* 7.4 Hz, 1H), 5.64 (s, 2H), 5.24 (s, 2H), 4.85 (s, 1H), 4.46 - 4.34 (m, 4H), 4.23 (d, *J* = 13.9 Hz, 1H), 3.62 - 3.07 (m, 8H), 2.94 (s, 1H), 1.38 (t, *J =* 7.1 Hz, 3H). Mass Calc'd: 589.3, found 590.3 (M+H)⁺.

### Step B - Synthesis of (S)-4-(4-((benzyloxy)carbonyl)-3-(cyanomethyl)piperazin-1-yl)-7-(naphthalen-1-yl)-5,6,7,8-tetrahydro-1,7-naphthyridine-2-carboxylic acid (Int-60b)

Ethyl (*S*)-4-(4-((benzyloxy)carbonyl)-3-(cyanomethyl)piperazin-1-yl)-7-(naphthalen-1-yl)-5,6,7,8-tetrahydro-1,7-naphthyridine-2-carboxylate **(Int-60a)** (500 mg, 0.848 mmol) in tetrahydrofuran (8 mL), methanol (2 mL) and water (2 mL) was treated with lithium hydroxide (203 mg, 8.48 mmol) and then stirred at room temperature for 20 h. The pH was adjusted to approximately 4 with 2 M aqueous NaHSO₄, and the mixture was extracted with ethyl acetate. The combined organic layers were washed with brine, dried with anhydrous sodium sulfate, filtered and the filtrate was concentrated to afford the crude (*S*)-4-(4-((benzyloxy)carbonyl)-3-(cyanomethyl)piperazin-1-yl)-7-(naphthalen-1-yl)-5,6,7,8-tetrahydro-1,7-naphthyridine-2-carboxylic acid **(Int-60b),** which was used without further purification. Mass Calc'd: 561.2, found 562.2 (M+H)⁺.

### Step C - Synthesis of (S)-4-(3-(cyanomethyl)piperazin-1-yl)-7-(naphthalen-1-yl)-5, 6, 7, 8-tetrahydro-1, 7-naphthyridine-2-carboxylic acid (Int-60c)

(*S*)-4-(4-((benzyloxy)carbonyl)-3-(cyanomethyl)piperazin-1-yl)-7-(naphthalen-1-yl)-5,6,7,8-tetrahydro-1,7-naphthyridine-2-carboxylic acid **(Int-60b)** (230 mg, 0.410 mmol) and 5 wt% palladium on carbon (43.6 mg, 0.020 mmol) in ethanol (6 mL) and tetrahydrofuran (2 mL) was stirred under hydrogen (1 atm) at 40 °C for 5 h. The mixture was filtered through celite with tetrahydrofuran rinse. The combined filtrate was concentrated to afford the crude (*S*)-4-(3-(cyanomethyl)piperazin-1-yl)-7-(naphthalen-1-yl)-5,6,7,8-tetrahydro-1,7-naphthyridine-2-carboxylic acid **(Int-60c),** which was used without further purification. Mass Calc'd: 427.2, found 428.1 (M+H)⁺.

### Step D - Synthesis of (S)-4-(4-acryloyl-3-(cyanomethyl)piperazin-1yl)-7-(naphthalen-1-yl)-5,6,7,8-tetrahydro-1, 7-naphthyridine-2-carboxylic acid (Int-60d)

(*S*)-4-(3-(cyanomethyl)piperazin-1-yl)-7-(naphthalen-1-yl)-5,6,7,8-tetrahydro-1,7-naphthyridine-2-carboxylic acid **(Int-60c)** (140 mg, 0.327 mmol) in dichloromethane (2.0 mL) treated at room temperature with *N*-methylmorpholine (0.180 mL, 1.64 mmol) and acrylic anhydride (206 mg, 1.64 mmol). The mixture was stirred at room temperature for 20 h, diluted with dichloromethane (10 mL) and washed with saturated aqueous sodium bicarbonate (10 mL). The organic layer was dried with anhydrous sodium sulfate, filtered and the filtrate was concentrated. The residue was purified by preparative RP-HPLC (with trifluoroacetic acid modifier) afford (*S*)-4-(4-acryloyl-3-(cyanomethyl)piperazin-1-yl)-7-(naphthalen-1-yl)-5,6,7,8-tetrahydro-1,7-naphthyridine-2-carboxylic acid **(Int-60d).** ¹H NMR (500 MHz, DMSO-*d*₆) δ 8.26 - 8.17 (m, 1H), 7.99 - 7.90 (m, 1H), 7.68 (d, *J =* 8.2 Hz, 1H), 7.60 - 7.46 (m, 4H), 7.25 (d, *J =* 7.4 Hz, 1H), 7.01 - 6.80 (m, 1H), 6.22 (d, *J =* 17.0 Hz, 1H), 5.81 (dd, *J =* 10.5, 1.8 Hz, 1H), 5.05 (s, 1H), 4.82 (s, 1H), 4.45 (q, *J =* 16.9 Hz, 2H), 4.09 (s, 1H), 3.76 - 3.39 (m, 4H), 3.39 - 3.12 (m, 5H), 3.06 (s, 2H). Mass Calc'd: 481.2, found 482.1 (M+H)⁺.

### Step E - Synthesis of (S)-4-(4-acryloyl-3-(cyanomethyl)piperazin-1-yl)-N-(2-(dimethylamino)ethyl)-7-(naphthalen-1-yl)-5,6,7,8-tetrahydro-1,7-naphthyridine-2-carboxamide (Example 60)

A mixture of (*S*)-4-(4-acryloyl-3-(cyanomethyl)piperazin-1-yl)-7-(naphthalen-1-yl)-5,6,7,8-tetrahydro-1,7-naphthyridine-2-carboxylic acid **(Int-60d)** (76 mg, 0.158 mmol) and *N,N-*dimethylethylenediamine (69.6 mg, 0.8 mmol) in *N*,*N*-dimethylformamide (1.0 mL) was treated at room temperature with ((6-chloro-1H-benzo[d][1,2,3]triazol-1-yl)oxy)tri(pyrrolidin-1-yl)phosphonium hexafluorophosphate (V) (175 mg, 0.316 mmol). The mixture was aged at room temperature for 4 h and then filtered. The filtrate was directly purified by RP-HPLC (with ammonium hydroxide modifier) to afford (S)-4-(4-acryloyl-3-(cyanomethyl)piperazin-1-yl)-N-(2-(dimethylamino)ethyl)-7-(naphthalen-1-yl)-5,6,7,8-tetrahydro-1,7-naphthyridine-2-carboxamide (Example **60**). ¹H NMR (500 MHz, DMSO-*d*₆) δ 8.56 - 8.40 (m, 1H), 8.27 - 8.14 (m, 1H), 8.01 - 7.87 (m, 1H), 7.67 (d, *J =* 8.2 Hz, 1H), 7.62 - 7.46 (m, 3H), 7.27 (d, *J =* 7.4 Hz, 1H), 6.88 (s, 1H), 6.21 (d, *J* = 15.5 Hz, 1H), 5.80 (d, *J =* 11.4 Hz, 1H), 5.09 (s, 1H), 4.81 (s, 1H), 4.51 (s, 1H), 4.37 (s, 2H), 4.10 (s, 1H), 3.25 - 2.29 (m, 20H). Mass Calc'd: 551.3, found 552.3 (M+H)⁺.

### Example 61

### Preparation of 4-((S)-4-acryloyl-3-(cyanomethyl)piperazin-1-yl)-N-((R)-1-aminopropan-2-yl)-7-(naphthalen-1-yl)-5,6,7,8-tetrahydro-1,7-naphthyridine-2-carboxamide (Example 61)

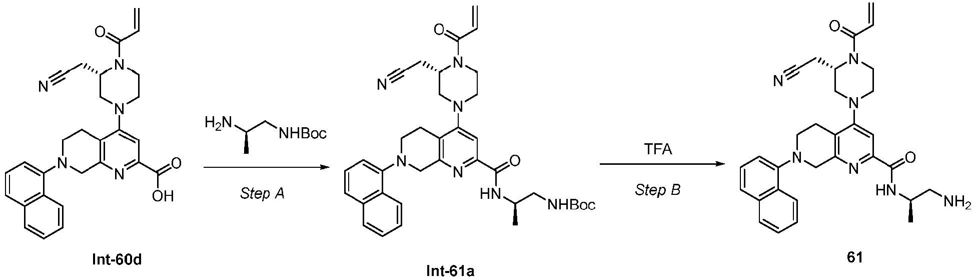

### Step A - Synthesis of tert-butyl ((R)-2-(4-((S)-4-acryloyl-3-(cyanomethyl)piperazin-1-yl)-7-(naphthalen-1-yl)-5,6,7,8-tetrahydro-1,7-naphthyridine-2-carboxamido)propyl)carbamate (Int-61a)

A mixture of (*S*)-4-(4-acryloyl-3-(cyanomethyl)piperazin-1-yl)-7-(naphthalen-1-yl)-5,6,7,8-tetrahydro-1,7-naphthyridine-2-carboxylic acid **(Int-60d)** (35 mg, 0.073 mmol) and (*R*)-tert-butyl (2-aminopropyl)carbamate (63.3 mg, 0.363 mmol) in *N,N*-dimethylformamide (1.0 mL) was treated at room temperature with triethylamine (0.101 mL, 0.727 mmol) and ((6-chloro-1H-benzo[d][1,2,3]triazol-1-yl)oxy)tri(pyrrolidin-1-yl)phosphonium hexafluorophosphate (V) (202 mg, 0.363 mmol), and was stirred at room temperature for 20 h. The mixture was diluted with DMSO (2 mL), neutralized with glacial AcOH (0.1 mL) and filtered. The filtrate was purified directly by RP-HPLC (with ammonium hydroxide modifier) to afford *tert*-butyl ((*R*)-2-(4-((*S*)-4-acryloyl-3-(cyanomethyl)piperazin-1-yl)-7-(naphthalen-1-yl)-5,6,7,8-tetrahydro-1,7-naphthyridine-2-carboxamido)propyl)carbamate **(Int-61a).** Mass Calc'd: 637.3, found 638.2 (M+H)⁺.

### Step B - Synthesis of 4-((S)-4-acryloyl-3-(cyanomethyl)piperazin-1-yl)-N-((R)-1-aminopropan-2-yl)-7-(naphthalen-1-yl)-5,6,7,8-tetrahydro-1,7-naphthyridine-2-carboxamide (Example 61)

*Tert*-butyl ((*R*)-2-(4-((*S*)-4-acryloyl-3-(cyanomethyl)piperazin-1-yl)-7-(naphthalen-1-yl)-5,6,7,8-tetrahydro-1,7-naphthyridine-2-carboxamido)propyl)carbamate **(Int-61a)** was dissolved in 20% v/v TFA in dichloromethane (2 mL), and the solution was aged at room temperature for 1 h and then concentrated. The residue was dissolved in ethanol (3 mL), filtered and the filtrate was purified by preparative RP-HPLC (with ammonium hydroxide modifier) to afford 4-((*S*)-4-acryloyl-3-(cyanomethyl)piperazin-1-yl)-N-((*R*)-1-aminopropan-2-yl)-7-(naphthalen-1-yl)-5,6,7,8-tetrahydro-1,7-naphthyridine-2-carboxamide (Example **61**). Mass Calc'd: 537.3, found 538.1 (M+H)⁺.

The following compound was prepared in a similar manner to Example 61.

| **Example No.** | **Structure** | **Compound Name** | **Exact Mass** |
|---|---|---|---|
| **62** | 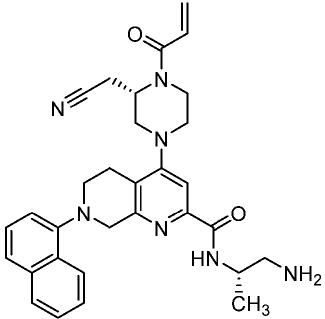 | 4-((*S*)-4-acryloyl-3-(cyanomethyl)piperazin-1-yl)-N-((*S*)-1-aminopropan-2-yl)-7-(naphthalen-1-yl)-5,6,7,8-tetrahydro-1,7-naphthyridine-2-carboxamide | Calc'd 537.3, found 538.2 (M+H)⁺ |

### Example 63

### Preparation of 2-((S)-1-acryloyl-4-(7-(8-methylnaphthalen-1-yl)-2-((R)-2-(pyrrolidin-1-ylmethyl)pyrrolidine-1-carbonyl)-5,6,7,8-tetrahydro-1,7-naphthyridin-4-yl)piperazin-2-yl)acetonitrile (Example 63)

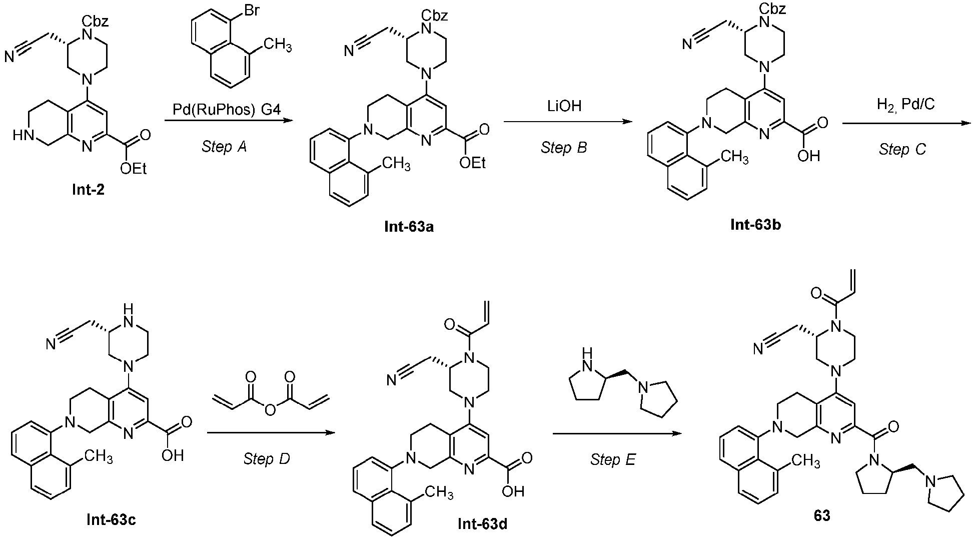

### Step A - Synthesis of ethyl (S)-4-(4-((benzyloxy)carbonyl)-3-(cyanomethyl)piperazin-1-yl)-7-(8-methylnaphthalen-1-yl)-5, 6, 7, 8-tetrahydro-1,7-naphthyridine-2-carboxylate (Int-63a)

To a 40 mL glass vial were added ethyl (S)-4-(4-((benzyloxy)carbonyl)-3-(cyanomethyl)piperazin-1-yl)-5,6, 7,8-tetrahydro-1, 7-naphthyridine-2-carboxylate **(Int-2)** (1.20 g, 2.58 mmol), 1-bromo-8-methylnaphthalene (0.856 g, 3.87 mmol), methanesulfonato(2-dicyclohexylphosphino-2',6'-di-i-propoxy-1,1'-biphenyl)(2'-methylamino-1,1'-biphenyl-2-yl)palladium(II) (219 mg, 0.258 mmol), cesium carbonate (2.94 g, 9.03 mmol), and anhydrous 1,4-dioxane (13 mL). The mixture was degassed by subsurface sparging with nitrogen for 5 minutes, then the vial was sealed under an atmosphere of nitrogen. The mixture was stirred at 100 °C for 2 h 47 min, then filtered through CELITE, rinsing with dichloromethane. The combined filtrate was concentrated to a residue that was purified by column chromatography on silica gel (20% to 60% ethyl acetate in hexane) to afford ethyl (*S*)-4-(4-((benzyloxy)carbonyl)-3-(cyanomethyl)piperazin-1-yl)-7-(8-methylnaphthalen-1-yl)-5,6,7,8-tetrahydro-1,7-naphthyridine-2-carboxylate **(Int-63a).** ¹H NMR (500 MHz, Methanol-*d*₄) Diagnostic signals: δ 7.74 - 7.60 (m, 3H), 7.49 - 7.29 (m, 8H), 7.23 (d, *J* = 7.0 Hz, 1H), 5.28 - 5.12 (m, 2H), 4.82 - 4.71 (m, 1H), 4.45 - 4.34 (m, 3H), 4.25 - 4.13 (m, 1H), 3.98 - 3.85 (m, 1H), 2.90 - 2.84 (m, 3H), 1.42 - 1.36 (m, 3H). Mass Calc'd: 603.3, found 604.3 (M+H)⁺.

### Step B - Synthesis of (S)-4-(4-((benzyloxy)carbonyl)-3-(cyanomethyl)piperazin-1-yl)-7-(8-methylnaphthalen-1-yl)-5,6, 7, 8-tetrahydro-1, 7-naphthyridine-2-carboxylic acid (Int-63b)

To a solution of ethyl (*S*)-4-(4-((benzyloxy)carbonyl)-3-(cyanomethyl)piperazin-1-yl)-7-(8-methylnaphthalen-1-yl)-5,6,7,8-tetrahydro-1,7-naphthyridine-2-carboxylate **(Int-63a)** (10.16 g, 16.84 mmol) in tetrahydrofuran (50 mL) was added methanol (17 mL), water (17 mL), and lithium hydroxide (2.02 g, 84.4 mmol). The mixture was stirred at room temperature for 47 min, then concentrated. To the resulting mixture was added dichloromethane (100 mL) and a solution of potassium hydrogen sulfate (13.75 g, 101 mmol) in water (100 mL). After sonication and thorough mixing, the biphasic layers were separated, the organic layer was washed with brine (50 mL), dried over anhydrous sodium sulfate, filtered and concentrated to afford (*S*)-4-(4-((benzyloxy)carbonyl)-3-(cyanomethyl)piperazin-1-yl)-7-(8-methylnaphthalen-1-yl)-5,6,7,8-tetrahydro-1,7-naphthyridine-2-carboxylic acid **(Int-63b),** which was used without further purification. Mass Calc'd: 575.3, found 576.3 (M+H)⁺.

### Step C - Synthesis of (S)-4-(3-(cyanomethyl)piperazin-1-yl)-7-(8-methylnaphthalen-1-yl)-5, 6,7,8-tetrahydro-1, 7-naphthyridine-2-carboxylic acid (Int-63c)

To a stirred mixture of (*S*)-4-(4-((benzyloxy)carbonyl)-3-(cyanomethyl)piperazin-1-yl)-7-(8-methylnaphthalen-1-yl)-5,6,7,8-tetrahydro-1,7-naphthyridine-2-carboxylic acid **(Int-63b)** (2.00 g, 3.47 mmol) in methanol (35 mL) was added palladium on carbon (10 wt% Pd dry basis, 50% water, 740 mg). The mixture was stirred under 1 atm of hydrogen at 50 °C for 2 h, then cooled to room temperature. To the mixture was added additional palladium on carbon (10 wt% Pd dry basis, 50% water, 740 mg), and a fresh hydrogen atmosphere was introduced above the mixture. The mixture was stirred under 1 atm of hydrogen at 50 °C for 4.5 h. The mixture was then concentrated, and the following was performed three times: the solid was sonicated under dichloromethane (15 mL), then concentrated. In this way was obtained (*S*)-4-(3-(cyanomethyl)piperazin-1-yl)-7-(8-methylnaphthalen-1-yl)-5,6,7,8-tetrahydro-1,7-naphthyridine-2-carboxylic acid **(Int-63c),** which was used directly without further purification. Mass Calc'd: 441.2, found 442.1 (M+H)⁺.

### Step D - Synthesis of (S)-4-(4-acryloyl-3-(cyanomethyl)piperazin-1-yl)-7-(8-methylnaphthalen-1-yl)-5,6,7,8-tetrahydro-1,7-naphthyridine-2-carboxylic acid (Int-63d)

To a stirred mixture of (*S*)-4-(3-(cyanomethyl)piperazin-1-yl)-7-(8-methylnaphthalen-1-yl)-5,6,7,8-tetrahydro-1,7-naphthyridine-2-carboxylic acid **(Int-63c)** (generated from 3.47 mmol of **Int-63b,** as described in Step C above) in anhydrous dichloromethane (70 mL) was added 4-methylmorpholine (1.15 mL, 10.5 mmol), then acrylic anhydride (0.50 mL, 4.3 mmol). The mixture was stirred at room temperature for 1.5 h, then was filtered through celite, and the celite was rinsed with dichloromethane (30 mL). The combined filtrate was washed with a solution of potassium bisulfate (1.89 g, 13.9 mmol) in water (100 mL). The layers were separated, the aqueous layer was extracted with dichloromethane (10 mL), the combined organic layers were washed with water (70 mL), dried over anhydrous sodium sulfate, filtered, and concentrated. The resulting solid was suspended in hexane, and was ground to a powder with a spatula and sonicated under hexane. The mixture was concentrated to afford (*S*)-4-(4-acryloyl-3-(cyanomethyl)piperazin-1-yl)-7-(8-methylnaphthalen-1-yl)-5,6,7,8-tetrahydro-1,7-naphthyridine-2-carboxylic acid **(Int-63d),** which was used without further purification. Mass Calc'd: 495.2, found 496.3 (M+H)⁺.

### Step E - Synthesis of 2-((S)-1-acryloyl-4-(7-(8-methylnaphthalen-1-yl)-2-((R)-2-(pyrrolidin-1-ylmethyl)pyrrolidine-1-carbonyl)-5,6,7,8-tetrahydro-1,7-naphthyridin-4-yl)piperazin-2-yl)acetonitrile (Example 63)

To a solution of (*S*)-4-(4-acryloyl-3-(cyanomethyl)piperazin-1-yl)-7-(8-methylnaphthalen-1-yl)-5,6,7,8-tetrahydro-1,7-naphthyridine-2-carboxylic acid **(Int-63d)** (25 mg, 0.050 mmol) in anhydrous dimethylformamide (0.40 mL) in a 4 mL glass vial ("vial A") was added triethylamine (0.028 mL, 0.20 mmol), then ((6-chloro-1H-benzo[d][1,2,3]triazol-1-yl)oxy)tri(pyrrolidin-1-yl)phosphonium hexafluorophosphate (V) (84.0 mg, 0.151 mmol). The resulting homogeneous solution was transferred to a 4 mL glass vial ("vial B") containing a mixture of (R)-1-(pyrrolidin-2-ylmethyl)pyrrolidine, 2HCl (57.3 mg, 0.252 mmol) and triethylamine (0.070 mL, 0.50 mmol), and a teflon-coated magnetic stirbar. Vial A was rinsed twice with anhydrous dimethylformamide (0.05 mL per rinse), and the rinses were transferred to vial B. The resulting mixture was sonicated briefly, then stirred at room temperature for 47 min. The mixture was then diluted with dimethylsulfoxide (3.0 mL), filtered, and purified directly by RP-HPLC (with ammonium hydroxide modifier) to afford 2-((*S*)-1-acryloyl-4-(7-(8-methylnaphthalen-1-yl)-2-((*R*)-2-(pyrrolidin-1-ylmethyl)pyrrolidine-1-carbonyl)-5,6,7,8-tetrahydro-1,7-naphthyridin-4-yl)piperazin-2-yl)acetonitrile (Example **63**). ¹H NMR (500 MHz, DMSO-*d*₆) Diagnostic signals: δ 7.79 - 7.74 (m, 1H), 7.74 - 7.67 (m, 1H), 7.51 - 7.42 (m, 1H), 7.41 - 7.29 (m, 2H), 7.29 - 7.23 (m, 1H), 7.18 - 7.06 (m, 1H), 6.99 - 6.76 (m, 1H), 6.19 (d, *J =* 16.9 Hz, 1H), 5.78 (d, *J* = 10.2 Hz, 1H). Mass Calc'd: 631.4, found 631.8 (M+H)⁺.

The following compounds were prepared in a similar manner to Example 63.

| **Example No.** | **Structure** | **Compound Name** | **Exact Mass** |
|---|---|---|---|
| **64** | 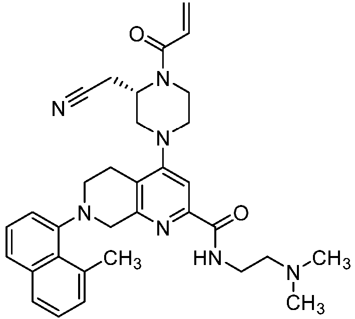 | (*S*)-4-(4-acryloyl-3-(cyanomethyl)piperazin-1-yl)-N-(2-(dimethylamino)ethyl)-7-(8-methylnaphthalen-1-yl)-5,6,7,8-tetrahydro-1,7-naphthyridine-2-carboxamide | Calc'd 565.3, found 566.3 (M+H)⁺ |
| **65** | 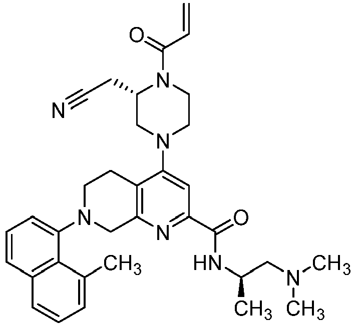 | 4-((*S*)-4-acryloyl-3-(cyanomethyl)piperazin-1-yl)-N-((*R*)-1-(dimethylamino)propan-2-yl)-7-(8-methylnaphthalen-1-yl)-5,6,7,8-tetrahydro-1,7-naphthyridine-2-carboxamide | Calc'd 579.3, found 580.2 (M+H)⁺ |
| **66** | 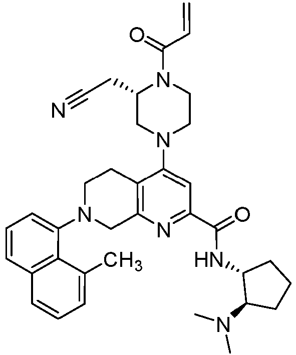 | 4-((*S*)-4-acryloyl-3-(cyanomethyl)piperazin-1-yl)-N-((1*R*,2*R*)-2-(dimethylamino)cyclopent yl)-7-(8-methylnaphthalen-1-yl)-5,6,7,8-tetrahydro-1,7-naphthyridine-2-carboxamide, as a mixture with 4-((*S*)-4-acryloyl-3-(cyanomethyl)piperazin-1-yl)-N-((1*S*,2*S*)-2-(dimethylamino)cyclopent yl)-7-(8-methylnaphthalen-1-yl)-5,6,7,8-tetrahydro-1,7-naphthyridine-2-carboxamide | Calc'd 605.4, found 606.3 (M+H)⁺ |

### Example 67

### Preparation of 4-((S)-4-acryloyl-3-(cyanomethyl)piperazin-1-yl)-N-((R)-1-aminopropan-2-yl)-7-(8-methylnaphthalen-1-yl)-5,6,7,8-tetrahydro-1,7-naphthyridine-2-carboxamide (Example 67)

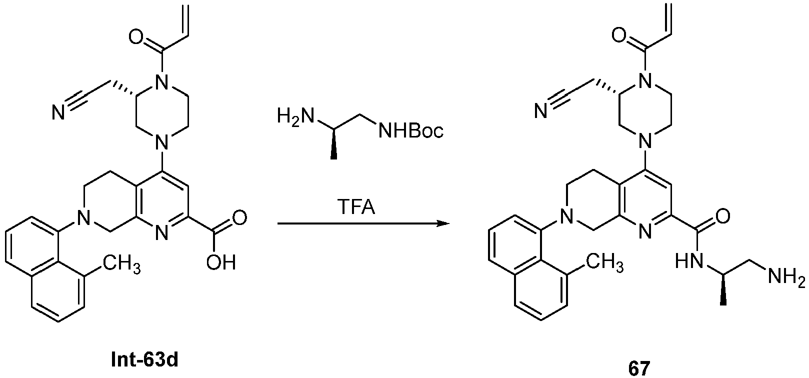

### Step A - Synthesis of 4-((S)-4-acryloyl-3-(cyanomethyl)piperazin-1-yl)-N-((R)-1-aminopropan-2-yl)-7-(8-methylnaphthalen-1-yl)-5,6,7,8-tetrahydro-1,7-naphthyridine-2-carboxamide (Example 67)

To a solution of (*S*)-4-(4-acryloyl-3-(cyanomethyl)piperazin-1-yl)-7-(8-methylnaphthalen-1-yl)-5,6,7,8-tetrahydro-1,7-naphthyridine-2-carboxylic acid **(Int-63d)** (12.4 mg, 0.025 mmol) in anhydrous dichloromethane (0.20 mL) in a 4 mL glass vial ("vial A") was added triethylamine (0.014 mL, 0.10 mmol), then ((6-chloro-1H-benzo[d][1,2,3]triazol-1-yl)oxy)tri(pyrrolidin-1-yl)phosphonium hexafluorophosphate (V) (41.6 mg, 0.075 mmol), then additional anhydrous dichloromethane (0.10 mL). The resulting homogeneous solution was transferred to a 4 mL glass vial ("vial B") containing tert-butyl (R)-(2-aminopropyl)carbamate (21.8 mg, 0.125 mmol) and a teflon-coated magnetic stirbar. Vial A was rinsed with anhydrous dichloromethane (0.05 mL), and the rinse was transferred to vial B. The resulting mixture was sonicated briefly, then stirred at room temperature for 41 min. Trifluoroacetic acid (0.090 mL, 1.2 mmol) was then added. The solution was stirred at room temperature for 5 h, then was allowed to stand at -20 °C for 92 h. The solution was then concentrated, the residue was dissolved in dimethylsulfoxide, and purified directly by RP-HPLC (with ammonium hydroxide modifier) to afford a solid, which was dissolved in dimethylsulfoxide and purified directly by RP-HPLC (with trifluoroacetic acid modifier) to afford 4-((*S*)-4-acryloyl-3-(cyanomethyl)piperazin-1-yl)-N-((*R*)-1-aminopropan-2-yl)-7-(8-methylnaphthalen-1-yl)-5,6,7,8-tetrahydro-1,7-naphthyridine-2-carboxamide (Example **67**). Mass Calc'd: 551.3, found 552.1 (M+H)⁺.

### Example 68

### Preparation of 4-((S)-4-acryloyl-3-(cyanomethyl)piperazin-1-yl)-N-((R)-1-(3,3-difluoroazetidin-1-yl)propan-2-yl)-7-(8-methylnaphthalen-1-yl)-5,6,7,8-tetrahydro-1,7-naphthyridine-2-carboxamide (Example 68)

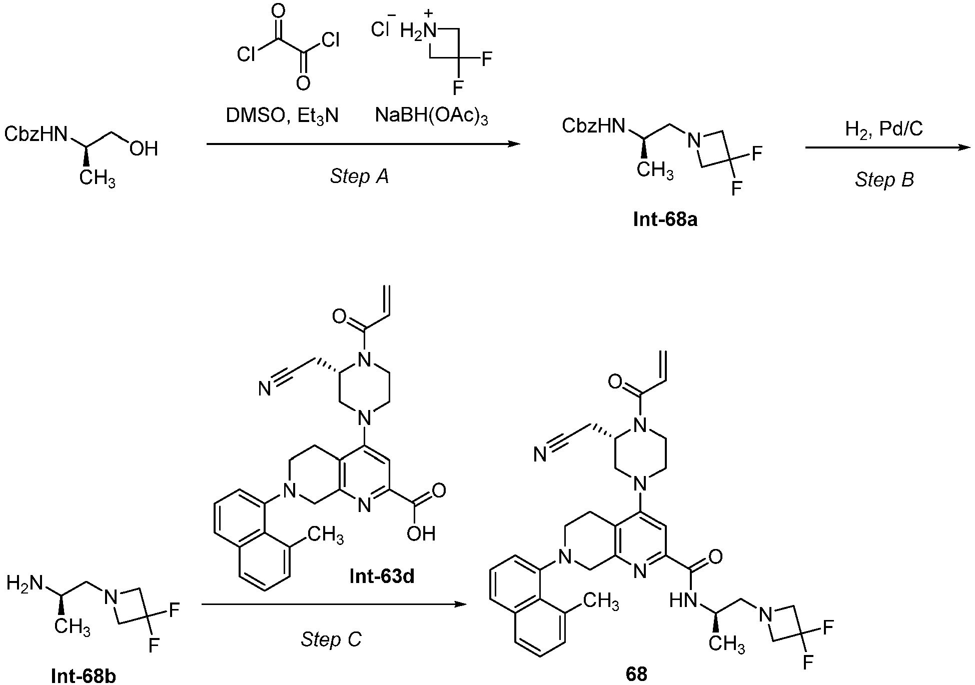

### Step A - Synthesis of benzyl (R)-(1-(3,3-difluoroazetidin-1-yl)propan-2-yl)carbamate (Int-68a)

Into a 500 mL round-bottomed flask, under nitrogen, was added dichloromethane (16 mL), oxalyl dichloride (2.0 M in dichloromethane, 16.6 mL, 33.3 mmol), and the solution was cooled in a dry ice / 2-propanol bath with magnetic stirring. In a 40 mL glass vial ("Vial A"), a solution of dimethylsulfoxide (4.73 mL, 66.6 mmol) in dichloromethane (24 mL) was prepared. The solution from Vial A was added to the reaction mixture, Vial A was rinsed with dichloromethane (8 mL), and this rinse was added to the reaction mixture. In a 100 mL round-bottomed flask ("Flask A"), a solution of benzyl (R)-(1-hydroxypropan-2-yl)carbamate (6.70 g, 32 mmol) in dichloromethane (48 mL) was prepared, then was added to the reaction mixture. Flask A was rinsed with dichloromethane (twice with 8 mL), and these rinses were added to the reaction mixture. Triethylamine (9.81 mL, 70.4 mmol) was then added to the reaction mixture. After 7 min, the reaction mixture was removed from the dry ice bath, and placed in an ice/water bath. After stirring for 1 h, the reaction mixture was removed from the ice/water bath, affording a reaction mixture henceforth referred to as "mixture A". To a 20 mL glass vial was added 3,3-difluoroazetidine hydrochloride (518 mg, 4.00 mmol) and a portion of "mixture A" (19 mL). After mixing, sodium triacetoxyborohydride (1.70 g, 8.00 mmol) was added. After stirring at room temperature for 22 h and 30 min, the reaction mixture was poured into 2M aqueous sodium carbonate. The aqueous layer was extracted with dichloromethane (10 mL), the combined organic layers were dried over anhydrous sodium sulfate, filtered, and concentrated to a residue, which was purified by column chromatography on silica gel (20% to 50% (3:1 ethyl acetate:ethanol v/v) in hexane), to afford benzyl (R)-(1-(3,3-difluoroazetidin-1-yl)propan-2-yl)carbamate **(Int-68a).** Mass Calc'd: 284.1, found 285.1 (M+H)⁺.

### Step B - Synthesis of (R)-1-(3,3-difluoroazetidin-1-yl)propan-2-amine (Int-68b)

To a 40 mL glass vial containing a solution of benzyl (*R*)-(1-(3,3-difluoroazetidin-1-yl)propan-2-yl)carbamate **(Int-68a)** (653 mg, 2.30 mmol) in methanol (20 mL) was added palladium on carbon (10 wt% Pd dry basis, 50% water, 489 mg). The mixture was stirred at room temperature under a balloon of hydrogen gas for 17 h, then was filtered. The filtrate was concentrated to a residue, to which dichloromethane was added. The mixture was concentrated to afford (R)-1-(3,3-difluoroazetidin-1-yl)propan-2-amine **(Int-68b),** which was used without further purification. Mass Calc'd: 150.1, found 151.2 (M+H)⁺.

### Step C - Synthesis of 4-((S)-4-acryloyl-3-(cyanomethyl)piperazin-1-yl)-N-((R)-1-(3, 3-difluoroazetidin-1-yl)propan-2-yl)-7-(8-methylnaphthalen-1-yl)-5, 6,7, 8-tetrahydro-1, 7-naphthyridine-2-carboxamide (Example 68)

To a solution of (*S*)-4-(4-acryloyl-3-(cyanomethyl)piperazin-1-yl)-7-(8-methylnaphthalen-1-yl)-5,6,7,8-tetrahydro-1,7-naphthyridine-2-carboxylic acid **(Int-63d)** (80 mg, 0.16 mmol) in anhydrous *N*,*N*-dimethylformamide (1.4 mL) in a 4 mL glass vial ("Vial A") was added triethylamine (0.034 mL, 0.24 mmol). To the resulting solution was added ((6-chloro-1H-benzo[d][1,2,3]triazol-1-yl)oxy)tri(pyrrolidin-1-yl)phosphonium hexafluorophosphate(V) (107 mg, 0.194 mmol), and the vial contents were mixed to afford a solution. To a separate 4 mL glass vial ("Vial B") was added (*R*)-1-(3,3-difluoroazetidin-1-yl)propan-2-amine **(Int-68b)** (48.5 mg, 0.323 mmol), then the contents of Vial A were added to Vial B. After standing at room temperature for 1 h, the reaction solution was diluted with dimethylsulfoxide (6.0 mL), and then was purified by RP-HPLC (with ammonium hydroxide modifier). The resulting column fractions were concentrated to afford a residue, which was purified by RP-HPLC (with trifluoroacetic acid modifier). The resulting column fractions were neutralized with saturated aqueous sodium bicarbonate, and the aqueous layer was extracted three times with 3:1 chloroform:2-propanol (v/v). The combined organic layers were dried over anhydrous sodium sulfate, filtered, and concentrated, to afford 4-((*S*)-4-acryloyl-3-(cyanomethyl)piperazin-1-yl)-N-((*R*)-1-(3,3-difluoroazetidin-1-yl)propan-2-yl)-7-(8-methylnaphthalen-1-yl)-5,6,7,8-tetrahydro-1,7-naphthyridine-2-carboxamide (Example **68**). ¹H NMR (500 MHz, MeOH-*d*₄) Diagnostic signals: δ 7.74 - 7.58 (m, 3H), 7.46 - 7.39 (m, 1H), 7.39 - 7.29 (m, 2H), 7.27 - 7.21 (m, 1H), 7.01 - 6.74 (m, 1H), 6.30 (d, *J =* 16.4 Hz, 1H), 5.84 (d, *J =* 9.4 Hz, 1H). Mass Calc'd: 627.3, found 628.3 (M+H)⁺.

The following compounds were prepared in a similar manner to Example **68.**

| **Example No.** | **Structure** | **Compound Name** |
|---|---|---|
| **69** | 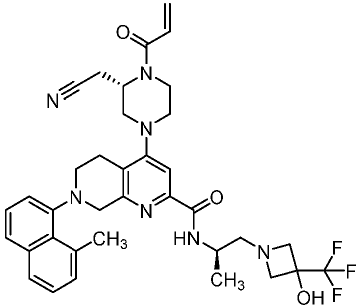 | 4-((*S*)-4-acryloyl-3-(cyanomethyl)piperazin-1-yl)-N-((*R*)-1-(3-hydroxy-3-(trifluoromethyl)azetidin-1-yl)propan-2-yl)-7-(8-methylnaphthalen-1-yl)-5,6,7,8-tetrahydro-1,7-naphthyridine-2-carboxamide |
| **70** | 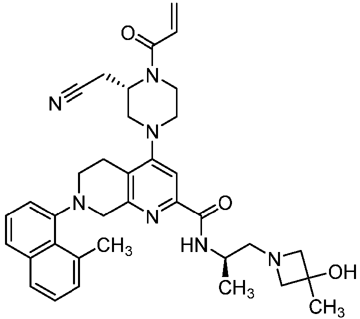 | 4-((*S*)-4-acryloyl-3-(cyanomethyl)piperazin-1-yl)-N-((*R*)-1-(3-hydroxy-3-methylazetidin-1-yl)propan-2-yl)-7-(8-methylnaphthalen-1-yl)-5,6,7,8-tetrahydro-1,7-naphthyridine-2-carboxamide |

### Characterization Data for Examples 69 and 70:

| **Example No.** | **Exact Mass** | **NMR** |
|---|---|---|
| **69** | Calc'd 675.3, found 676.3 (M+H)⁺ | ¹H NMR (500 MHz, MeOH-*d*₄) Diagnostic signals: δ 7.74 - 7.59 (m, 3H), 7.47 - 7.39 (m, 1H), 7.38 - 7.30 (m, 2H), 7.24 (m, 1H), 6.99 - 6.75 (m, 1H), 6.29 (d, *J =* 16.6 Hz, 1H), 5.84 (d, *J =* 10.5 Hz, 1H), 1.24 - 1.16 (m, 3H). |
| **70** | Calc'd 621.3, found 622.3 (M+H)⁺ | ¹H NMR (500 MHz, DMSO-*d*₆) Diagnostic signals: δ 8.30 - 8.16 (m, 1H), 7.77 (d, *J =* 8.1 Hz, 1H), 7.74 - 7.67 (m, 1H), 7.56 - 7.43 (m, 2H), 7.42 - 7.32 (m, 2H), 7.27 (d, *J =* 7.0 Hz, 1H), 7.00 - 6.79 (m, 1H), 6.20 (d, *J =* 16.8 Hz, 1H), 5.80 (dd, *J =* 10.5, 2.1 Hz, 1H), 1.30 - 1.22 (m, 3H), 1.14 - 1.05 (m, 3H). |

### Example 71 and Example 72

### Preparation of 4-((S)-4-acryloyl-3-(cyanomethyl)piperazin-1-yl)-N-((R or S)-1-(3,3-difluoropyrrolidin-1-yl)propan-2-yl)-7-(8-methylnaphthalen-1-yl)-5,6,7,8-tetrahydro-1,7-naphthyridine-2-carboxamide, "Diastereomer A" (Example 71), and 4-((S)-4-acryloyl-3-(cyanomethyl)piperazin-1-yl)-N-((S or R)-1-(3,3-difluoropyrrolidin-1-yl)propan-2-yl)-7-(8-methylnaphthalen-1-yl)-5,6,7,8-tetrahydro-1,7-naphthyridine-2-carboxamide, "Diastereomer B" (Example 72)

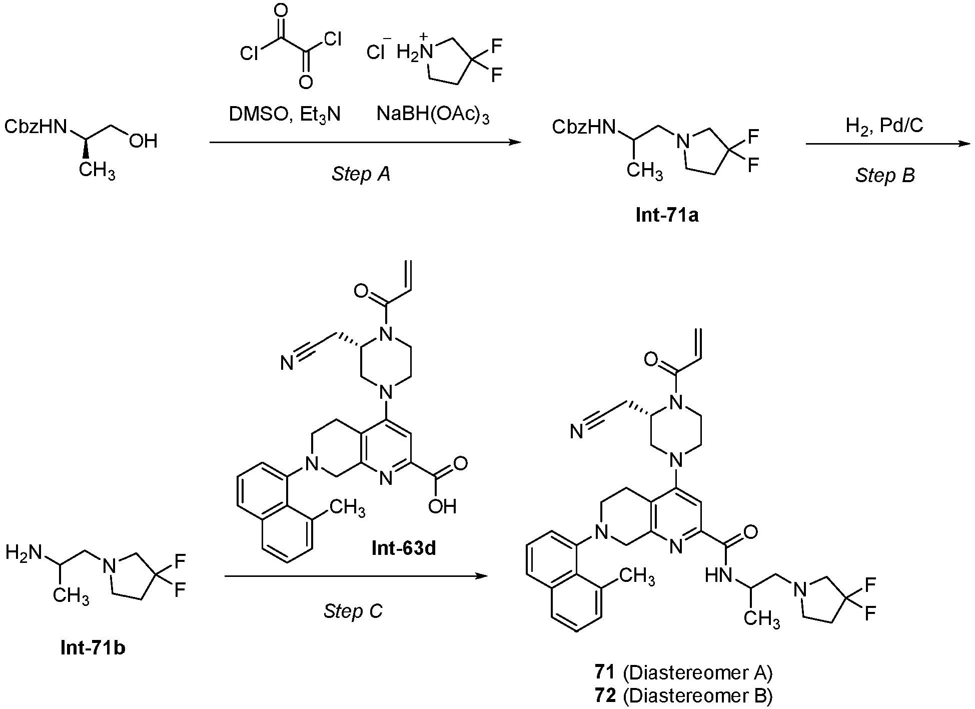

### Step A - Synthesis of benzyl (1-(3,3-difluoropyrrolidin-1-yl)propan-2-yl)carbamate (Int-71a)

To a 20 mL glass vial under nitrogen was added dichloromethane (1.5 mL) and oxalyl dichloride (2.0M in dichloromethane, 0.53 mL, 1.06 mmol). The magnetically-stirred solution was cooled in a dry ice/2-propanol bath, then dimethylsulfoxide (0.078 mL, 1.1 mmol) was added. In a 4 mL glass vial ("Vial A"), a solution of benzyl (*R*)-(1-hydroxypropan-2-yl)carbamate (209 mg, 0.999 mmol) in dichloromethane (2.0 mL) was prepared, and the solution from vial A was then added dropwise to the reaction mixture. Vial A was rinsed with dichloromethane (twice with 0.5 mL), and these rinses were added to the reaction mixture. After stirring the reaction mixture for 15 min in the dry ice/2-propanol bath, triethylamine (0.31 mL, 2.22 mmol) was added. After stirring further for 12 min, the reaction flask was removed from the dry ice/2-propanol bath and allowed to warm to room temperature while surrounded by ambient air. After stirring further for 1 h and 28 min, 3,3-difluoropyrrolidine hydrochloride (143 mg, 0.999 mmol) was added, and the reaction mixture was sonicated for 2 min. After stirring at room temperature for 14 h and 39 min, sodium triacetoxyborohydride (635 mg, 3.00 mmol) was added. The mixture was sonicated for 1 min, then stirred at room temperature for 1 h and 27 min. To the mixture was then added 2 M aqueous sodium carbonate (10 mL). After mixing, the separated aqueous layer was extracted with dichloromethane (5 mL), and the combined organic layers were then dried over anhydrous sodium sulfate, filtered, and concentrated to afford a residue, which was purified by column chromatography on silica gel (0% to 5% methanol in dichloromethane), to afford benzyl (1-(3,3-difluoropyrrolidin-1-yl)propan-2-yl)carbamate **(Int-71a).** Mass Calc'd: 298.2, found 299.2 (M+H)⁺.

### Step B - Synthesis of 1-(3,3-difluoropyrrolidin-1-yl)propan-2-amine (Int-71b)

To a 20 mL glass vial containing benzyl (1-(3,3-difluoropyrrolidin-1-yl)propan-2-yl)carbamate **(Int-71a)** (203 mg, 0.680 mmol) was added methanol (3.4 mL) and palladium on carbon (10 wt% Pd dry basis, 50% water, 145 mg). The reaction mixture was stirred under a balloon of hydrogen gas for 2.5 h, then filtered, and the filtrate was concentrated to afford 1-(3,3-difluoropyrrolidin-1-yl)propan-2-amine **(Int-71b),** which was used without further purification. Mass Calc'd: 164.1, found 165.1 (M+H)⁺.

### Step C - Synthesis of 4-((S)-4-acryloyl-3-(cyanomethyl)piperazin-1-yl)-N-((R or S)-1-(3,3-difluoropyrrolidin-1-yl)propan-2-yl)-7-(8-methylnaphthalen-1-yl)-5,6,7,8-tetrahydro-1,7-naphthyridine-2-carboxamide, "Diastereomer A" (Example 71), and 4-((S)-4-acryloyl-3-(cyanomethyl)piperazin-1-yl)-N-((S or R)-1-(3, 3-difluoropyrrolidin-1-yl)propan-2-yl)-7-(8-methylnaphthalen-1-yl)-5,6,7,8-tetrahydro-1,7-naphthyridine-2-carboxamide, "Diastereomer B" (Example 72)

To a solution of (*S*)-4-(4-acryloyl-3-(cyanomethyl)piperazin-1-yl)-7-(8-methylnaphthalen-1-yl)-5,6,7,8-tetrahydro-1,7-naphthyridine-2-carboxylic acid **(Int-63d)** (120 mg, 0.242 mmol) in anhydrous N,N-dimethylformamide (2.2 mL) in a 4 mL glass vial ("Vial A") was added triethylamine (0.051 mL, 0.37 mmol). To the resulting solution was added ((6-chloro-1H-benzo[d][1,2,3]triazol-1-yl)oxy)tri(pyrrolidin-1-yl)phosphonium hexafluorophosphate(V) (161 mg, 0.291 mmol), and the vial contents were mixed to afford a solution. To a separate 4 mL glass vial ("Vial B") was added 1-(3,3-difluoropyrrolidin-1-yl)propan-2-amine **(Int-71b)** (59.6 mg, 0.363 mmol), then the contents of Vial A were added to Vial B. After standing at room temperature for 1 h, the reaction solution was diluted with dimethylsulfoxide (6.0 mL), and then was purified by RP-HPLC (with ammonium hydroxide modifier). The resulting column fractions were concentrated to afford a residue, which was purified by SFC (column: Daicel Chiralpak IA (21 mm x 250 mm, 5 um); modifier: 2-propanol with 0.1% NH₄OH; percent modifier in CO₂: 40%) to afford the first eluting peak from SFC purification: 4-((*S*)-4-acryloyl-3-(cyanomethyl)piperazin-1-yl)-N-((*R* or *S*)-1-(3,3-difluoropyrrolidin-1-yl)propan-2-yl)-7-(8-methylnaphthalen-1-yl)-5,6,7,8-tetrahydro-1,7-naphthyridine-2-carboxamide, "Diastereomer A" (Example **71**), and the second-eluting peak from SFC purification: 4-((*S*)-4-acryloyl-3-(cyanomethyl)piperazin-1-yl)-N-((*S* or *R*)-1-(3,3-difluoropyrrolidin-1-yl)propan-2-yl)-7-(8-methylnaphthalen-1-yl)-5,6,7,8-tetrahydro-1,7-naphthyridine-2-carboxamide, "Diastereomer B" (Example **72**).
Example **71:** ¹H NMR (500 MHz, DMSO-*d*₆) Diagnostic signals: δ 8.33 - 8.25 (m, 1H), 7.76 (d, *J =* 8.1 Hz, 1H), 7.72 - 7.67 (m, 1H), 7.54 - 7.42 (m, 2H), 7.41 - 7.31 (m, 2H), 7.26 (d, *J =* 6.8 Hz, 1H), 7.00 - 6.78 (m, 1H), 6.20 (d, *J =* 16.6 Hz, 1H), 5.79 (dd, *J =* 10.5, 2.1 Hz, 1H), 4.26 (dd, *J =* 16.4, 6.8 Hz, 1H), 2.23 - 2.10 (m, 2H), 1.16 - 1.09 (m, 3H). Mass Calc'd: 641.3, found 642.3 (M+H)⁺.
Example 72: ¹H NMR (500 MHz, DMSO-*d*₆) Diagnostic signals: δ 8.33 - 8.25 (m, 1H), 7.76 (d, *J =* 8.1 Hz, 1H), 7.72 - 7.67 (m, 1H), 7.55 - 7.43 (m, 2H), 7.40 - 7.32 (m, 2H), 7.26 (d, *J =* 7.0 Hz, 1H), 6.99 - 6.78 (m, 1H), 6.20 (d, *J =* 16.6 Hz, 1H), 5.79 (dd, *J =* 10.5, 2.1 Hz, 1H), 4.26 (dd, *J =* 16.3, 8.1 Hz, 1H), 2.23 - 2.10 (m, 2H), 1.17 - 1.09 (m, 3H). Mass Calc'd: 641.3, found 642.3 (M+H)⁺.

### Example 73

### Preparation of 4-((S)-4-acryloyl-3-(cyanomethyl)piperazin-1-yl)-N-((R or S)-1-((R or S)-3-hydroxy-3-methylpyrrolidin-1-yl)propan-2-yl)-7-(8-methylnaphthalen-1-yl)-5,6,7,8-tetrahydro-1,7-naphthyridine-2-carboxamide, "Diastereomer A" (Example 73)

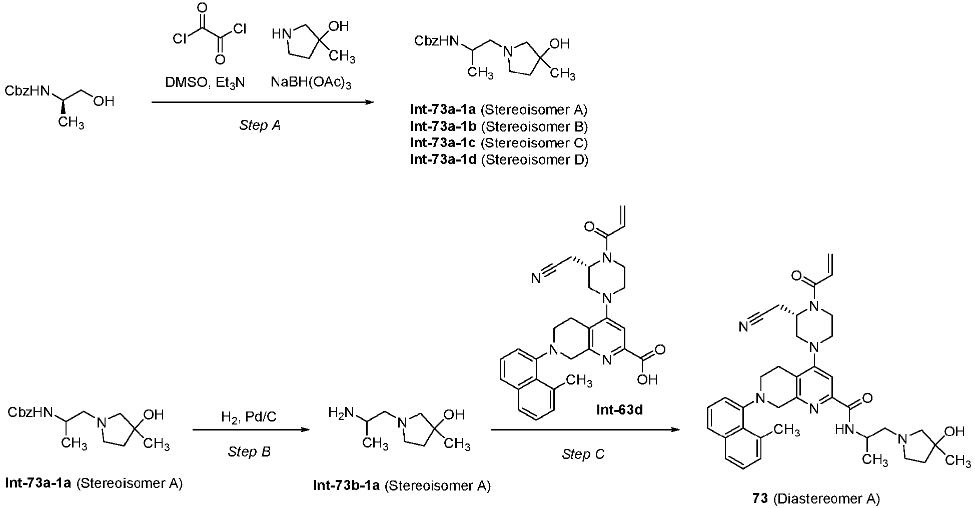

### Step A - Synthesis of benzyl ((R or S)-1-((R or S)-3-hydroxy-3-methylpyrrolidin-1-yl)propan-2-yl)carbamate, "Stereoisomer A" (Int-73a-1a), and ((S or R)-1-((R or S)-3-hydroxy-3-methylpyrrolidin-1-yl)propan-2-yl)carbamate, "Stereoisomer B" (Int-73a-1b), and ((R or S)-1-((S or R)-3-hydroxy-3-methylpyrrolidin-1-yl)propan-2-yl)carbamate, "Stereoisomer C" (Int-73a-1c), and ((S or R)-1-((S or R)-3-hydroxy-3-methylpyrrolidin-1-yl)propan-2-yl)carbamate, "Stereoisomer D" (Int-73a-1d)

To a 40 mL glass vial ("Vial A") was added dichloromethane (2 mL) and oxalyl dichloride (2.0M in dichloromethane, 2.04 mL, 4.08 mmol). The magnetically-stirred solution was cooled in a dry ice/2-propanol bath. In a 4 mL glass vial ("Vial B"), a solution of dimethylsulfoxide (0.58 mL, 8.2 mmol) in dichloromethane (3 mL) was prepared. The solution from Vial B was added to Vial A dropwise, then Vial B was rinsed with dichloromethane (twice with 0.5 mL), and the rinses were added to Vial A. The reaction mixture was then stirred for 30 min in the dry ice/2-propanol bath. In a 20 mL glass vial ("Vial C"), a solution of benzyl (R)-(1-hydroxypropan-2-yl)carbamate (836 mg, 4.00 mmol) in dichloromethane (6.0 mL) was prepared. The solution from Vial C was added to Vial A, then Vial C was rinsed with dichloromethane (twice with 1.0 mL), and the rinses were added to Vial A. The reaction mixture was then stirred for 30 min in the dry ice/2-propanol bath. Triethylamine (1.22 mL, 8.75 mmol) was then added, and the reaction mixture in Vial A was then removed from the dry ice/2-propanol bath and allowed to warm to room temperature. The mixture was then stirred for 1 h, and 3-methylpyrrolidin-3-ol (404 mg, 4.00 mmol) was added. After mixing and sonicating the reaction mixture, sodium triacetoxyborohydride (1.69 g, 7.99 mmol) was added. The reaction mixture was stirred at room temperature for 30 min, then was allowed to stand at -20 °C overnight. After warming to room temperature, 2M aqueous sodium carbonate (20 mL) was added. After mixing and separating the layers, the aqueous layer was extracted with dichloromethane (10 mL), the combined organic layers were dried over sodium sulfate, filtered and concentrated to a residue that was purified by column chromatography on silica gel (0% to 80% (3: 1 ethyl acetate:ethanol, v/v) in hexane, to afford a residue that was further purified by SFC: (column: Daicel Chiralpak IG (21 mm x 250 mm, 5 um); modifier: methanol with 0.1% NH₄OH; percent modifier in CO₂: 20%), to afford the first-eluting peak from SFC purification, "Residue A", and the second-eluting peak from SFC purification, benzyl ((*R* or *S*)-1-((*R* or *S*)-3-hydroxy-3-methylpyrrolidin-1-yl)propan-2-yl)carbamate, "Stereoisomer A" (**Int-73a-1a**), and the third-eluting peak from SFC purification, ((*S* or *R*)-1-((*R* or *S*)-3-hydroxy-3-methylpyrrolidin-1-yl)propan-2-yl)carbamate, "Stereoisomer B" **(Int-73a-1b).** "Residue A" was then further purified by SFC: (column: ES Industries ChromegaChiral CC4 (21mm x 250mm, 5 um); modifier: methanol with 0.1% NH₄OH; percent modifier in CO₂: 10%), to afford the first-eluting peak from SFC purification, ((*R* or *S*)-1-((*S* or *R*)-3-hydroxy-3-methylpyrrolidin-1-yl)propan-2-yl)carbamate, "Stereoisomer C" **(Int-73a-1c),** and the second-eluting peak from SFC purification, ((*S* or *R*)-1-((*S* or *R*)-3-hydroxy-3-methylpyrrolidin-1-yl)propan-2-yl)carbamate, "Stereoisomer D" **(Int-73a-1d).**
**Int-73a-1a:** Mass Calc'd: 292.2, found 293.2 (M+H)⁺.
**Int-73a-1b:** Mass Calc'd: 292.2, found 293.2 (M+H)⁺.
**Int-73a-1c:** Mass Calc'd: 292.2, found 293.2 (M+H)⁺.
**Int-73a-1d:** Mass Calc'd: 292.2, found 293.2 (M+H)⁺.

### Step B - Synthesis of (R or S)-1-((R or S)-2-aminopropyl)-3-methylpyrrolidin-3-ol, "Stereoisomer A" (Int-73b-1a)

To a solution of benzyl ((*R* or *S*)-1-((*R* or *S*)-3-hydroxy-3-methylpyrrolidin-1-yl)propan-2-yl)carbamate, "Stereoisomer A" (**Int-73a-1a**) (188.6 mg, 0.6450 mmol) in methanol (6.5 mL) was added palladium on carbon (10 wt% Pd dry basis, 50% water, 137 mg). The reaction mixture was stirred under a balloon of hydrogen gas for 17 h and 45 min, then filtered, and the filtrate was concentrated. The residue was dissolved in dichloromethane, and the solution was concentrated, to afford (*R* or *S*)-1-((*R* or *S*)-2-aminopropyl)-3-methylpyrrolidin-3-ol, "Stereoisomer A" (**Int-73b-1a**), which was used without further purification. Mass Calc'd: 158.1, found 159.2 (M+H)⁺.

### Step C - Synthesis of 4-((S)-4-acryloyl-3-(cyanomethyl)piperazin-1-yl)-N-((R or S)-1-((R or S)-3-hydroxy-3-methylpyrrolidin-1-yl)propan-2-yl)-7-(8-methylnaphthalen-1-yl)-5,6,7,8-tetrahydro-1,7-naphthyridine-2-carboxamide, "Diastereomer A" (Example 73)

To a solution of (*S*)-4-(4-acryloyl-3-(cyanomethyl)piperazin-1-yl)-7-(8-methylnaphthalen-1-yl)-5,6,7,8-tetrahydro-1,7-naphthyridine-2-carboxylic acid **(Int-63d)** (80 mg, 0.16 mmol) in anhydrous *N*,*N*-dimethylformamide (1.01 mL) in a 4 mL glass vial ("Vial A") was added triethylamine (0.034 mL, 0.24 mmol). To the resulting solution was added ((6-chloro-1H-benzo[d][1,2,3]triazol-1-yl)oxy)tri(pyrrolidin-1-yl)phosphonium hexafluorophosphate(V) (107 mg, 0.194 mmol), and the vial contents were mixed to afford a solution. In a separate 4 mL glass vial ("Vial B") was prepared a solution of 1-(2-aminopropyl)-3-methylpyrrolidin-3-ol, "Stereoisomer A" (**Int-73b-1a**) (37.3 mg, 0.210 mmol) in anhydrous N,N-dimethylformamide (0.20 mL), then the solution from Vial B was added to Vial A, and Vial B was rinsed with anhydrous N,N-dimethylformamide (twice with 0.20 mL), and the rinses were added to Vial A. The resulting homogeneous solution in Vial A was allowed to stand at room temperature for 1 h and 8 min, then was filtered and diluted with DMSO (6 mL), and the filtrate was purified by RP-HPLC (with ammonium hydroxide modifier) to afford 4-((*S*)-4-acryloyl-3-(cyanomethyl)piperazin-1-yl)-N-((*R* or *S*)-1-((*R* or *S*)-3-hydroxy-3-methylpyrrolidin-1-yl)propan-2-yl)-7-(8-methylnaphthalen-1-yl)-5,6,7,8-tetrahydro-1,7-naphthyridine-2-carboxamide, "Diastereomer A" (Example **73**). ¹H NMR (500 MHz, DMSO-*d*₆) Diagnostic signals: δ 8.32 - 8.23 (m, 1H), 7.77 (d, *J =* 8.0 Hz, 1H), 7.72 - 7.67 (m, 1H), 7.55 - 7.42 (m, 2H), 7.42 - 7.31 (m, 2H), 7.29 - 7.21 (m, 1H), 7.00 - 6.77 (m, 1H), 6.20 (d, *J =* 16.9 Hz, 1H), 5.79 (dd, *J =* 10.5, 2.1 Hz, 1H), 4.44 (d, *J* = 8.6 Hz, 1H), 4.24 (t, *J =* 16.5 Hz, 1H), 1.70 - 1.53 (m, 2H), 1.21 - 1.07 (m, 6H). Mass Calc'd: 635.4, found 636.4 (M+H)⁺.

The following compounds were prepared from the indicated intermediates, in a similar manner to Example **73.**

| **Example No.** | **Structure** | **Compound Name** | **Intermediate Name** |
|---|---|---|---|
| **74** | 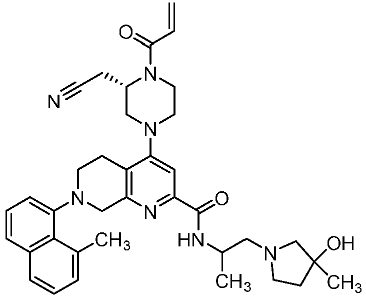 | 4-((*S*)-4-acryloyl-3-(cyanomethyl)piperazi n-1-yl)-N-((*S* or R)-1-((*R* or *S*)-3-hydroxy-3-methylpyrrolidin-1-yl)propan-2-yl)-7-(8-methylnaphthalen-1-yl)-5,6,7,8-tetrahydro-1,7-naphthyridine-2-carboxamide, "Diastereomer B" | ((*S* or *R*)-1-((*R* or *S*)-3-hydroxy-3-methylpyrrolidi n-1-yl)propan-2-yl)carbamate, "Stereoisomer B" **(Int-73a-1b)** |
| **75** | 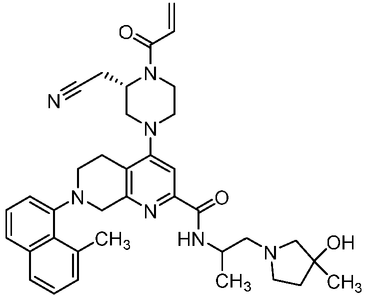 | 4-((*S*)-4-acryloyl-3-(cyanomethyl)piperazi n-1-yl)-N-((*R* or *S*)-1-((*S* or *R*)-3-hydroxy-3-methylpyrrolidin-1-yl)propan-2-yl)-7-(8-methylnaphthalen-1-yl)-5,6,7,8-tetrahydro-1,7-naphthyridine-2-carboxamide, "Diastereomer C" | ((*R* or *S*)-1-((*S* or *R*)-3-hydroxy-3-methylpyrrolidi n-1-yl)propan-2-yl)carbamate, "Stereoisomer C" **(Int-73a-1c)** |
| **76** | 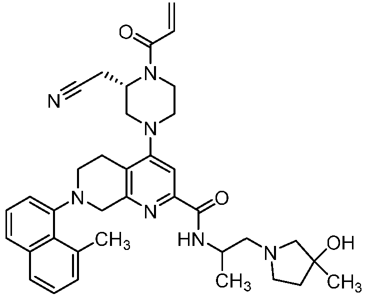 | 4-((*S*)-4-acryloyl-3-(cyanomethyl)piperazi n-1-yl)-N-((*S* or *R*)-1-((*S* or *R*)-3-hydroxy-3-methylpyrrolidin-1-yl)propan-2-yl)-7-(8-methylnaphthalen-1-yl)-5,6,7,8-tetrahydro-1,7-naphthyridine-2-carboxamide, "Diastereomer D" | ((*S* or *R*)-1-((*S* or *R*)-3-hydroxy-3-methylpyrrolidi n-1-yl)propan-2-yl)carbamate, "Stereoisomer D" **(Int-73a-1d)** |

### Characterization Data for Examples 74 - 76:

| **Example No.** | **Exact Mass** | **NMR** |
|---|---|---|
| **74** | Calc'd 635.4, found 636.4 (M+H)⁺ | ¹H NMR (500 MHz, DMSO-*d*₆) Diagnostic signals: δ 8.34 - 8.22 (m, 1H), 7.76 (d, *J =* 8.1 Hz, 1H), 7.73 - 7.66 (m, 1H), 7.56 - 7.42 (m, 2H), 7.41 - 7.31 (m, 2H), 7.30 - 7.22 (m, 1H), 7.00 - 6.77 (m, 1H), 6.20 (d, *J =* 16.8 Hz, 1H), 5.79 (dd, *J =* 10.5, 2.1 Hz, 1H), 4.42 (d, *J =* 10.7 Hz, 1H), 4.24 (dd, *J =* 16.4, 3.1 Hz, 1H), 1.71 - 1.54 (m, 2H), 1.20 - 1.08 (m, 6H). |
| **75** | Calc'd 635.4, found 636.3 (M+H)⁺ | ¹H NMR (500 MHz, DMSO-*d*₆) Diagnostic signals: δ 8.33 - 8.21 (m, 1H), 7.77 (d, *J =* 8.2 Hz, 1H), 7.73 - 7.66 (m, 1H), 7.54 - 7.41 (m, 2H), 7.41 - 7.31 (m, 2H), 7.29 - 7.22 (m, 1H), 7.00 - 6.77 (m, 1H), 6.20 (d, *J =* 16.7 Hz, 1H), 5.79 (dd, *J =* 10.5, 2.2 Hz, 1H), 4.44 (d, *J =* 12.8 Hz, 1H), 4.24 (d, *J =* 16.6 Hz, 1H), 1.70 - 1.54 (m, 2H), 1.20 - 1.08 (m, 6H). |
| **76** | Calc'd 635.4, found 636.3 (M+H)⁺ | ¹H NMR (500 MHz, DMSO-*d*₆) Diagnostic signals: δ 8.32 - 8.22 (m, 1H), 7.76 (d, *J =* 8.1 Hz, 1H), 7.73 - 7.66 (m, 1H), 7.54 - 7.42 (m, 2H), 7.40 - 7.32 (m, 2H), 7.29 - 7.22 (m, 1H), 7.00 - 6.77 (m, 1H), 6.20 (d, *J =* 16.8 Hz, 1H), 5.79 (dd, *J =* 10.5, 2.1 Hz, 1H), 4.43 (d, *J =* 4.7 Hz, 1H), 4.24 (dd, *J =* 16.5, 10.9 Hz, 1H), 1.70 - 1.54 (m, 2H), 1.20 - 1.08 (m, 6H). |

### Example 77

### Preparation of 4-((S)-3-(cyanomethyl)-4-(2-fluoroacryloyl)piperazin-1-yl)-N-((R or S)-1-((S or R)-3-hydroxy-3-methylpyrrolidin-1-yl)propan-2-yl)-7-(8-methylnaphthalen-1-yl)-5,6,7,8-tetrahydro-1,7-naphthyridine-2-carboxamide, "Diastereomer B" (Example 77)

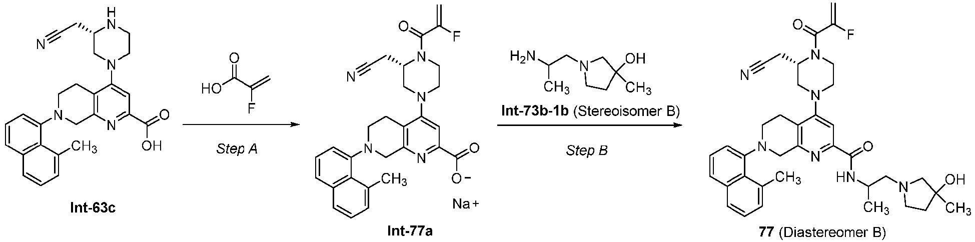

### Step A - Synthesis of sodium (S)-4-(3-(cyanomethyl)-4-(2-fluoroacryloyl)piperazin-1-yl)-7-(8-methylnaphthalen-1-yl)-5,6,7,8-tetrahydro-1,7-naphthyridine-2-carboxylate (Int-77a)

To a 40 mL glass vial ("Vial A") was added 2-fluoroacrylic acid (510 mg, 5.66 mmol), dichloromethane (17.5 mL), and N-(3-dimethylaminopropyl)-N'-ethylcarbodiimide hydrochloride (543 mg, 2.83 mmol). The mixture in Vial A was then sonicated for 3 min, and stirred at room temperature for 12 min. To a 250 mL round-bottomed flask ("Flask A") was added (*S*)-4-(3-(cyanomethyl)piperazin-1-yl)-7-(8-methylnaphthalen-1-yl)-5,6,7,8-tetrahydro-1,7-naphthyridine-2-carboxylic acid **(Int-63c)** (1.00 g, 2.27 mmol), dichloromethane (22.5 mL), and 4-methylmorpholine (1.00 mL, 9.06 mmol). While magnetically stirring the contents of Flask A at room temperature, the contents of Vial A were added dropwise to Flask A over 7 min. Vial A was rinsed with dichloromethane (twice with 2.5 mL), and the rinses were added to Flask A. The reaction mixture was stirred at room temperature for 24 min, then water (45 mL) was added, and the mixture was mixed thoroughly in a separatory funnel. The separated aqueous layer was extracted with dichloromethane (5 mL). The combined organic layers were dried over sodium sulfate, filtered, and the filtrate was concentrated to afford a residue, which was purified by RP-HPLC (with trifluoroacetic acid modifier). The combined fractions obtained from RP-HPLC purification (150 mL) were diluted with 3:1 chloroform:2-propanol v/v (40 mL), water (40 mL), and saturated aqueous sodium bicarbonate (40 mL). After mixing thoroughly, the layers were separated, and the aqueous layer was extracted with 3:1 chloroform:2-propanol v/v (three times with 40 mL, then six times with 20 mL, then with 35 mL, then twice with 20 mL). The combined organic layers were dried over sodium sulfate, filtered, and concentrated to afford a residue. The residue was suspended in dichloromethane and filtered, and the filtrate was concentrated to afford sodium (*S*)-4-(3-(cyanomethyl)-4-(2-fluoroacryloyl)piperazin-1-yl)-7-(8-methylnaphthalen-1-yl)-5,6,7,8-tetrahydro-1,7-naphthyridine-2-carboxylate **(Int-77a).** Mass Calc'd: 513.2, found 514.2 (M+H)⁺.

### Step B - Synthesis of 4-((S)-3-(cyanomethyl)-4-(2-fluoroacryloyl)piperazin-1-yl)-N-((R or S)-1-((S or R)-3-hydroxy-3-methylpyrrolidin-1-yl)propan-2-yl)-7-(8-methylnaphthalen-1-yl)-5, 6,7,8-tetrahydro-1,7-naphthyridine-2-carboxamide, "Diastereomer B" (Example 77)

To a solution of sodium (*S*)-4-(3-(cyanomethyl)-4-(2-fluoroacryloyl)piperazin-1-yl)-7-(8-methylnaphthalen-1-yl)-5,6,7,8-tetrahydro-1,7-naphthyridine-2-carboxylate **(Int-77a)** (40 mg, 0.075 mmol) in anhydrous *N*,*N*-dimethylformamide (0.50 mL) in a 4 mL glass vial ("Vial A") was added triethylamine (0.016 mL, 0.12 mmol). To the resulting solution was added ((6-chloro-1H-benzo[d][1,2,3]triazol-1-yl)oxy)tri(pyrrolidin-1-yl)phosphonium hexafluorophosphate(V) (49.7 mg, 0.0896 mmol), and the vial contents were mixed to afford a solution. In a separate 4 mL glass vial ("Vial B") was prepared a solution of (*S* or *R*)-1-((*R* or *S*)-2-aminopropyl)-3-methylpyrrolidin-3-ol, "Stereoisomer B" **(Int-73b-1b,** which was prepared analogously to **Int-73b-1a**, starting from **Int-73a-1b)** (15.4 mg, 0.0973 mmol) in anhydrous *N*,*N-*dimethylformamide (0.10 mL), then the solution from Vial B was added to Vial A, and Vial B was rinsed with anhydrous N,N-dimethylformamide (three times with 0.05 mL), and the rinses were added to Vial A. The reaction mixture in Vial A was stirred at room temperature for 34 min, then was diluted with DMSO (2 mL) and filtered, and the filter was rinsed with DMSO (1 mL). The filtrates were combined and purified by RP-HPLC (with ammonium hydroxide modifier) to afford 4-((*S*)-3-(cyanomethyl)-4-(2-fluoroacryloyl)piperazin-1-yl)-*N*-((*R* or*S*)-1-((*S* or *R*)-3-hydroxy-3-methylpyrrolidin-1-yl)propan-2-yl)-7-(8-methylnaphthalen-1-yl)-5,6,7,8-tetrahydro-1,7-naphthyridine-2-carboxamide, "Diastereomer B" (Example 77). ¹H NMR (500 MHz, DMSO-*d*₆) Diagnostic signals: δ 8.34 - 8.22 (m, 1H), 7.76 (d, *J =* 8.2 Hz, 1H), 7.73 - 7.66 (m, 1H), 7.55 - 7.43 (m, 2H), 7.41 - 7.32 (m, 2H), 7.29 - 7.23 (m, 1H), 5.41 (dd, *J =* 18.0, 4.1 Hz, 1H), 5.29 (d, *J* = 50.2 Hz, 1H), 4.42 (d, *J* = 10.4 Hz, 1H), 4.24 (d, *J* = 16.8 Hz, 1H), 1.71 - 1.54 (m, 2H), 1.20 - 1.08 (m, 6H). Mass Calc'd: 653.4, found 654.3 (M+H)⁺.

The following compounds were prepared in a similar manner to Example **77.**

| **Example No.** | **Structure** | **Compound Name** |
|---|---|---|
| **78** | 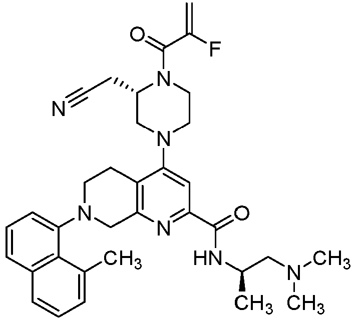 | 4-((*S*)-3-(cyanomethyl)-4-(2-fluoroacryloyl)piperazin-1-yl)-N-((*R*)-1-(dimethylamino)propan-2-yl)-7-(8-methylnaphthalen-1-yl)-5,6,7,8-tetrahydro-1,7-naphthyridine-2-carboxamide |
| **79** | 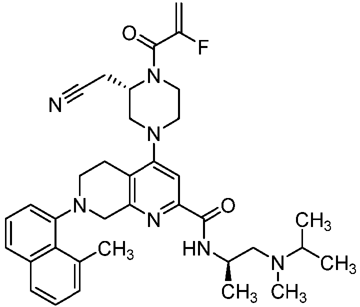 | 4-((*S*)-3-(cyanomethyl)-4-(2-fluoroacryloyl)piperazin-1-yl)-N-((*R*)-1-(isopropyl(methyl)amino)propan-2-yl)-7-(8-methylnaphthalen-1-yl)-5,6,7,8-tetrahydro-1,7-naphthyridine-2-carboxamide |

### Characterization Data for Examples 78 and 79:

| **Example No.** | **Exact Mass** | **NMR** |
|---|---|---|
| **78** | Calc'd 597.3, found 598.3 (M+H)⁺ | ¹H NMR (500 MHz, DMSO-*d*₆) Diagnostic signals: δ 8.25 - 8.15 (m, 1H), 7.77 (d, *J =* 8.1 Hz, 1H), 7.73 - 7.66 (m, 1H), 7.56 - 7.42 (m, 2H), 7.40 - 7.33 (m, 2H), 7.30 - 7.23 (m, 1H), 5.41 (dd, *J =* 18.0, 4.1 Hz, 1H), 5.29 (d, *J =* 50.1 Hz, 1H), 4.24 (dd, *J =* 16.7, 7.0 Hz, 1H), 2.84 (s, 3H), 2.12 (s, 3H), 2.10 (s, 3H), 1.11 (d, *J =* 6.5 Hz, 3H). |
| **79** | Calc'd 625.3, found 626.3 (M+H)⁺ | ¹H NMR (500 MHz, DMSO-*d*₆) Diagnostic signals: δ 8.27 - 8.17 (m, 1H), 7.77 (d, *J =* 8.2 Hz, 1H), 7.73 - 7.67 (m, 1H), 7.56 - 7.43 (m, 2H), 7.41 - 7.31 (m, 2H), 7.29 - 7.22 (m, 1H), 5.41 (dd, *J =* 17.9, 4.1 Hz, 1H), 5.29 (d, *J* = 49.4 Hz, 1H), 4.22 (d, *J =* 16.6 Hz, 1H), 2.87 - 2.81 (m, 3H), 2.16 - 2.08 (m, 3H), 1.13 (d, *J =* 6.5 Hz, 3H), 0.92 - 0.83 (m, 6H). |

The following compounds were prepared in a similar manner to Example **61,** but starting from **Int-63d** instead of **Int-60d.**

| **Example No.** | **Structure** | **Compound Name** | **Exact Mass** |
|---|---|---|---|
| **80** | 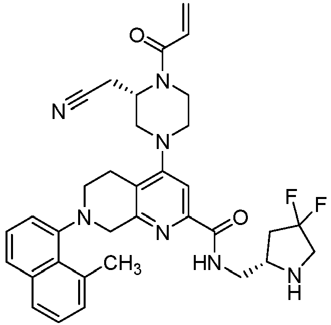 | 4-((*S*)-4-acryloyl-3-(cyanomethyl)piperazin-1-yl)-N-(((*S*)-4,4-difluoropyrrolidin-2-yl)methyl)-7-(8-methylnaphthalen-1-yl)-5,6,7,8-tetrahydro-1,7-naphthyridine-2-carboxamide | Calc'd 613.3, found 614.3 (M+H)⁺ |
| **81** | 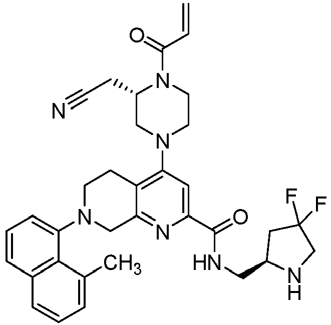 | 4-((*S*)-4-acryloyl-3-(cyanomethyl)piperazin-1-yl)-N-(((*R*)-4,4-difluoropyrrolidin-2-yl)methyl)-7-(8-methylnaphthalen-1-yl)-5,6,7,8-tetrahydro-1,7-naphthyridine-2-carboxamide | Calc'd 613.3, found 614.3 (M+H)⁺ |

The following compound was prepared in a similar manner to Example **63.**

| **Example No.** | **Structure** | **Compound Name** | **Exact Mass** |
|---|---|---|---|
| **82** | 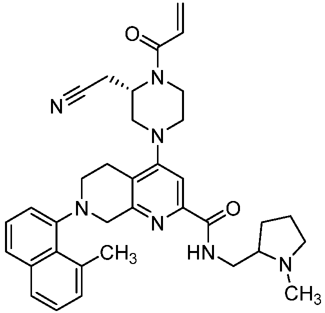 | 4-((*S*)-4-acryloyl-3-(cyanomethyl)piperazin-1-yl)-7-(8-methylnaphthalen-1-yl)-N-(((*R*)-1-methylpyrrolidin-2-yl)methyl)-5,6,7,8-tetrahydro-1,7-naphthyridine-2-carboxamide, as a mixture with 4-((*S*)-4-acryloyl-3-(cyanomethyl)piperazin-1-yl)-7-(8-methylnaphthalen-1-yl)-N-(((*S*)-1-methylpyrrolidin-2-yl)methyl)-5,6,7,8-tetrahydro-1,7-naphthyridine-2-carboxamide | Calc'd 591.3, found 592.6 (M+H)⁺ |

### Example 83

### Preparation of (R)-4-(4-acryloylpiperazin-1-yl)-7-(5-chloro-6-methyl-1H-indazol-4-yl)-N-(1-(dimethylamino)propan-2-yl)-5,6,7,8-tetrahydro-1,7-naphthyridine-2-carboxamide (Example 83)

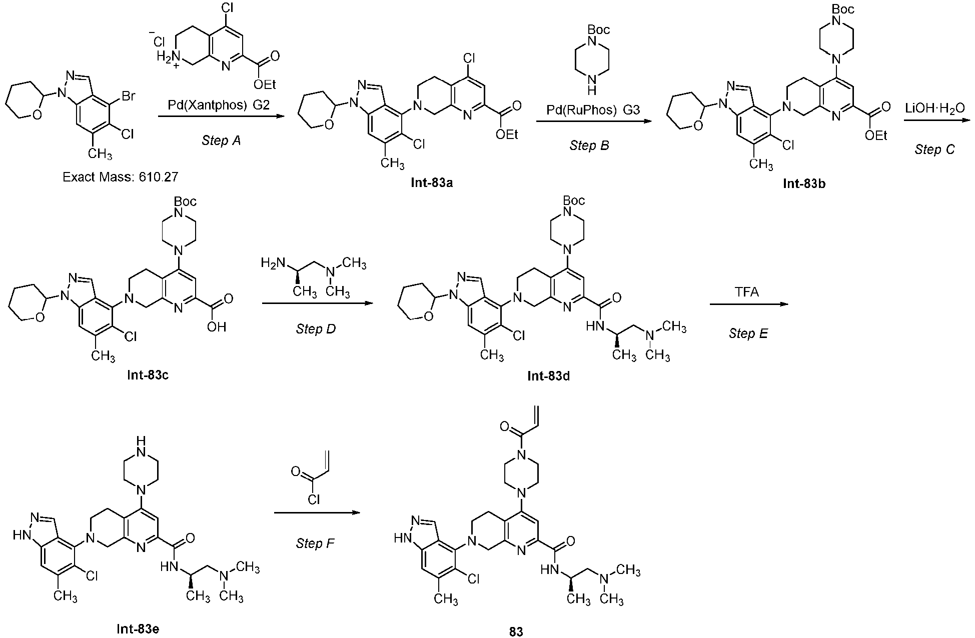

### Step A - Synthesis of ethyl 4-chloro-7-(5-chloro-6-methyl-1-(tetrahydro-2H-pyran-2-yl)-1H-indazol-4-yl)-5,6,7,8-tetrahydro-1,7-naphthyridine-2-carboxylate (Int-83a)

To a mixture of ethyl 4-chloro-5,6,7,8-tetrahydro-1,7-naphthyridine-2-carboxylate, HCl salt (500 mg, 1.80 mmol, 1.0 equiv.), 4-bromo-5-chloro-6-methyl-1-(tetrahydro-2*H*-pyran-2-yl)-1H-indazole (1.5 equiv.), and Cs₂CO₃ (4.0 equiv.) in toluene (15 mL) was added chloro[(4,5-bis(diphenylphosphino)-9,9-dimethylxanthene)-2-(2'-amino-1,1'-biphenyl)]palladium(II) (Pd(Xantphos) G2) (0.10 equiv.), and the mixture was degassed with nitrogen, and the mixture was stirred at 110 °C for 2 h. The reaction mixture was diluted with EtOAc and washed with water, and the aqueous layer was extracted with EtOAc. The combined organic layers were dried over anhydrous sodium sulfate, filtered and concentrated. The residue was purified by column chromatography on silica gel (0 to 30% EtOAc in petroleum ether) to afford ethyl 4-chloro-7-(5-chloro-6-methyl-1-(tetrahydro-2H-pyran-2-yl)-1H-indazol-4-yl)-5,6,7,8-tetrahydro-1,7-naphthyridine-2-carboxylate **(Int-83a).** Mass Calc'd: 488.1, found 489.0 (M+H)⁺.

### Step B - Synthesis of ethyl 4-(4-(tert-butoxycarbonyl)piperazin-1-yl)-7-(5-chloro-6-methyl-1-(tetrahydro-2H-pyran-2-yl)-1H-indazol-4-yl)-5,6,7,8-tetrahydro-1,7-naphthyridine-2-carboxylate (Int-83b)

To a stirred solution of ethyl 4-chloro-7-(5-chloro-6-methyl-1-(tetrahydro-2H-pyran-2-yl)-1H-indazol-4-yl)-5,6,7,8-tetrahydro-1,7-naphthyridine-2-carboxylate **(Int-83a)** (0.1 g, 0.2 mmol, 1.0 equiv.) in 1,4-dioxane (2.5 mL) was added tert-butyl piperazine-1-carboxylate (3.0 equiv.), cesium carbonate (3.0 equiv.) and (2-dicyclohexylphosphino-2,6'-diisopropoxy-1,1'-biphenyl)[2-(2'-amino-1,1'-biphenyl)]palladium(II) methanesulfonate (Pd(RuPhos) G3) (0.10 equiv.) at 20 °C, and the mixture was stirred at 70 °C for 16 h under an atmosphere of nitrogen. The reaction mixture was filtered and the filtrate was purified by preparative TLC plate (SiO₂, Pet. Ether / EtOAc = 3/1) to give ethyl 4-(4-(tert-butoxycarbonyl)piperazin-1-yl)-7-(5-chloro-6-methyl-1-(tetrahydro-2H-pyran-2-yl)-1H-indazol-4-yl)-5,6,7,8-tetrahydro-1,7-naphthyridine-2-carboxylate **(Int-83b).** Mass Calc'd: 638.3, found 639.7 (M+H)⁺.

### Step C - Synthesis of 4-(4-(tert-butoxycarbonyl)piperazin-1-yl)-7-(5-chloro-6-methyl-1-(tetrahydro-2H-pyran-2-yl)-1H-indazol-4-yl)-5,6,7,8-tetrahydro-1,7-naphthyridine-2-carboxylic acid (Int-83c)

To a solution of ethyl 4-(4-(tert-butoxycarbonyl)piperazin-1-yl)-7-(5-chloro-6-methyl-1-(tetrahydro-2H-pyran-2-yl)-1H-indazol-4-yl)-5,6,7,8-tetrahydro-1,7-naphthyridine-2-carboxylate **(Int-83b)** (60 mg, 0.094 mmol) in MeOH (2 mL) and water (0.5 mL) was added lithium hydroxide monohydrate (19.7 mg, 0.469 mmol), and the mixture was stirred at 25 °C for 10 min. The reaction mixture was concentrated in vacuum to afford 4-(4-(tert-butoxycarbonyl)piperazin-1-yl)-7-(5-chloro-6-methyl-1-(tetrahydro-2H-pyran-2-yl)-1H-indazol-4-yl)-5,6,7,8-tetrahydro-1,7-naphthyridine-2-carboxylic acid **(Int-83c).** Mass Calc'd: 610.3, found 611.3 (M+H)⁺.

### Step D - Synthesis of tert-butyl 4-(7-(5-chloro-6-methyl-1-(tetrahydro-2H-pyran-2-yl)-1H-indazol-4-yl)-2-(((R)-1-(dimethylamino)propan-2-yl)carbamoyl)-5,6,7,8-tetrahydro-1, 7-naphthyridin-4-yl)piperazine-1-carboxylate (Int-83d)

To a stirred solution of 4-(4-(tert-butoxycarbonyl)piperazin-1-yl)-7-(5-chloro-6-methyl-1-(tetrahydro-2H-pyran-2-yl)-1H-indazol-4-yl)-5,6,7,8-tetrahydro-1,7-naphthyridine-2-carboxylic acid **(Int-83c)** (60 mg, 0.098 mmol) in DMF (2 mL) was added HATU (56.0 mg, 0.147 mmol), N,N-diisopropylethylamine (0.051 mL, 0.295 mmol) and (*R*)-N1,N1-dimethylpropane-1,2-diamine (20.06 mg, 0.196 mmol) at 20 °C, and the mixture was stirred at 20 °C for 2 h under a nitrogen atmosphere. The reaction mixture was concentrated, and the residue was purified by preparative TLC plate (SiO₂, DCM/MeOH=5/1) to afford tert-butyl 4-(7-(5-chloro-6-methyl-1-(tetrahydro-2H-pyran-2-yl)-1H-indazol-4-yl)-2-(((*R*)-1-(dimethylamino)propan-2-yl)carbamoyl)-5,6,7,8-tetrahydro-1,7-naphthyridin-4-yl)piperazine-1-carboxylate **(Int-83d).** Mass Calc'd: 694.4, found 695.4 (M+H)⁺.

### Step E - Synthesis of (R)-7-(S-chloro-6-methyl-1H-indazol-4-yl)-N-(1-(dimethylamino)propan-2-yl)-4-(piperazin-1-yl)-5,6,7,8-tetrahydro-1,7-naphthyridine-2-carboxamide (Int-83e)

To a stirred solution of tert-butyl 4-(7-(5-chloro-6-methyl-1-(tetrahydro-2H-pyran-2-yl)-1H-indazol-4-yl)-2-(((*R*)-1-(dimethylamino)propan-2-yl)carbamoyl)-5,6,7,8-tetrahydro-1,7-naphthyridin-4-yl)piperazine-1-carboxylate **(Int-83d)** (20 mg, 0.029 mmol) in DCM (1mL) was added TFA (0.5 mL) at 20 °C, and the mixture was stirred at 20 °C for 5 min under a nitrogen atmosphere. The reaction mixture was concentrated, and the residue was purified by RP-HPLC (with trifluoroacetic acid modifier), to afford (*R*)-7-(5-chloro-6-methyl-1H-indazol-4-yl)-N-(1-(dimethylamino)propan-2-yl)-4-(piperazin-1-yl)-5,6,7,8-tetrahydro-1,7-naphthyridine-2-carboxamide **(Int-83e),** as a salt with trifluoroacetic acid. ¹H NMR (400 MHz, Methanol-*d*₄) δ 8.13 (s, 1H), 7.70 (s, 1H), 7.28 (s, 1H), 4.70 (s, 2H), 4.67 (br s, 1H), 3.71 (br t, *J =* 4.7 Hz, 2H), 3.44 (br s, 4H), 3.41 (br s, 4H), 3.30 - 3.25 (m, 1H), 3.08 (br s, 3H), 3.00 (br s, 3H), 2.92 (br s, 3H), 2.51 (s, 3H), 1.34 (d, *J =* 6.7 Hz, 3H). Mass Calc'd: 510.3, found 511.3 (M+H)⁺.

### Step F - Synthesis of (R)-4-(4-acryloylpiperazin-1-yl)-7-(5-chloro-6-methyl-1H-indazol-4-yl)-N-(1-(dimethylamino)propan-2-yl)-5,6,7,8-tetrahydro-1,7-naphthyridine-2-carboxamide (Example 83)

To a stirred solution of (*R*)-7-(5-chloro-6-methyl-1*H*-indazol-4-yl)-*N*-(1-(dimethylamino)propan-2-yl)-4-(piperazin-1-yl)-5,6,7,8-tetrahydro-1,7-naphthyridine-2-carboxamide (27 mg, 0.053 mmol) in THF (0.5 mL) and water (0.1 mL) was added N,N-diisopropylethylamine (0.046 mL, 0.26 mmol) and acryloyl chloride (4.8 mg, 0.053 mmol) in sequence at 0 °C, and the mixture was stirred at 0 °C for 5 min. The reaction mixture was quenched with water (1 mL), and extracted with ethyl acetate (10 mL). The combined organic layers were washed with brine (2 mL), dried over Na₂SO₄, filtered and the solvent was evaporated under reduced pressure. The residue was purified by RP-HPLC (with ammonium hydroxide and ammonium bicarbonate modifiers) to afford (*R*)-4-(4-acryloylpiperazin-1-yl)-7-(5-chloro-6-methyl-1H-indazol-4-yl)-N-(1-(dimethylamino)propan-2-yl)-5,6,7,8-tetrahydro-1,7-naphthyridine-2-carboxamide (Example **83**). ¹H NMR (400 MHz, Methanol-*d*₄) δ 8.15 (s, 1H), 7.62 (s, 1H), 7.25 (s, 1H), 6.83 (dd, *J =* 10.6, 16.8 Hz, 1H), 6.25 (dd, *J =* 2.0, 16.8 Hz, 1H), 5.79 (dd, *J* = 2.0, 11.0 Hz, 1H), 4.67 (s, 2H), 4.41 - 4.28 (m, 1H), 3.85 (br s, 4H), 3.69 (br t, *J* = 5.1 Hz, 2H), 3.17 (br s, 4H), 3.07 (br s, 2H), 2.72 - 2.62 (m, 1H), 2.51 (s, 3H), 2.39 (dd, *J =* 5.3, 12.3 Hz, 1H), 2.31 (s, 6H), 1.25 (d, *J =* 6.7 Hz, 3H). Mass Calc'd: 564.3, found 565.2 (M+H)⁺.

### Example 84

### Preparation of 4-((S)-4-acryloyl-3-(cyanomethyl)piperazin-1-yl)-7-(5-chloro-6-methyl-1H-indazol-4-yl)-N-((R)-1-(dimethylamino)propan-2-yl)-5,6,7,8-tetrahydro-1,7-naphthyridine-2-carboxamide (Example 84)

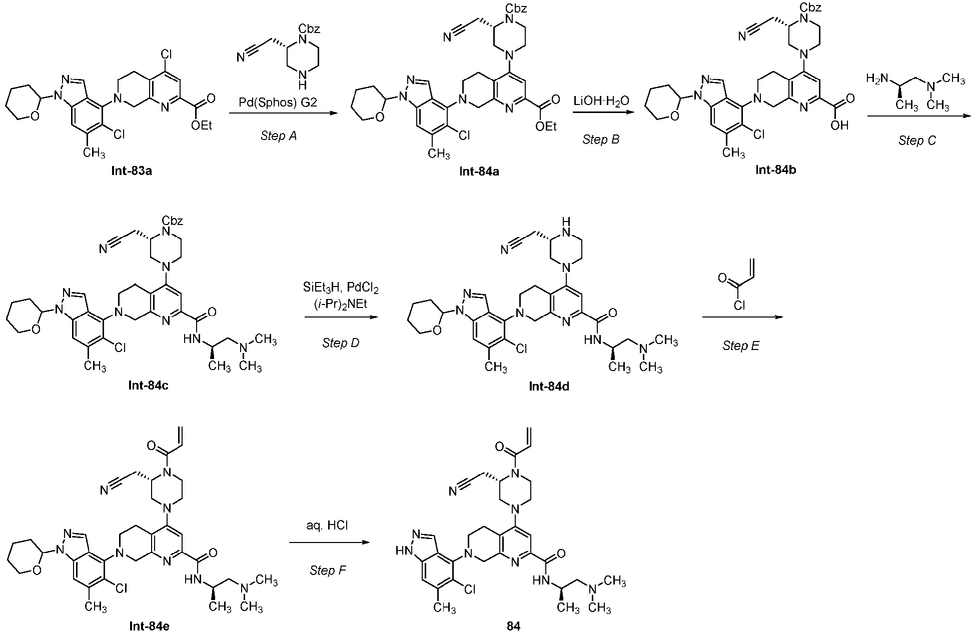

### Step A - Synthesis of ethyl 4-((S)-4-((benzyloxy)carbonyl)-3-(cyanomethyl)piperazin-1-yl)-7-(5-chloro-6-methyl-1-(tetrahydro-2H-pyran-2-yl)-1H-indazol-4-yl)-5,6,7,8-tetrahydro-1,7-naphthyridine-2-carboxylate (Int-84a)

To a mixture of ethyl 4-chloro-7-(5-chloro-6-methyl-1-(tetrahydro-2H-pyran-2-yl)-1H-indazol-4-yl)-5,6,7,8-tetrahydro-1,7-naphthyridine-2-carboxylate **(Int-83a)** (110 mg, 0.225 mmol) in dioxane (1.0 mL) was added benzyl (*S*)-2-(cyanomethyl)piperazine-1-carboxylate (69.9 mg, 0.270 mmol), cesium carbonate (220 mg, 0.674 mmol) and Chloro(2-dicyclohexylphosphino-2',6'-dimethoxy-1,1'-biphenyl)[2-(2'-amino-1,1'-biphenyl)]palladium(II) (16.2 mg, 0.022 mmol) in a glove box. The mixture was then heated to 80 °C for 16 hours under an atmosphere of nitrogen. After 16 hours, the mixture was allowed to cool to room temperature, filtered, and the filtrate was concentrated under reduced pressure. The resulting residue was purified by column chromatography on silica gel (0-46% ethyl acetate gradient in petroleum ether) to afford ethyl 4-((*S*)-4-((benzyloxy)carbonyl)-3-(cyanomethyl)piperazin-1-yl)-7-(5-chloro-6-methyl-1-(tetrahydro-2H-pyran-2-yl)-1H-indazol-4-yl)-5,6,7,8-tetrahydro-1,7-naphthyridine-2-carboxylate **(Int-84a).** ¹H NMR (400 MHz, CDCl₃) δ 8.02 (d, *J =* 6.8 Hz, 1H), 7.59 (s, 1H), 7.42 - 7.30 (m, 5H), 7.22 (s, 1H), 5.66 - 5.60 (m, 1H), 5.19 (s, 2H), 4.83 - 4.68 (m, 3H), 4.46 (q, *J =* 7.1 Hz, 2H), 4.25 - 4.13 (m, 1H), 4.01 (br d, *J =* 11.0 Hz, 1H), 3.77 - 3.69 (m, 1H), 3.65 (br d, *J =* 4.4 Hz, 2H), 3.28 (br s, 3H), 3.15 - 3.08 (m, 1H), 3.01 (br s, 3H), 2.83 (br d, *J* = 10.3 Hz, 2H), 2.51 (s, 4H), 2.45 - 2.29 (m, 1H), 2.18 - 2.10 (m, 1H), 1.74 (br s, 2H), 1.66 - 1.61 (m, 1H), 1.41 (s, 3H). Mass Calc'd: 711.3, found 712.2 (M+H)⁺.

### Step B - Synthesis of 4-((S)-4-((benzyloxy)carbonyl)-3-(cyanomethyl)piperazin-1-yl)-7-(5-chloro-6-methyl-1-(tetrahydro-2H-pyran-2-yl)-1H-indazol-4-yl)-5,6,7,8-tetrahydro-1,7-naphthyridine-2-carboxylic acid (Int-84b)

To a solution of ethyl 4-((*S*)-4-((benzyloxy)carbonyl)-3-(cyanomethyl)piperazin-1-yl)-7-(5-chloro-6-methyl-1-(tetrahydro-2H-pyran-2-yl)-1H-indazol-4-yl)-5,6,7,8-tetrahydro-1,7-naphthyridine-2-carboxylate **(Int-84a)** (70 mg, 0.098 mmol) in methanol (0.50 mL) was added lithium hydroxide monohydrate (21 mg, 0.49 mmol) and water (50 µL). After 1 hour at 23 °C, the mixture was concentrated under reduced pressure. The resulting residue was purified by RP-HPLC (with trifluoroacetic acid modifier) to afford 4-((S)-4-((benzyloxy)carbonyl)-3-(cyanomethyl)piperazin-1-yl)-7-(5-chloro-6-methyl-1-(tetrahydro-2H-pyran-2-yl)-1H-indazol-4-yl)-5,6,7,8-tetrahydro-1,7-naphthyridine-2-carboxylic acid **(Int-84b).** Mass Calc'd: 683.3, found 684.3 (M+H)⁺.

### Step C - Synthesis of benzyl (2S)-4-(7-(5-chloro-6-methyl-1-(tetrahydro-2H-pyran-2-yl)-1H-indazol-4-yl)-2-(((R)-1-(dimethylamino)propan-2-yl)carbamoyl)-5,6,7,8-tetrahydro-1, 7-naphthyridin-4-yl)-2-(cyanomethyl)piperazine-1-carboxylate (Int-84c)

To a mixture of 4-((S)-4-((benzyloxy)carbonyl)-3-(cyanomethyl)piperazin-1-yl)-7-(5-chloro-6-methyl-1-(tetrahydro-2H-pyran-2-yl)-1H-indazol-4-yl)-5,6,7,8-tetrahydro-1,7-naphthyridine-2-carboxylic acid **(Int-84b)** (60 mg, 0.088 mmol) in DMF (1 mL) was added N,N-diisopropylethylamine (0.046 mL, 0.26 mmol), HATU (100 mg, 0.263 mmol) and (*R*)-N1,N1-dimethylpropane-1,2-diamine (26.9 mg, 0.263 mmol) at 25 °C. The mixture was stirred at 25 °C for 1.5 h. The mixture was then concentrated under reduced pressure. The resulting residue was purified by preparative TLC (SiO₂, DCM: MeOH = 7 : 1) to afford benzyl (2S)-4-(7-(5-chloro-6-methyl-1-(tetrahydro-2H-pyran-2-yl)-1H-indazol-4-yl)-2-(((*R*)-1-(dimethylamino)propan-2-yl)carbamoyl)-5,6,7,8-tetrahydro-1,7-naphthyridin-4-yl)-2-(cyanomethyl)piperazine-1-carboxylate **(Int-84c).** Mass Calc'd: 767.4, found 768.3 (M+H)⁺.

### Step D - Synthesis of 7-(5-chloro-6-methyl-1-(tetrahydro-2H-pyran-2-yl)-1H-indazol-4-yl)-4-((S)-3-(cyanomethyl)piperazin-1-yl)-N-((R)-1-(dimethylamino)propan-2-yl)-5,6,7,8-tetrahydro-1,7-naphthyridine-2-carboxamide (Int-84d)

To a mixture of benzyl (2*S*)-4-(7-(5-chloro-6-methyl-1-(tetrahydro-2H-pyran-2-yl)-1H-indazol-4-yl)-2-(((*R*)-1-(dimethylamino)propan-2-yl)carbamoyl)-5,6,7,8-tetrahydro-1,7-naphthyridin-4-yl)-2-(cyanomethyl)piperazine-1-carboxylate **(Int-84c)** (50 mg, 0.065 mmol) in DCM (0.5 mL) was added N,N-diisopropylethylamine (0.034 mL, 0.20 mmol), triethylsilane (91 mg, 0.78 mmol) and palladium(II) chloride (1.2 mg, 6.5 µmol). After stirring for 2 hours at 25 °C, the mixture was filtered, and the filtrate was concentrated under reduced pressure to afford 7-(5-chloro-6-methyl-1-(tetrahydro-2H-pyran-2-yl)-1H-indazol-4-yl)-4-((S)-3-(cyanomethyl)piperazin-1-yl)-N-((*R*)-1-(dimethylamino)propan-2-yl)-5,6,7,8-tetrahydro-1,7-naphthyridine-2-carboxamide **(Int-84d).** Mass Calc'd: 633.3, found 634.4 (M+H)⁺.

### Step E - Synthesis of 4-((S)-4-acryloyl-3-(cyanomethyl)piperazin-1-yl)-7-(5-chloro-6-methyl-1-(tetrahydro-2H-pyran-2-yl)-1H-indazol-4-yl)-N-((R)-1-(dimethylamino)propan-2-yl)-5,6,7,8-tetrahydro-1,7-naphthyridine-2-carboxamide (Int-84e)

To a mixture of 7-(5-chloro-6-methyl-1-(tetrahydro-2H-pyran-2-yl)-1H-indazol-4-yl)-4-((S)-3-(cyanomethyl)piperazin-1-yl)-N-((R)-1-(dimethylamino)propan-2-yl)-5,6,7,8-tetrahydro-1,7-naphthyridine-2-carboxamide **(Int-84d)** (30 mg, 0.047 mmol) in DCM (0.5 mL) was added N,N-diisopropylethylamine (0.025 mL, 0.14 mmol) and acryloyl chloride (7.7 µL, 0.095 mmol) at 25 °C. The mixture was stirred at 25 °C for 10 min. The reaction mixture was purified by preparative TLC (SiO₂, MeOH: DCM = 10 : 1) to afford 4-((*S*)-4-acryloyl-3-(cyanomethyl)piperazin-1-yl)-7-(5-chloro-6-methyl-1-(tetrahydro-2H-pyran-2-yl)-1H-indazol-4-yl)-N-((*R*)-1-(dimethylamino)propan-2-yl)-5,6,7,8-tetrahydro-1,7-naphthyridine-2-carboxamide **(Int-84e).** Mass Calc'd: 687.3, found 688.5 (M+H)⁺.

### Step F - Synthesis of 4-((S)-4-acryloyl-3-(cyanomethyl)piperazin-1-yl)-7-(5-chloro-6-methyl-1H-indazol-4-yl)-N-((R)-1-(dimethylamino)propan-2-yl)-5,6,7,8-tetrahydro-1,7-naphthyridine-2-carboxamide (Example 84)

To a mixture of 4-((S)-4-acryloyl-3-(cyanomethyl)piperazin-1-yl)-7-(5-chloro-6-methyl-1-(tetrahydro-2H-pyran-2-yl)-1H-indazol-4-yl)-N-((*R*)-1-(dimethylamino)propan-2-yl)-5,6,7,8-tetrahydro-1,7-naphthyridine-2-carboxamide **(Int-84e)** (10 mg, 0.015 mmol) in MeCN (1 mL) and water (0.1 mL) was added hydrochloric acid (12 N in water, 0.5 mL, 6 mmol) at 25 °C, and the mixture was stirred at 25 °C for 2 h. The mixture was concentrated under reduced pressure, and the resulting residue was purified by RP-HPLC (with 0.04% ammonium hydroxide and 10 mM ammonium bicarbonate modifiers) to afford 4-((*S*)-4-acryloyl-3-(cyanomethyl)piperazin-1-yl)-7-(5-chloro-6-methyl-1H-indazol-4-yl)-N-((*R*)-1-(dimethylamino)propan-2-yl)-5,6,7,8-tetrahydro-1,7-naphthyridine-2-carboxamide (Example **84**). ¹H NMR (500 MHz, CDCl₃) δ 10.77 - 10.51 (m, 1H), 8.13 (s, 1H), 8.08 (br s, 1H), 7.69 (s, 1H), 7.11 (s, 1H), 6.60 (br s, 1H), 6.41 (br d, *J =* 17.4 Hz, 1H), 5.84 (br d, *J =* 9.5 Hz, 1H), 5.25 - 5.10 (m, 1H), 4.64 (br s, 1H), 4.58 (br s, 1H), 4.33 (br s, 1H), 4.08 - 3.91 (m, 1H), 3.66 (br s, 2H), 3.51 - 3.43 (m, 1H), 3.35 (br d, *J =* 11.6 Hz, 1H), 3.16 - 2.79 (m, 7H), 2.63 (br s, 1H), 2.50 (s, 3H), 2.34 (br s, 7H), 1.31 (br d, *J =* 6.4 Hz, 3H). Mass Calc'd: 603.3, found 604.3 (M+H)⁺.

### Example 85

### Preparation of 4-((S)-4-acryloyl-3-(cyanomethyl)piperazin-1-yl)-N-((R)-1-(dimethylamino)propan-2-yl)-3-methyl-7-(8-methylnaphthalen-1-yl)-5,6,7,8-tetrahydro-1,7-naphthyridine-2-carboxamide (Example 85)

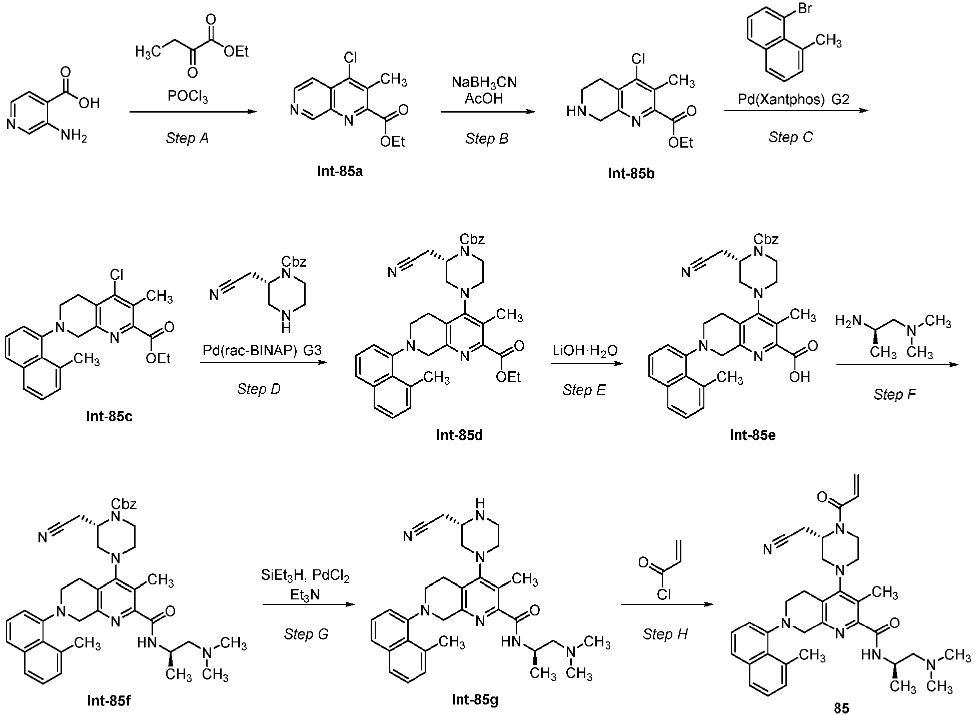

### Step A - Synthesis of ethyl 4-chloro-3-methyl-1, 7-naphthyridine-2-carboxylate (Int-85a)

To a mixture of 3-aminoisonicotinic acid (10 g, 72 mmol) and ethyl 2-oxobutanoate (11.3 g, 86.9 mmol) was added POCl₃ (33.7 mL, 362 mmol). The mixture was heated to 50 °C for 3 hours. The reaction mixture was then allowed to cool to room temperature, and partially concentrated under reduced pressure. The mixture was then poured into water (200 mL) slowly and neutralized by the addition of solid sodium bicarbonate to pH 7. The resulting mixture was extracted with EtOAc (3 x 600 mL). The combined organic layers were dried over anhydrous sodium sulfate, filtered and concentrated under reduced pressure. The resulting residue was purified by column chromatography on silica gel (43% ethyl acetate in petroleum ether) to afford ethyl 4-chloro-3-methyl-1,7-naphthyridine-2-carboxylate **(Int-85a).** ¹H NMR (400 MHz, CDCl₃) δ 9.56 (d, *J =* 0.7 Hz, 1H), 8.75 (d, *J =* 5.9 Hz, 1H), 8.01 (d, *J =* 5.9 Hz, 1H), 4.56 (q, *J =* 7.3 Hz, 2H), 2.72 (s, 3H), 1.49 (t, *J =* 7.2 Hz, 3H). Mass Calc'd: 250.1, found 250.9 (M+H)⁺.

### Step B - Synthesis of ethyl 4-chloro-3-methyl-5, 6, 7, 8-tetrahydro-1, 7-naphthyridine-2-carboxylate (Int-85b)

To a mixture of ethyl 4-chloro-3-methyl-1,7-naphthyridine-2-carboxylate **(Int-85a)** (2.15 g, 8.58 mmol) in acetic acid (10 mL) was added sodium cyanoborohydride (0.647 g, 10.3 mmol), and the mixture was stirred at 25 °C for 0.5 h. The solvent was then concentrated under reduced pressure. The resulting mixture was then diluted with water (80 mL) and neutralized by the addition of solid sodium bicarbonate to a pH > 7. The mixture was extracted with dichloromethane (3 x 200 mL), the combined organic layers were dried over anhydrous sodium sulfate, filtered and the filtrate was concentrated under reduced pressure. The resulting residue was purified by RP-HPLC to afford ethyl 4-chloro-3-methyl-5,6,7,8-tetrahydro-1,7-naphthyridine-2-carboxylate **(Int-85b).** ¹H NMR (400 MHz, CDCl₃) δ 10.62 - 10.38 (m, 1H), 4.46 (q, *J =* 7.1 Hz, 2H), 4.41 (s, 2H), 3.51 (br t, *J =* 6.1 Hz, 2H), 3.20 (br t, *J =* 6.1 Hz, 2H), 2.54 (s, 3H), 1.43 (t, *J =* 7.2 Hz, 3H). Mass Calc'd: 254.1, found 255.1 (M+H)⁺.

### Step C - Synthesis of ethyl 4-chloro-3-methyl-7-(8-methylnaphthalen-1-yl)-5, 6, 7, 8-tetrahydro-1,7-naphthyridine-2-carboxylate (Int-85c)

To a mixture of ethyl 4-chloro-3-methyl-5,6,7,8-tetrahydro-1,7-naphthyridine-2-carboxylate **(Int-85b)** (500 mg, 1.96 mmol) in toluene (8 mL) was added 1-bromo-8-methylnaphthalene (651 mg, 2.94 mmol), Cs₂CO₃ (1.92 g, 5.89 mmol) and chloro[(4,5-bis(diphenylphosphino)-9,9-dimethylxanthene)-2-(2'-amino-1,1'-biphenyl)]palladium(II) (Pd(Xantphos) G2) (191 mg) at 25 °C under a nitrogen atmosphere. The mixture was stirred at 100 °C for 15 h. The mixture was then allowed to cool to room temperature, filtered and the solvent was concentrated under reduced pressure. The resulting residue was purified by silica gel column chromatography (0-18% ethyl acetate in petroleum ether) to afford ethyl 4-chloro-3-methyl-7-(8-methylnaphthalen-1-yl)-5,6,7,8-tetrahydro-1,7-naphthyridine-2-carboxylate **(Int-85c).** Mass Calc'd: 394.1, found 395.0 (M+H)⁺.

### Step D - Synthesis of ethyl (S)-4-(4-((benzyloxy)carbonyl)-3-(cyanomethyl)piperazin-1-yl)-3-methyl-7-(8-methylnaphthalen-1-yl)-5,6,7, 8-tetrahydro-1, 7-naphthyridine-2-carboxylate (Int-85d)

To a mixture of ethyl 4-chloro-3-methyl-7-(8-methylnaphthalen-1-yl)-5,6,7,8-tetrahydro-1,7-naphthyridine-2-carboxylate **(Int-85c)** (250 mg, 0.633 mmol) in 1,4-dioxane (8 mL) was added benzyl (*S*)-2-(cyanomethyl)piperazine-1-carboxylate (246 mg, 0.950 mmol), cesium carbonate (619 mg, 1.90 mmol) and racemic Pd(rac-BINAP) G3 (CAS Number: 2151915-22-7, 63 mg, 0.063 mmol) at room temperature under an atmosphere of nitrogen. The mixture was heated to 80 °C for 40 hours. The mixture was then allowed to cool to room temperature, diluted with water (4 mL), and extracted with EtOAc (2 x 6 mL). The combined organic layers were dried over anhydrous sodium sulfate, filtered, and the filtrate was concentrated under reduced pressure. The resulting residue was purified by silica gel column chromatography (0-25% ethyl acetate gradient in petroleum ether) to afford ethyl (S)-4-(4-((benzyloxy)carbonyl)-3-(cyanomethyl)piperazin-1-yl)-3-methyl-7-(8-methylnaphthalen-1-yl)-5,6,7,8-tetrahydro-1,7-naphthyridine-2-carboxylate **(Int-85d).** Mass Calc'd: 617.3, found 618.4 (M+H)⁺.

### Step E - Synthesis of (S)-4-(4-((benzyloxy)carbonyl)-3-(cyanomethyl)piperazin-1-yl)-3-methyl-7-(8-methylnaphthalen-1-yl)-5,6, 7,8-tetrahydro-1, 7-naphthyridine-2-carboxylic acid (Int-85e)

To a solution of ethyl (,S)-4-(4-((benzyloxy)carbonyl)-3-(cyanomethyl)piperazin-1-yl)-3-methyl-7-(8-methylnaphthalen-1-yl)-5,6,7,8-tetrahydro-1,7-naphthyridine-2-carboxylate **(Int-85d)** (90 mg, 0.15 mmol) in methanol (1 mL) was added water (0.1 mL) and lithium hydroxide monohydrate (61.1 mg, 1.46 mmol) at room temperature. After 2 hours at room temperature, the mixture was acidified with aqueous HCl (1 M) to pH 7. The mixture was concentrated under reduced pressure to afford (*S*)-4-(4-((benzyloxy)carbonyl)-3-(cyanomethyl)piperazin-1-yl)-3-methyl-7-(8-methylnaphthalen-1-yl)-5,6,7,8-tetrahydro-1,7-naphthyridine-2-carboxylic acid **(Int-85e),** which was used without further purification. Mass Calc'd: 589.3, found 590.3 (M+H)⁺.

### Step F - Synthesis of benzyl (S)-2-(cyanomethyl)-4-(2-(((R)-1-(dimethylamino)propan-2-yl)carbamoyl)-3-methyl-7-(8-methylnaphthalen-1-yl)-5,6,7,8-tetrahydro-1,7-naphthyridin-4-yl)piperazine-1-carboxylate (Int-85f)

To a mixture of (*S*)-4-(4-((benzyloxy)carbonyl)-3-(cyanomethyl)piperazin-1-yl)-3-methyl-7-(8-methylnaphthalen-1-yl)-5,6,7,8-tetrahydro-1,7-naphthyridine-2-carboxylic acid **(Int-85e)** (82 mg, 0.14 mmol) in DMF (1 mL) was added (*R*)-N1,N1-dimethylpropane-1,2-diamine (23 mg, 0.23 mmol), triethylamine (0.058 mL, 0.42 mmol) and HATU (63.4 mg, 0.167 mmol) at room temperature. After 1 hour at room temperature, the mixture was concentrated under reduced pressure. The resulting residue was purified by preparative TLC (SiO₂, ethyl acetate) to afford benzyl (*S*)-2-(cyanomethyl)-4-(2-(((*R*)-1-(dimethylamino)propan-2-yl)carbamoyl)-3-methyl-7-(8-methylnaphthalen-1-yl)-5,6,7,8-tetrahydro-1,7-naphthyridin-4-yl)piperazine-1-carboxylate **(Int-85f).** Mass Calc'd: 673.4, found 674.6 (M+H)⁺.

### Step G - Synthesis of 4-((S)-3-(cyanomethyl)piperazin-1-yl)-N-((R)-1-(dimethylamino)propan-2-yl)-3-methyl-7-(8-methylnaphthalen-1-yl)-5,6,7,8-tetrahydro-1,7-naphthyridine-2-carboxamide (Int-85g)

To a mixture of benzyl (*S*)-2-(cyanomethyl)-4-(2-(((*R*)-1-(dimethylamino)propan-2-yl)carbamoyl)-3-methyl-7-(8-methylnaphthalen-1-yl)-5,6,7,8-tetrahydro-1,7-naphthyridin-4-yl)piperazine-1-carboxylate **(Int-85f)** (65 mg, 0.096 mmol) in DCM (1 mL) was added triethylamine (0.040 mL, 0.29 mmol), triethylsilane (0.18 mL, 1.2 mmol) and palladium(II) chloride (1.7 mg, 9.7 µmol) at room temperature. After 1 hour, the mixture was quenched with methanol (1 mL), filtered, and the filtrate was concentrated under reduced pressure to afford 4-((*S*)-3-(cyanomethyl)piperazin-1-yl)-N-((*R*)-1-(dimethylamino)propan-2-yl)-3-methyl-7-(8-methylnaphthalen-1-yl)-5,6,7,8-tetrahydro-1,7-naphthyridine-2-carboxamide **(Int-85g).** Mass Calc'd: 539.3, found 540.5 (M+H)⁺.

### Step H - Synthesis of 4-((S)-4-acryloyl-3-(cyanomethyl)piperazin-1-yl)-N-((R)-1-(dimethylamino)propan-2-yl)-3-methyl-7-(8-methylnaphthalen-1-yl)-5,6,7,8-tetrahydro-1, 7-naphthyridine-2-carboxamide (Example 85)

To a mixture of 4-((S)-3-(cyanomethyl)piperazin-1-yl)-N-((R)-1-(dimethylamino)propan-2-yl)-3-methyl-7-(8-methylnaphthalen-1-yl)-5,6,7,8-tetrahydro-1,7-naphthyridine-2-carboxamide **(Int-85g)** (40 mg, 0.074 mmol) in DCM (1 mL) was added N,N-diisopropylethylamine (0.013 mL, 0.074 mmol) and acryloyl chloride (6.0 µL, 0.074 mmol) at room temperature under an atmosphere of nitrogen. After 10 minutes at room temperature, the mixture was concentrated under reduced pressure. The resulting residue was purified by RP-HPLC (with 0.04% ammonium hydroxide and 10 mM ammonium bicarbonate modifiers) to afford 4-((S)-4-acryloyl-3-(cyanomethyl)piperazin-1-yl)-N-((R)-1-(dimethylamino)propan-2-yl)-3-methyl-7-(8-methylnaphthalen-1-yl)-5,6,7,8-tetrahydro-1,7-naphthyridine-2-carboxamide (Example **85).** ¹H NMR (500 MHz, Methanol-*d*₄) δ 7.70 - 7.66 (m, 1H), 7.63 (s, 1H), 7.42 (s, 1H), 7.31 (s, 2H), 7.21 (br d, *J =* 6.7 Hz, 1H), 6.95 - 6.75 (m, 1H), 6.28 (br d, *J =* 16.8 Hz, 1H), 5.83 (br d, *J =* 10.1 Hz, 1H), 5.19 - 4.91 (m, 1H), 4.91 - 4.89 (m, 1H), 4.84 (br d, *J =* 3.7 Hz, 2H), 4.25 (br s, 2H), 4.11 - 3.84 (m, 1H), 3.74 (s, 1H), 3.60 (br s, 2H), 3.46 - 3.35 (m, 1H), 3.29 - 2.91 (m, 7H), 2.86 - 2.82 (m, 3H), 2.54 (br s, 2H), 2.45 - 2.40 (m, 1H), 2.37 - 2.31 (m, 1H), 2.29 - 2.27 (m, 5H), 1.22 (d, *J =* 6.7 Hz, 3H). Mass Calc'd: 593.3, found 594.3 (M+H)⁺.

### Example 86

### Preparation of 4-((S)-4-acryloyl-3-(cyanomethyl)piperazin-1-yl)-N-((S)-2-aminopropyl)-7-(8-methylnaphthalen-1-yl)-5,6,7,8-tetrahydro-1,7-naphthyridine-2-carboxamide (Example 86)

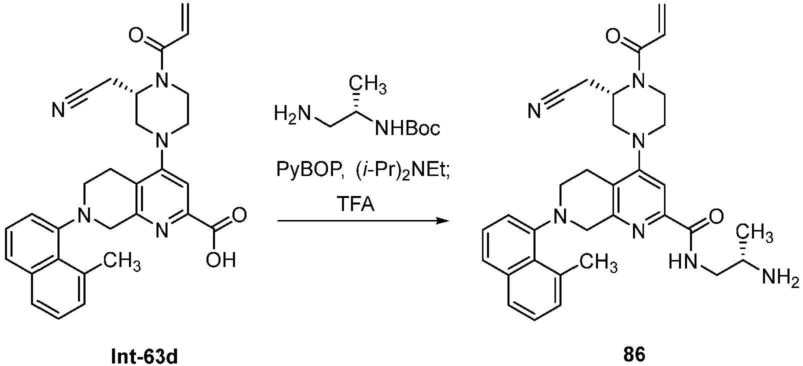

### Synthesis of 4-((S)-4-acryloyl-3-(cyanomethyl)piperazin-1-yl)-N-((S)-2-aminopropyl)-7-(8-methylnaphthalen-1-yl)-5, 6, 7, 8-tetrahydro-1, 7-naphthyridine-2-carboxamide (Example 86)

For synthesis of a library of amides including example **86:** A stock solution, "Solution A", was prepared by dissolving (S)-4-(4-acryloyl-3-(cyanomethyl)piperazin-1-yl)-7-(8-methylnaphthalen-1-yl)-5,6,7,8-tetrahydro-1,7-naphthyridine-2-carboxylic acid, TFA salt (prepared by purification of **Int-63d** by RP-HPLC using trifluoroacetic acid modifier) (80 mg, 0.13 mmol) in anhydrous dichloromethane (4.0 mL). A stock solution, "Solution B", was prepared by dissolving benzotriazol-1-yl-oxytripyrrolidinophosphonium hexafluorophosphate (82 mg, 0.16 mmol) in anhydrous dichloromethane (4.0 mL).

To a 1.0 mL glass micro vial containing a micro magnetic stir bar and tert-butyl (*S*)-(1-aminopropan-2-yl)carbamate (0.69 mg, 3.9 µmol) was added 100 µL from above-prepared stock solution, "Solution A". To the resulting solution was added 100 uL from above prepared stock solution, "Solution B", followed by the addition of N,N-Diisopropylethylamine (1.7 µL, 9.8 µmol). The contents of the 1.0 mL micro-vial were stirred at room temperature for 0.5 h, then a 1:1 TFA : DCM mixture (100 µL) was added to the microvial. After 0.5 h the contents of the micro-vial were concentrated, and the residue was diluted with dimethylsulfoxide (105 uL). The resulting mixture was filtered, and the filtrate was purified by RP-HPLC (with trifluoroacetic acid modifier) to afford 4-((*S*)-4-acryloyl-3-(cyanomethyl)piperazin-1-yl)-N-((*S*)-2-aminopropyl)-7-(8-methylnaphthalen-1-yl)-5,6,7,8-tetrahydro-1,7-naphthyridine-2-carboxamide (Example **86)** as a salt with trifluoroacetic acid. Mass Calc'd: 551.3, found 552 (M+H)⁺.

The following compounds were prepared as salts with trifluoroacetic acid, in a similar manner to Example **86.**

| **Example No.** | **Structure** | **Compound Name** | **Exact Mass** |
|---|---|---|---|
| **87** | 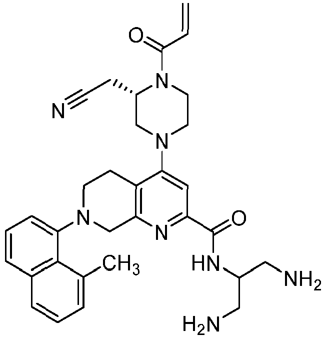 | (*S*)-4-(4-acryloyl-3-(cyanomethyl)piperazin-1-yl)-N-(1,3-diaminopropan-2-yl)-7-(8-methylnaphthalen-1-yl)-5,6,7,8-tetrahydro-1,7-naphthyridine-2-carboxamide | Calc'd 566.3, found 567.8 (M+H)⁺ |
| **88** | 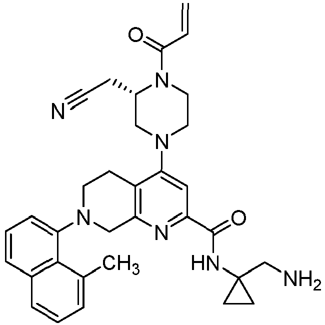 | (*S*)-4-(4-acryloyl-3-(cyanomethyl)piperazin-1-yl)-N-(1-(aminomethyl)cycloprop yl)-7-(8-methylnaphthalen-1-yl)-5,6,7,8-tetrahydro-1,7-naphthyridine-2-carboxamide | Calc'd 563.3, found 564.6 (M+H)⁺ |
| **89** | 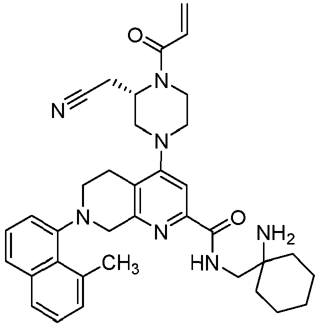 | (*S*)-4-(4-acryloyl-3-(cyanomethyl)piperazin-1-yl)-N-((1-aminocyclohexyl)methyl )-7-(8-methylnaphthalen-1-yl)-5,6,7,8-tetrahydro-1,7-naphthyridine-2-carboxamide | Calc'd 605.3, found 606.7 (M+H)⁺ |
| **90** | 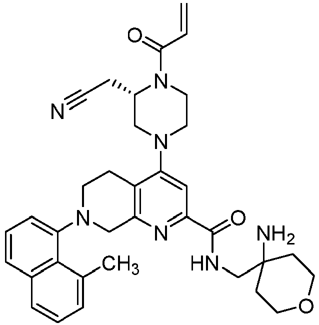 | (*S*)-4-(4-acryloyl-3-(cyanomethyl)piperazin-1-yl)-N-((4-aminotetrahydro-2H-pyran-4-yl)methyl)-7-(8-methylnaphthalen-1-yl)-5,6,7,8-tetrahydro-1,7-naphthyridine-2-carboxamide | Calc'd 607.3, found 608.7 (M+H)⁺ |
| **91** | 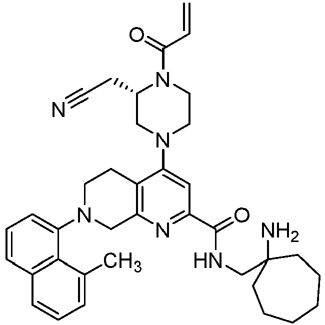 | (*S*)-4-(4-acryloyl-3-(cyanomethyl)piperazin-1-yl)-N-((1-aminocycloheptyl)methy l)-7-(8-methylnaphthalen-1-yl)-5,6,7,8-tetrahydro-1,7-naphthyridine-2-carboxamide | Calc'd 619.4, found 620.7 (M+H)⁺ |
| **92** | 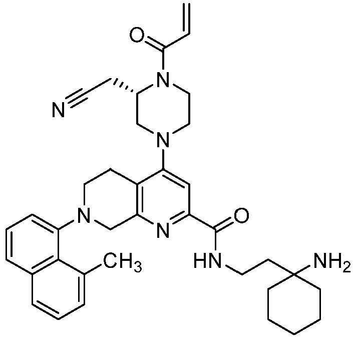 | (*S*)-4-(4-acryloyl-3-(cyanomethyl)piperazin-1-yl)-N-(2-(1-aminocyclohexyl)ethyl)-7-(8-methylnaphthalen-1-yl)-5,6,7,8-tetrahydro-1,7-naphthyridine-2-carboxamide | Calc'd 619.4, found 620.6 (M+H)⁺ |
| **93** | 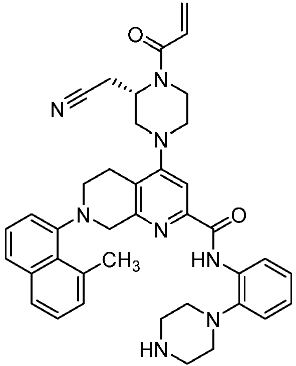 | (*S*)-4-(4-acryloyl-3-(cyanomethyl)piperazin-1-yl)-7-(8-methylnaphthalen-1-yl)-N-(2-(piperazin-1-yl)phenyl)-5,6,7,8-tetrahydro-1,7-naphthyridine-2-carboxamide | Calc'd 654.3, found 655.7 (M+H)⁺ |
| **94** | 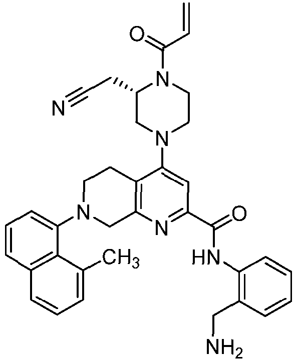 | (*S*)-4-(4-acryloyl-3-(cyanomethyl)piperazin-1-yl)-N-(2-(aminomethyl)phenyl)-7-(8-methylnaphthalen-1-yl)-5,6,7,8-tetrahydro-1,7-naphthyridine-2-carboxamide | Calc'd 599.3, found 600.5 (M+H)⁺ |
| **95** | 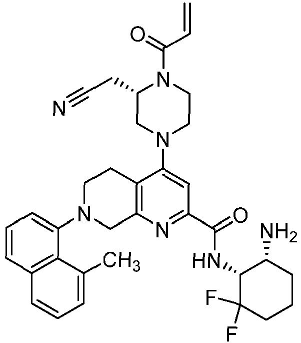 | 4-((*S*)-4-acryloyl-3-(cyanomethyl)piperazin-1-yl)-N-((1*R*,6*R*)-6-amino-2,2-difluorocyclohexyl)-7-(8-methylnaphthalen-1-yl)-5,6,7,8-tetrahydro-1,7-naphthyridine-2-carboxamide | Calc'd 627.3, found 628.5 (M+H)⁺ |
| **96** | 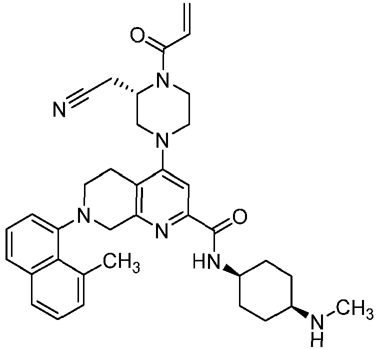 | 4-((*S*)-4-acryloyl-3-(cyanomethyl)piperazin-1-yl)-N-(cis-4-(methylamino)cyclohexy l)-7-(8-methylnaphthalen-1-yl)-5,6,7,8-tetrahydro-1,7-naphthyridine-2-carboxamide | Calc'd 605.3, found 606 (M+H)⁺ |
| **97** | 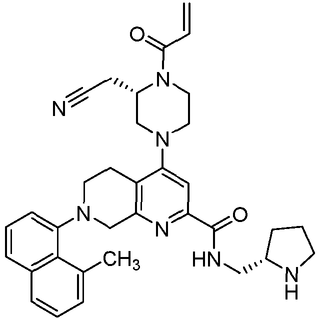 | 4-((*S*)-4-acryloyl-3-(cyanomethyl)piperazin-1-yl)-7-(8-methylnaphthalen-1-yl)-N-(((*S*)-pyrrolidin-2-yl)methyl)-5,6,7,8-tetrahydro-1,7-naphthyridine-2-carboxamide | Calc'd 577.3, found 578.7 (M+H)⁺ |
| **98** | 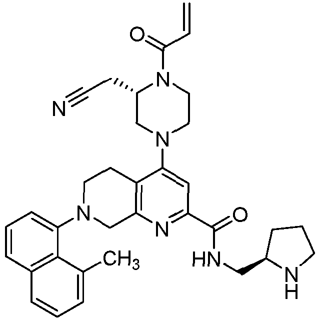 | 4-((5)-4-acryloyl-3-(cyanomethyl)piperazin-1-yl)-7-(8-methylnaphthalen-1-yl)-N-(((*R*)-pyrrolidin-2-yl)methyl)-5,6,7,8-tetrahydro-1,7-naphthyridine-2-carboxamide | Calc'd 577.3, found 578.6 (M+H)⁺ |
| **99** | 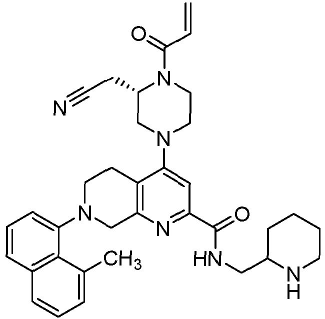 | 4-((*S*)-4-acryloyl-3-(cyanomethyl)piperazin-1-yl)-7-(8-methylnaphthalen-1-yl)-N-(((*R*)-piperidin-2-yl)methyl)-5,6,7,8-tetrahydro-1,7-naphthyridine-2-carboxamide, as a mixture with 4-((S)-4-acryloyl-3-(cyanomethyl)piperazin-1-yl)-7-(8-methylnaphthalen-1-yl)-N-(((S)-piperidin-2-yl)methyl)-5,6,7,8-tetrahydro-1,7-naphthyridine-2-carboxamide | Calc'd 591.3, found 592.6 (M+H)⁺ |
| **100** | 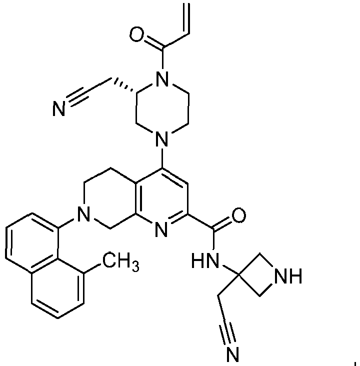 | (*S*)-4-(4-acryloyl-3-(cyanomethyl)piperazin-1-yl)-N-(3-(cyanomethyl)azetidin-3-yl)-7-(8-methylnaphthalen-1-yl)-5,6,7,8-tetrahydro-1,7-naphthyridine-2-carboxamide | Calc'd 588.3, found 589.5 (M+H)⁺ |
| **101** | 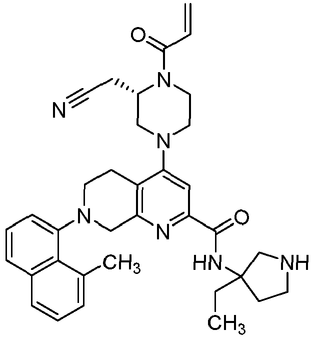 | 4-((*S*)-4-acryloyl-3-(cyanomethyl)piperazin-1-yl)-N-((R)-3-ethylpyrrolidin-3-yl)-7-(8-methylnaphthalen-1-yl)-5,6,7,8-tetrahydro-1,7-naphthyridine-2-carboxamide, as a mixture with 4-((S)-4-acryloyl-3-(cyanomethyl)piperazin-1-yl)-N-((*S*)-3-ethylpyrrolidin-3-yl)-7-(8-methylnaphthalen-1-yl)-5,6,7,8-tetrahydro-1,7-naphthyridine-2-carboxamide | Calc'd 591.3, found 592.6 (M+H)⁺ |
| **102** | 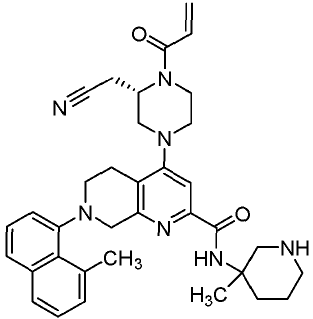 | 4-((*S*)-4-acryloyl-3-(cyanomethyl)piperazin-1-yl)-7-(8-methylnaphthalen-1-yl)-N-((*R*)-3-methylpiperidin-3-yl)-5,6,7,8-tetrahydro-1,7-naphthyridine-2-carboxamide, as a mixture with 4-((*S*)-4-acryloyl-3-(cyanomethyl)piperazin-1-yl)-7-(8-methylnaphthalen-1-yl)-N-((,S)-3-methylpiperidin-3-yl)-5,6,7,8-tetrahydro-1,7-naphthyridine-2-carboxamide | Calc'd 591.3, found 592.7 (M+H)⁺ |
| **103** | 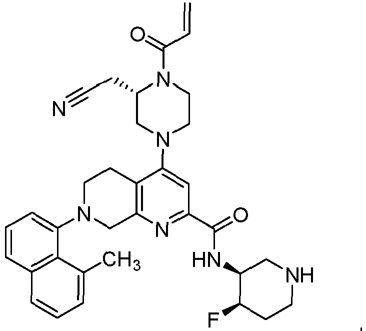 | 4-((*S*)-4-acryloyl-3-(cyanomethyl)piperazin-1-yl)-N-((3*S*,4*R*)-4-fluoropiperidin-3-yl)-7-(8-methylnaphthalen-1-yl)-5,6,7,8-tetrahydro-1,7-naphthyridine-2-carboxamide | Calc'd 595.3, found 596.6 (M+H)⁺ |
| **104** | 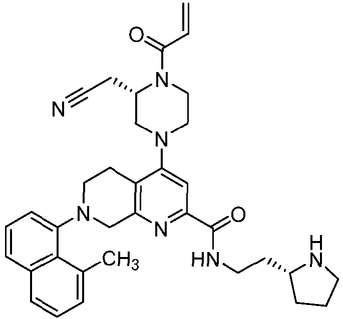 | 4-((*S*)-4-acryloyl-3-(cyanomethyl)piperazin-1-yl)-7-(8-methylnaphthalen-1-yl)-N-(2-((*R*)-pyrrolidin-2-yl)ethyl)-5,6,7,8-tetrahydro-1,7-naphthyridine-2-carboxamide | Calc'd 591.3, found 592.7 (M+H)⁺ |
| **105** | 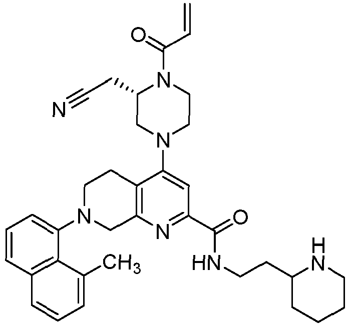 | 4-((*S*)-4-acryloyl-3-(cyanomethyl)piperazin-1-yl)-7-(8-methylnaphthalen-1-yl)-N-(2-((*R*)-piperidin-2-yl)ethyl)-5,6,7,8-tetrahydro-1,7-naphthyridine-2-carboxamide, as a mixture with 4-((S)-4-acryloyl-3-(cyanomethyl)piperazin-1-yl)-7-(8-methylnaphthalen-1-yl)-N-(2-((*S*)-piperidin-2-yl)ethyl)-5,6,7,8-tetrahydro-1,7-naphthyridine-2-carboxamide | Calc'd 605.3, found 606.7 (M+H)⁺ |
| **106** | 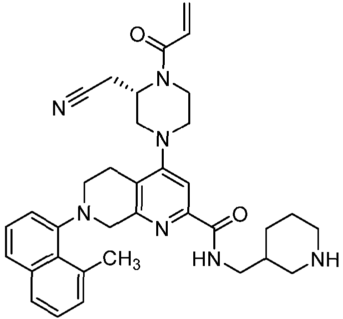 | 4-((*S*)-4-acryloyl-3-(cyanomethyl)piperazin-1-yl)-7-(8-methylnaphthalen-1-yl)-N-(((*R*)-piperidin-3-yl)methyl)-5,6,7,8-tetrahydro-1,7-naphthyridine-2-carboxamide, as a mixture with 4-((S)-4-acryloyl-3-(cyanomethyl)piperazin-1-yl)-7-(8-methylnaphthalen-1-yl)-N-(((S)-piperidin-3-yl)methyl)-5,6,7,8-tetrahydro-1,7-naphthyridine-2-carboxamide | Calc'd 591.3, found 592.6 (M+H)⁺ |
| **107** | 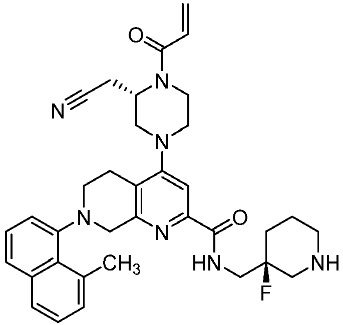 | 4-((*S*)-4-acryloyl-3-(cyanomethyl)piperazin-1-yl)-N-(((*S*)-3-fluoropiperidin-3-yl)methyl)-7-(8-methylnaphthalen-1-yl)-5,6,7,8-tetrahydro-1,7-naphthyridine-2-carboxamide | Calc'd 609.3, found 610.6 (M+H)⁺ |

### BIOLOGICAL ASSAYS:

### Procedure for SOS-catalyzed nucleotide exchange assay

The SOS-catalyzed nucleotide exchange assay utilizes a preformed complex of recombinant biotinylated KRAS protein containing G12C/C51S/C80L/C118S mutations (183 amino acids; biotin on K10; leader sequence which is an AviTag; referred to as SEQ ID NO: 1 or "Biotinylated KRAS G12C protein" hereafter), Bodipy-GDP, and Terbium-streptavidin. Compounds are added to this complex, and then after 60 minute incubation time the mixture is treated with recombinant SOS protein and unlabeled GTP. Small molecule inhibitors stabilize the Bodipy-GDP complex whereas the untreated protein rapidly exchanges Bodipy-GDP for unlabeled GTP resulting in reduced TR-FRET signal.

Biotinylated KRAS G12C protein (SEQ ID NO:1) is diluted to 2 µM in an EDTA buffer (20 mM HEPES, 150 mM sodium chloride, 10 mM EDTA, and 0.01% Tween) and incubated at room temperature for one hour. This mixture is then further diluted to 90 nM in an assay buffer (20 mM HEPES, 150 mM sodium chloride, 10 mM magnesium chloride, and 0.005% Tween) containing 15nM of Terbium-Streptavidin (Invitrogen, catalog# PV3577) and 900 nM of Bodipy-GDP and incubated at room temperature for six hours. This solution is referred to as Biotinylated KRAS G12C mixture.

Each test compound (10 mM stock in DMSO) is diluted in DMSO to make a 10-point, 3-fold dilution series in a 384-well low dead volume microplate (Labcyte, catalog# LP-0200). Once titrations are made, 10 nL of the diluted compounds is acoustically dispensed into a 384-well assay plate (Corning, catalog# 3820) using an Echo 550 (Labcyte).

Each well of the assay plate receives 3 µL Biotinylated KRAS G12C mixture that had been incubating for six hours and 3 µL of assay buffer using a BioRAPTR (Beckman Coulter) and is incubated at room temperature for 60 minutes. Each well then receives 3 µL of 240 nM recombinant human SOS protein and 9 mM GTP (Sigma, G8877) in assay buffer and is incubated at room temperature for 60 minutes. The time-resolved fluorescence resonance energy transfer signal for the assay plate is measured on an Envision (PerkinElmer) plate reader: Excitation filter = 340 nm; emission1 = 495 nm; emission2 = 520 nm; dichroic mirror = D400/D505; delay time = 100 µs. The signal of each well is determined as the ratio of the emission at 520 nm to that at 495 nm. Percent effect of each well is determined after normalization to control wells containing DMSO (no effect) or a saturating concentration of inhibitor (max effect). The apparent effect as a function of compound concentration is fit to a four parameter logistic equation.

**Table 1. In vitro apparent potency (IC₅₀) in the SOS-catalyzed nucleotide exchange assay with preincubation time of 60 minutes prior to addition of SOS.**

| **Compound** | **IC₅₀ (nM) at 60 min** |
|---|---|
| 1 | 3916 |
| 2 | 3150 |
| 4 | 9401 |
| 5 | 6019 |
| 6 | 1695 |
| 7 | 1585 |
| 12 | 66 |
| 13 | 826 |
| 14 | 614 |
| 16 | 962 |
| 18 | 145 |
| 19 | 3735 |
| 21 | 3182 |
| 23 | 90 |
| 24 | 1486 |
| 25 | 74 |
| 26 | 187 |
| 28 | 7320 |
| 29 | 259 |
| 30 | 4899 |
| 31 | 426 |
| 32 | 4474 |
| 33 | 6654 |
| 34 | 3575 |
| 36 | 1520 |
| 37 | 468 |
| 38 | 4766 |
| 40 | 3540 |
| 41 | 9595 |
| 42 | 3774 |
| 44 | 7866 |
| 45 | 5350 |
| 46 | 8882 |
| 47 | 7746 |
| 48 | 7293 |
| 49 | 2176 |
| 50 | 3574 |
| 52 | 5496 |
| 53 | 5364 |
| 54 | 9149 |
| 56 | 3647 |
| 57 | 3409 |
| 58 | 2475 |
| 60 | 8.8 |
| 61 | 13 |
| 62 | 51 |
| 63 | 8.0 |
| 64 | 2.7 |
| 65 | 2.1 |
| 66 | 1.0 |
| 67 | 5.9 |
| 68 | 29 |
| 69 | 22 |
| 70 | 2.2 |
| 71 | 32 |
| 72 | 529 |
| 73 | 9.7 |
| 74 | 5.4 |
| 75 | 7.2 |
| 76 | 6.2 |
| 77 | 5.0 |
| 78 | 8.3 |
| 79 | 12 |
| 80 | 91 |
| 81 | 29 |
| 82 | 4.8 |
| 83 | 90 |
| 84 | 0.9 |
| 85 | 180 |
| 86 | 11 |
| 87 | 15 |
| 88 | 4.3 |
| 89 | 15 |
| 90 | 21 |
| 91 | 12 |
| 92 | 20 |
| 93 | 1843 |
| 94 | 12 |
| 95 | 793 |
| 96 | 56 |
| 97 | 15 |
| 98 | 6.2 |
| 99 | 8.3 |
| 100 | 22 |
| 101 | 4.5 |
| 102 | 5.4 |
| 103 | 26 |
| 104 | 12 |
| 105 | 17 |
| 106 | 33 |
| 107 | 54 |

**Table 2. In vitro apparent potency (% inhibition at 10,000 nM) in the SOS-catalyzed nucleotide exchange assay with 60 minutes of preincubation prior to addition of SOS.**

| **Compound** | **% Inhibition at 10,000 nM (60 min)** |
|---|---|
| 4 | 23 |
| 9 | 33 |
| 15 | 33 |
| 17 | 16 |
| 20 | 30 |
| 22 | 37 |
| 27 | 29 |
| 35 | 39 |
| 43 | 33 |
| 51 | 36 |
| 55 | 31 |
| 59 | 38 |

### Protein sequence

### Biotinylated KRAS G12C protein

GLNDIFEAQKIEWHETEYKLVVVGACGVGKSALTIQLIQNHFVDEYDPTIEDSYRKQVVI
DGETSLLDILDTAGQEEYSAMRDQYMRTGEGFLLVFAINNTKSFEDIHHYREQIKRVKD
SEDVPMVLVGNKSDLPSRTVDTKQAQDLARSYGIPFIETSAKTRQGVDDAFYTLVREIR
KHKEK (SEQ ID NO: 1)

### Procedure for cellular phospho-ERK assay

NCI-H358 cells (ATCC^{®} CRL-5807^{™}) were cultured in T150 flask in growth medium (RPMI medium 1640-GlutaMAX^{™}-I (ThermoFisher Scientific 61870) containing 10% fetal bovine serum (ThermoFisher Scientific 10091148)). The cells were harvested in growth medium after TrypLE (ThermoFisher scientific 12604021) digestion and were seeded in 384-well collagen coated cell culture plate (Corning 356702) at a density of 15,000 cells/well, and incubated at 37°C, 5% CO₂ overnight. The compound dose-response titrations were prepared and appropriate amounts of compounds were dispensed in 384-well intermediate plate using Echo 550 liquid handler. RPMI medium 1640-GlutaMAX^{™}-I were added to the intermediate plate and transferred to 384-well cell culture plate, which was incubated at 37°C, 5% CO₂ for 2 hours. After removal of medium from the plate, cells were lysed in lysis buffer from Alpha *SureFire*^{®} *Ultra*^{™} Multiplex p-Erk and total Erk assay kit (PerkinElmer MPSU-PTERK) containing Halt^{™} Protease and Phosphotase inhibitor cocktail (ThermoFisher Scientific 78446) at room temperature with constant shaking at 300 rpm for 30 minutes. The cell lysates were then transferred to OptiPlate-384 plate (PerkinElmer 6005620) and the phosphorylation of Erk and total Erk levels were detected by Alpha SureFire Ultra Multiplex p-Erk kit (PerkinElmer MPSU-PTERK) following the manufacturer's protocol. Assay plates were read on an EnVision Multimode Plate Reader (PerkinElmer) and the ratio of p-Erk vs total Erk in each well was used as the final readout. Dose response curves were analyzed using a 4-parameter logistic model to calculate IC₅₀ values using spotfire software.

**Table 3. In vitro potency in the cellular phospho-ERK assay after 2 hour incubation.**

| **Compound** | **IC₅₀ (nM)** |
|---|---|
| 12 | 5938 |
| 23 | 9772 |
| 25 | 5644 |
| 60 | 375 |
| 61 | 1236 |
| 62 | 2408 |
| 63 | 427 |
| 64 | 106 |
| 65 | 16 |
| 66 | 41 |
| 67 | 433 |
| 68 | 2891 |
| 69 | 2005 |
| 70 | 57 |
| 71 | 5551 |
| 72 | 25450 |
| 73 | 257 |
| 74 | 45 |
| 75 | 68 |
| 76 | 207 |
| 77 | 611 |
| 78 | 1251 |
| 79 | 1276 |
| 80 | 6357 |
| 81 | 2971 |
| 82 | 324 |
| 83 | 1605 |
| 84 | 96 |
| 85 | 3547 |
| 86 | 1653 |
| 87 | 26740 |
| 88 | 1005 |
| 89 | 5682 |
| 90 | 2337 |
| 91 | 7859 |
| 92 | 7204 |
| 93 | 24380 |
| 94 | 6630 |
| 96 | 5469 |
| 97 | 2475 |
| 98 | 1044 |
| 99 | 1741 |
| 100 | 5879 |
| 101 | 1801 |
| 102 | 809 |
| 103 | 14940 |
| 104 | 4898 |
| 105 | 4893 |
| 106 | 5238 |
| 107 | 7346 |

## Claims

1. A compound of Formula (I) 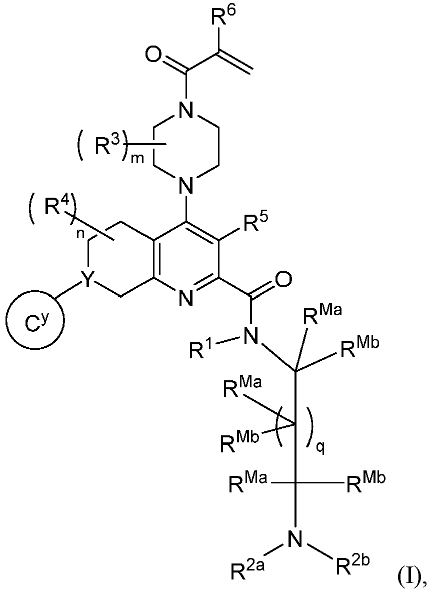 wherein:
Y is N;
R¹ is:
(a) H,
(b) C₁-C₄ alkyl, wherein said C₁-C₄ alkyl is unsubstituted or substituted by 1 to 2 hydroxy, cyano, carboxy, amino, or C1-C4 alkoxy, or
(c) C₁-C₄ fluoroalkyl, wherein said C₁-C₄ fluoroalkyl is unsubstituted or substituted by 1 to 2 hydroxy, cyano, carboxy, amino, or C₁-C₄ alkoxy;
each occurrence of R^{Ma} and R^{Mb} is independently:
(a) H,
(b) C₁-C₄ alkyl, wherein said C₁-C₄ alkyl is unsubstituted or substituted by 1 to 2 fluoro, hydroxy, cyano, carboxy, amino, or C₁-C₄ alkoxy;
(c) C₁-C₄ fluoroalkyl, wherein said C₁-C₄ fluoroalkyl is unsubstituted or substituted by 1 to 2 hydroxy, cyano, carboxy, amino, or C₁-C₄ alkoxy;
(d) alternatively, R^{Ma} and R^{Mb} , when geminally substituted on a common carbon atom, together with the atom to which they are attached, form a ring M^{c}, wherein ring M^{c} is a 3- to 8-membered saturated ring optionally containing one heteroatom selected from N and O,
wherein ring M^{c} is unsubstituted or substituted by 1 to 6 fluoro, hydroxy, cyano, carboxy, amino, oxo, C₁-C₃ alkoxy, C₁-C₃ alkyl, or C₁-C₃ fluoroalkyl;
wherein said C₁-C₃ alkyl or C₁-C₃ fluoroalkyl peripheral substituent of ring M^{c} is unsubstituted or substituted by 1 to 2 hydroxy, cyano, carboxy, amino, oxo, or C1-C3 alkoxy;
(e) alternatively, R^{Ma} and R^{Mb}, when substituted on vicinal or hominal carbon atoms, together with the atoms to which they are attached, form a ring M^{d}, wherein ring M^{d} is phenyl, a 5- to 6-membered heteroaryl containing one to three heteroatoms selected from N, O, and S, or a 3- to 6-membered saturated ring optionally containing one heteroatom selected from N and O,
wherein ring M^{d} is unsubstituted or substituted by 1 to 6 fluoro, hydroxy, cyano, carboxy, amino, oxo, C₁-C₃ alkoxy, C₁-C₃ alkyl, or C₁-C₃ fluoroalkyl;
wherein said C₁-C₃ alkyl or C₁-C₃ fluoroalkyl peripheral substituent of ring M^{d} is unsubstituted or substituted by 1 to 2 hydroxy, cyano, carboxy, amino, oxo, or C1-C3 alkoxy;
(f) alternatively, any R^{Ma} and R¹, together with the atoms to which they are attached, form ring M^{e}, wherein ring M^{e} is a 3- to 6-membered heterocycloalkyl ring optionally containing one additional heteroatom selected from N and O,
wherein ring M^{e} is unsubstituted or substituted by 1 to 6 fluoro, hydroxy, cyano, carboxy, amino, oxo, C₁-C₃ alkoxy, C₁-C₃ alkyl, or C₁-C₃ fluoroalkyl;
wherein said C₁-C₃ alkyl or C₁-C₃ fluoroalkyl peripheral substituent of ring M^{e} is unsubstituted or substituted by 1 to 2 hydroxy, cyano, carboxy, amino, oxo, or C₁-C₃ alkoxy; or
(g) alternatively, any R^{Ma} and R^{2b}, together with the atoms to which they are attached, form ring M^{f}, wherein ring M^{f} is a 4- to 7-membered heterocycloalkyl ring optionally containing one additional heteroatom selected from N and O,
wherein ring M^{f} is unsubstituted or substituted by 1 to 6 fluoro, hydroxy, cyano, carboxy, amino, oxo, C₁-C₃ alkoxy, C₁-C₃ alkyl, or C₁-C₃ fluoroalkyl;
wherein said C₁-C₃ alkyl or C₁-C₃ fluoroalkyl peripheral substituent of ring M^{f} is unsubstituted or substituted by 1 to 2 hydroxy, cyano, carboxy, amino, oxo, or C₁-C₃ alkoxy;
R^{2a} is:
(a) H,
(b) C₁-C₄ alkyl, wherein said C₁-C₄ alkyl is unsubstituted or substituted by 1 to 2 hydroxy, cyano, carboxy, amino, or C₁-C₄ alkoxy, or
(c) C1-C4 fluoroalkyl, wherein said C1-C4 fluoroalkyl is unsubstituted or substituted by 1 to 2 hydroxy, cyano, carboxy, amino, or C₁-C₄ alkoxy;
R^{2b} is:
(a) H,
(b) C₁-C₄ alkyl, wherein said C₁-C₄ alkyl is unsubstituted or substituted by 1 to 2 hydroxy, cyano, carboxy, amino, or C₁-C₄ alkoxy,
(c) C1-C4 fluoroalkyl, wherein said C1-C4 fluoroalkyl is unsubstituted or substituted by 1 to 2 hydroxy, cyano, carboxy, amino, or C₁-C₄ alkoxy; or
(d) R^{2b} and R^{Ma}, together with the atoms to which they are attached, form ring M^{f} as described above,
or alternatively R^{2a} and R^{2b}, together with the nitrogen atom to which they are attached, form ring C^{Z}, wherein ring C^{Z} is a 3- to 7-membered heterocycloalkyl optionally containing one additional heteroatom selected from N and O,
wherein ring C^{Z} is unsubstituted or substituted by 1 to 6 fluoro, hydroxy, cyano, carboxy, amino, oxo, C₁-C₃ alkoxy, C₁-C₃ alkyl, or C₁-C₃ fluoroalkyl;
wherein said C₁-C₃ alkyl or C₁-C₃ fluoroalkyl peripheral substituent of ring C^{Z} is unsubstituted or substituted by 1 to 2 hydroxy, cyano, carboxy, amino, oxo, or C1-C3 alkoxy;
ring C^{y} is:
(a) C₆-C₁₀ aryl, or
(b) a five- to ten-membered heteroaryl containing one to four heteroatoms selected from N, O, and S;
wherein ring CY is unsubstituted or substituted by one to four halo, hydroxy, C₁-C₃ alkyl, C₁-C₃ fluoroalkyl, C₁-C₃ alkoxy, cyano, or C₁-C₄ hydroxyalkyl;
R³ is C₁-C₄ alkyl, C₁-C₄ cyanoalkyl, C₁-C₄ fluoroalkyl, C₁-C₄ hydroxyalkyl, oxo, carboxy, or alternatively, two R³ substituents, together with the carbon atoms to which they are attached, can form a bicyclo- or spirocyclic ring system with the illustrated piperazine ring;
R⁴ is C₁-C₃ alkyl, C₁-C₃ cyanoalkyl, C₁-C₃ fluoroalkyl, hydroxy, oxo, or fluoro;
R⁵ is H, halo, cyano, C₁-C₃ alkyl, C₁-C₃ fluoroalkyl or C₁-C₃ alkoxy;
R⁶ is H or halo;
the subscript m is 0, 1, 2, or 3;
the subscript n is 0, 1 or 2; and
the subscript q is 0 or 1;
or a pharmaceutically acceptable salt thereof.

2. The compound of claim 1 or a pharmaceutically salt thereof, wherein each occurrence of R^{Ma} and R^{Mb} is independently:
(a) H,
(b) C₁-C₄ alkyl, wherein said C₁-C₄ alkyl is unsubstituted or substituted by 1 to 2 fluoro, hydroxy, cyano, carboxy, amino, or C₁-C₄ alkoxy;
(c) C1-C4 fluoroalkyl, wherein said C1-C4 fluoroalkyl is unsubstituted or substituted by 1 to 2 hydroxy, cyano, carboxy, amino, or C₁-C₄ alkoxy;
(d) alternatively, R^{Ma} and R^{Mb} , when geminally substituted on a common carbon atom, together with the atom to which they are attached, form a ring M^{c}, wherein ring M^{c} is a 3- to 6-membered saturated ring optionally containing one heteroatom selected from N and O,
wherein ring M^{c} is unsubstituted or substituted by 1 to 6 fluoro, hydroxy, cyano, carboxy, amino, oxo, C₁-C₃ alkoxy, C₁-C₃ alkyl, or C₁-C₃ fluoroalkyl;
wherein said C₁-C₃ alkyl or C₁-C₃ fluoroalkyl peripheral substituent of ring M^{c} is unsubstituted or substituted by 1 to 2 hydroxy, cyano, carboxy, amino, oxo, or C₁-C₃ alkoxy;
(e) alternatively, R^{Ma} and R^{Mb}, when substituted on vicinal or hominal carbon atoms, together with the atoms to which they are attached, form a ring M^{d}, wherein ring M^{d} is phenyl, a 5- to 6-membered heteroaryl containing one to three heteroatoms selected from N, O, and S, or a 3- to 6-membered saturated ring optionally containing one heteroatom selected from N and O,
wherein ring M^{d} is unsubstituted or substituted by 1 to 6 fluoro, hydroxy, cyano, carboxy, amino, oxo, C₁-C₃ alkoxy, C₁-C₃ alkyl, or C₁-C₃ fluoroalkyl;
wherein said C₁-C₃ alkyl or C₁-C₃ fluoroalkyl peripheral substituent of ring M^{d} is unsubstituted or substituted by 1 to 2 hydroxy, cyano, carboxy, amino, oxo, or C₁-C₃ alkoxy;
(f) alternatively, any R^{Ma} and R¹, together with the atoms to which they are attached, form ring M^{e}, wherein ring M^{e} is a 3- to 6-membered heterocycloalkyl ring optionally containing one additional heteroatom selected from N and O,
wherein ring M^{e} is unsubstituted or substituted by 1 to 6 fluoro, hydroxy, cyano, carboxy, amino, oxo, C₁-C₃ alkoxy, C₁-C₃ alkyl, or C₁-C₃ fluoroalkyl;
wherein said C₁-C₃ alkyl or C₁-C₃ fluoroalkyl peripheral substituent of ring M^{e} is unsubstituted or substituted by 1 to 2 hydroxy, cyano, carboxy, amino, oxo, or C1-C3 alkoxy; or
(g) alternatively, any R^{Ma} and R^{2b}, together with the atoms to which they are attached, form ring M^{f}, wherein ring M^{f} is a 4- to 7-membered heterocycloalkyl ring optionally containing one additional heteroatom selected from N and O,
wherein ring M^{f} is unsubstituted or substituted by 1 to 6 fluoro, hydroxy, cyano, carboxy, amino, oxo, C₁-C₃ alkoxy, C₁-C₃ alkyl, or C₁-C₃ fluoroalkyl;
wherein said C₁-C₃ alkyl or C₁-C₃ fluoroalkyl peripheral substituent of ring M^{f} is unsubstituted or substituted by 1 to 2 hydroxy, cyano, carboxy, amino, oxo, or C₁-C₃ alkoxy; and
R⁶ is H.

3. The compound of claim 1 or 2 or a pharmaceutically acceptable salt thereof, wherein:
(a) the subscript q is 0; and/or
(b) R¹ is H.

4. The compound of claim 1 or a pharmaceutically acceptable salt thereof, wherein Y is N, the subscript q is 0, and R¹ is H; optionally wherein each R^{Ma} and R^{Mb} are independently:
(a) H,
(b) C₁-C₄ alkyl, wherein said C₁-C₄ alkyl is unsubstituted or substituted by 1 to 2 fluoro, hydroxy, cyano, carboxy, amino, or C₁-C₄ alkoxy; or
(c) C₁-C₄ fluoroalkyl, wherein said C₁-C₄ fluoroalkyl is unsubstituted or substituted by 1 to 2 hydroxy, cyano, carboxy, amino, or C₁-C₄ alkoxy.

5. The compound of claim 4 or a pharmaceutically acceptable salt thereof, wherein R^{Ma} and R^{Mb} are each independently H or C₁-C₄ alkyl and/or R^{2a} and R^{2b} are each independently:
(a) H,
(b) C₁-C₄ alkyl, wherein said C₁-C₄ alkyl is unsubstituted or substituted by 1 to 2 hydroxy, cyano, carboxy, amino, or C₁-C₄ alkoxy, or
(c) C₁-C₄ fluoroalkyl, wherein said C₁-C₄ fluoroalkyl is unsubstituted or substituted by 1 to 2 hydroxy, cyano, carboxy, amino, or C₁-C₄ alkoxy;
optionally wherein R^{2a} and R^{2b} are each independently H or C₁-C₄ alkyl.

6. The compound of claim 1 or a pharmaceutically acceptable salt thereof, wherein the group 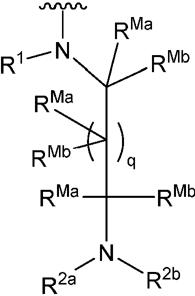 is selected from:

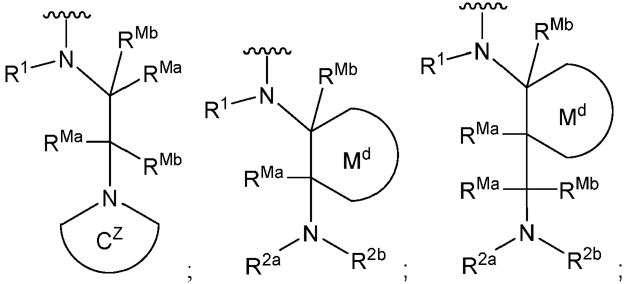

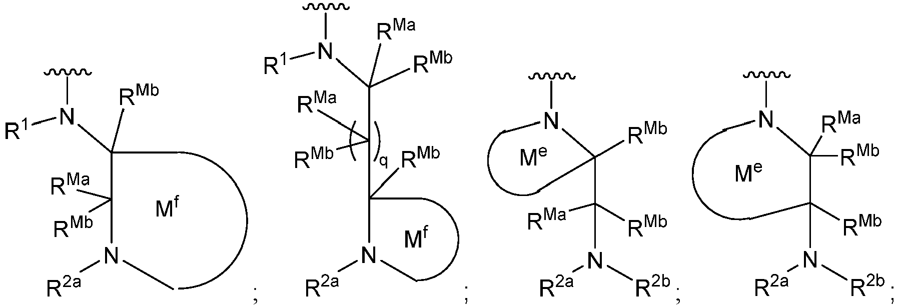

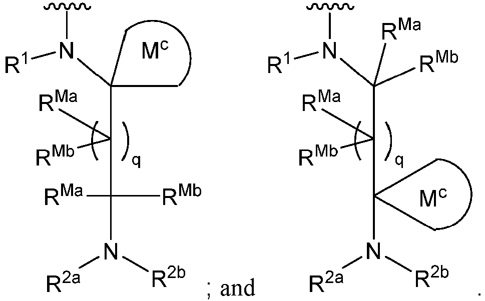

7. The compound of claim 6 or a pharmaceutically acceptable salt thereof, wherein:
(a) the group 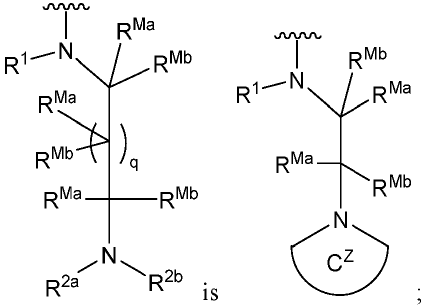 wherein ring C^{Z} is azetidine, pyrrolidine, piperidine, piperazine or morpholine; or
(b) the group 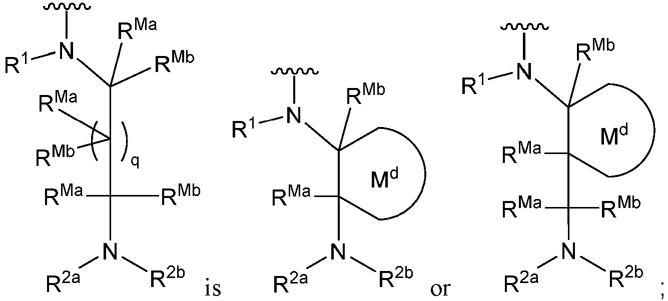 wherein:
i) ring M^{d} is a 3- to 6-membered cycloalkyl ring or a 3- to 6-membered saturated heterocycloalkyl ring containing one heteroatom selected from N and O, optionally wherein ring M^{d} is a 3- to 6-membered cycloalkyl ring; or
ii) ring M^{d} is phenyl.

8. The compound of claim 6 or a pharmaceutically acceptable salt thereof, wherein the group 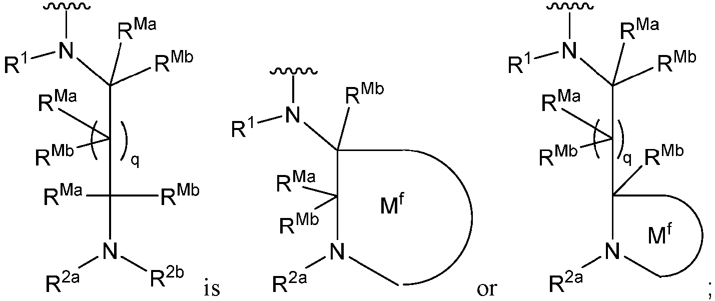 wherein: (a) ring M^{f} is an azetidine, pyrrolidine, piperidine, or azepine ring; optionally a pyrrolidine or piperidine ring; and/or (b) R^{2a} is methyl.

9. The compound of claim 6 or a pharmaceutically acceptable salt thereof, wherein:
(a) the group 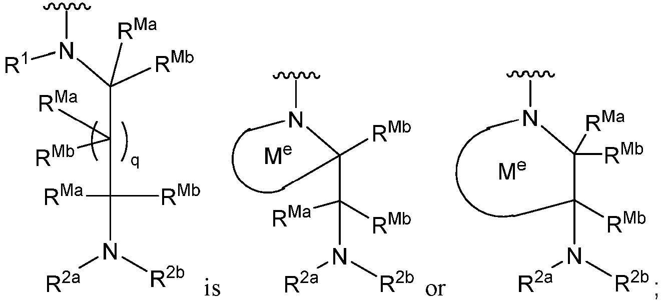 wherein ring M^{e} is a pyrrolidine ring; or
(b) the group 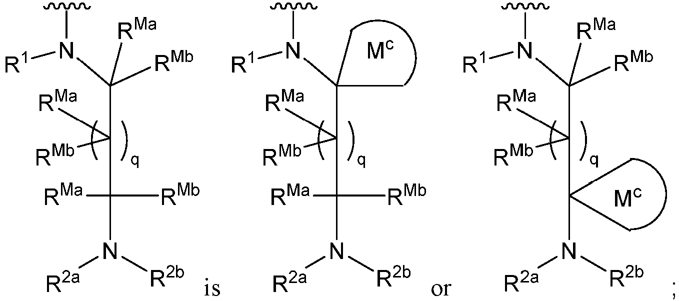 wherein ring M^{c} is cyclopropane, the subscript q is 0, and R¹ is H.

10. The compound of any one of claims 1-9 or a pharmaceutically acceptable salt thereof, wherein:
(a) the subscript m is 0 or 1; and/or
(b) the subscript n is 0; and/or
(c) R⁵ is H.

11. The compound of any one of claims 1-10 selected from the group consisting of: 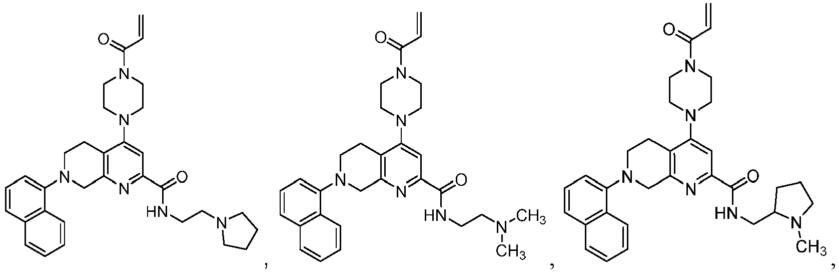 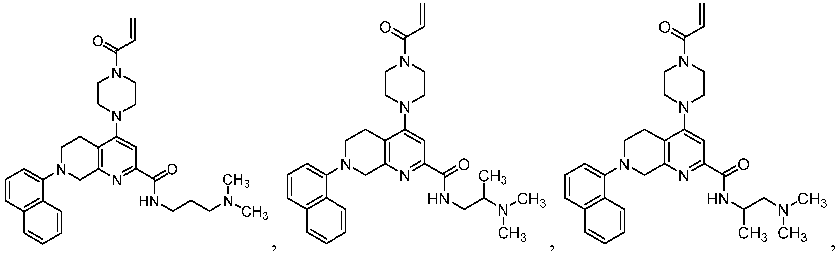 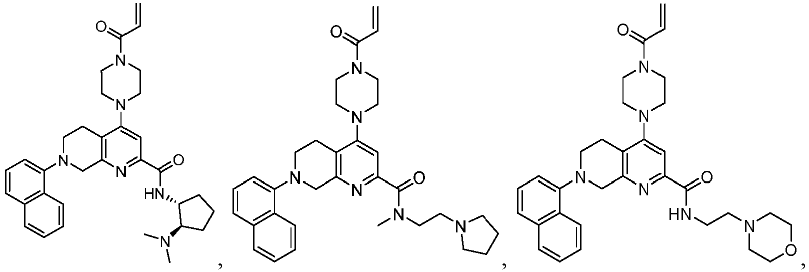 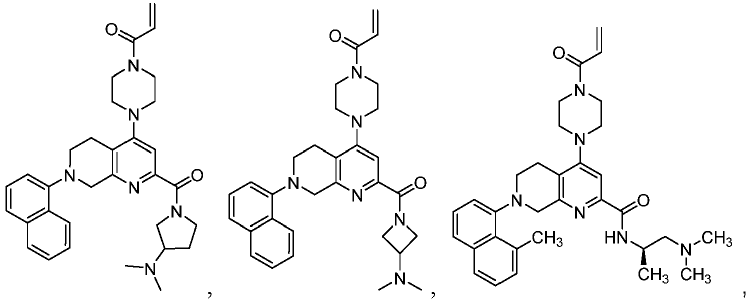 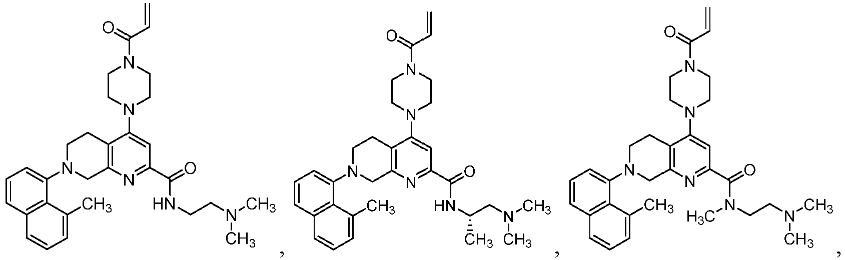 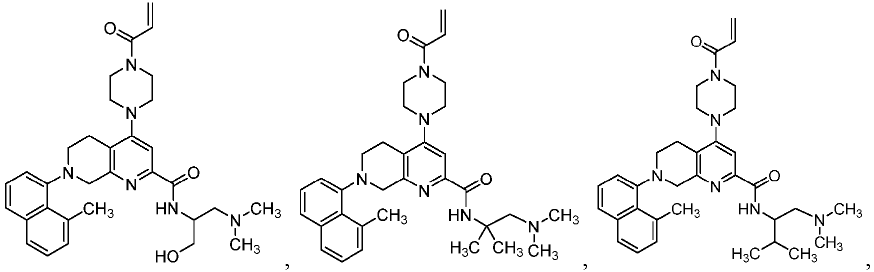 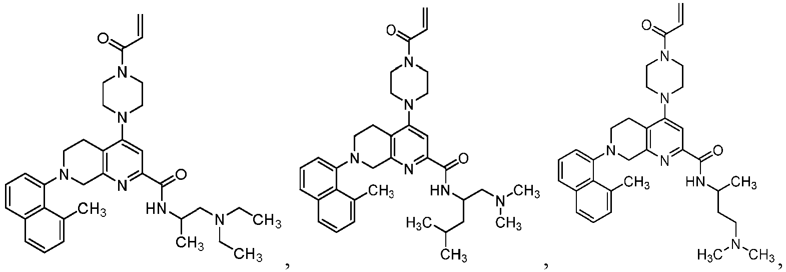 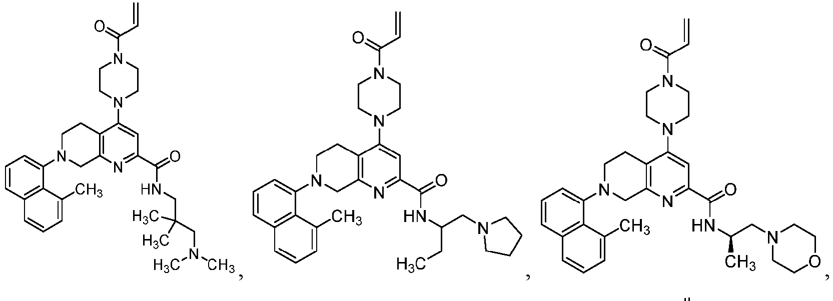 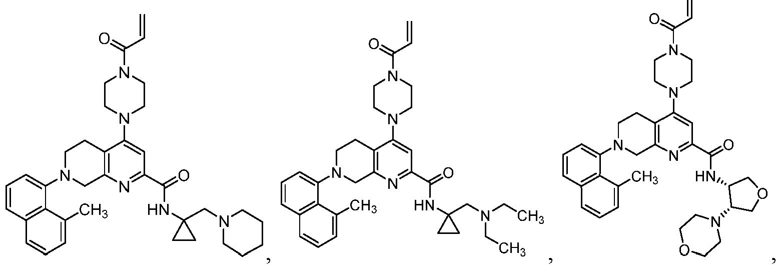 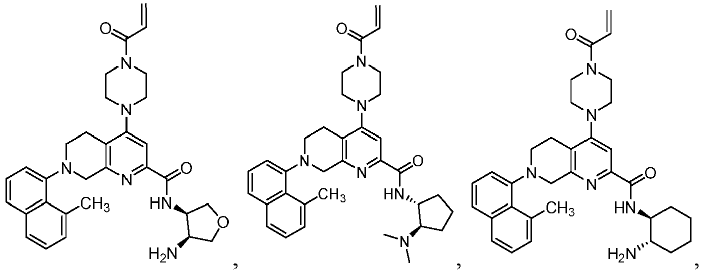 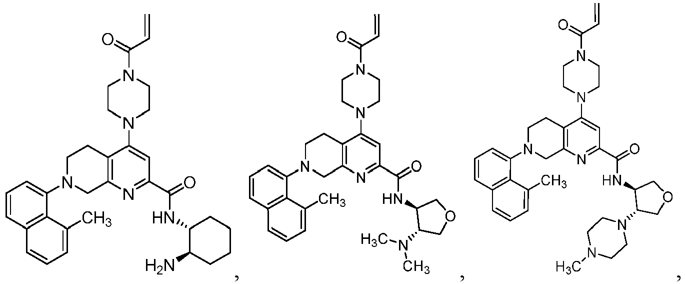 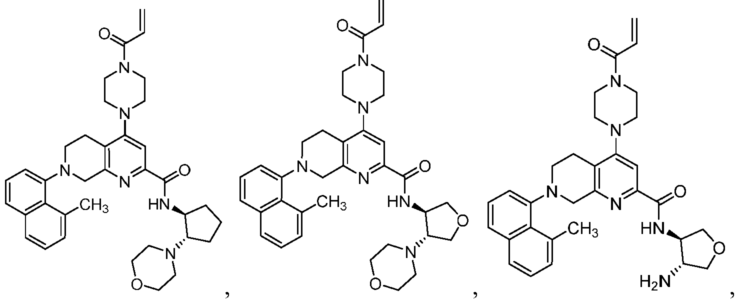 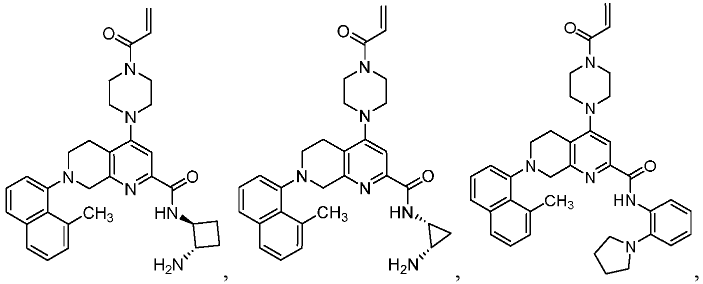 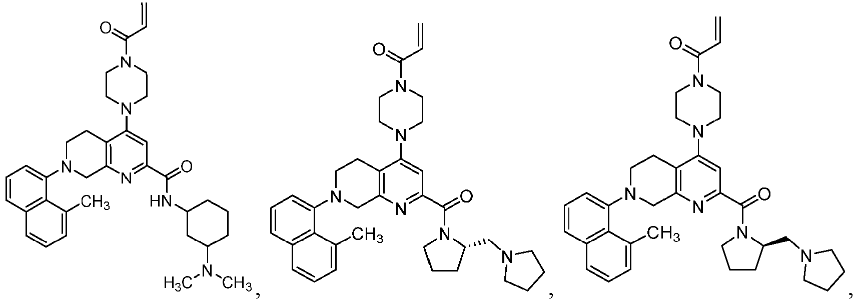 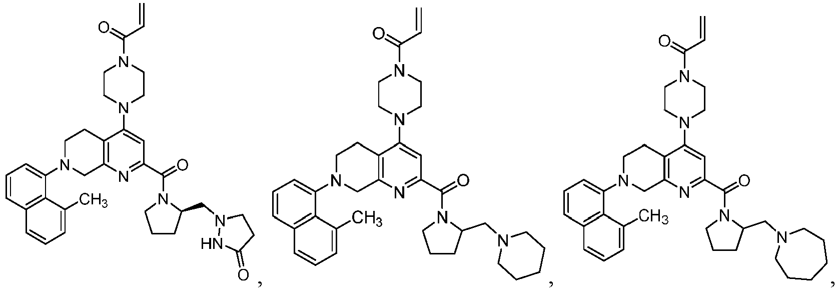 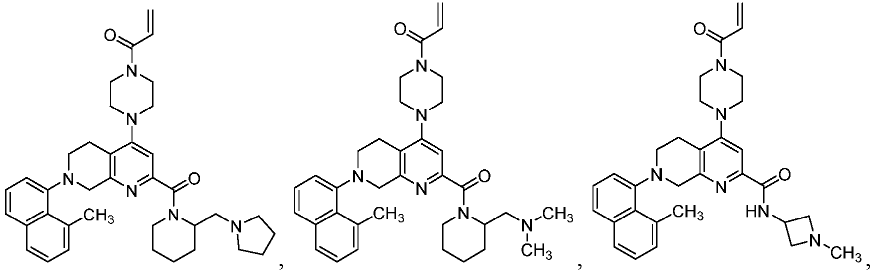 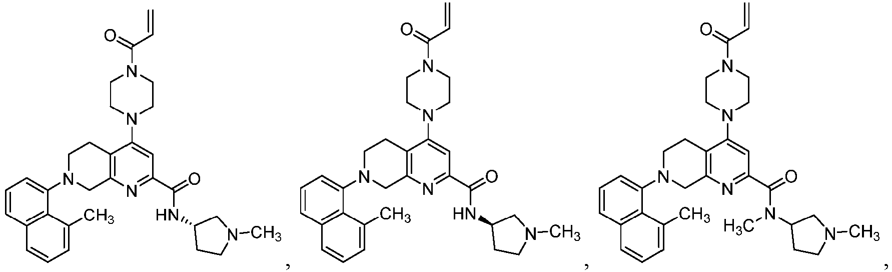 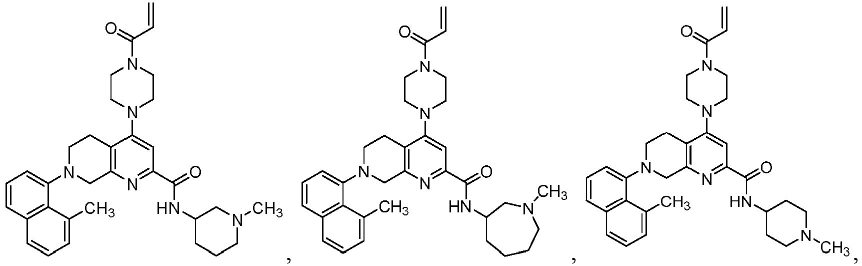 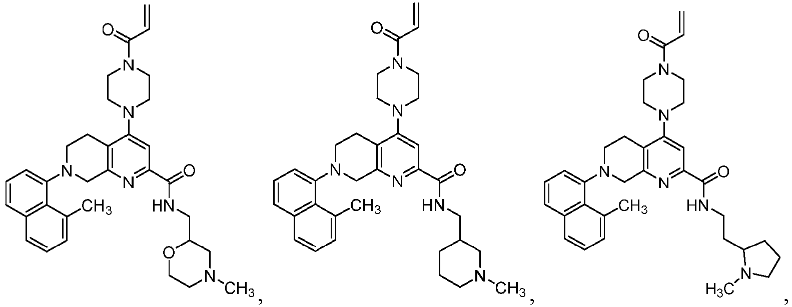 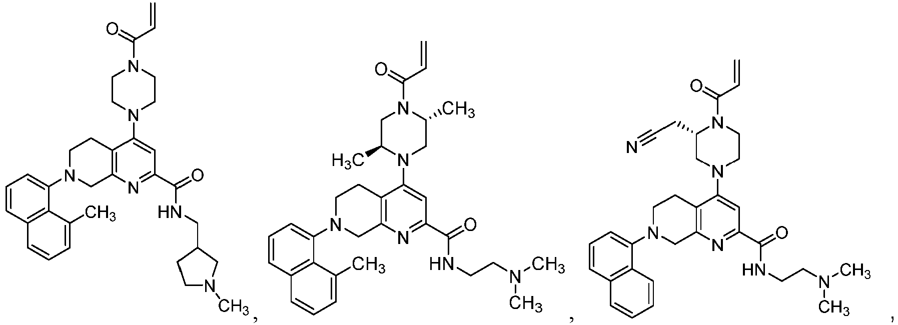 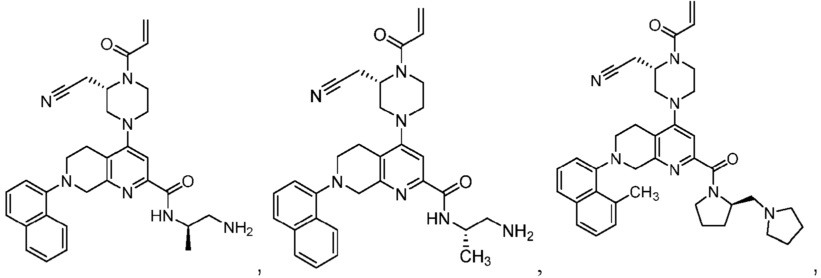 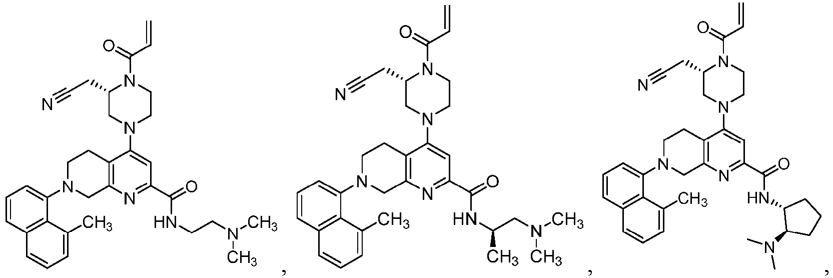 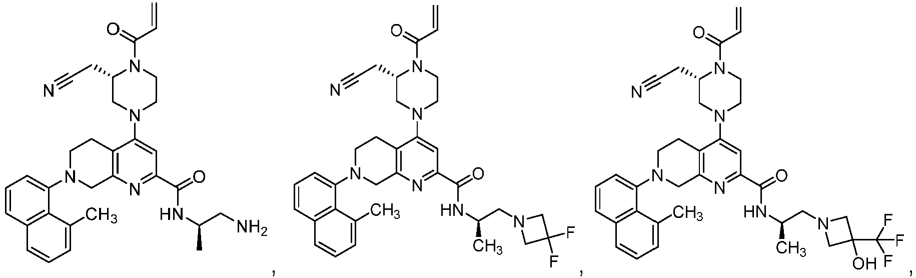 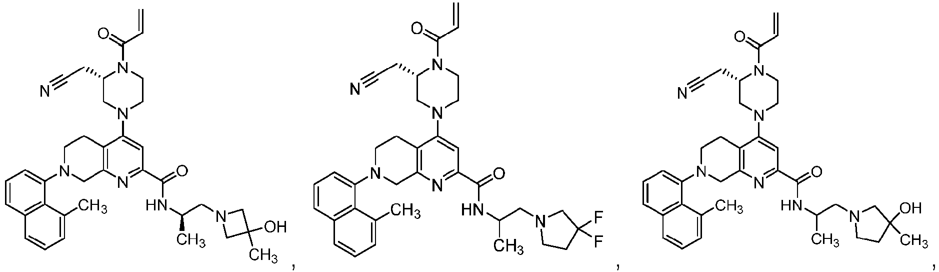 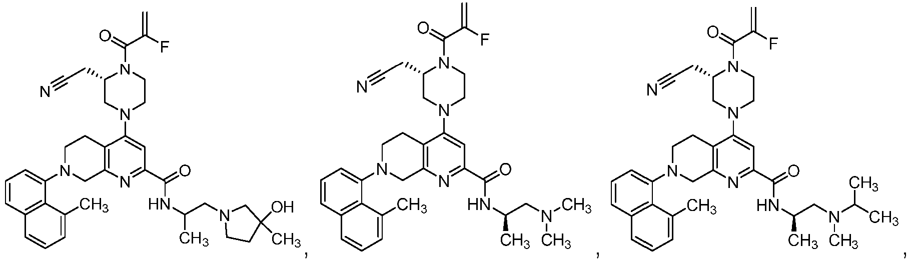 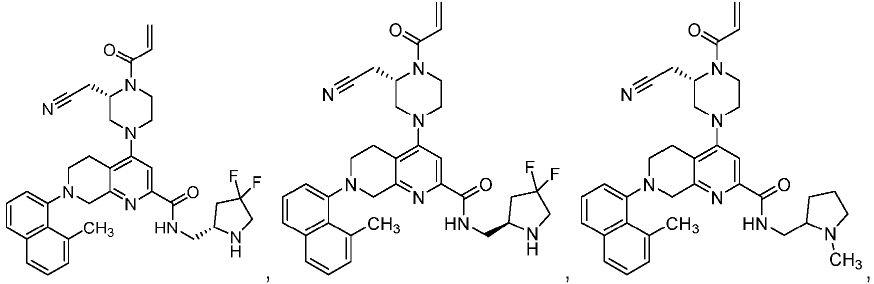 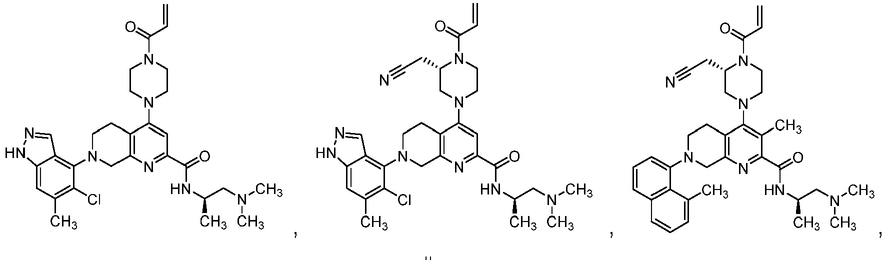 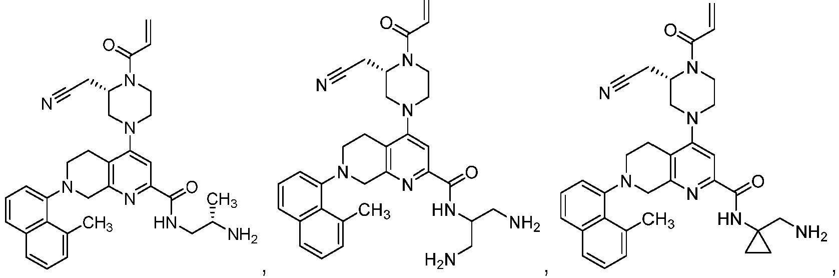 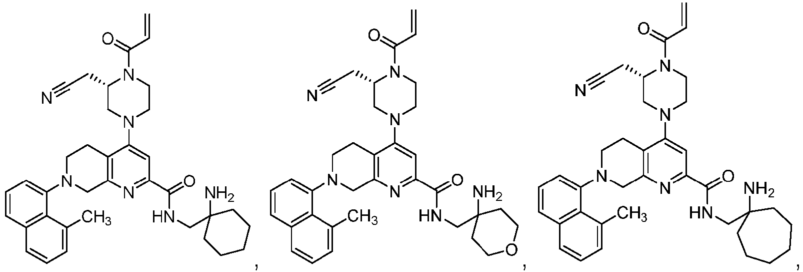 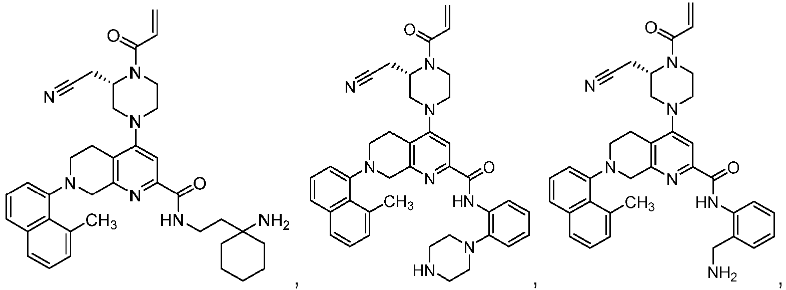 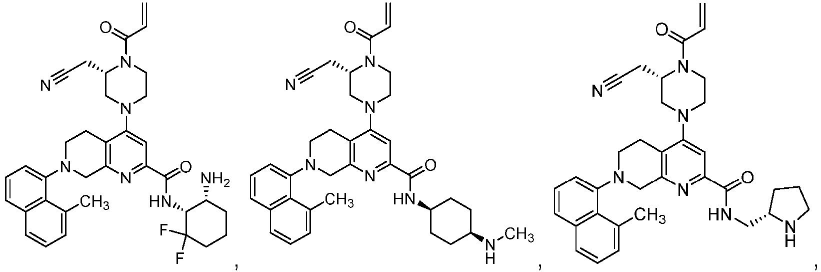 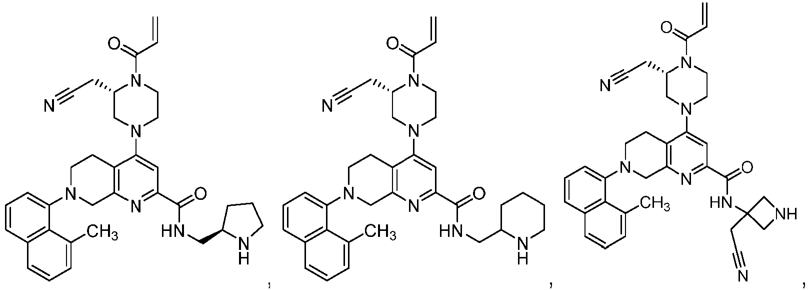 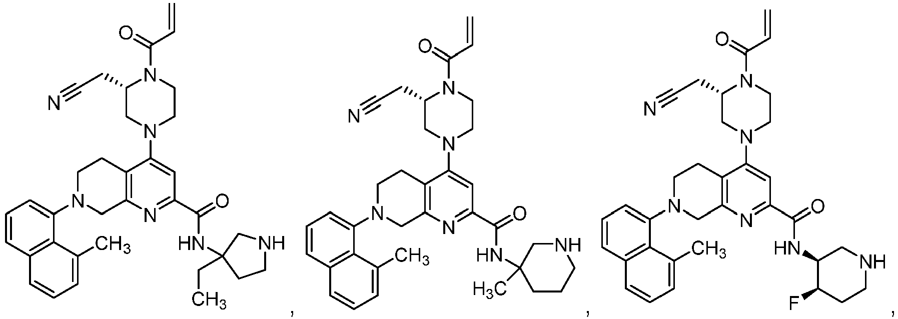 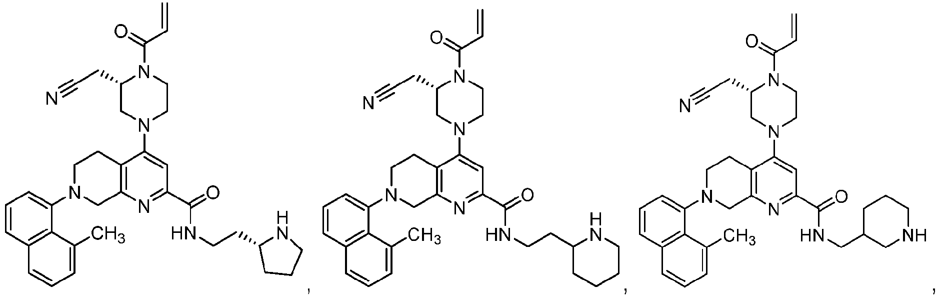 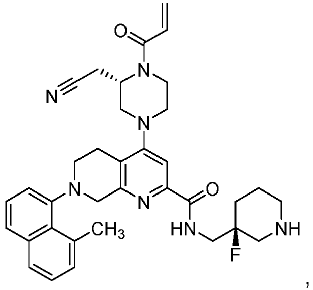 or a pharmaceutically acceptable salt thereof.

12. A pharmaceutical composition comprising the compound of any of claims 1-11, or the pharmaceutically acceptable salt thereof, and a pharmaceutically acceptable carrier.

13. The compound of any one of claims 1-11, or the pharmaceutically acceptable salt thereof, for use in therapy.

14. The compound of any one of claims 1-11, or the pharmaceutically acceptable salt thereof, or a pharmaceutical composition comprising the compound of any one of claims 1-11, or the pharmaceutically acceptable salt thereof, for use in treating cancer.

15. The compound of any one of claims 1-11, or the pharmaceutically acceptable salt thereof, and an additional anti-cancer agent, or a pharmaceutical composition comprising the compound of any one of claims 1-11, or the pharmaceutically acceptable salt thereof, and an additional anti-cancer agent, for use in the treatment of cancer.

## Patentansprüche

1. Verbindung der Formel (I) 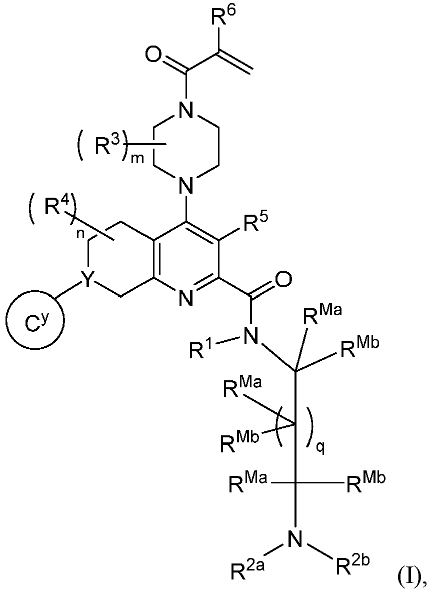 wobei:
Y für N steht;
R¹ für Folgendes steht:
(a) H,
(b) C₁-C₄-Alkyl, wobei das C₁-C₄-Alkyl unsubstituiert oder durch 1 bis 2 Hydroxy, Cyano, Carboxy, Amino oder C₁-C₄-Alkoxy substituiert ist, oder
(c) C₁-C₄-Fluoralkyl, wobei das C₁-C₄-Fluoralkyl unsubstituiert oder durch 1 bis 2 Hydroxy, Cyano, Carboxy, Amino oder C₁-C₄-Alkoxy substituiert ist;
jedes Vorkommen von R^{Ma} und R^{Mb} unabhängig für Folgendes steht:
(a) H,
(b) C₁-C₄-Alkyl, wobei das C₁-C₄-Alkyl unsubstituiert oder durch 1 bis 2 Fluor, Hydroxy, Cyano, Carboxy, Amino oder C₁-C₄-Alkoxy substituiert ist;
(c) C₁-C₄-Fluoralkyl, wobei das C₁-C₄-Fluoralkyl unsubstituiert oder durch 1 bis 2 Hydroxy, Cyano, Carboxy, Amino oder C₁-C₄-Alkoxy substituiert ist;
(d) alternativ dazu R^{Ma} und R^{Mb} dann, wenn sie geminal an einem gemeinsamen Kohlenstoffatom substituiert sind, zusammen mit dem Atom, an das sie gebunden sind, einen Ring M^{c} bilden, wobei es sich bei dem Ring M^{c} um einen 3-bis 8-gliedrigen gesättigten Ring handelt, der gegebenenfalls ein aus N und O ausgewähltes Heteroatom enthält,
wobei der Ring M^{c} unsubstituiert oder durch 1 bis 6 Fluor, Hydroxy, Cyano, Carboxy, Amino, Oxo, C₁-C₃-Alkoxy, C₁-C₃-Alkyl oder C₁-C₃-Fluoralkyl substituiert ist;
wobei der periphere C₁-C₃-Alkyl- oder C₁-C₃-Fluoralkylsubstituent von Ring M^{c} unsubstituiert oder durch 1 bis 2 Hydroxy, Cyano, Carboxy, Amino, Oxo oder C₁-C₃-Alkoxy substituiert ist;
(e) alternativ dazu R^{Ma} und R^{Mb} dann, wenn sie an vicinalen oder hominalen Kohlenstoffatomen substituiert sind, zusammen mit den Atomen, an die sie gebunden sind, einen Ring M^{d} bilden,
wobei der Ring M^{d} für Phenyl, ein 5- bis 6-gliedriges Heteroaryl mit einem bis drei aus N, O und S ausgewählten Heteroatomen oder einen 3- bis 6-gliedrigen gesättigten Ring, der gegebenenfalls ein aus N und O ausgewähltes Heteroatom enthält, steht,
wobei der Ring M^{d} unsubstituiert oder durch 1 bis 6 Fluor, Hydroxy, Cyano, Carboxy, Amino, Oxo, C₁-C₃-Alkoxy, C₁-C₃-Alkyl oder C₁-C₃-Fluoralkyl substituiert ist;
wobei der periphere C₁-C₃-Alkyl- oder C₁-C₃-Fluoralkylsubstituent von Ring M^{d} unsubstituiert oder durch 1 bis 2 Hydroxy, Cyano, Carboxy, Amino, Oxo oder C₁-C₃-Alkoxy substituiert ist;
(f) alternativ dazu jedes R^{Ma} und R¹ zusammen mit den Atomen, an die sie gebunden sind, einen Ring M^{e} bilden, wobei der Ring M^{e} für einen 3- bis 6-gliedrigen Heterocycloalkylring, der gegebenenfalls ein zusätzliches, aus N und O ausgewähltes Heteroatom enthält, steht,
wobei der Ring M^{e} unsubstituiert oder durch 1 bis 6 Fluor, Hydroxy, Cyano, Carboxy, Amino, Oxo, C₁-C₃-Alkoxy, C₁-C₃-Alkyl oder C₁-C₃-Fluoralkyl substituiert ist;
wobei der periphere C₁-C₃-Alkyl- oder C₁-C₃-Fluoralkylsubstituent von Ring M^{e} unsubstituiert oder durch 1 bis 2 Hydroxy, Cyano, Carboxy, Amino, Oxo oder C₁-C₃-Alkoxy substituiert ist; oder
(g) alternativ dazu jedes ^{Ma} und R^{2b} zusammen mit den Atomen, an die sie gebunden sind, einen Ring M^{f} bilden, wobei der Ring M^{f} für einen 4- bis 7-gliedrigen Heterocycloalkylring, der gegebenenfalls ein zusätzliches, aus N und O ausgewähltes Heteroatom enthält, steht,
wobei der Ring M^{f} unsubstituiert oder durch 1 bis 6 Fluor, Hydroxy, Cyano, Carboxy, Amino, Oxo, C₁-C₃-Alkoxy, C₁-C₃-Alkyl oder C₁-C₃-Fluoralkyl substituiert ist;
wobei der periphere C₁-C₃-Alkyl- oder C₁-C₃-Fluoralkylsubstituent von Ring M^{f} unsubstituiert oder durch 1 bis 2 Hydroxy, Cyano, Carboxy, Amino, Oxo oder C₁-C₃-Alkoxy substituiert ist;
R^{2a} für Folgendes steht:
(a) H,
(b) C₁-C₄-Alkyl, wobei das C₁-C₄-Alkyl unsubstituiert oder durch 1 bis 2 Hydroxy, Cyano, Carboxy, Amino oder C₁-C₄-Alkoxy substituiert ist, oder
(c) C₁-C₄-Fluoralkyl, wobei das C₁-C₄-Fluoralkyl unsubstituiert oder durch 1 bis 2 Hydroxy, Cyano, Carboxy, Amino oder C₁-C₄-Alkoxy substituiert ist;
R^{2b} für Folgendes steht:
(a) H,
(b) C₁-C₄-Alkyl, wobei das C₁-C₄-Alkyl unsubstituiert oder durch 1 bis 2 Hydroxy, Cyano, Carboxy, Amino oder C₁-C₄-Alkoxy substituiert ist,
(c) C₁-C₄-Fluoralkyl, wobei das C₁-C₄-Fluoralkyl unsubstituiert oder durch 1 bis 2 Hydroxy, Cyano, Carboxy, Amino oder C₁-C₄-Alkoxy substituiert ist; oder
(d) R^{2b} und R^{Ma} zusammen mit den Atomen, an die sie gebunden sind, einen Ring M^{f} gemäß obiger Beschreibung bilden,
oder alternativ dazu R^{2a} und R^{2b} zusammen mit dem Stickstoffatom, an das sie gebunden sind, einen Ring C^{z} bilden, wobei der Ring C^{z} für ein 3- bis 7-gliedriges Heterocycloalkyl, das gegebenenfalls mindestens ein zusätzliches, aus N und O ausgewähltes Heteroatom enthält, steht,
wobei der Ring C^{z} unsubstituiert oder durch 1 bis 6 Fluor, Hydroxy, Cyano, Carboxy, Amino, Oxo, C₁-C₃-Alkoxy, C₁-C₃-Alkyl oder C₁-C₃-Fluoralkyl substituiert ist;
wobei der periphere C₁-C₃-Alkyl- oder C₁-C₃-Fluoralkylsubstituent von Ring C^{z} unsubstituiert oder durch 1 bis 2 Hydroxy, Cyano, Carboxy, Amino, Oxo oder C₁-C₃-Alkoxy substituiert ist;
Ring C^{y} für Folgendes steht:
(a) C₆-C₁₀-Aryl oder
(b) ein fünf- bis zehngliedriges Heteroaryl mit einem bis vier aus N, O und S ausgewählten Heteroatomen;
wobei der Ring C^{y} unsubstituiert oder durch eins bis vier Halogen, Hydroxy, C₁-C₃-Alkyl, C₁-C₃-Fluoralkyl, C₁-C₃-Alkoxy, Cyano oder C₁-C₄-Hydroxyalkyl substituiert ist;
R³ für C₁-C₄-Alkyl, C₁-C₄-Cyanoalkyl, C₁-C₄-Fluoralkyl, C₁-C₄-Hydroxyalkyl, Oxo, Carboxy steht oder alternativ dazu zwei R³-Substituenten zusammen mit den Kohlenstoffatomen,
an die sie gebunden sind, mit dem abgebildeten Piperazinring ein bicyclisches oder spirocyclisches Ringsystem bilden können;
R⁴ für C₁-C₃-Alkyl, C₁-C₃-Cyanoalkyl, C₁-C₃-Fluoralkyl, Hydroxy, Oxo oder Fluor steht;
R⁵ für H, Halogen, Cyano, C₁-C₃-Alkyl, C₁-C₃-Fluoralkyl oder C₁-C₃-Alkoxy steht;
R⁶ für H oder Halogen steht;
der tiefgestellte Index m für 0, 1, 2 oder 3 steht;
der tiefgestellte Index n für 0, 1 oder 2 steht; und
der tiefgestellte Index q für 0 oder 1 steht;
oder ein pharmazeutisch unbedenkliches Salz davon.

2. Verbindung nach Anspruch 1 oder ein pharmazeutisch unbedenkliches Salz davon, wobei jedes Vorkommen von R^{Ma} und R^{Mb} unabhängig für Folgendes steht:
(a) H,
(b) C₁-C₄-Alkyl, wobei das C₁-C₄-Alkyl unsubstituiert oder durch 1 bis 2 Fluor, Hydroxy, Cyano, Carboxy, Amino oder C₁-C₄-Alkoxy substituiert ist;
(c) C₁-C₄-Fluoralkyl, wobei das C₁-C₄-Fluoralkyl unsubstituiert oder durch 1 bis 2 Hydroxy, Cyano, Carboxy, Amino oder C₁-C₄-Alkoxy substituiert ist;
(d) alternativ dazu R^{Ma} und R^{Mb} dann, wenn sie geminal an einem gemeinsamen Kohlenstoffatom substituiert sind, zusammen mit dem Atom, an das sie gebunden sind, einen Ring M^{c} bilden, wobei es sich bei dem Ring M^{c} um einen 3-bis 6-gliedrigen gesättigten Ring handelt, der gegebenenfalls ein aus N und O ausgewähltes Heteroatom enthält,
wobei der Ring M^{c} unsubstituiert oder durch 1 bis 6 Fluor, Hydroxy, Cyano, Carboxy, Amino, Oxo, C₁-C₃-Alkoxy, C₁-C₃-Alkyl oder C₁-C₃-Fluoralkyl substituiert ist;
wobei der periphere C₁-C₃-Alkyl- oder C₁-C₃-Fluoralkylsubstituent von Ring M^{c} unsubstituiert oder durch 1 bis 2 Hydroxy, Cyano, Carboxy, Amino, Oxo oder C₁-C₃-Alkoxy substituiert ist;
(e) alternativ dazu R^{Ma} und R^{Mb} dann, wenn sie an vicinale oder hominale Kohlenstoffatome gebunden sind, zusammen mit den Atomen, an die sie gebunden sind, einen Ring M^{d} bilden, wobei der Ring M^{d} für Phenyl, ein 5- bis 6-gliedriges Heteroaryl mit einem bis drei aus N, O und S ausgewählten Heteroatomen oder einen 3- bis 6-gliedrigen gesättigten Ring, der gegebenenfalls ein aus N und O ausgewähltes Heteroatom enthält, steht,
wobei der Ring M^{d} unsubstituiert oder durch 1 bis 6 Fluor, Hydroxy, Cyano, Carboxy, Amino, Oxo, C₁-C₃-Alkoxy, C₁-C₃-Alkyl oder C₁-C₃-Fluoralkyl substituiert ist;
wobei der periphere C₁-C₃-Alkyl- oder C₁-C₃-Fluoralkylsubstituent von Ring M^{d} unsubstituiert oder durch 1 bis 2 Hydroxy, Cyano, Carboxy, Amino, Oxo oder C₁-C₃-Alkoxy substituiert ist;
(f) alternativ dazu jedes R^{Ma} und R¹ zusammen mit den Atomen, an die sie gebunden sind, einen Ring M^{e} bilden, wobei der Ring M^{e} für einen 3- bis 6-gliedrigen Heterocycloalkylring, der gegebenenfalls ein zusätzliches, aus N und O ausgewähltes Heteroatom enthält, steht,
wobei der Ring M^{e} unsubstituiert oder durch 1 bis 6 Fluor, Hydroxy, Cyano, Carboxy, Amino, Oxo, C₁-C₃-Alkoxy, C₁-C₃-Alkyl oder C₁-C₃-Fluoralkyl substituiert ist;
wobei der periphere C₁-C₃-Alkyl- oder C₁-C₃-Fluoralkylsubstituent von Ring M^{e} unsubstituiert oder durch 1 bis 2 Hydroxy, Cyano, Carboxy, Amino, Oxo oder C₁-C₃-Alkoxy substituiert ist; oder
(g) alternativ dazu jedes ^{Ma} und R^{2b} zusammen mit den Atomen, an die sie gebunden sind, einen Ring M^{f} bilden, wobei der Ring M^{f} für einen 4- bis 7-gliedrigen Heterocycloalkylring, der gegebenenfalls ein zusätzliches, aus N und O ausgewähltes Heteroatom enthält, steht,
wobei der Ring M^{f} unsubstituiert oder durch 1 bis 6 Fluor, Hydroxy, Cyano, Carboxy, Amino, Oxo, C₁-C₃-Alkoxy, C₁-C₃-Alkyl oder C₁-C₃-Fluoralkyl substituiert ist;
wobei der periphere C₁-C₃-Alkyl- oder C₁-C₃-Fluoralkylsubstituent von Ring M^{f} unsubstituiert oder durch 1 bis 2 Hydroxy, Cyano, Carboxy, Amino, Oxo oder C₁-C₃-Alkoxy substituiert ist; und
R⁶ für H steht.

3. Verbindung nach Anspruch 1 oder 2 oder ein pharmazeutisch unbedenkliches Salz davon, wobei:
(a) der tiefgestellte Index q für 0 steht; und/oder
(b) R¹ für H steht.

4. Verbindung nach Anspruch 1 oder ein pharmazeutisch unbedenkliches Salz davon, wobei Y für N steht, der tiefgestellte Index q für 0 steht und R¹ für H steht; gegebenenfalls wobei R^{Ma} und R^{Mb} jeweils unabhängig für Folgendes stehen:
(a) H,
(b) C₁-C₄-Alkyl, wobei das C₁-C₄-Alkyl unsubstituiert oder durch 1 bis 2 Fluor, Hydroxy, Cyano, Carboxy, Amino oder C₁-C₄-Alkoxy substituiert ist; oder
(c) C₁-C₄-Fluoralkyl, wobei das C₁-C₄-Fluoralkyl unsubstituiert oder durch 1 bis 2 Hydroxy, Cyano, Carboxy, Amino oder C₁-C₄-Alkoxy substituiert ist.

5. Verbindung nach Anspruch 4 oder ein pharmazeutisch unbedenkliches Salz davon, wobei R^{Ma} und R^{Mb} jeweils unabhängig für H oder C₁-C₄-Alkyl stehen und/oder R^{2a} und R^{2b} jeweils unabhängig für Folgendes stehen:
(a) H,
(b) C₁-C₄-Alkyl, wobei das C₁-C₄-Alkyl unsubstituiert oder durch 1 bis 2 Hydroxy, Cyano, Carboxy, Amino oder C₁-C₄-Alkoxy substituiert ist, oder
(c) C₁-C₄-Fluoralkyl, wobei das C₁-C₄-Fluoralkyl unsubstituiert oder durch 1 bis 2 Hydroxy, Cyano, Carboxy, Amino oder C₁-C₄-Alkoxy substituiert ist; gegebenenfalls wobei R^{2a} und R^{2b} jeweils unabhängig für H oder C₁-C₄-Alkyl stehen.

6. Verbindung nach Anspruch 1 oder ein pharmazeutisch unbedenkliches Salz davon, wobei die Gruppe 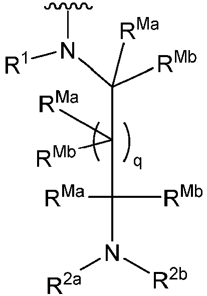 aus Folgendem ausgewählt ist:

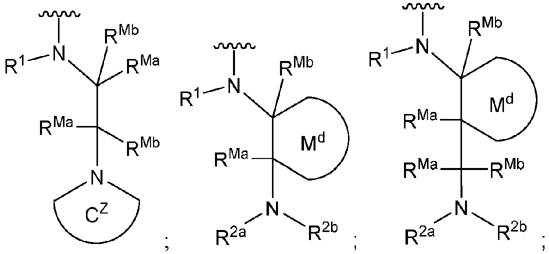

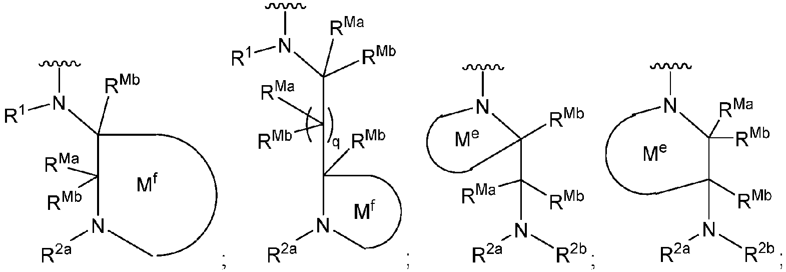

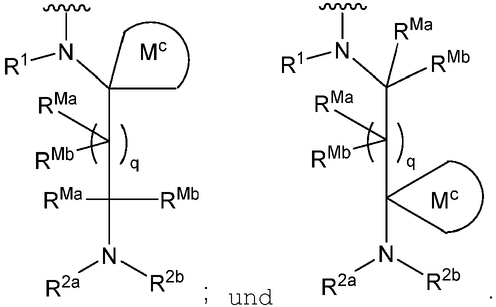

7. Verbindung nach Anspruch 6 oder ein pharmazeutisch unbedenkliches Salz davon, wobei:
(a) die Gruppe 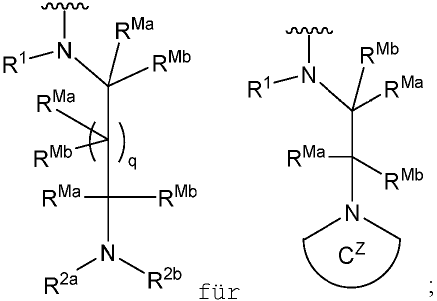 steht, wobei der Ring C^{Z} für Azetidin, Pyrrolidin, Piperidin, Piperazin oder Morpholin steht; oder
(b) die Gruppe 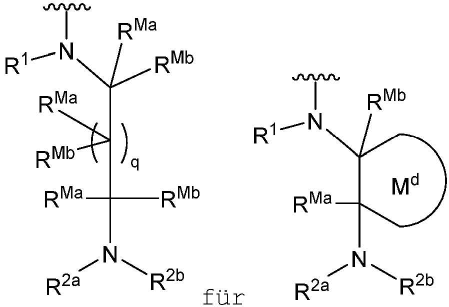 oder 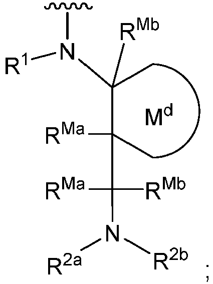 steht.
wobei:
i) der Ring M^{d} für einen 3- bis 6-gliedrigen Cycloalkylring oder einen 3- bis 6-gliedrigen gesättigten Heterocycloalkylring mit einem aus N und O ausgewählten Heteroatomen steht, gegebenenfalls wobei der Ring M^{d} für einen 3- bis 6-gliedrigen Cycloalkylring steht; oder
ii) der Ring M^{d} für Phenyl steht.

8. Verbindung nach Anspruch 6 oder ein pharmazeutisch unbedenkliches Salz davon, wobei die Gruppe 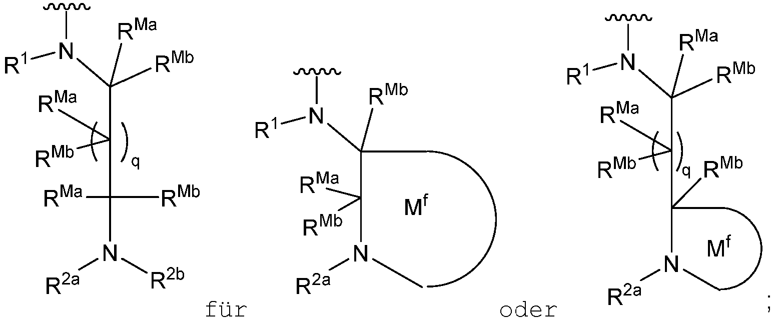 steht,
wobei: (a) der Ring M^{f} für einen Azetidin-, Pyrrolidin-, Piperidin- oder Azepinring, gegebenenfalls einen Pyrrolidin- oder Piperidinring, steht; und/oder (b) R^{2a} für Methyl steht.

9. Verbindung nach Anspruch 6 oder ein pharmazeutisch unbedenkliches Salz davon, wobei:
(a) die Gruppe 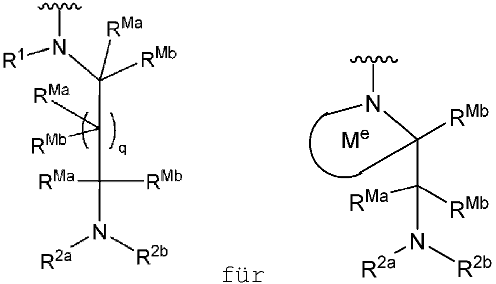 oder 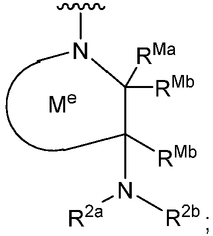 steht, wobei der Ring M^{e} für einen Pyrrolidinring steht; oder
(b) die Gruppe 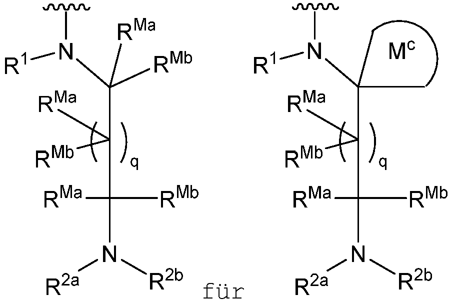 oder 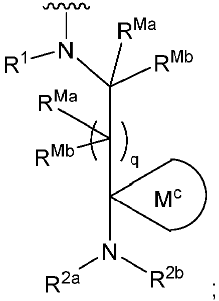 steht, wobei der Ring M^{c} für Cyclopropan steht, der tiefgestellte Index q für 0 steht und R¹ für H steht.

10. Verbindung nach einem der Ansprüche 1-9 oder ein pharmazeutisch unbedenkliches Salz davon, wobei:
(a) der tiefgestellte Index m für 0 oder 1 steht; und/oder
(b) der tiefgestellte Index n für 0 steht; und/oder
(c) R⁵ für H steht.

11. Verbindung nach einem der Ansprüche 1-10, ausgewählt aus der Gruppe bestehend aus: 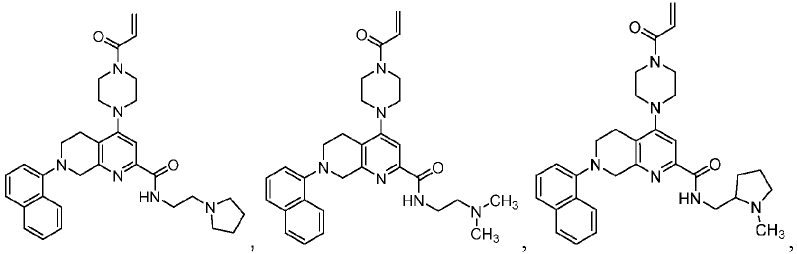 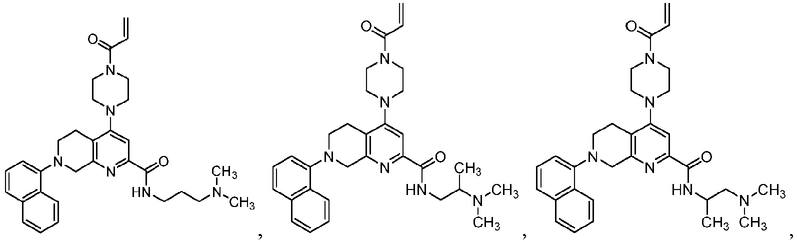 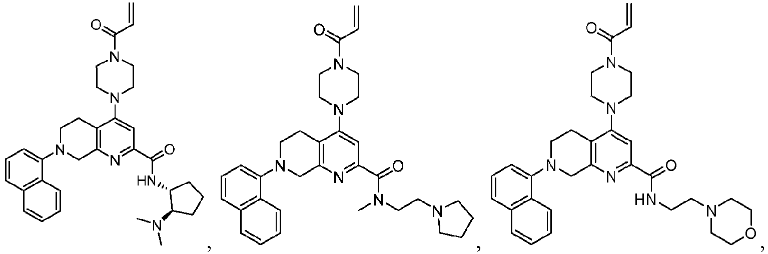 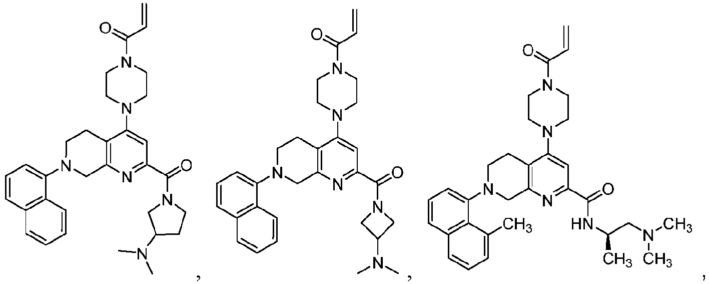 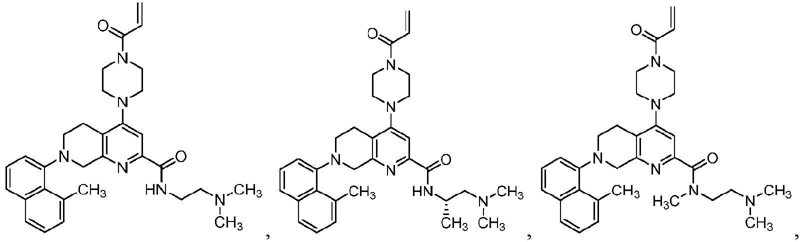 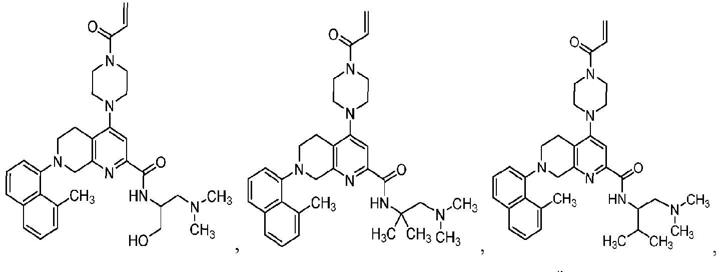 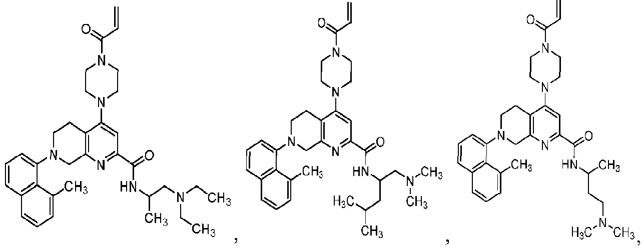 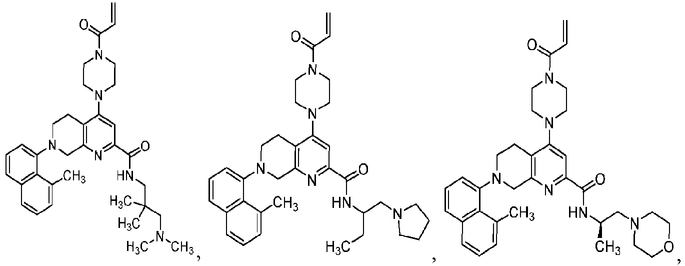 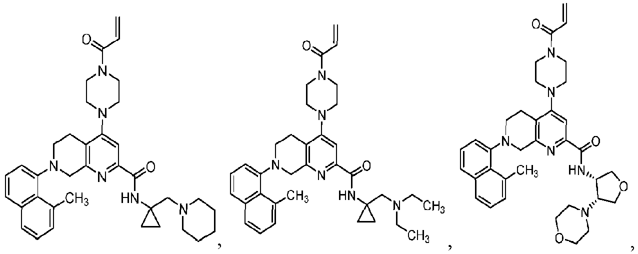 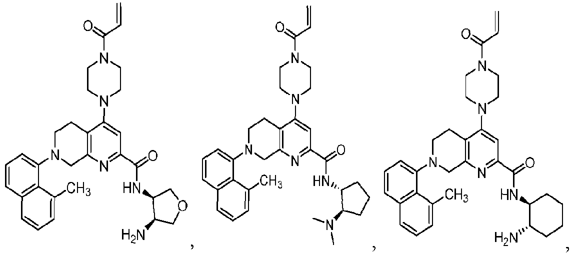 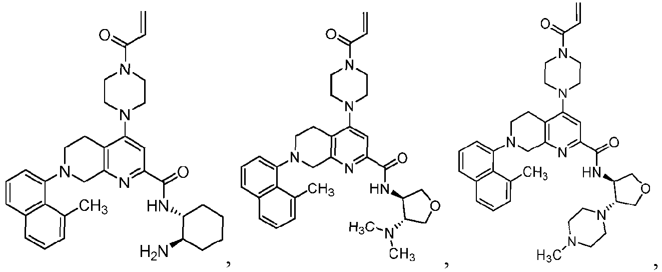 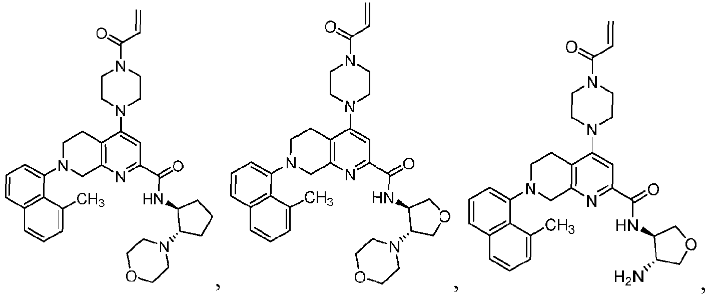 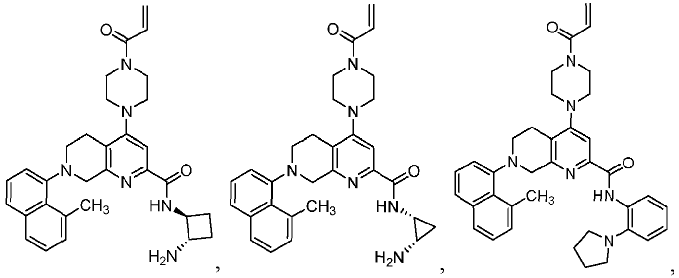 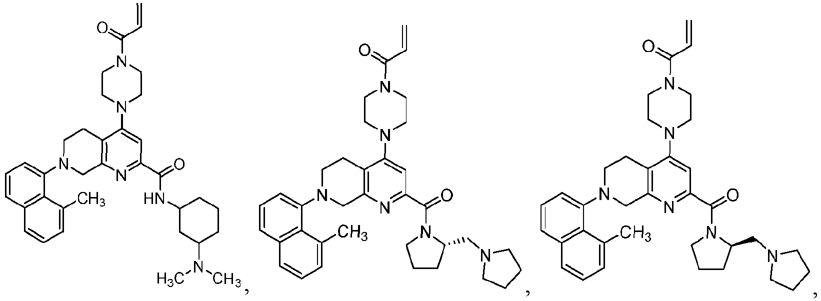 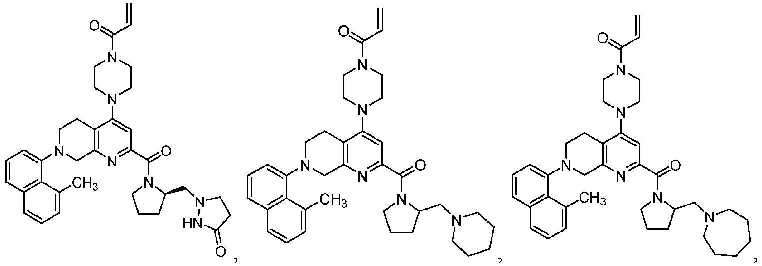 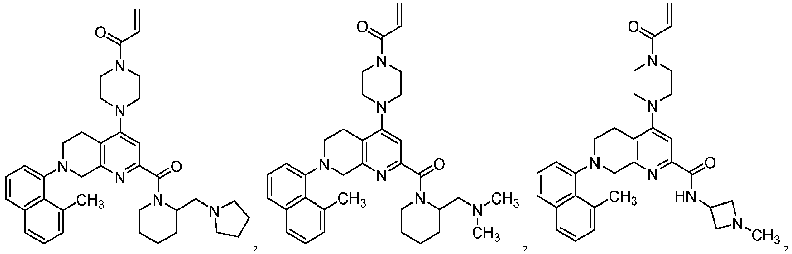 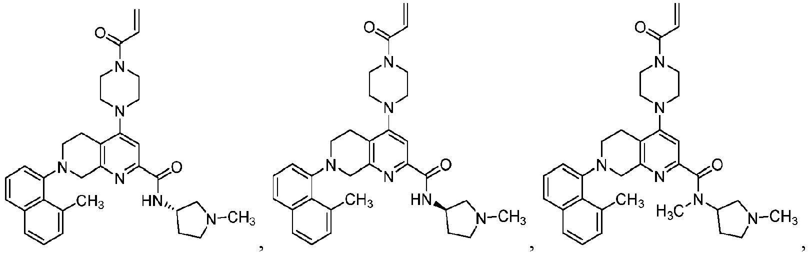 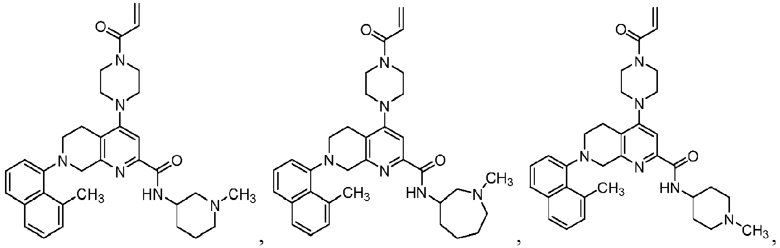 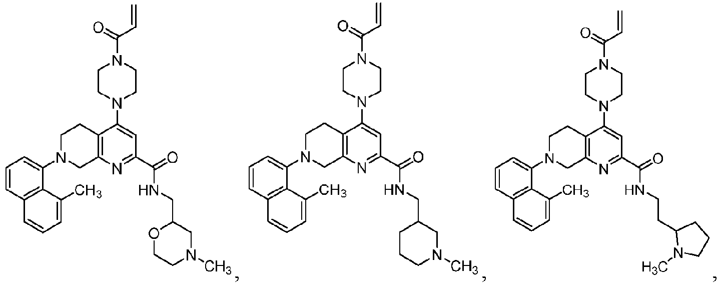 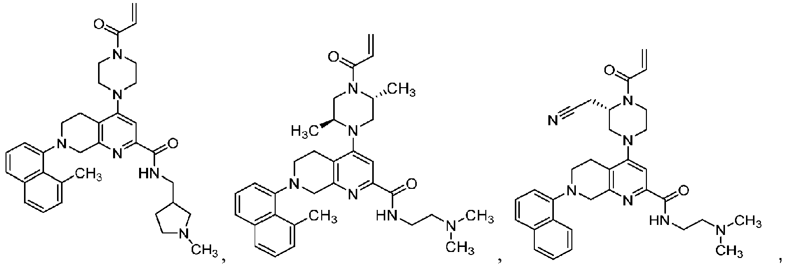 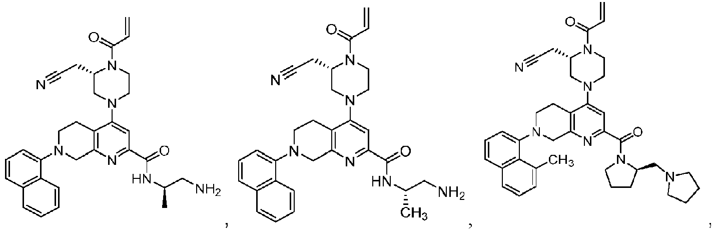 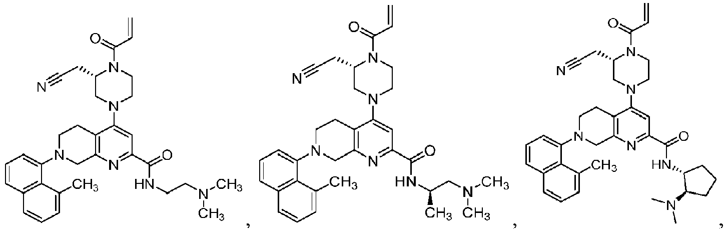 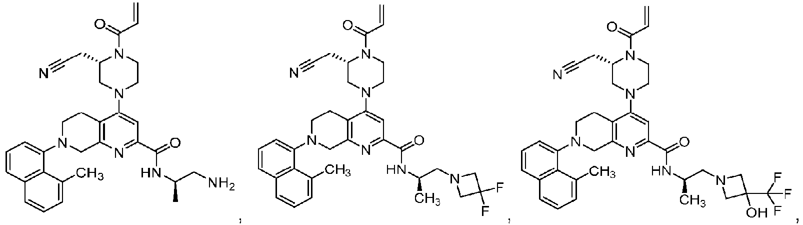 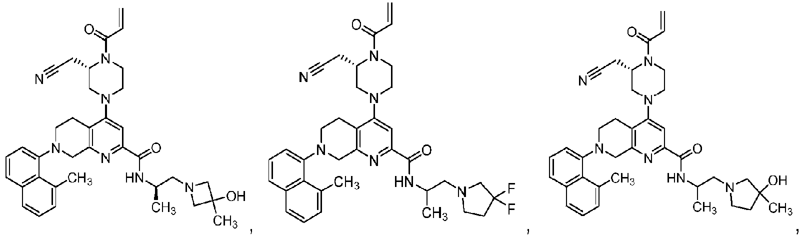 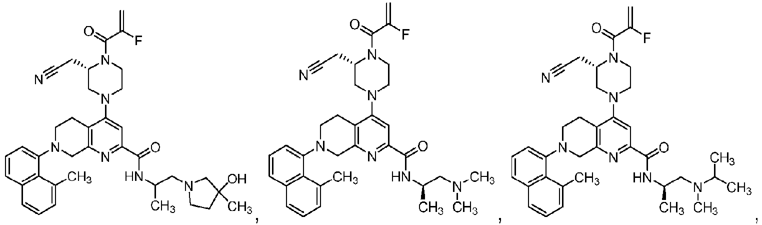 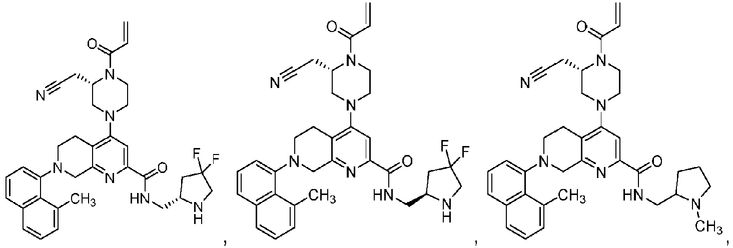 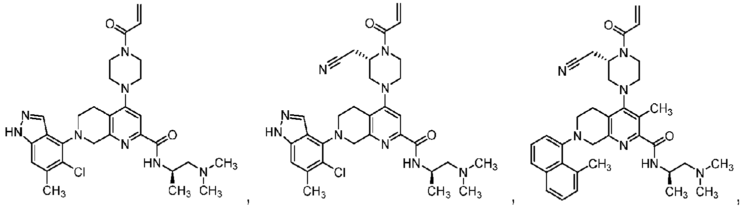 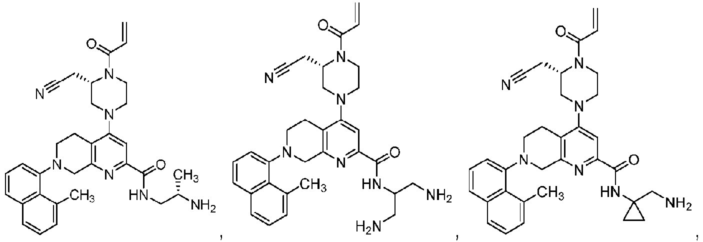 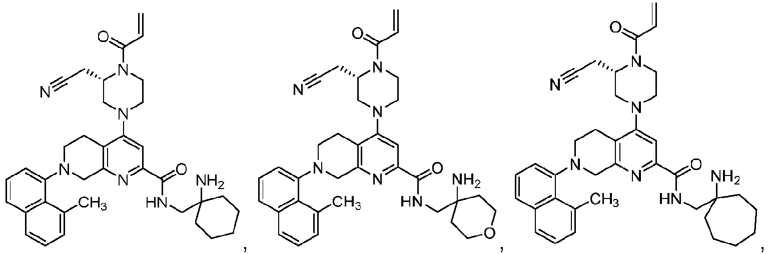 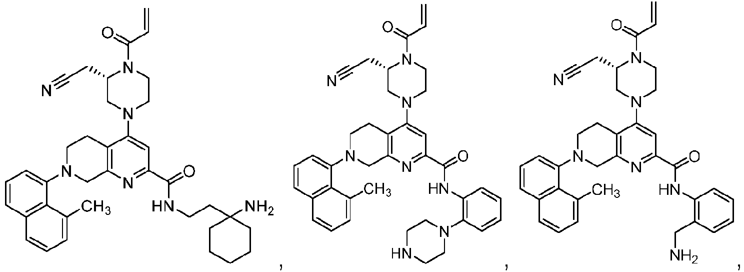 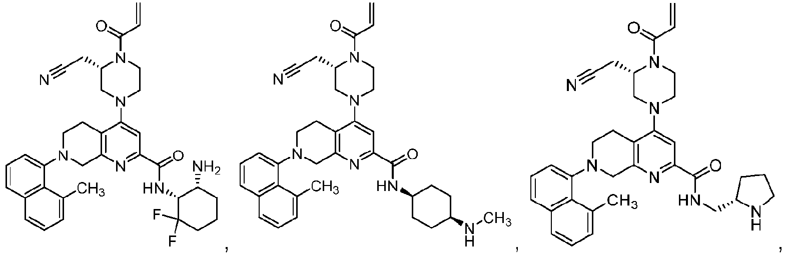 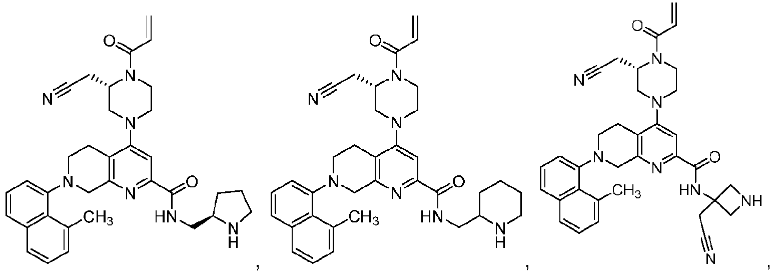 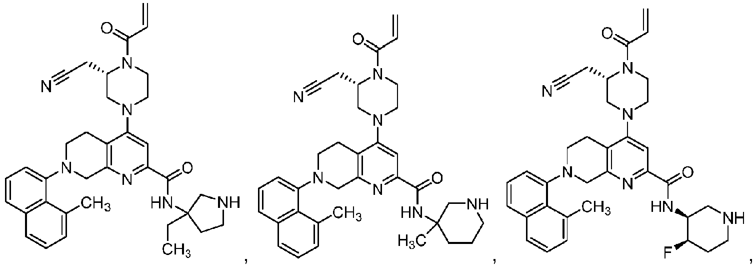 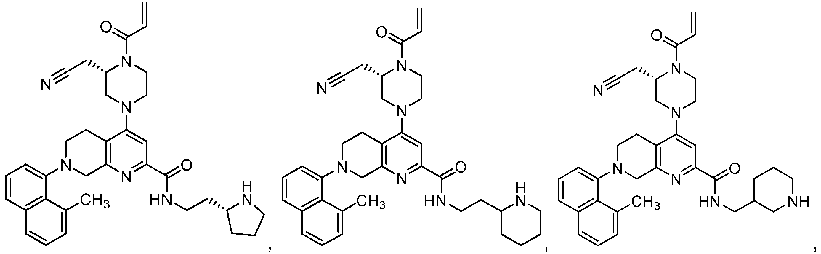 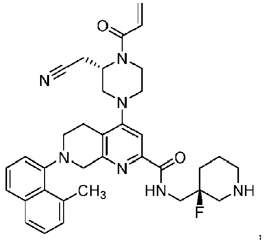 oder ein pharmazeutisch unbedenkliches Salz davon.

12. Pharmazeutische Zusammensetzung, umfassend die Verbindung nach einem der Ansprüche 1-11 oder das pharmazeutisch unbedenkliche Salz davon und einen pharmazeutisch unbedenklichen Träger.

13. Verbindung nach einem der Ansprüche 1-11 oder pharmazeutisch unbedenkliches Salz davon zur Verwendung bei der Therapie.

14. Verbindung nach einem der Ansprüche 1-11 oder pharmazeutisch unbedenkliches Salz davon oder pharmazeutische Zusammensetzung, die die Verbindung nach einem der Ansprüche 1-11 oder das pharmazeutisch unbedenkliche Salz davon umfasst, zur Verwendung bei der Behandlung von Krebs.

15. Verbindung nach einem der Ansprüche 1-11 oder pharmazeutisch unbedenkliches Salz davon und ein zusätzliches Antikrebsmittel oder pharmazeutische Zusammensetzung, die die Verbindung nach einem der Ansprüche 1-11 oder das pharmazeutisch unbedenkliche Salz davon und ein zusätzliches Antikrebsmittel umfasst, zur Verwendung bei der Behandlung von Krebs.

## Revendications

1. Composé de formule (I) 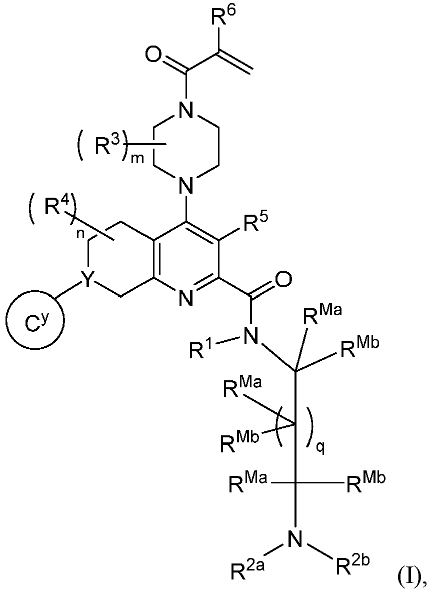 dans lequel :
Y représente N ;
R¹ représente :
(a) H,
(b) alkyle en C₁-C₄, ledit alkyle en C₁-C₄ étant non substitué ou substitué par 1 à 2 groupes hydroxy, cyano, carboxy, amino ou alcoxy en C₁-C₄, ou
(c) fluoroalkyle en C₁-C₄, ledit fluoroalkyle en C₁-C₄ étant non substitué ou substitué par 1 à 2 groupes hydroxy, cyano, carboxy, amino ou alcoxy en C₁-C₄ ;
chaque occurrence de R^{Ma} et R^{Mb} représente indépendamment :
(a) H,
(b) alkyle en C₁-C₄, ledit alkyle en C₁-C₄ étant non substitué ou substitué par 1 à 2 groupes fluoro, hydroxy, cyano, carboxy, amino ou alcoxy en C₁-C₄ ;
(c) fluoroalkyle en C₁-C₄, ledit fluoroalkyle en C₁-C₄ étant non substitué ou substitué par 1 à 2 groupes hydroxy, cyano, carboxy, amino ou alcoxy en C₁-C₄ ;
(d) en variante, R^{Ma} et R^{Mb}, lorsqu'ils sont substitués en position géminale sur un atome de carbone commun, avec l'atome auquel ils sont liés, forment un cycle M^{c}, le cycle M^{c} étant un cycle saturé de 3 à 8 chaînons contenant facultativement un hétéroatome choisi parmi N et O,
dans lequel le cycle M^{c} est non substitué ou substitué par 1 à 6 groupes fluoro, hydroxy, cyano, carboxy, amino, oxo, alcoxy en C₁-C₃, alkyle en C₁-C₃ ou fluoroalkyle en C₁-C₃ ;
dans lequel ledit substituant périphérique alkyle en C₁-C₃ ou fluoroalkyle en C₁-C₃ du cycle M^{c} est non substitué ou substitué par 1 à 2 groupes hydroxy, cyano, carboxy, amino, oxo ou alcoxy en C₁-C₃ ;
(e) en variante, R^{Ma} et R^{Mb}, lorsqu'ils sont substitués sur des atomes de carbone vicinaux ou géminaux, avec les atomes auxquels ils sont liés, forment un cycle M^{d},
dans lequel le cycle M^{d} représente un groupe phényle, hétéroaryle de 5 à 6 chaînons contenant un à trois hétéroatomes choisis parmi N, O et S, ou un cycle saturé de 3 à 6 chaînons contenant facultativement un hétéroatome choisi parmi N et O,
dans lequel le cycle M^{d} est non substitué ou substitué par 1 à 6 groupes fluoro, hydroxy, cyano, carboxy, amino, oxo, alcoxy en C₁-C₃, alkyle en C₁-C₃ ou fluoroalkyle en C₁-C₃ ;
dans lequel ledit substituant périphérique alkyle en C₁-C₃ ou fluoroalkyle en C₁-C₃ du cycle M^{d} est non substitué ou substitué par 1 à 2 groupes hydroxy, cyano, carboxy, amino, oxo ou alcoxy en C₁-C₃ ;
(f) en variante, des groupes R^{Ma} et R¹, avec les atomes auxquels ils sont liés, forment un cycle M^{e}, le cycle M^{e} étant un cycle hétérocycloalkyle de 3 à 6 chaînons contenant facultativement un hétéroatome supplémentaire choisi parmi N et O,
dans lequel le cycle M^{e} est non substitué ou substitué par 1 à 6 groupes fluoro, hydroxy, cyano, carboxy, amino, oxo, alcoxy en C₁-C₃, alkyle en C₁-C₃ ou fluoroalkyle en C₁-C₃ ;
dans lequel ledit substituant périphérique alkyle en C₁-C₃ ou fluoroalkyle en C₁-C₃ du cycle M^{e} est non substitué ou substitué par 1 à 2 groupes hydroxy, cyano, carboxy, amino, oxo ou alcoxy en C₁-C₃ ; ou
(g) en variante, des groupes R^{Ma} et R^{2b}, avec les atomes auxquels ils sont liés, forment un cycle M^{f}, le cycle M^{f} étant un cycle hétérocycloalkyle de 4 à 7 chaînons contenant facultativement un hétéroatome supplémentaire choisi parmi N et O,
dans lequel le cycle M^{f} est non substitué ou substitué par 1 à 6 groupes fluoro, hydroxy, cyano, carboxy, amino, oxo, alcoxy en C₁-C₃, alkyle en C₁-C₃ ou fluoroalkyle en C₁-C₃ ;
dans lequel ledit substituant périphérique alkyle en C₁-C₃ ou fluoroalkyle en C₁-C₃ du cycle M^{f} est non substitué ou substitué par 1 à 2 groupes hydroxy, cyano, carboxy, amino, oxo ou alcoxy en C₁-C₃ ;
R^{2a} représente :
(a) H,
(b) alkyle en C₁-C₄, ledit alkyle en C₁-C₄ étant non substitué ou substitué par 1 à 2 groupes hydroxy, cyano, carboxy, amino ou alcoxy en C₁-C₄, ou
(c) fluoroalkyle en C₁-C₄, ledit fluoroalkyle en C₁-C₄ étant non substitué ou substitué par 1 à 2 groupes hydroxy, cyano, carboxy, amino ou alcoxy en C₁-C₄ ;
R^{2b} représente :
(a) H,
(b) alkyle en C₁-C₄, ledit alkyle en C₁-C₄ étant non substitué ou substitué par 1 à 2 groupes hydroxy, cyano, carboxy, amino ou alcoxy en C₁-C₄,
(c) fluoroalkyle en C₁-C₄, ledit fluoroalkyle en C₁-C₄ étant non substitué ou substitué par 1 à 2 groupes hydroxy, cyano, carboxy, amino ou alcoxy en C₁-C₄ ; ou
(d) R^{2b} et R^{Ma}, avec les atomes auxquels ils sont liés, forment un cycle M^{f} comme décrit ci-dessus,
ou en variante R^{2a} et R^{2b}, avec les atomes auxquels ils sont liés, forment un cycle C^{Z}, le cycle C^{Z} étant un hétérocycloalkyle de 3 à 7 chainons contenant facultativement un hétéroatome supplémentaire choisi parmi N et O,
dans lequel le cycle C^{Z} est non substitué ou substitué par 1 à 6 groupes fluoro, hydroxy, cyano, carboxy, amino, oxo, alcoxy en C₁-C₃, alkyle en C₁-C₃ ou fluoroalkyle en C₁-C₃ ;
dans lequel ledit substituant périphérique alkyle en C₁-C₃ ou fluoroalkyle en C₁-C₃ du cycle C^{Z} est non substitué ou substitué par 1 à 2 groupes hydroxy, cyano, carboxy, amino, oxo ou alcoxy en C₁-C₃ ;
le cycle C^{y} représente :
(a) un groupe aryle en C₆-C₁₀, ou
(b) un hétéroaryle de cinq à dix chaînons contenant un à quatre hétéroatomes choisis parmi N, O et S ;
dans lequel le cycle C^{y} est non substitué ou substitué par un à quatre groupes halogéno, hydroxy, alkyle en C₁-C₃, fluoroalkyle en C₁-C₃, alcoxy en C₁-C₃, cyano, ou hydroxyalkyle en C₁-C₄ ;
R³ représente un groupe alkyle en C₁-C₄, cyanoalkyle en C₁-C₄, fluoroalkyle en C₁-C₄, hydroxyalkyle en C₁-C₄, oxo, carboxy ou, en variante, deux substituants R³, avec les atomes de carbone auxquels ils sont liés, peuvent former un système bicyclique ou spirocyclique avec le cycle pipérazine représenté ;
R⁴ représente un groupe alkyle en C₁-C₃, cyanoalkyle en C₁-C₃, fluoroalkyle en C₁-C₃, hydroxy, oxo ou fluoro ;
R⁵ représente H, halogéno, cyano, alkyle en C₁-C₃, fluoroalkyle en C₁-C₃ ou alcoxy en C₁-C₃ ;
R⁶ représente H ou halogéno ;
l'indice m est 0, 1, 2 ou 3 ;
l'indice n est 0, 1 ou 2 ; et
l'indice q est 0 ou 1 ;
ou un sel pharmaceutiquement acceptable de celui-ci.

2. Composé selon la revendication 1 ou un sel pharmaceutiquement acceptable de celui-ci, dans lequel chaque occurrence de R^{Ma} et R^{Mb} représente indépendamment :
(a) H,
(b) alkyle en C₁-C₄, ledit alkyle en C₁-C₄ étant non substitué ou substitué par 1 à 2 groupes fluoro, hydroxy, cyano, carboxy, amino ou alcoxy en C₁-C₄ ;
(c) fluoroalkyle en C₁-C₄, ledit fluoroalkyle en C₁-C₄ étant non substitué ou substitué par 1 à 2 groupes hydroxy, cyano, carboxy, amino ou alcoxy en C₁-C₄ ;
(d) en variante, R^{Ma} et R^{Mb}, lorsqu'ils sont substitués en position géminale sur un atome de carbone commun, avec l'atome auquel ils sont liés, forment un cycle M^{c}, le cycle M^{c} étant un cycle saturé de 3 à 6 chaînons contenant facultativement un hétéroatome choisi parmi N et O,
dans lequel le cycle M^{c} est non substitué ou substitué par 1 à 6 groupes fluoro, hydroxy, cyano, carboxy, amino, oxo, alcoxy en C₁-C₃, alkyle en C₁-C₃ ou fluoroalkyle en C₁-C₃ ;
dans lequel ledit substituant périphérique alkyle en C₁-C₃ ou fluoroalkyle en C₁-C₃ du cycle M^{c} est non substitué ou substitué par 1 à 2 groupes hydroxy, cyano, carboxy, amino, oxo ou alcoxy en C₁-C₃ ;
(e) en variante, R^{Ma} et R^{Mb}, lorsqu'ils sont substitués sur des atomes de carbone vicinaux ou géminaux, avec les atomes auxquels ils sont liés, forment un cycle M^{d}, le cycle M^{d} représentant un groupe phényle, hétéroaryle de 5 ou 6 chaînons contenant un hétéroatome choisi parmi N et O et S, ou un cycle saturé de 3 à 6 chaînons contenant facultativement un hétéroatome choisi parmi N et O,
dans lequel le cycle M^{d} est non substitué ou substitué par 1 à 6 groupes fluoro, hydroxy, cyano, carboxy, amino, oxo, alcoxy en C₁-C₃, alkyle en C₁-C₃ ou fluoroalkyle en C₁-C₃ ;
dans lequel ledit substituant périphérique alkyle en C₁-C₃ ou fluoroalkyle en C₁-C₃ du cycle M^{d} est non substitué ou substitué par 1 à 2 groupes hydroxy, cyano, carboxy, amino, oxo ou alcoxy en C₁-C₃ ;
(f) en variante, des groupes R^{Ma} et R¹, avec les atomes auxquels ils sont liés, forment un cycle M^{e}, le cycle M^{e} étant un cycle hétérocycloalkyle de 3 à 6 chaînons contenant facultativement un hétéroatome supplémentaire choisi parmi N et O,
dans lequel le cycle M^{e} est non substitué ou substitué par 1 à 6 groupes fluoro, hydroxy, cyano, carboxy, amino, oxo, alcoxy en C₁-C₃, alkyle en C₁-C₃ ou fluoroalkyle en C₁-C₃ ;
dans lequel ledit substituant périphérique alkyle en C₁-C₃ ou fluoroalkyle en C₁-C₃ du cycle M^{e} est non substitué ou substitué par 1 à 2 groupes hydroxy, cyano, carboxy, amino, oxo ou alcoxy en C₁-C₃ ; ou
(g) en variante, des groupes R^{Ma} et R^{2b}, avec les atomes auxquels ils sont liés, forment un cycle M^{f}, le cycle M^{f} étant un cycle hétérocycloalkyle de 4 à 7 chaînons contenant facultativement un hétéroatome supplémentaire choisi parmi N et O,
dans lequel le cycle M^{f} est non substitué ou substitué par 1 à 6 groupes fluoro, hydroxy, cyano, carboxy, amino, oxo, alcoxy en C₁-C₃, alkyle en C₁-C₃ ou fluoroalkyle en C₁-C₃ ;
dans lequel ledit substituant périphérique alkyle en C₁-C₃ ou fluoroalkyle en C₁-C₃ du cycle M^{f} est non substitué ou substitué par 1 à 2 groupes hydroxy, cyano, carboxy, amino, oxo ou alcoxy en C₁-C₃ ; et
R⁶ représente H.

3. Composé selon la revendication 1 ou 2, ou un sel pharmaceutiquement acceptable de celui-ci, dans lequel :
(a) l'indice q est 0 ; et/ou
(b) R¹ représente H.

4. Composé selon la revendication 1 ou un sel pharmaceutiquement acceptable de celui-ci, dans lequel Y représente N, l'indice q est 0 et R¹ représente H ; facultativement dans lequel chaque R^{Ma} et chaque R^{Mb} représentent indépendamment :
(a) H,
(b) alkyle en C₁-C₄, ledit alkyle en C₁-C₄ étant non substitué ou substitué par 1 à 2 groupes fluoro, hydroxy, cyano, carboxy, amino ou alcoxy en C₁-C₄ ; ou
(c) fluoroalkyle en C₁-C₄, ledit fluoroalkyle en C₁-C₄ étant non substitué ou substitué par 1 à 2 groupes hydroxy, cyano, carboxy, amino ou alcoxy en C₁-C₄.

5. Composé selon la revendication 4 ou un sel pharmaceutiquement acceptable de celui-ci, dans lequel R^{Ma} et R^{Mb} représentent chacun indépendamment H ou alkyle en C₁-C₄ et/ou R^{2a} et R^{2b} représentent chacun indépendamment :
(a) H,
(b) alkyle en C₁-C₄, ledit alkyle en C₁-C₄ étant non substitué ou substitué par 1 à 2 groupes hydroxy, cyano, carboxy, amino ou alcoxy en C₁-C₄, ou
(c) fluoroalkyle en C₁-C₄, ledit fluoroalkyle en C₁-C₄ étant non substitué ou substitué par 1 à 2 groupes hydroxy, cyano, carboxy, amino ou alcoxy en C₁-C₄ ; facultativement dans lequel R^{2a} et R^{2b} représentent chacun indépendamment H ou alkyle en C₁-C₄.

6. Composé selon la revendication 1, ou un sel pharmaceutiquement acceptable de celui-ci, dans lequel le groupe 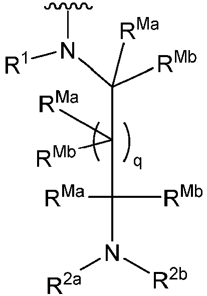 est choisi parmi :

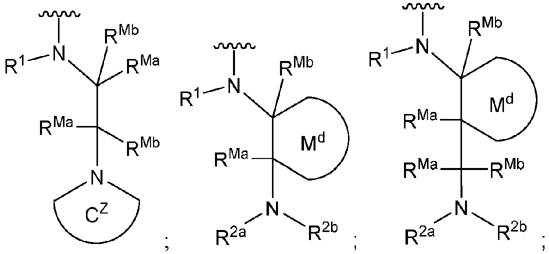

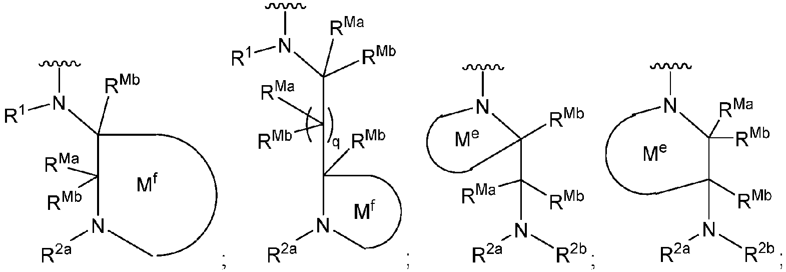

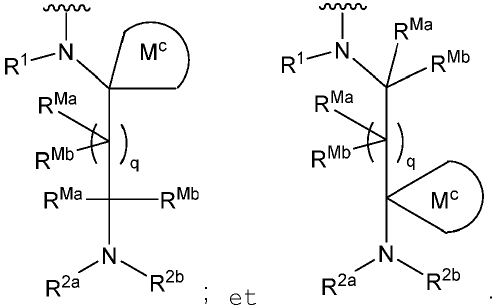

7. Composé selon la revendication 6, ou un sel pharmaceutiquement acceptable de celui-ci, dans lequel :
(a) le groupe 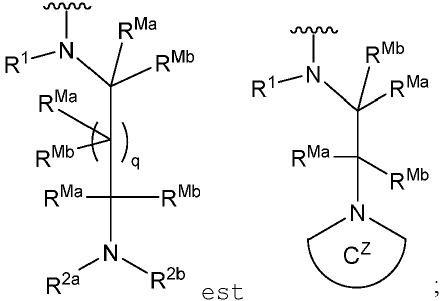 dans lequel le cycle C^{Z} représente un groupe azétidine, pyrrolidine, pipéridine, pipérazine ou morpholine ; ou
(b) le groupe 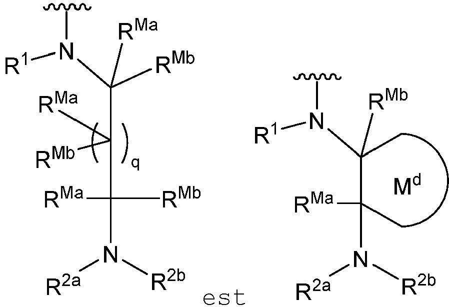 ou 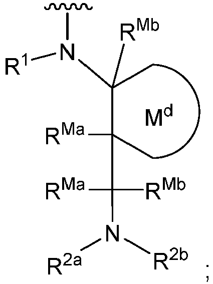 dans lequel :
i) le cycle M^{d} représente un cycle cycloalkyle de 3 à 6 chainons ou un cycle hétérocycloalkyle saturé de 3 à 6 chainons contenant un hétéroatome choisi parmi N et O, facultativement dans lequel le cycle M^{d} représente un cycle cycloalkyle de 3 à 6 chainons ; ou
ii) le cycle M^{d} représente un groupe phényle.

8. Composé selon la revendication 6, ou un sel pharmaceutiquement acceptable de celui-ci, dans lequel le groupe 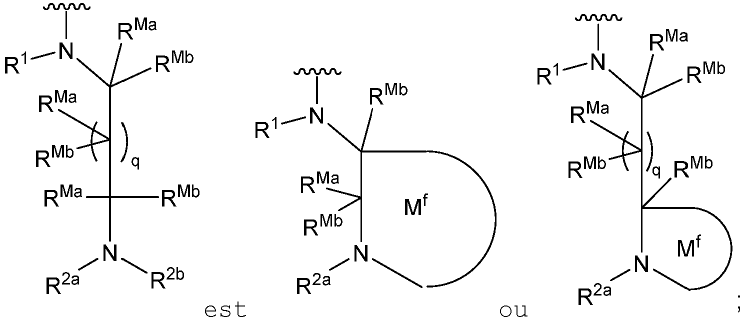 dans lequel : (a) le cycle M^{f} représente un groupe azétidine, pyrrolidine, pipéridine ou azépine ; facultativement un cycle pyrrolidine ou pipéridine ; et/ou (b) R^{2a} représente un groupe méthyle.

9. Composé selon la revendication 6, ou un sel pharmaceutiquement acceptable de celui-ci, dans lequel :
(a) le groupe 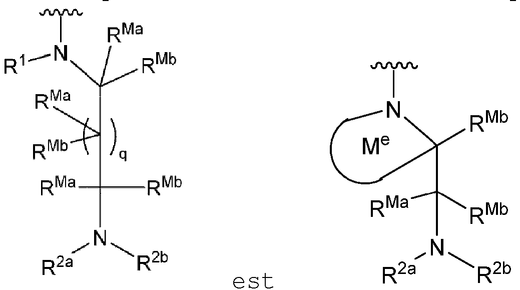 ou 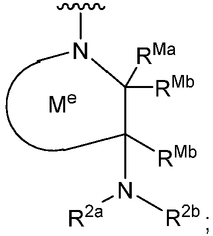 dans lequel le cycle M^{e} représente un cycle pyrrolidine ; ou
(b) le groupe 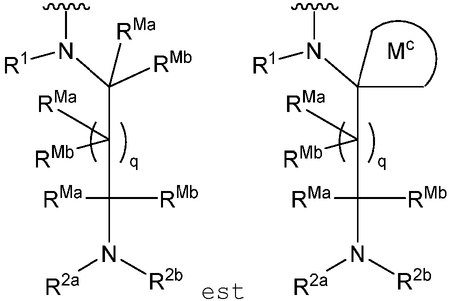 ou 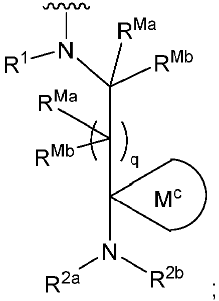 dans lequel le cycle M^{c} représente un groupe cyclopropane, l'indice q est 0, et R¹ représente H.

10. Composé selon l'une quelconque des revendications 1 à 9, ou un sel pharmaceutiquement acceptable de celui-ci, dans lequel :
(a) l'indice m est 0 ou 1 ; et/ou
(b) l'indice n est 0 ; et/ou
(c) R⁵ est H.

11. Composé selon l'une quelconque des revendications 1 à 10, choisi dans le groupe constitué par : 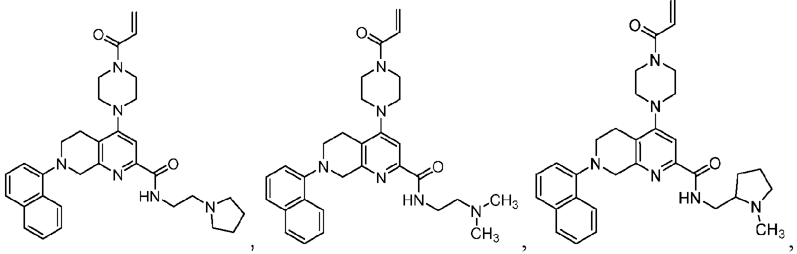 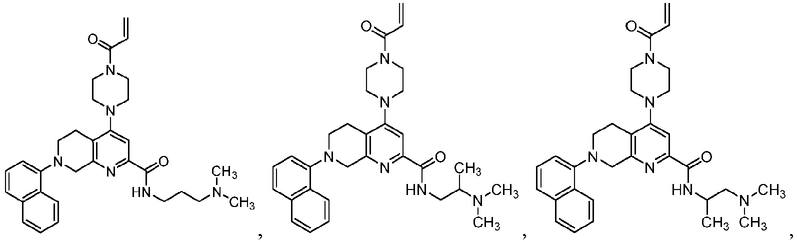 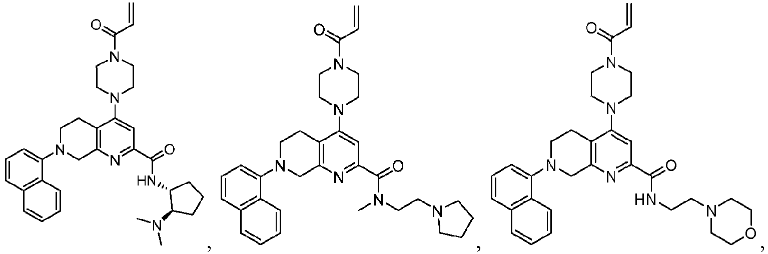 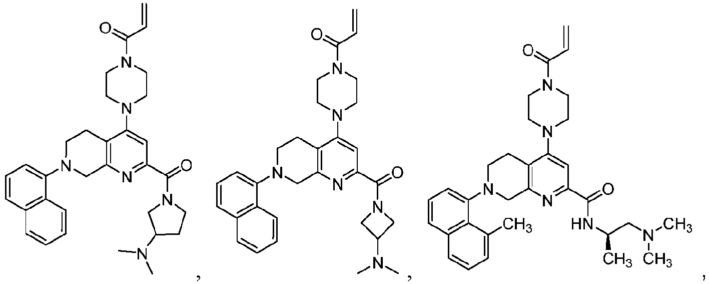 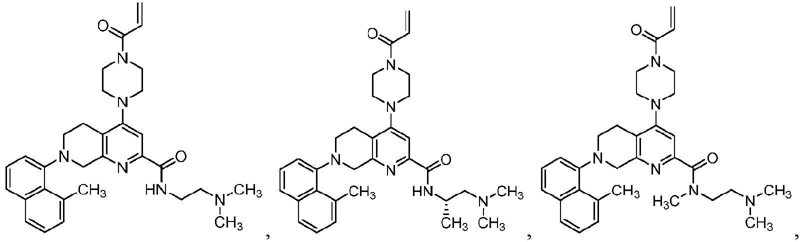 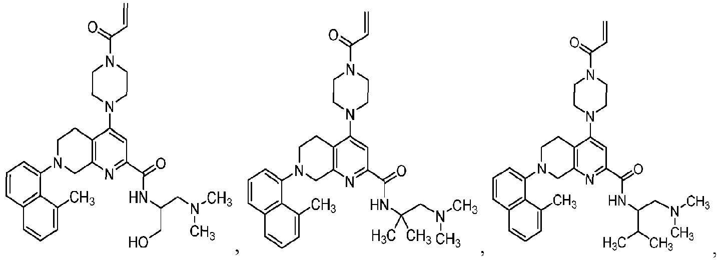 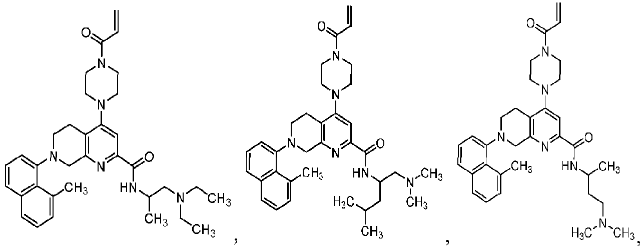 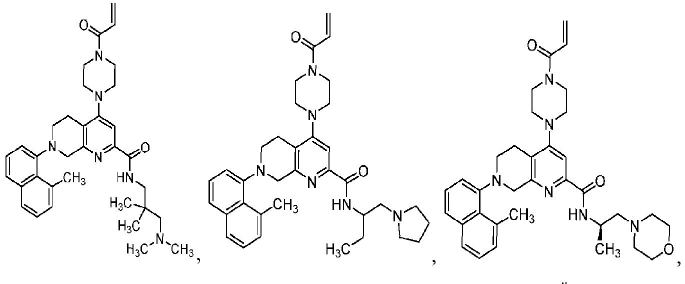 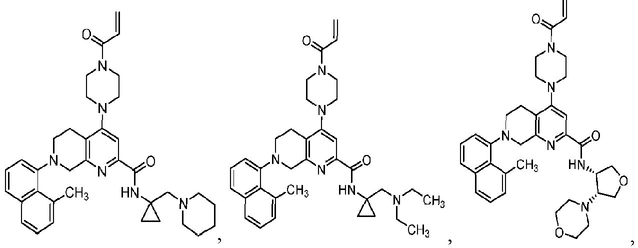 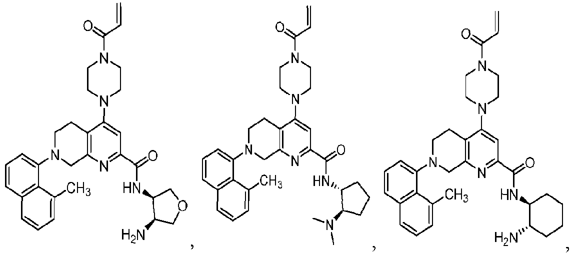 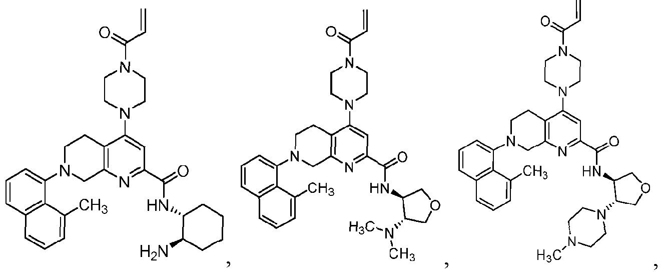 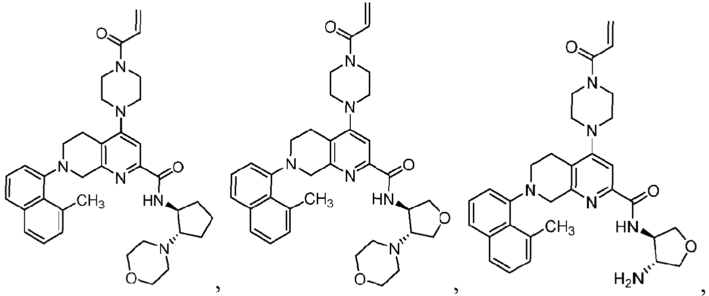 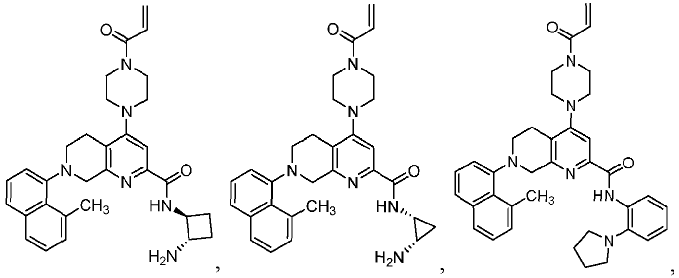 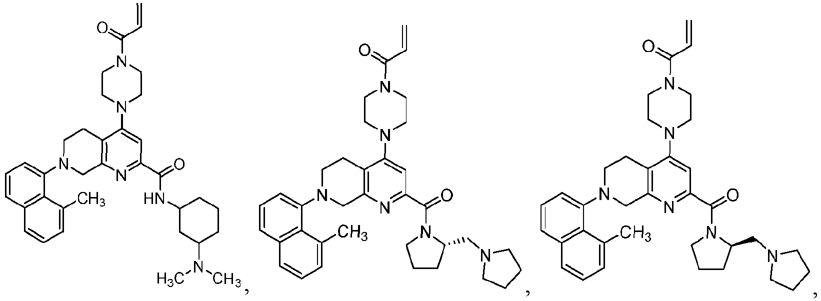 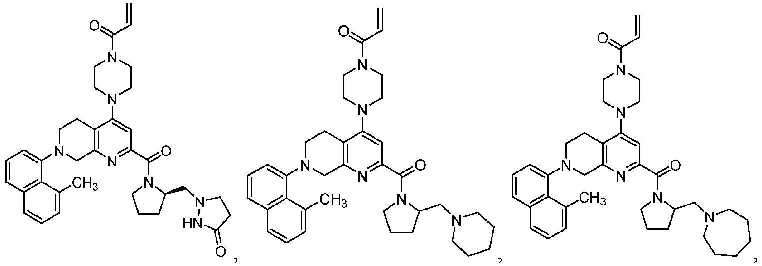 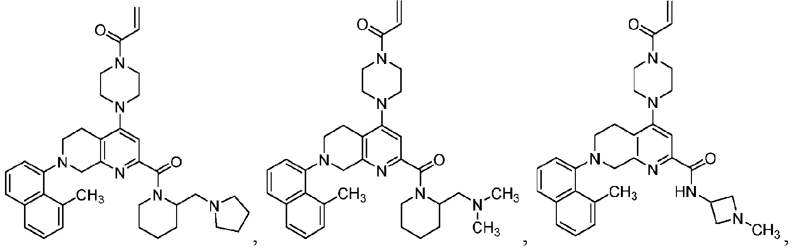 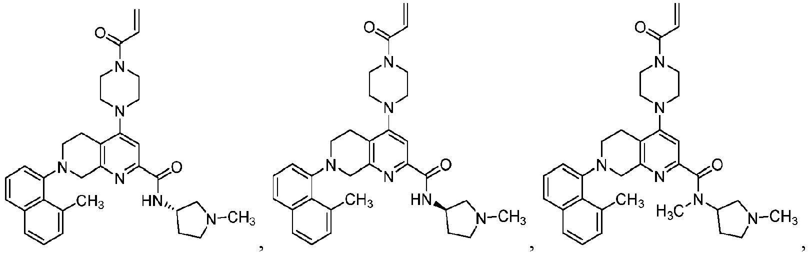 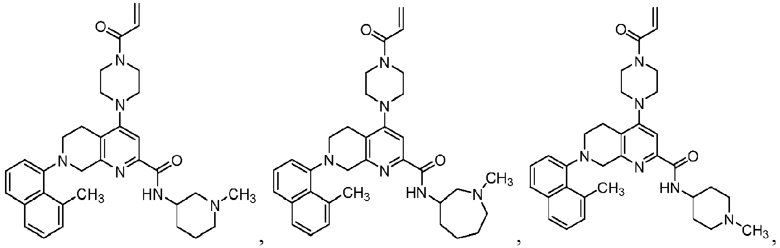 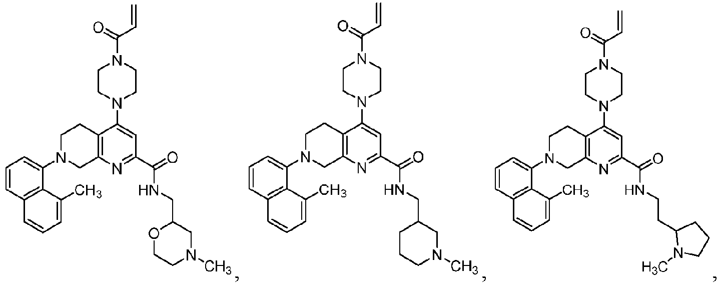 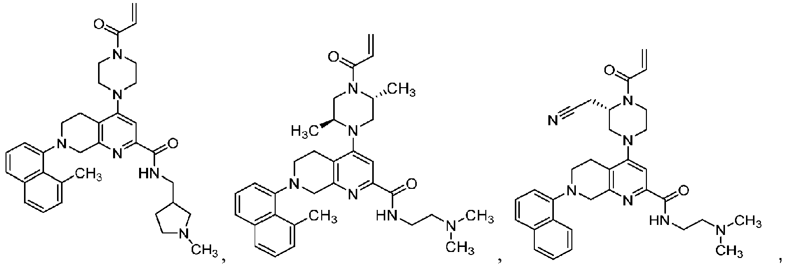 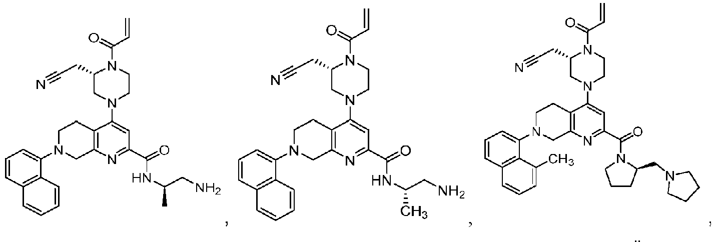 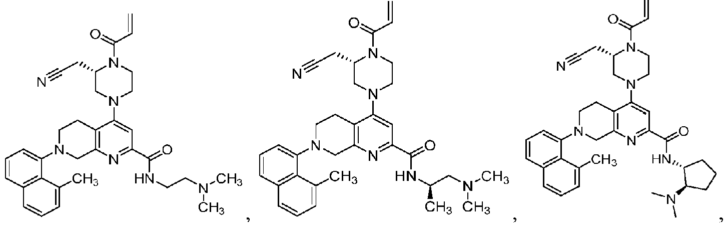 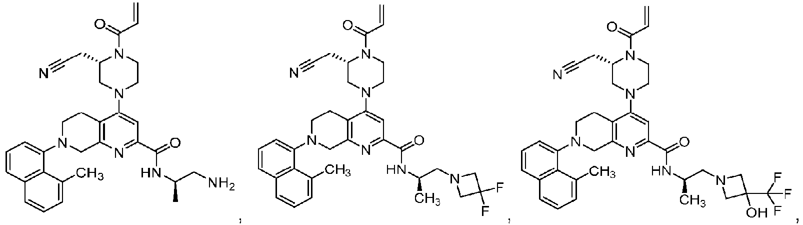 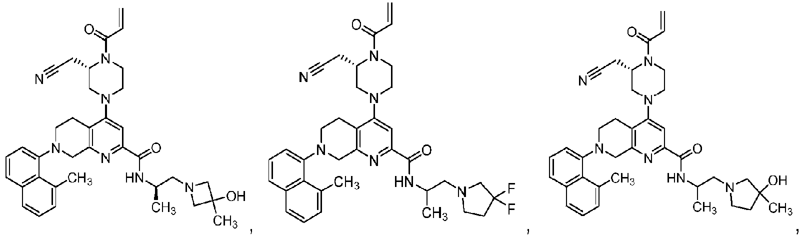 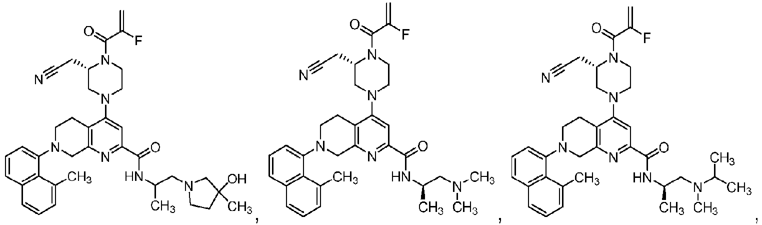 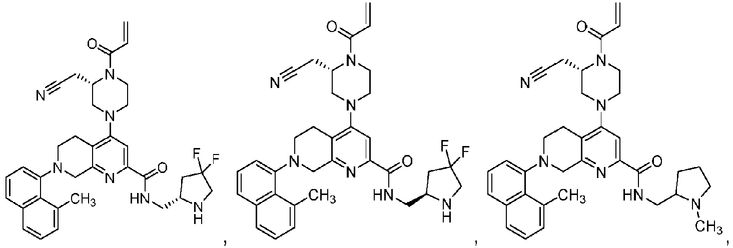 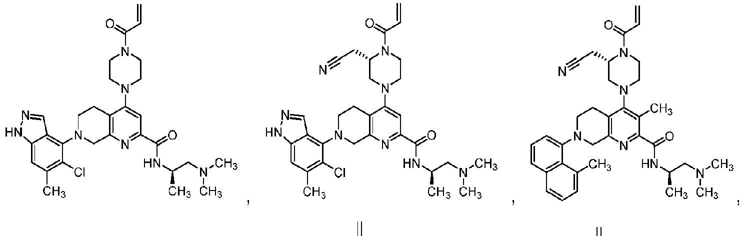 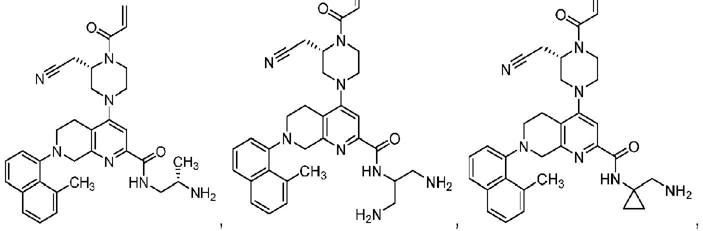 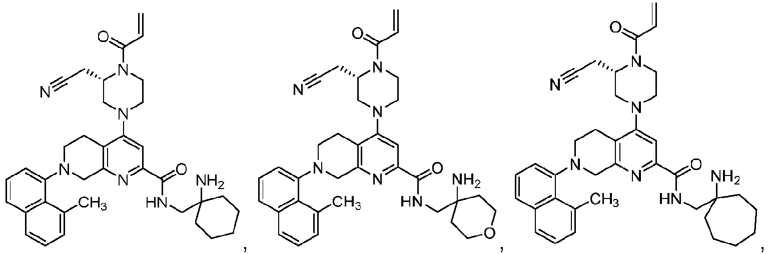 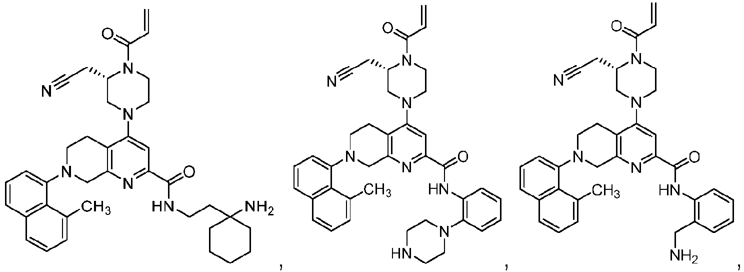 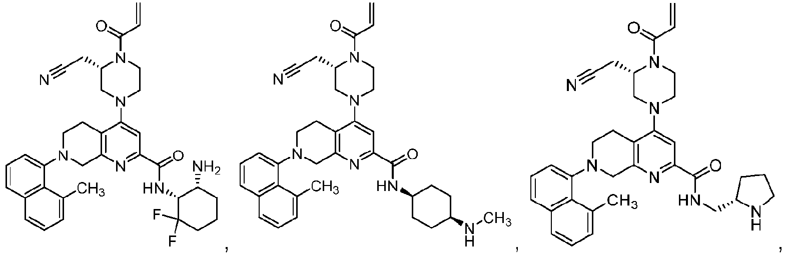 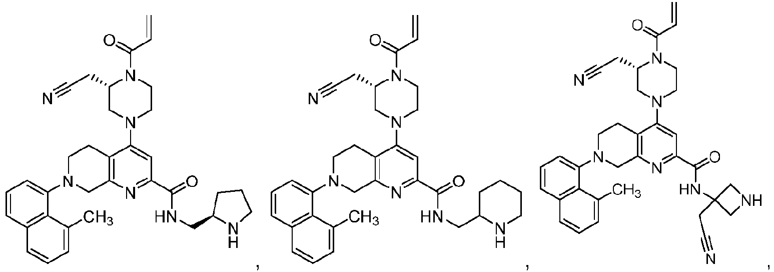 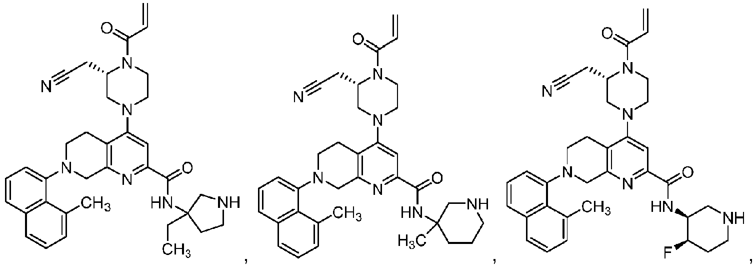 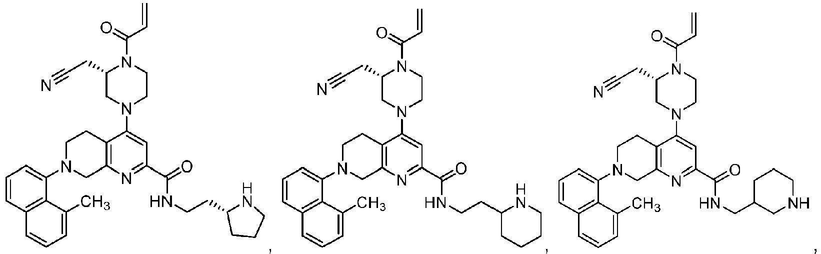 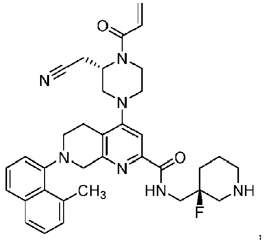 ou un sel pharmaceutiquement acceptable de celui-ci.

12. Composition pharmaceutique comprenant le composé selon l'une quelconque des revendications 1 à 11 ou un sel pharmaceutiquement acceptable de celui-ci, et un véhicule pharmaceutiquement acceptable.

13. Composé selon l'une quelconque des revendications 1 à 11, ou un sel pharmaceutiquement acceptable de celui-ci, pour une utilisation en thérapie.

14. Composé selon l'une quelconque des revendications 1 à 11, ou un sel pharmaceutiquement acceptable de celui-ci, ou une composition pharmaceutique comprenant le composé selon l'une quelconque des revendications 1 à 11, ou un sel pharmaceutiquement acceptable de celui-ci, pour une utilisation dans le traitement du cancer.

15. Composé selon l'une quelconque des revendications 1 à 11, ou un sel pharmaceutiquement acceptable de celui-ci, et un agent anticancéreux supplémentaire, ou une composition pharmaceutique comprenant le composé selon l'une quelconque des revendications 1 à 11, ou un sel pharmaceutiquement acceptable de celui-ci, et un agent anticancéreux supplémentaire, pour une utilisation dans le traitement du cancer.
